# EUROPEAN PATENT APPLICATION

(11) **EP 4 403 557 A1**
(43) Date of publication of application: **24.07.2024**
(21) Application number: 22869187.9
(22) Date of filing: 13.09.2022
(51) Int. Cl.: C07D 487/06

(54) **COMPOUND THAT INHIBITS PROGRAMMED CELL NECROSIS, AND PREPARATION METHOD THEREFOR**

(30) Priority: 14.09.2021 CN 202111074627
(71) Applicant: Shanghai Institute of Organic Chemistry, Chinese Academy of Sciences, Shanghai 200032 (CN)
(72) Inventor: WANG, Zhaoyin, Shanghai 200032 (CN); YANG, Shuailong, Shanghai 200032 (CN); ZHAO, Zhimin, Shanghai 200032 (CN)
(74) Representative: dompatent von Kreisler Selting Werner - Partnerschaft von Patent- und Rechtsanwälten mbB
(86) International application number: PCT/CN2022/118387
(87) International publication number: WO 2023/040818

(57) **Abstract**

The present invention discloses RIPK1 inhibitors for inhibiting necroptosis and their preparation methods. The RIPK1 inhibitors of the present invention are as shown in general formula I, wherein, X¹, X², X³, X⁴, X⁵, Z¹, Z², Z³, L¹, L², L³, R, R¹, ring D and n are as disclosed in the specifications and claims. The present invention also discloses the preparation methods of general formula I and the test results of inhibition of necroptosis and RIPK1 activity. The said compounds in general formula I of the present invention can be used for used for treating and preventing inflammatory and degenerative diseases.

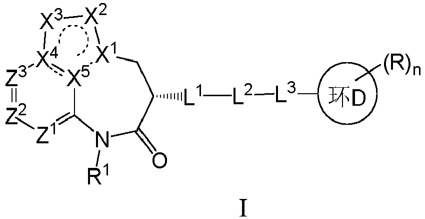

## Description

### Cross Reference to Related Applications

This application claims the priority of the Chinese invention patent application numbered 202111074627.8, submitted on September 14, 2021, with its content being incorporated into this application by cross reference.

### Technical Field

The present invention relates to an inhibitor of necroptosis, specifically relating to its preparation method and using method for treating and preventing inflammatory and degenerative diseases.

### Background Art

Necroptosis is a highly inflammatory form of cell death, which can be induced by various promoting factors, such as tumor necrosis factor (TNF) and FAS ligand. This process occurs in multiple types of cells and is considered the primary mode of cell death in pathological conditions, which is directly associated with various inflammatory and degenerative diseases. These diseases include neurodegenerative diseases, stroke, coronary heart disease, myocardial infarction, retinal degenerative diseases, inflammatory intestinal diseases, nephropathy, hepatopathy, and various other related diseases.

RIPK1 (Receptor Interacting Protein Kinase 1) is involved not only in necroptosis, but also in many crucial intracellular inflammatory signaling pathways. Besides, scientific experiments have shown that it can induce widespread inflammatory cell processes by causing cell rupture. RIPK1 is a major regulator of the cell determinant in NF-κB signaling transduction and death response, wherein, the NF-κB signaling transduction responds to widespread inflammatory and pro-apoptotic stimuli in human diseases.

Therefore, the RIPK1 inhibitor can effectively inhibit necroptosis for preventing and treating related inflammatory and degenerative diseases.

### Summary of the Invention

The purpose of the present invention is to provide novel RIPK1 inhibitors that inhibit necroptosis.

Another purpose of the present invention is to provide the preparation method for the inhibitors.

The first aspect of the present invention provides compounds as shown in general formula I, or its various isomers or pharmaceutically acceptable salts.

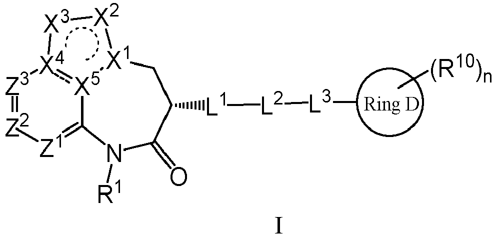

Wherein,
Z¹, Z², and Z³ are independently N, C(R);
R is independently H, halogen, -OH, -CN, -COOH, C₁-C₆ alkyl, -C₀-C₆ alkylene alkoxy, C₁-C₆ alkyl-, C₁-C₆ halogenated alkyl, C₁-C₆ alkoxy, C₁-C₆ halogenated alkoxy, C₂-C₁₀ alkoxyl-alkyl, C₂-C₁₀ halogenated alkoxyl-alkyl, C₁-C₆ hydroxyalkyl, -C₀-C₆ alkylene, -S-C₁-C₆ alkyl-, -C₀-C₆ alkylene, -C₆-C₁₀ aryl, -C₀-C₆ alkylene-X-C₆-C₁₀ aryl, -C₀-C₆ alkylene-5-10-membered heteroaryl, -C₀-C₆ alkoxy-X-5-10-membered heteroaryl, -C₀-C₆ alkylene-3-10-membered non-aromatic heterocyclyl (wherein, the heteroatoms are independently one or more of sulfur, oxygen, NH, or NR^{g}), -C₀-C₆ alkylene, -C₃-C₁₀ cycloalkyl, -C₀-C₆ alkylene, -C₃-C₁₀ cycloalkenyl, -C₀-C₆ alkylene-COR^{c}, -C₀-C₆ alkylene-CO₂-C₁-C₆ alkyl, -C₀-C₆ alkylene-CONR^{a}R^{b}, -C₀-C₆ alkylene-SO₂NR^{a}R^{b}, -C₀-C₆ alkyleneS(O)₂R^{c}, SF₅, -C₀-C₆ alkylene-NR^{a}R^{b}, -C₀-C₆ alkylene-NHC(O)R^{c}, -C₀-C₆ alkylene-NHC(O)C(O)NR^{a}R^{b}, -C₀-C₆ alkoxy-NHC(O)C(O)OR^{a}, -C₀-C₆ alkylene-NHC(O)NR^{a}R^{b}, -P(O)Me₂, -P(O)(OMe)₂, -C₀-C₆ alkylene-C₃-C₆ cycloalkyloxy, -C₀-C₆ alkylene-C₁-C₆ alkoxy, -C₀-C₆ alkylene-C₁-C₆ halogenated alkoxy, -C₀-C₆ alkylene-C=C-R², -O-C₁-C₆ alkylene-C=C-R², -S-C₁-C₆ alkylene-C≡C-R², and -C₀-C₆ alkylene-CH=CH-R²; R can be unsubstituted or substituted by 1 to 4 R^{f}; when R is connected to a nitrogen atom, R is not a halogen.

R can be specifically selected from the following groups: 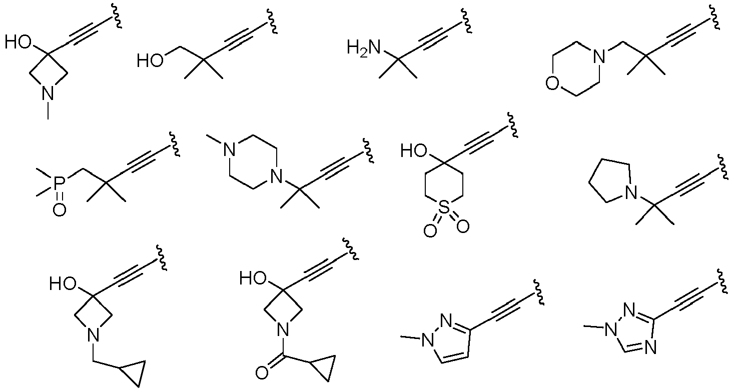 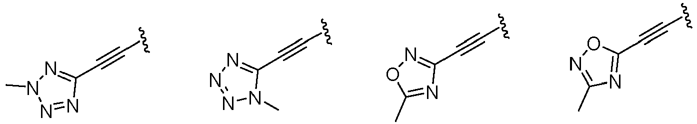 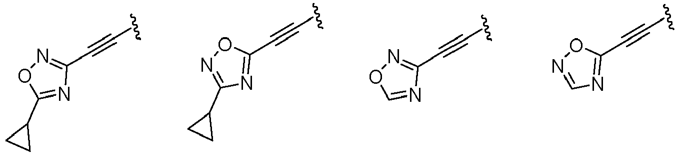 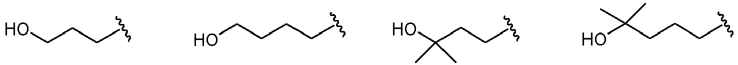 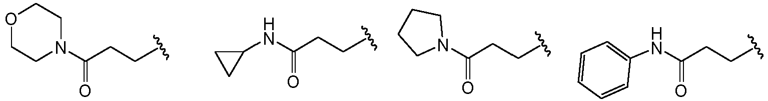 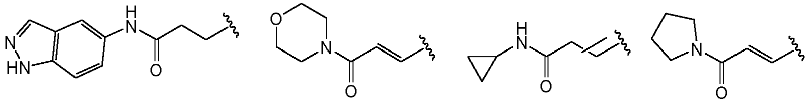 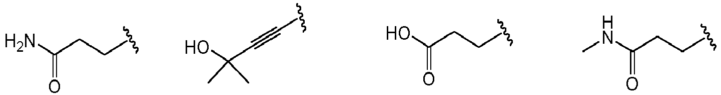 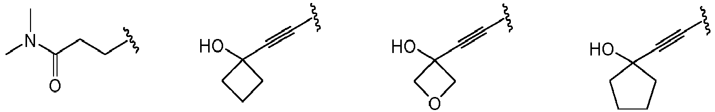 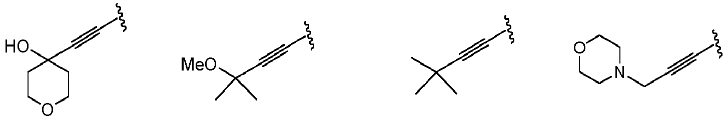 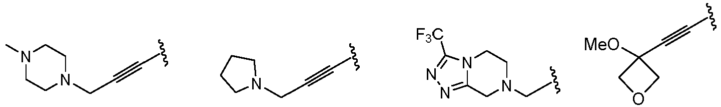 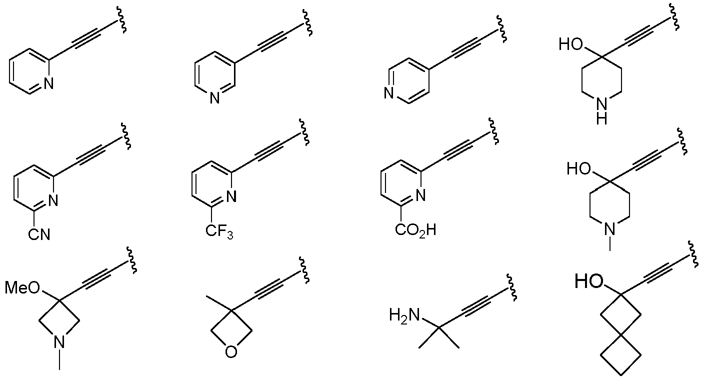 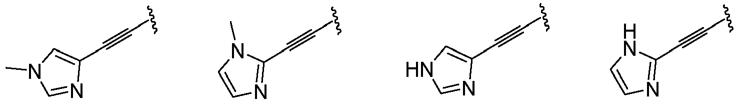 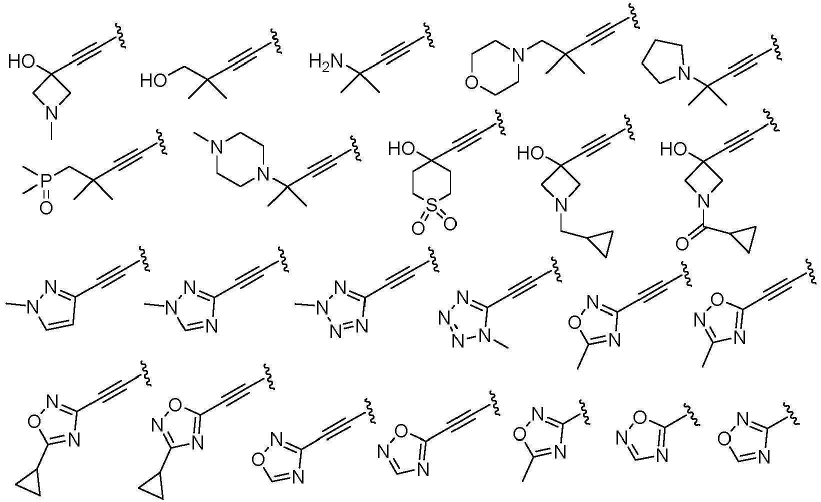 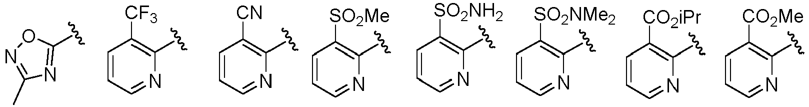 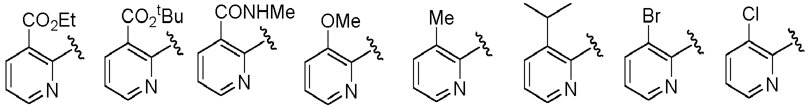 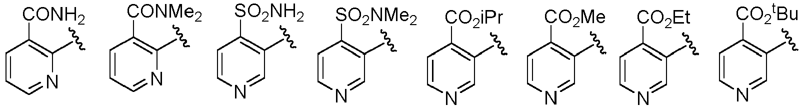 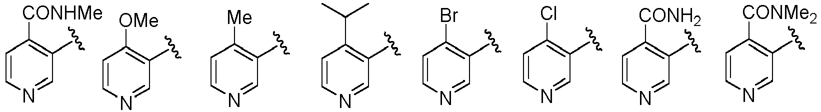 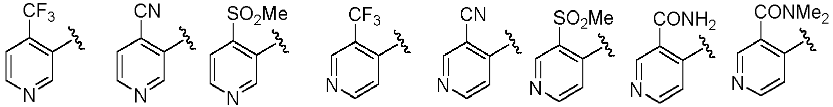 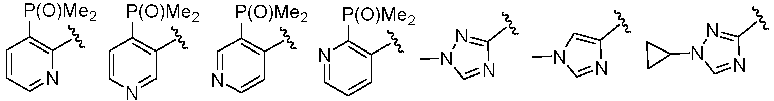 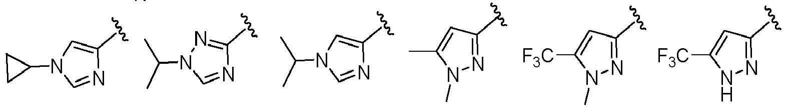
X can be O, S, SO, S(O)₂, NH, C(O), CH₂, CF₂, CH(CH₃), CH(OH), or N(CH₃);
R¹ and R^{1a} are independently of H, C₁-C₆ alkyl or -C₀-C₃ alkylene-C₃-C₆ cycloalkyl;
R² is hydrogen, C₁-C₁₀ alkyl, -C₀-C₆ alkylene-C₆-C₁₀ aryl, -C₀-C₆ alkylene-5-10-membered heteroaryl, -C₀-C₆ alkylene-3-10-membered non-aromatic heterocyclyl (wherein, the heteroatoms are independent one or more of sulfur, oxygen, NH, or NR^{g}), -C₀-C₆ alkylene-C₃-C₁₀ cycloalkyl, or -C₀-C₆ alkylene-C₁-C₁₀ alkoxy; R² can be independently unsubstituted or substituted by 1 to 4 R^{f};
R^{f}, at each occurrence, is independently halogen, -OH, carbonyl, -CN, -COOH, C₁-C₆ alkyl, - C₀-C₆ alkylene alkoxy, C₁-C₆ alkyl, C₁-C₆ halogenated alkyl, C₁-C₆ alkoxy, C₁-C₆ halogenated alkoxy, C₂-C₁₀ alkoxyl-alkyl, C₂-C₁₀ halogenated alkoxyl-alkyl, C₁-C₆ hydroxyalkyl, -C₀-C₆ alkylene-S-Ci-C₆ alkyl, -SF₅, -C₀-C₆ alkylene-COR^{c}, -C₀-C₆ alkylene-CO₂C₁-C₆ alkyl, -C₀-C₆ alkylene-CONR^{a}R^{b}, -C₀-C₆ alkylene-SO₂NR^{a}R^{b}, -C₀-C₆ alkylene-S(O)₂R^{c}, -C₀-C₆ alkylene-NR^{a}R^{b}, -C₀-C₆ alkylene-C(O)NR^{a}R^{b}, -C₀-C₆ alkylene-NHC(O)R^{c}, -C₀-C₆ alkylene-NHC(O)C(O)NR^{a}R^{b}, -C₀-C₆ alkylene-NHC(O)C(O)OR^{a}, -C₀-C₆ alkylene-NHC(O)NR^{a}R^{b}, -C₀-C₆ alkylene-P(O)Me₂, -C₀-C₆ alkylene-P(O)(OMe)₂. Two neighboring R^{f} or two R^{f} connected to the same carbon atom together form a 3- to 8-membered ring or a 4- to 8-membered heterocyclic ring, which may contain heteroatoms such as sulfur, oxygen, NH, or NR^{g};
R^{a} and R^{b} are independently hydrogen, substituted or unsubstituted C₁-C₁₀ alkyl, substituted or unsubstituted C₃-C₁₀ cycloalkyl, substituted or unsubstituted C₂-C₁₀ alkenyl, substituted or unsubstituted C₆-C₁₀ aryl, or substituted or unsubstituted C₃-C₁₀ heteroaryl respectively; R^{a} and R^{b} can form a 3- to 8-membered ring or a 4- to 8-membered heterocyclic ring when connected to a nitrogen or carbon atom, which may contain heteroatoms such as sulfur, oxygen, NH, or NR^{g}; the said 3- to 8-membered ring or 4- to 8-membered heterocyclic ring can be substituted by one or multiple R^{e};
R^{c} is independently H, C₁-C₆ alkyl, C₃-C₆ cycloalkyl, C₁-C₆ halogenated alkyl, C₃-C₁₀ cycloalkenyl, C₁-C₆ alkoxy, C₁-C₆ cycloalkyloxy, C₀-C₆ alkylene hydroxyl, C₆-C₁₀ aryl, 5- to 10-membered heteroaryl, 3- to 10-membered non-aromatic heterocyclyl, -C₀-C₆ alkylene-CF₃, -C₁-C₆ alkylene-CN, -C₁-C₆ alkylene-C₁-C₆ alkoxy, C₁-C₆ alkylene-NR^{a}R^{b}, C₁-C₆ alkylene-NR^{b}C(O)R^{a}, C₁-C₆ alkylene-NR^{b}S(O)₂R^{a}, C₁-C₆ alkylene-carboxyl, -C₁-C₆ alkylene-CO₂C₁-C₆ alkyl, C₁-C₆ alkylene-COR^{a}, -C₀-C₆ alkylene-CONR^{a}R^{b}; wherein, the said C₆-C₁₀ aryl, 5- to 10-membered heteroaryl, 3- to 10-membered non-aromatic heterocyclyl, C₃-C₆ cycloalkyl, or C₃-C₁₀ cycloalkenyl is each independently unsubstituted or substituted by one or two substituents independently selected from the following: halogen, C₁-C₆ alkyl, C₃-C₆ cycloalkyl, C₁-C₄ alkoxy, C₀-C₆ alkylene-NR^{a}R^{b}, -C₀-C₆ alkylene-CN, -C₀-C₆ alkylene-OH;
R^{g} is C₁-C₁₀ alkyl, C₁-C₁₀ heteroalkyl, C₃-C₁₀ cycloalkyl, C₆-C₁₀ aryl, or C₃-C₁₀ heteroaryl;
X¹, X², X³, X⁴, and X⁵ are independently selected from C, CR, N, and NR, provided that the rules of valence bonding are conformed; 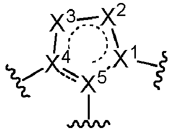 is selected from the following:

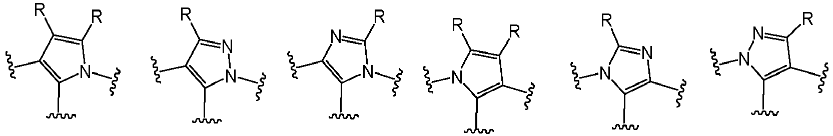

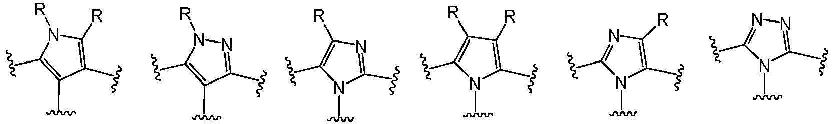

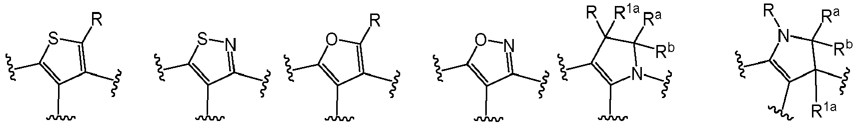

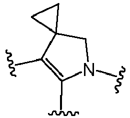

The said 3- to 10-membered non-aromatic heterocyclyl can also have the bicyclic or spiral ring structures as follows:

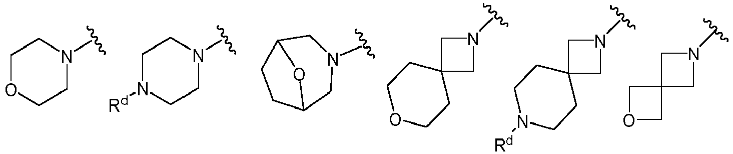

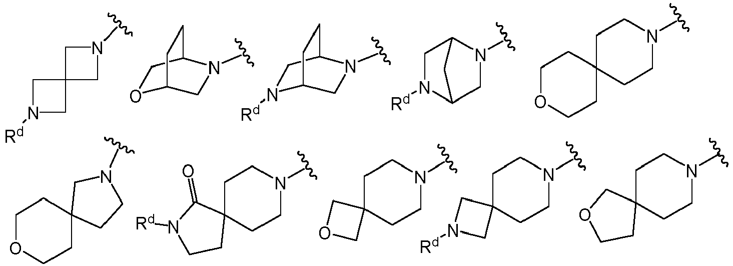

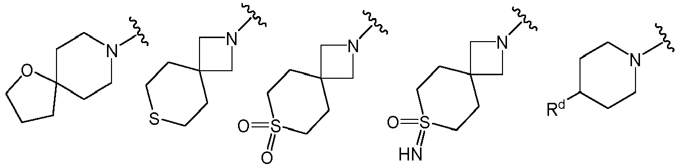

L¹ is structured as follows:

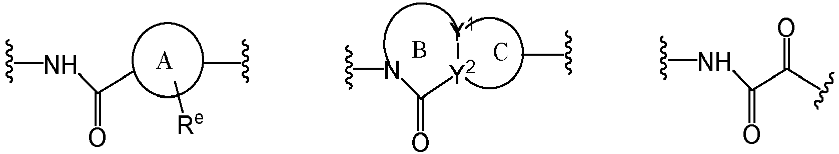

Ring A is a substituted or unsubstituted benzene ring, a 5- to 6-membered heteroaromatic ring, or a 5- to6-membered non-aromatic heterocyclic ring; Ring A can be furan, thiophene, isoxazole, oxazole, thiazole, oxadiazole, pyrrole, pyrazole, imidazole, triazole, or tetrazole;

Preferably, Ring A is selected from the following structures: 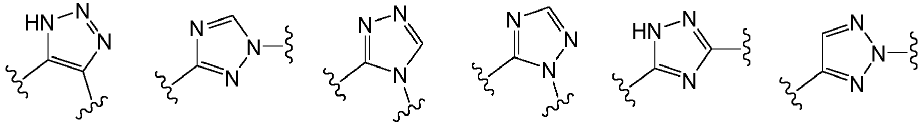 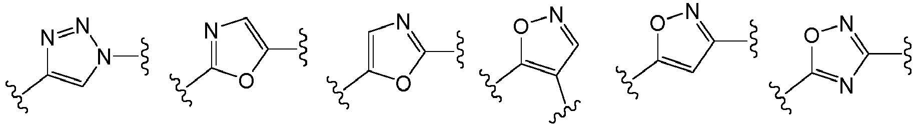 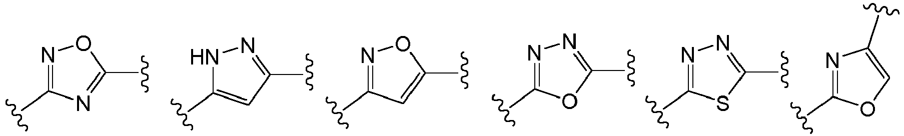 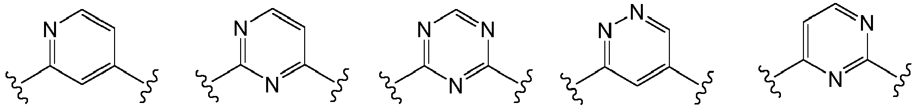 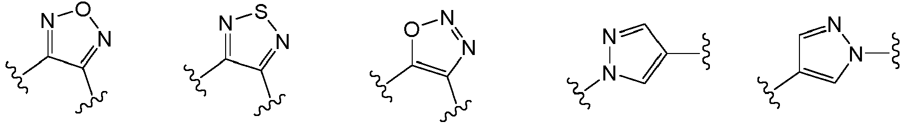
Ring A can be substituted by one or two halogens, -CN, C₁-C₆ alkyl, or C₁-C₆ halogenated alkyl;
Ring B is a substituted or unsubstituted 5- to 7-membered heterocyclic ring containing nitrogen atom;
Ring C is a substituted or unsubstituted benzene ring, or a 5- to 6-membered heteroaromatic ring;
Y¹ and Y² are independently carbon atoms or nitrogen atoms;
Preferably, 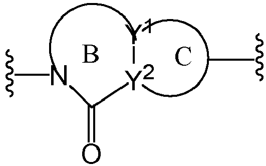 is structured as follows: 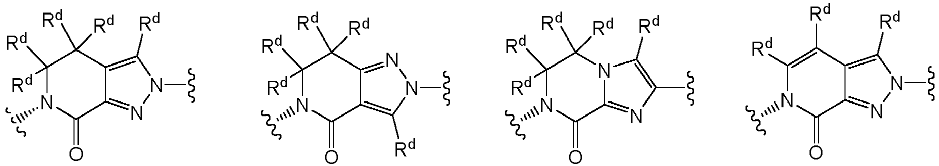 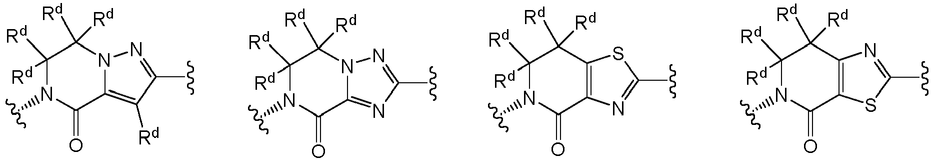 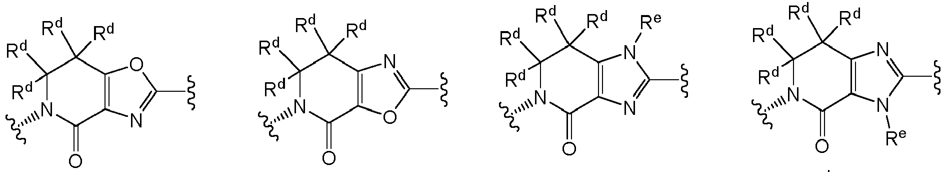 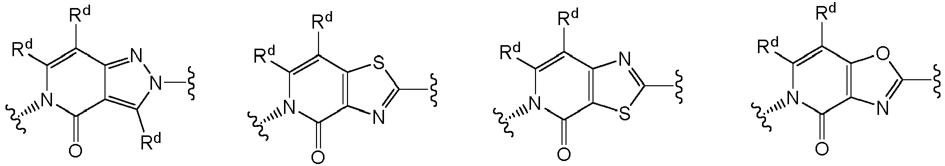 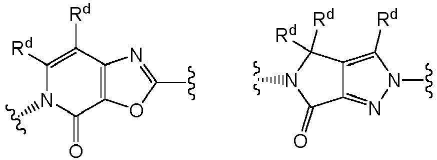
R^{d} is independently selected from H, halogen, C₁-C₆ alkyl, or C₁-C₆ halogenated alkyl;
R^{e} is H, C₁-C₆ alkyl, or C₁-C₆ halogenated alkyl;
L² is O, NR^{g}, CR^{a}R^{b}, or absent;
L³ is C₀-C₆ alkylene, C₂-C₆ alkenylene, C₃-C₆ cycloalkyl, benzene ring, 5- to 6-membered heteroaromatic ring, or 5- to 6-membered non-aromatic heterocyclic ring; L³ can be substituted by 1 to 3 R⁵;
R⁵ is independently H, halogen, -OH, -CN, carbonyl, C₁-C₆ alkyl, C₁-C₆ halogenated alkyl, C₁-C₆ alkoxy, or C₁-C₆ halogenated alkoxy;
Ring D is C₃-C₆ cycloalkyl, phenyl, naphthyl, C₃-C₆ cycloalkyl and phenyl, 5- to 6-membered heteroaryl, or 5- to 6-membered non-aromatic heterocyclyl;
R¹⁰ is independently selected from halogen, CN, C₁-C₆ alkyl, C₁-C₆ halogenated alkyl, OC₁-C₆ alkyl, or C₃-C₆ cycloalkyl;
n is 0, 1, 2, or 3.

In another preferred embodiment, the said compound of general formula I is the compound shown in general formula II:

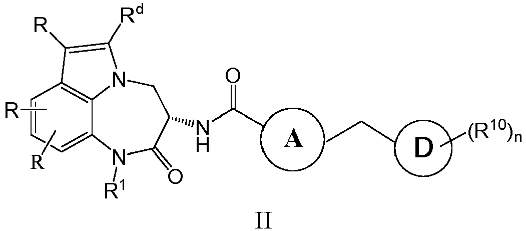

Wherein, the independent definitions of R are as stated for general formula I; R¹ is H, methyl or ethyl.
R^{d} is independently H, halogen, C₁-C₆ alkyl, or C₁-C₆ halogenated alkyl;
Ring A is structured as follows: 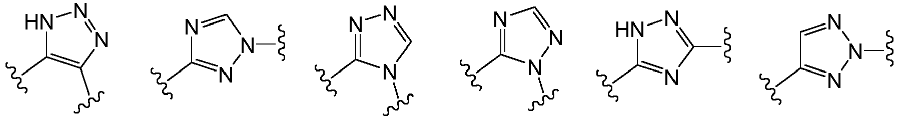 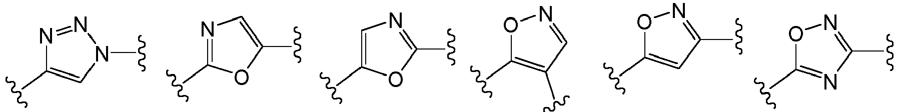 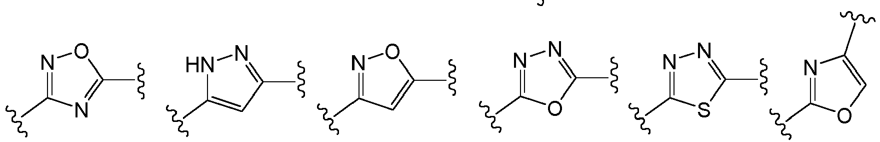 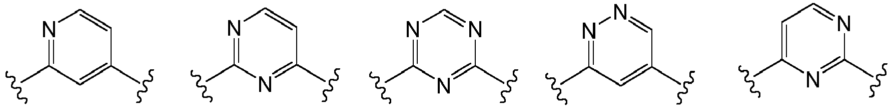
Ring D is a benzene ring or thiophene;
R¹⁰ is a halogen or -CN.
n is 0, 1, 2, or 3.

In another preferred embodiment, the said compound of general formula I is the compound shown in general formula III:

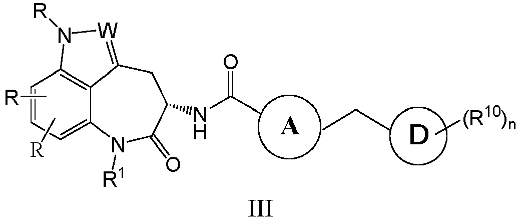

Wherein, the independent definitions of R are as stated for general formula I, provided that R is not a halogen when R is connected to a nitrogen atom; R¹ is H, methyl or ethyl.
W is N or CR^{d};
R^{d} is H, halogen, C₁-C₆ alkyl, or C₁-C₆ halogenated alkyl;
Ring A is structured as follows: 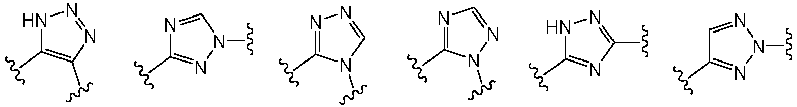 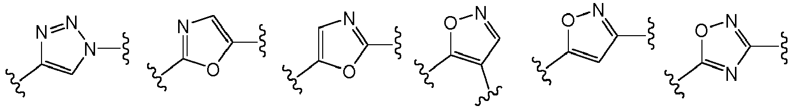 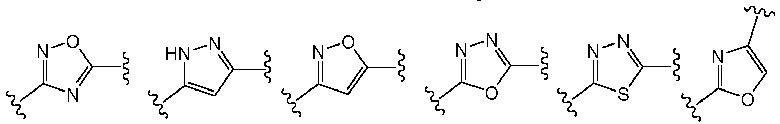 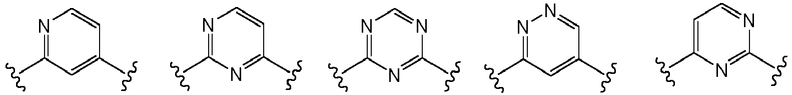 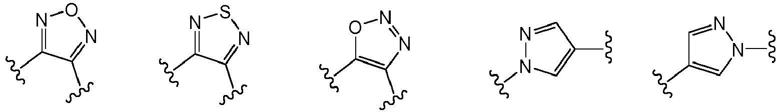
Ring D is a benzene ring or thiophene;
R¹⁰ is an independently halogen or -CN;
n is 0, 1, 2, or 3.

In another preferred embodiment, the said compound of general formula I is the compound shown in general formula IV:

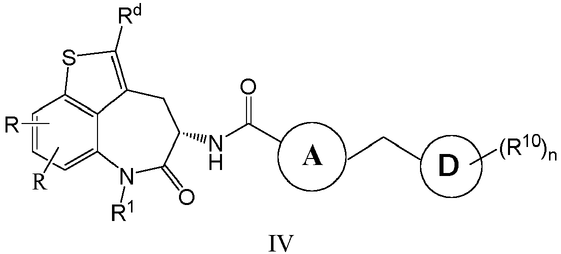

Wherein, the independent definitions of R are as stated in general formula I; R¹ is H, methyl or ethyl.
R^{d} is independently H, halogen, C₁-C₆ alkyl, or C₁-C₆ halogenated alkyl;
Ring A is structured as follows: 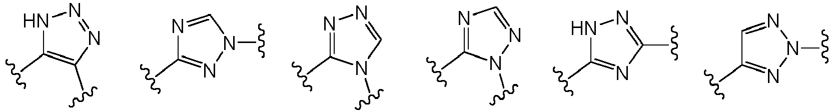 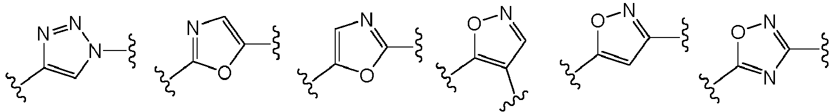 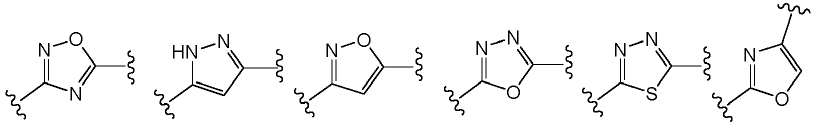 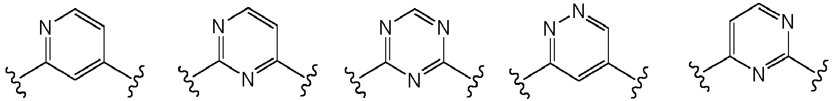 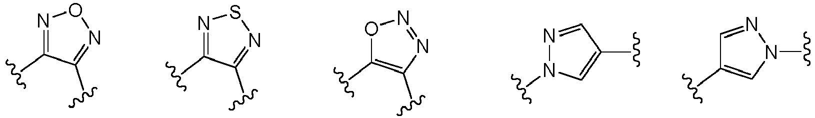
Ring D is a benzene ring or thiophene;
R¹⁰ is an independently halogen or -CN;
n is 0, 1, 2, or 3.

In another preferred embodiment, the said compound of general formula I is the compounds shown in general formula V:

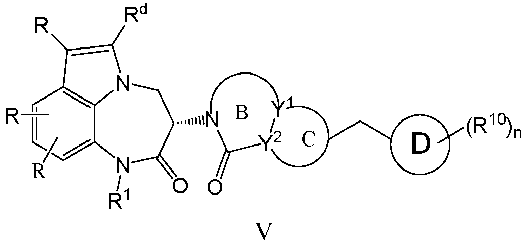

Wherein, the independent definitions of Rand R¹⁰ are as stated for general formula I; R¹ is H, methyl or ethyl. 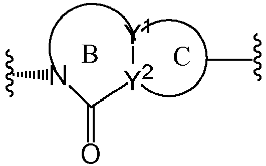 is structured as follows: 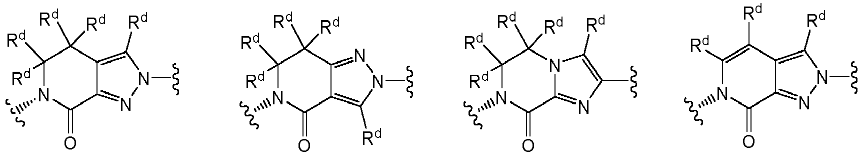 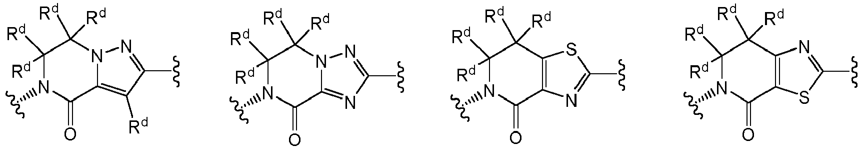 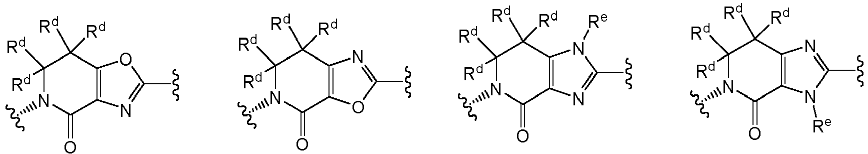 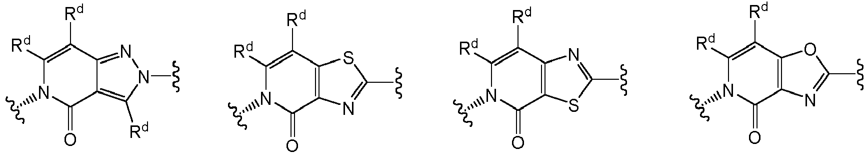 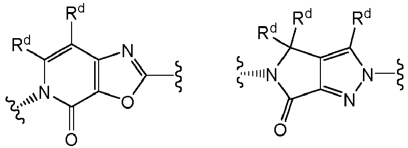
R^{d} is independently H, halogen, C₁-C₆ alkyl, or C₁-C₆ halogenated alkyl;
R^{e} is independently H, C₁-C₆ alkyl, or C₁-C₆ halogenated alkyl;
Ring D is a benzene ring or thiophene;
R¹⁰ is an independently halogen or -CN.
n is 0, 1, 2, or 3.

In another preferred embodiment, the said compound of general formula I is the compound shown in general formula VI:

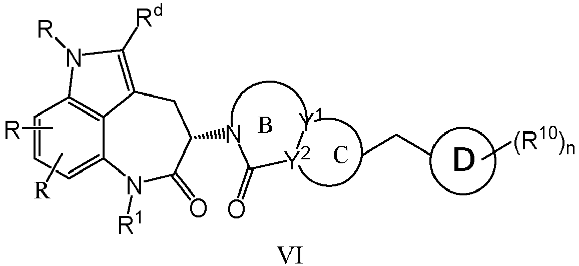

Wherein, the independent definitions of R and R¹⁰ are as stated for general formula I, provided that R is not a halogen when R is connected to a nitrogen atom; R¹ is H, methyl or ethyl. 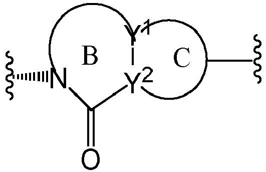 is structured as follows: 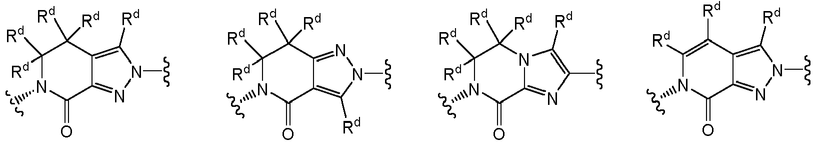 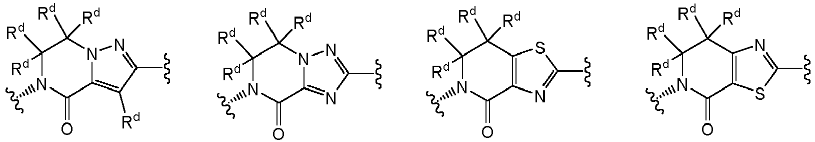 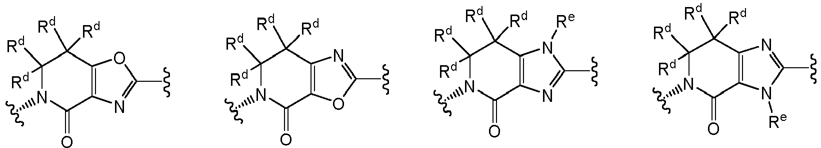 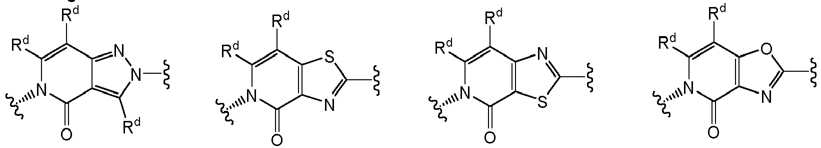 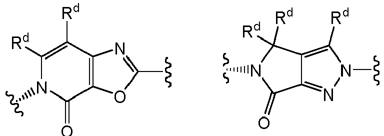
R^{d} is independently H, halogen, C₁-C₆ alkyl, or C₁-C₆ halogenated alkyl;
R^{e} is H, C₁-C₆ alkyl, or C₁-C₆ halogenated alkyl;
Ring D is a benzene ring or thiophene;
R¹⁰ is an independently halogen or -CN.
n is 0, 1, 2, or 3.

In another preferred embodiment, the said compound of general formula I is the compound shown in general formula VII:

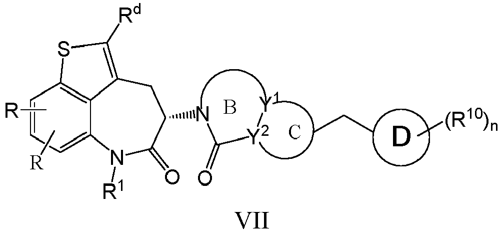

Wherein, the independent definitions of R are as stated for general formula I, provided that R is not a halogen when R is connected to a nitrogen atom; R¹ is H, methyl or ethyl. 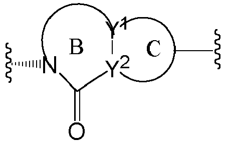 is structured as follows: 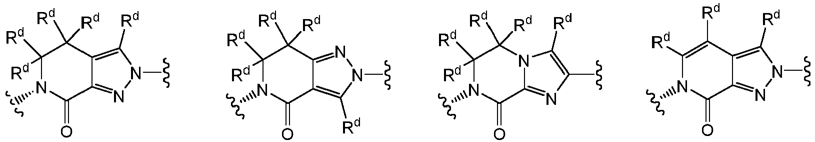 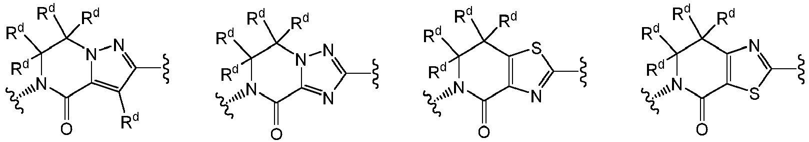 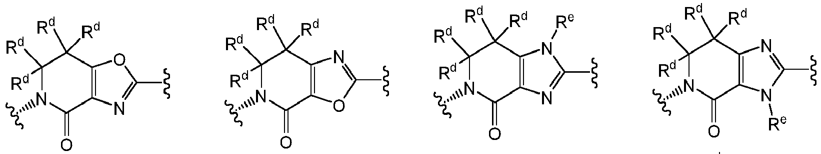 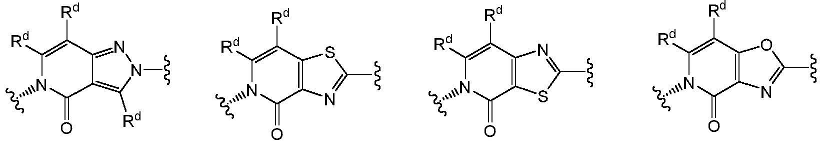 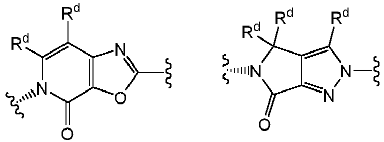
R^{d} is independently H, halogen, C₁-C₆ alkyl, or C₁-C₆ halogenated alkyl;
R^{e} is H, C₁-C₆ alkyl, or C₁-C₆ halogenated alkyl;
Ring D is a benzene ring or thiophene;
R¹⁰ is an independently halogen or -CN.
n is 0, 1, 2, or 3.

In another preferred embodiment, the said compound of general formula I is the compound shown in general formula VIII:

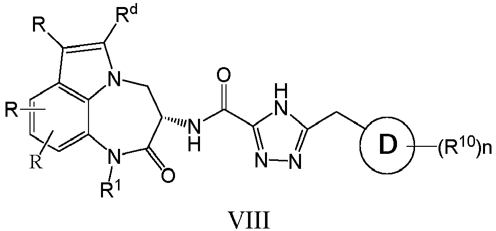

Wherein, the independent definitions of R are as stated for general formula I; R¹ is H, methyl or ethyl.
Ring D is a benzene ring or thiophene;
R^{d} is H, halogen, C₁-C₆ alkyl, or C₁-C₆ halogenated alkyl;
R¹⁰ is an independently halogen or -CN;
n is 0, 1, 2, or 3.

In another preferred embodiment, the said compound of general formula I is the compound shown in general formula IX: 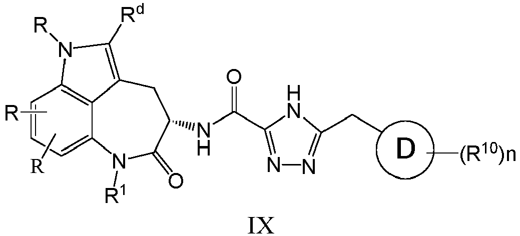
Wherein, the independent definitions of R are as stated for general formula I, provided that R is not a halogen when R is connected to a nitrogen atom; R¹ is H or methyl;
R¹⁰ is a halogen or -CN;
R^{d} is H, halogen, C₁-C₆ alkyl, or C₁-C₆ halogenated alkyl;
Ring D is a benzene ring or thiophene;
n is 0, 1, 2, or 3.

In another preferred embodiment, the said compound of general formula I is the compound shown in general formula X:

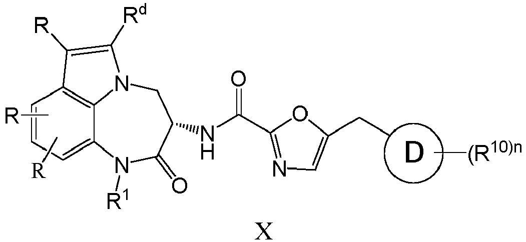

Wherein, the independent definitions of R are as stated for general formula I; R¹ is H, methyl or ethyl.
Ring D is a benzene ring or thiophene;
R^{d} is H, halogen, C₁-C₆ alkyl, or C₁-C₆ halogenated alkyl;
R¹⁰ is a halogen or -CN;
n is 0, 1, 2, or 3.

In another preferred embodiment, the said compound of general formula I is the compound shown in general formula XI:

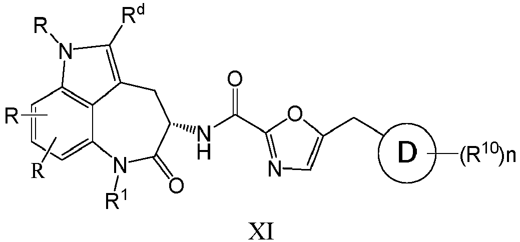

Wherein, the independent definitions of R are as stated for general formula I; R¹ is H, methyl or ethyl.
Ring D is a benzene ring or thiophene;
R^{d} is H, halogen, C₁-C₆ alkyl, or C₁-C₆ halogenated alkyl;
R¹⁰ is a halogen or -CN;
n is 0, 1, 2, or 3.

In another preferred embodiment, the said compound of general formula I is the compound shown in general formula XII:

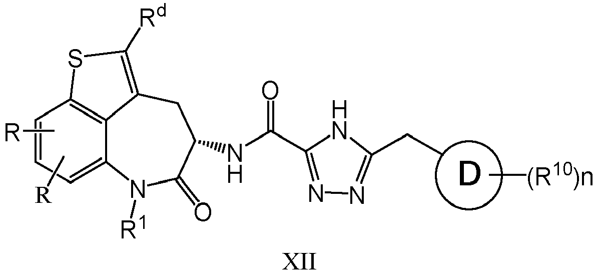

Wherein, the independent definitions of R are as stated for general formula I; R¹ is H, methyl or ethyl.
Ring D is a benzene ring or thiophene;
R^{d} is H, halogen, C₁-C₆ alkyl, or C₁-C₆ halogenated alkyl;
R¹⁰ is a halogen or -CN;
n is 0, 1, 2, or 3.

In another preferred embodiment, R in the compounds of general formulas I to XII is selected from the following groups: halogen,

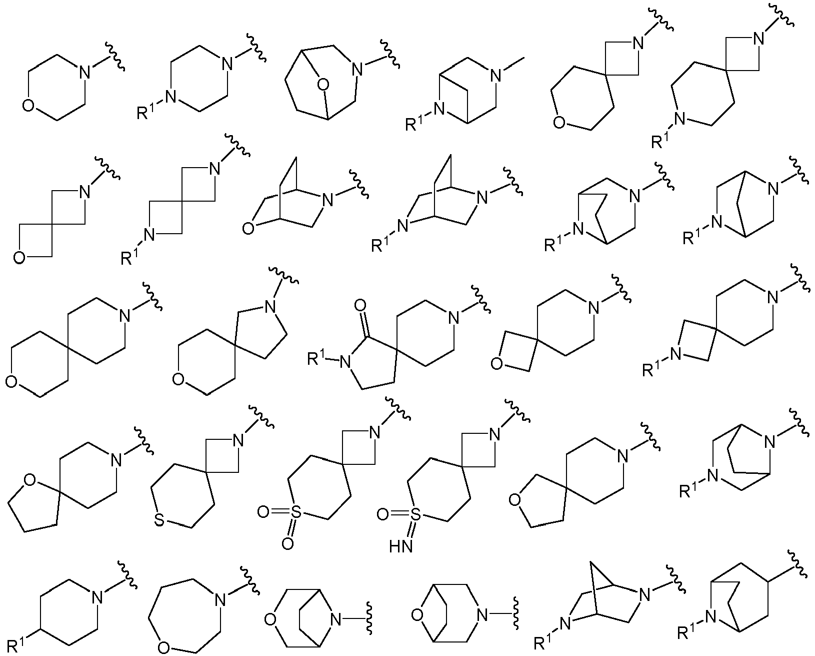

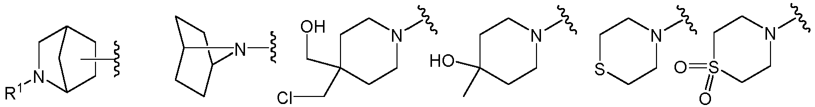

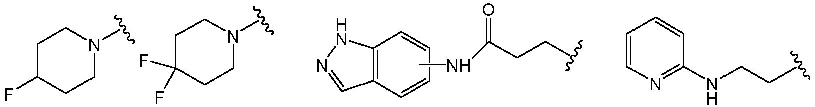

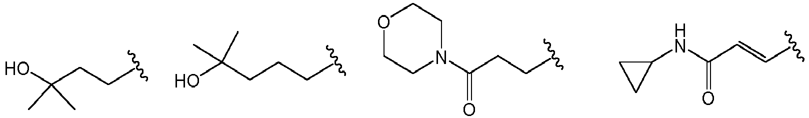

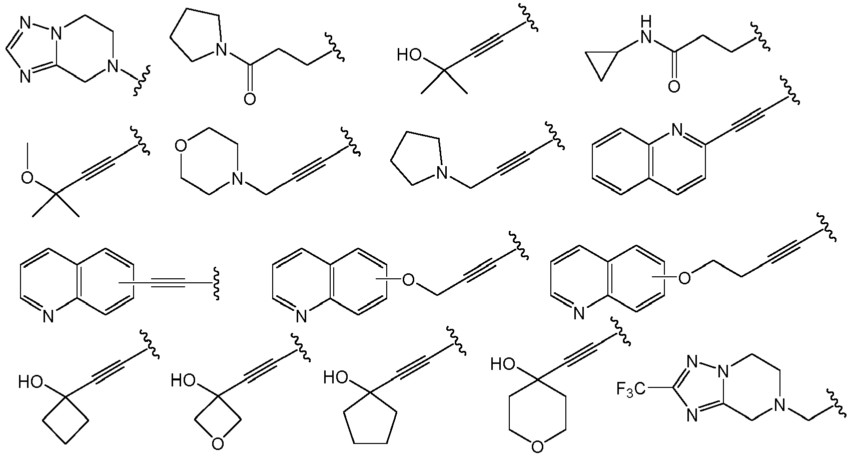

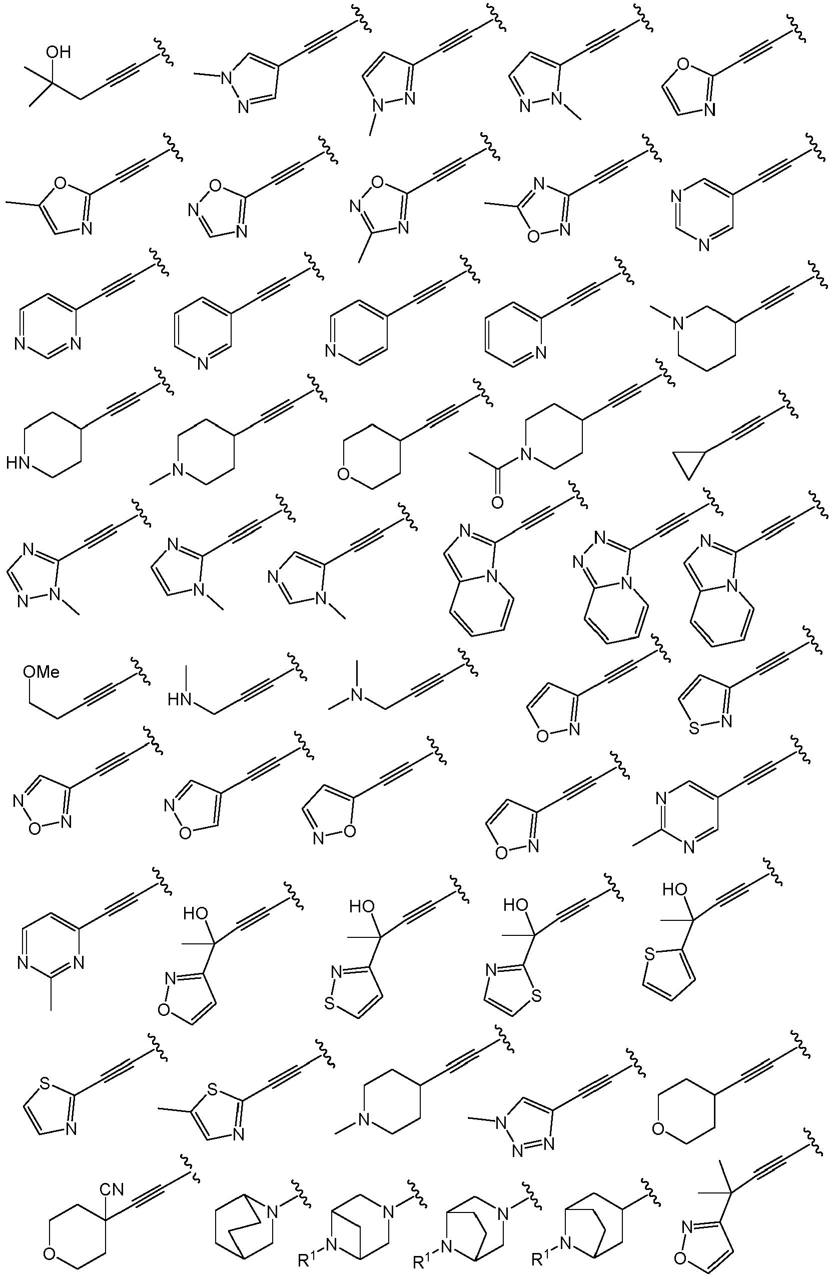

In another preferred embodiment, the said compounds of general formulas I to XII are as follows:

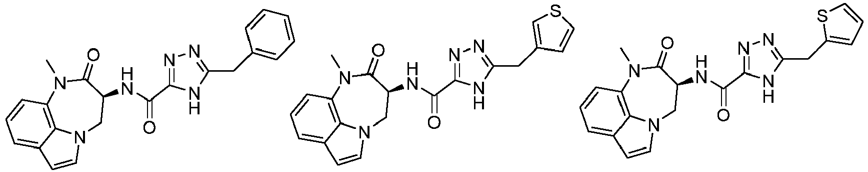

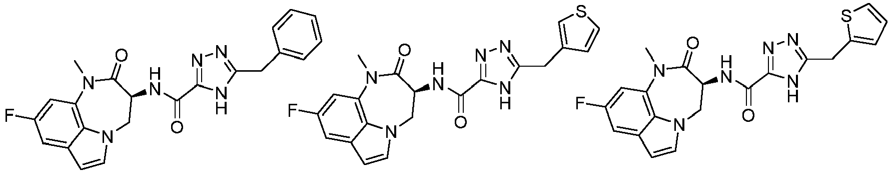

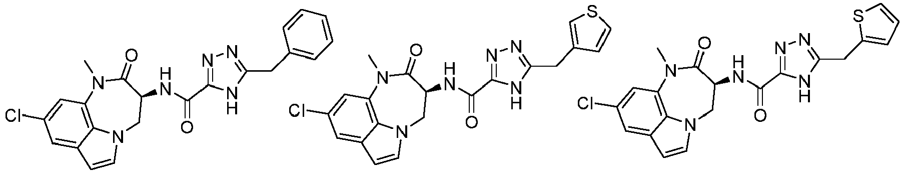

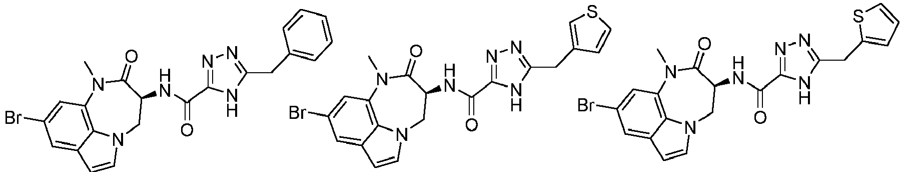

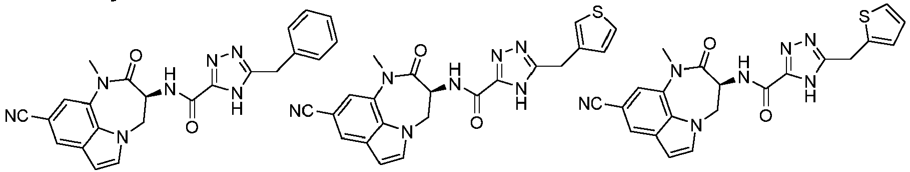

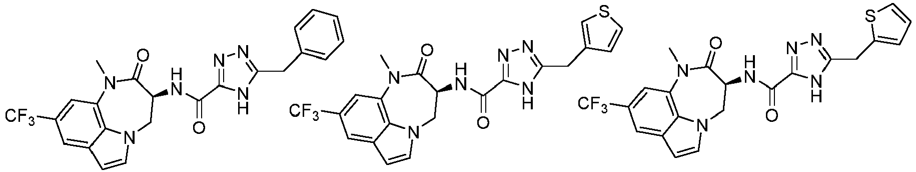

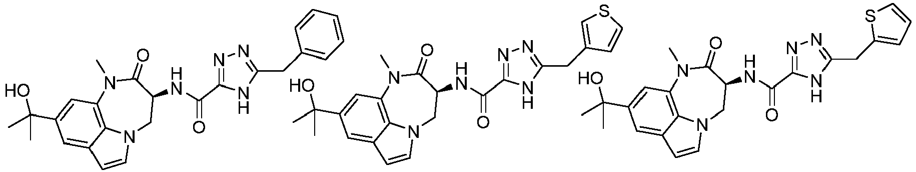

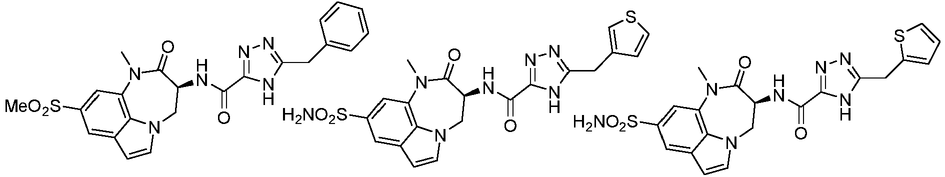

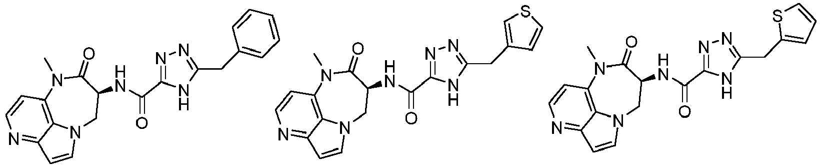

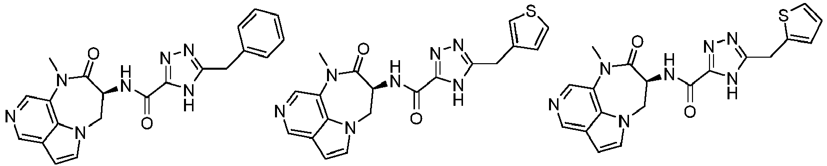

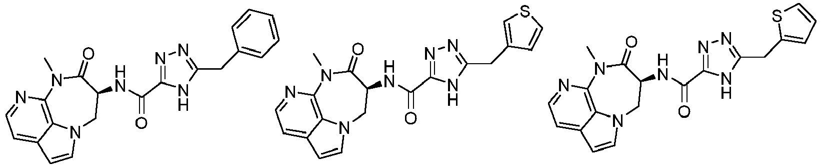

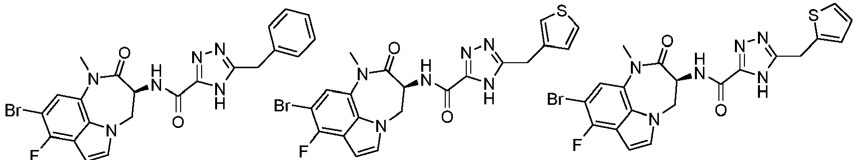

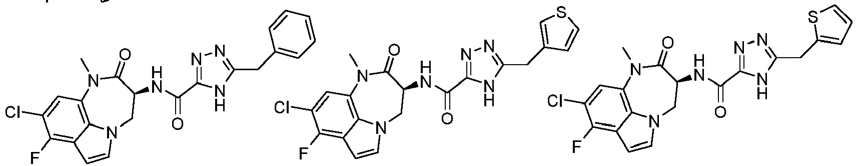

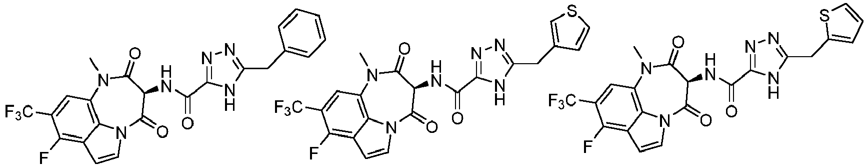

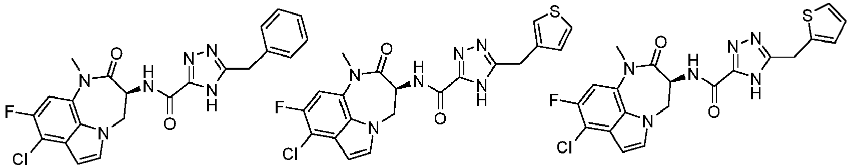

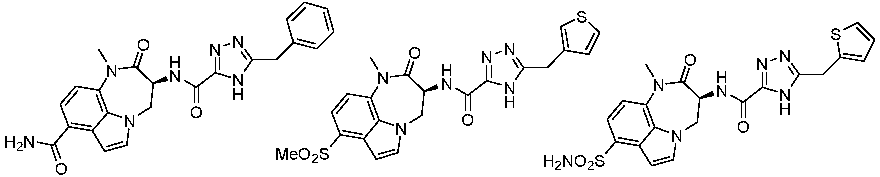

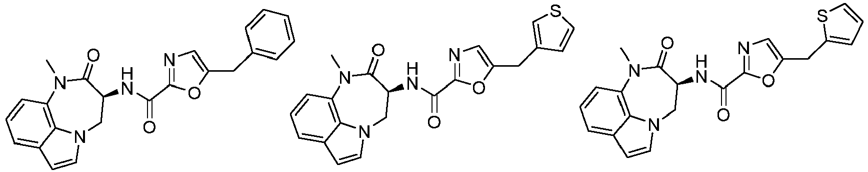

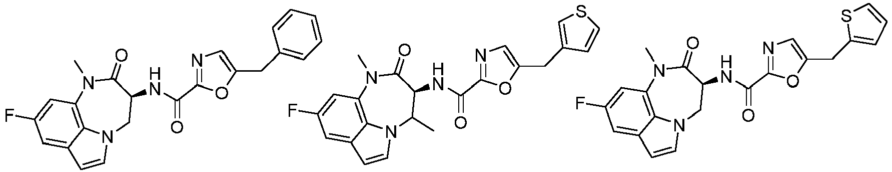

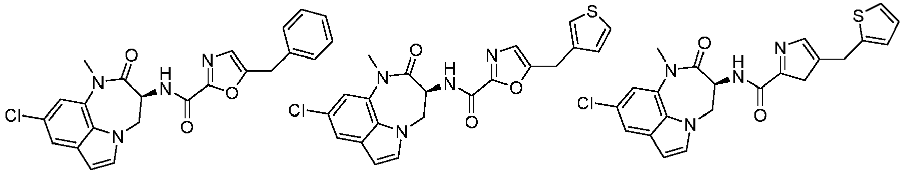

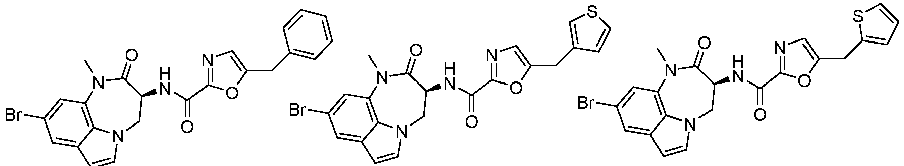

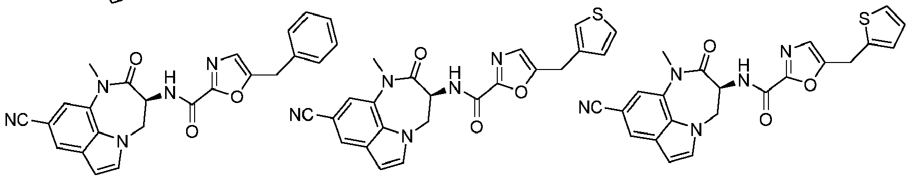

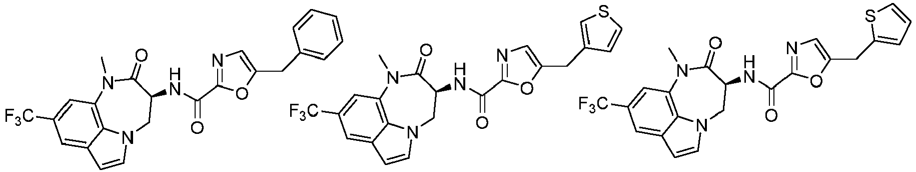

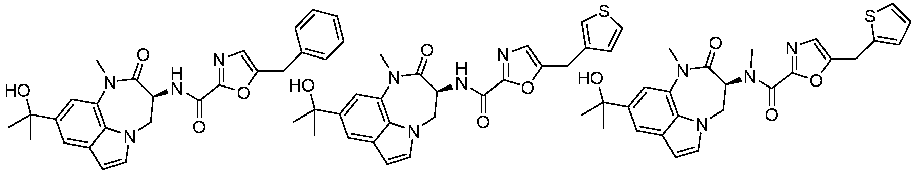

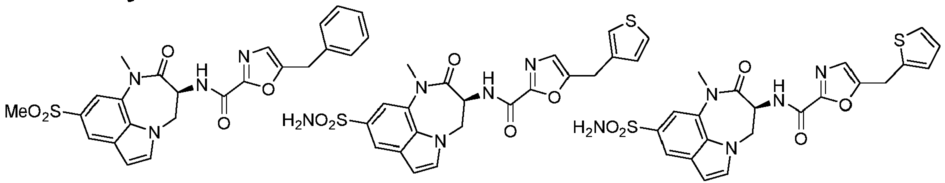

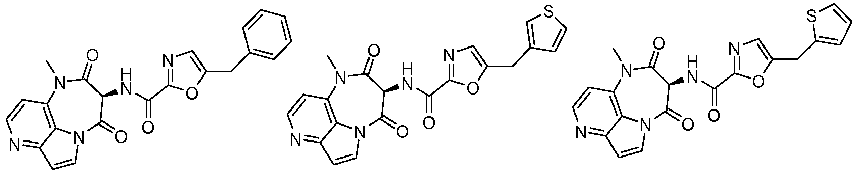

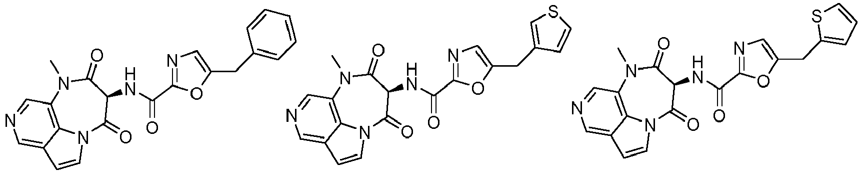

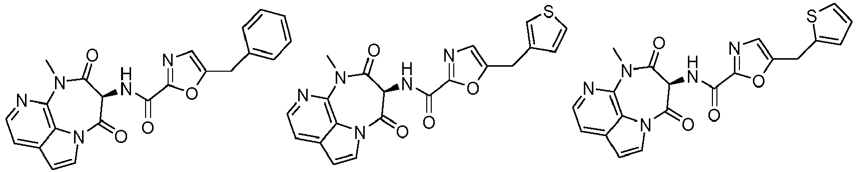

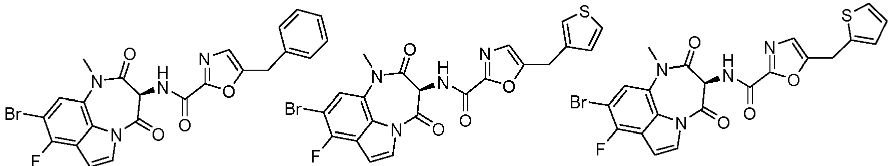

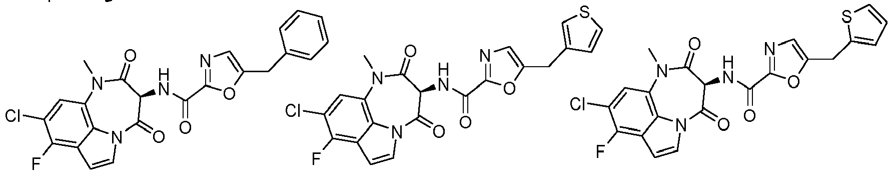

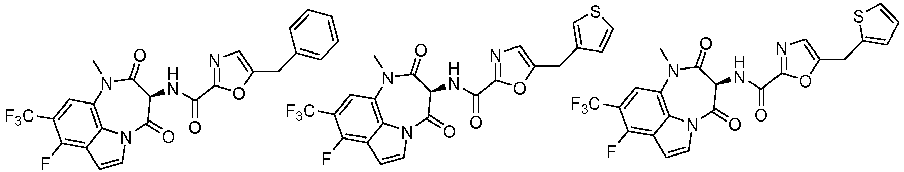

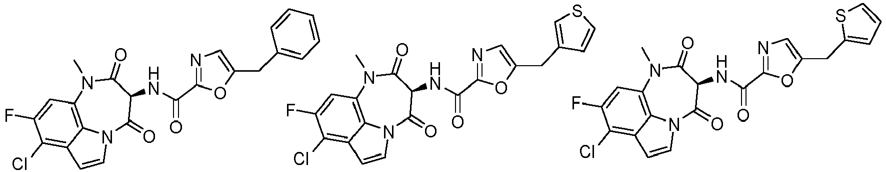

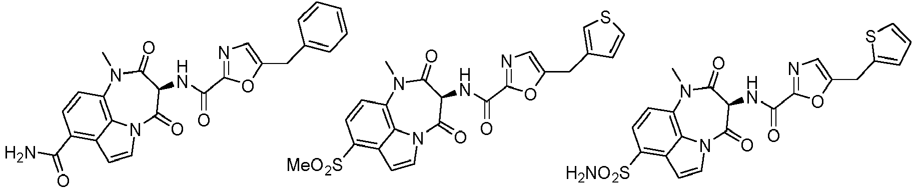

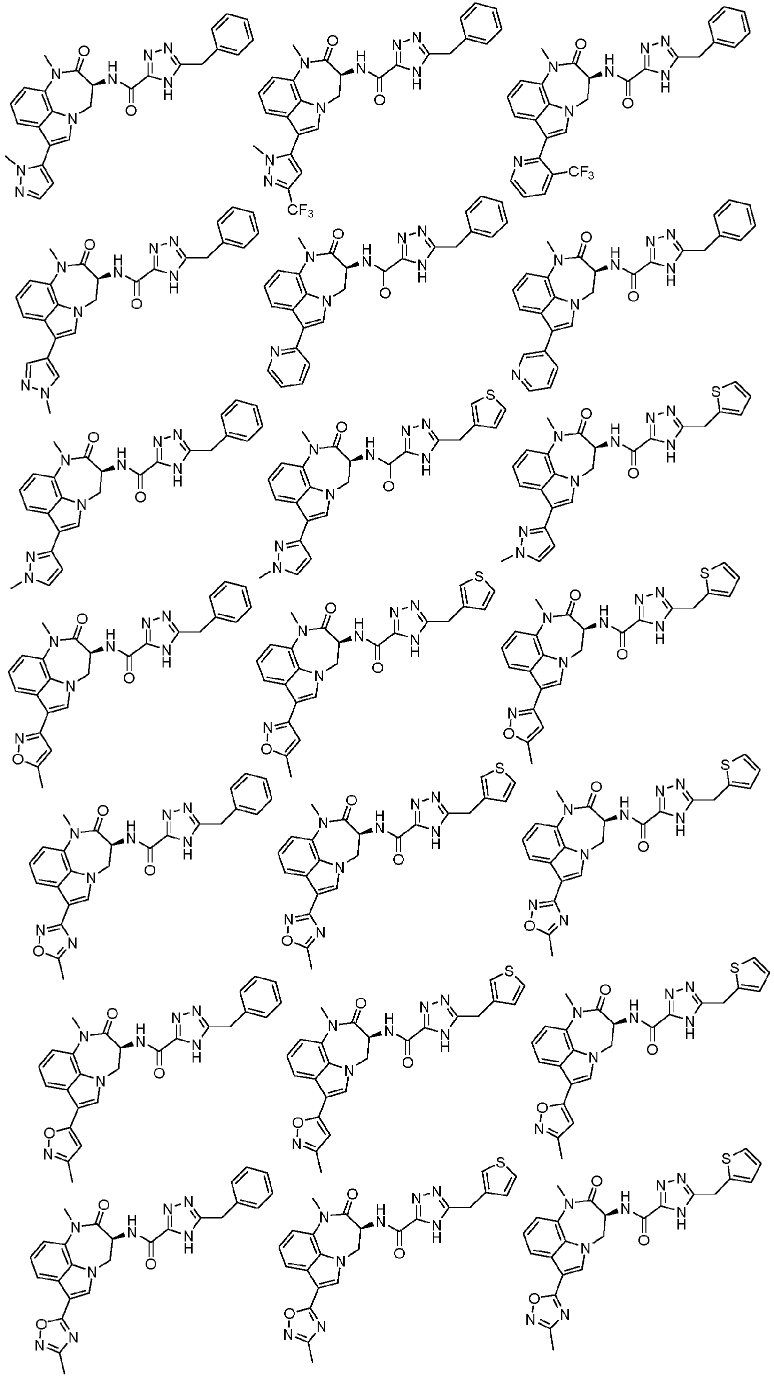

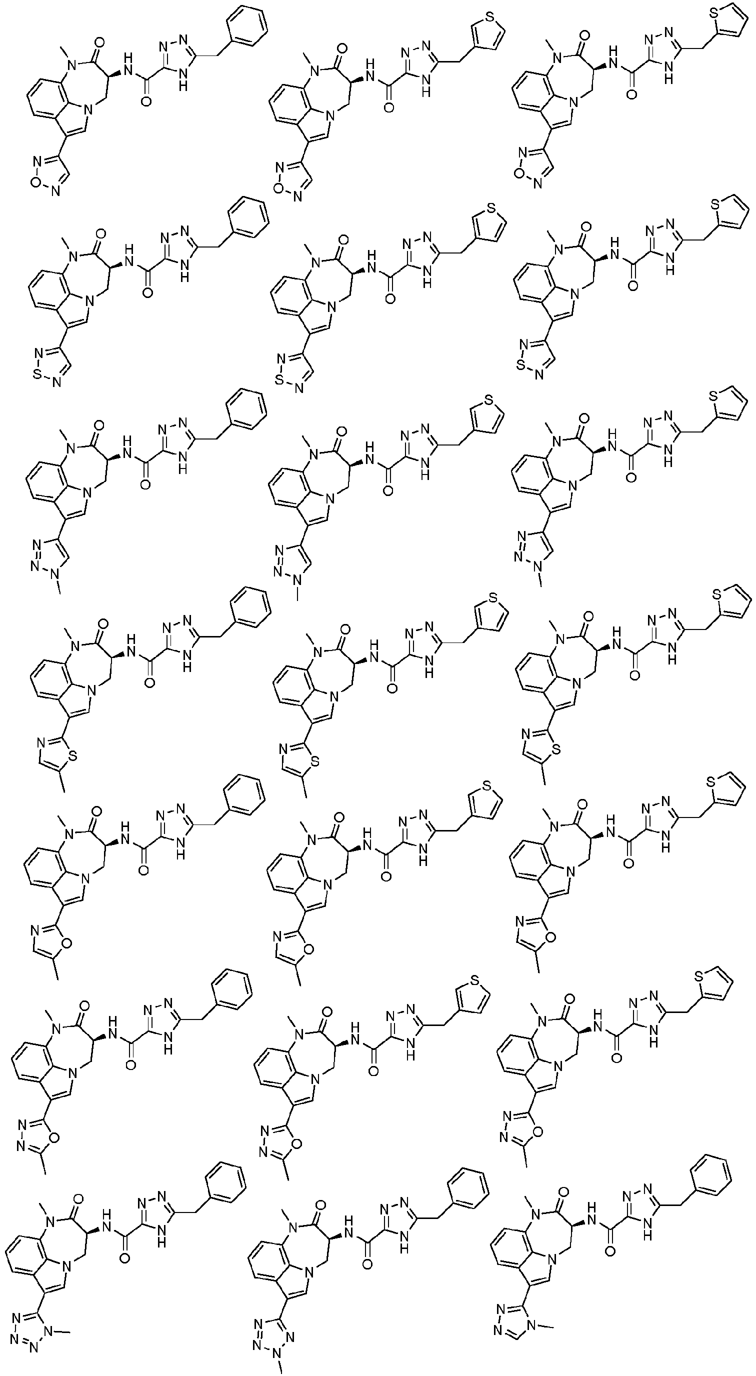

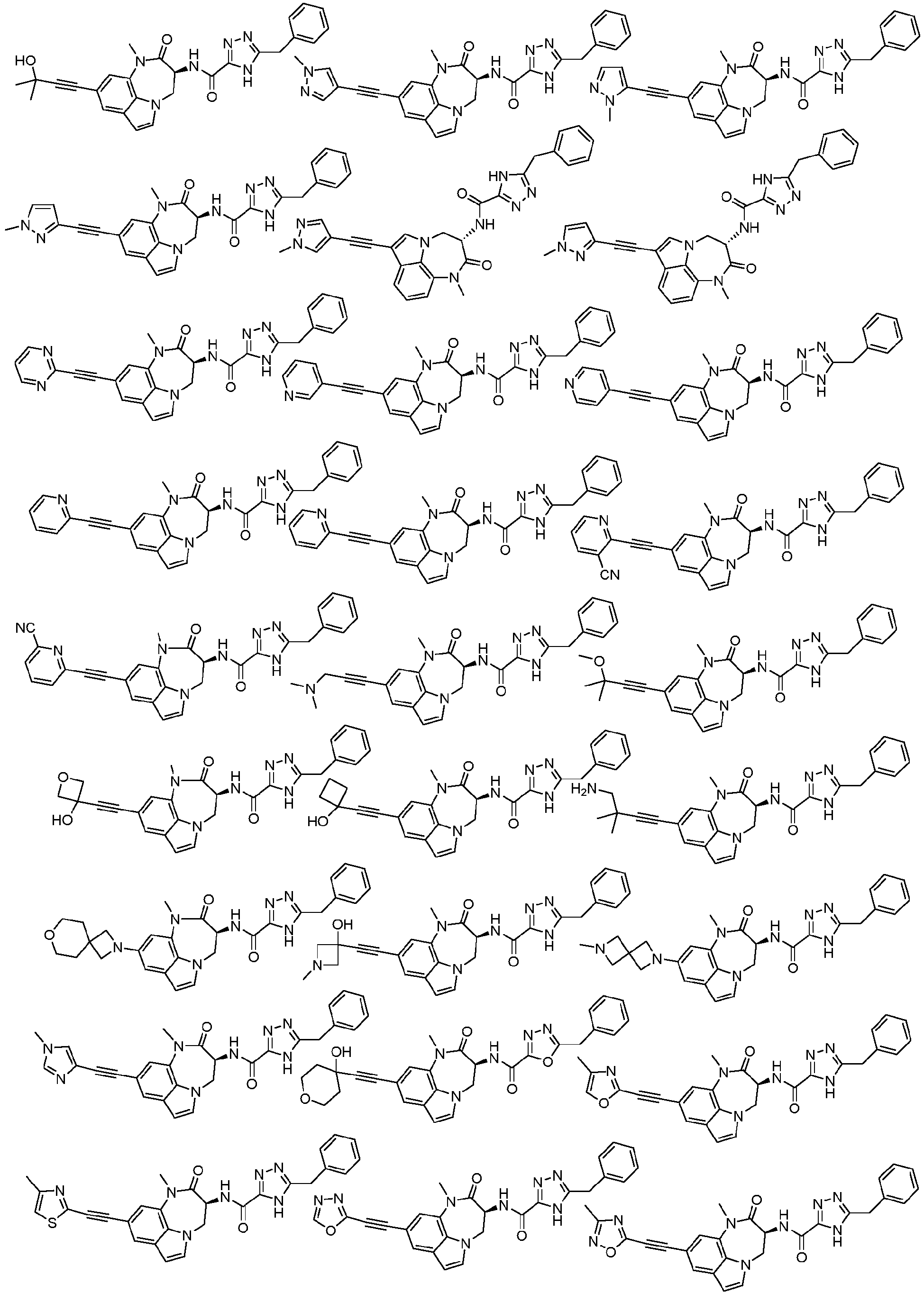

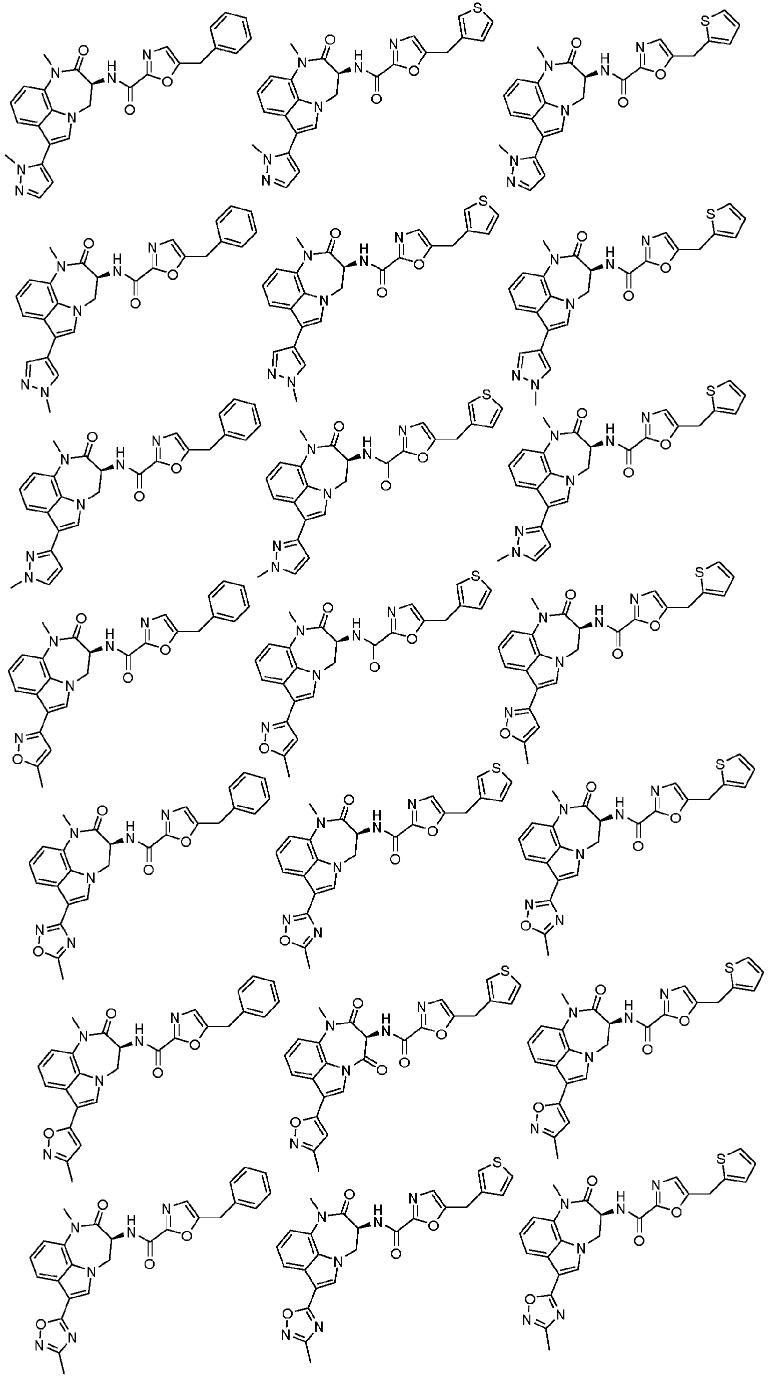

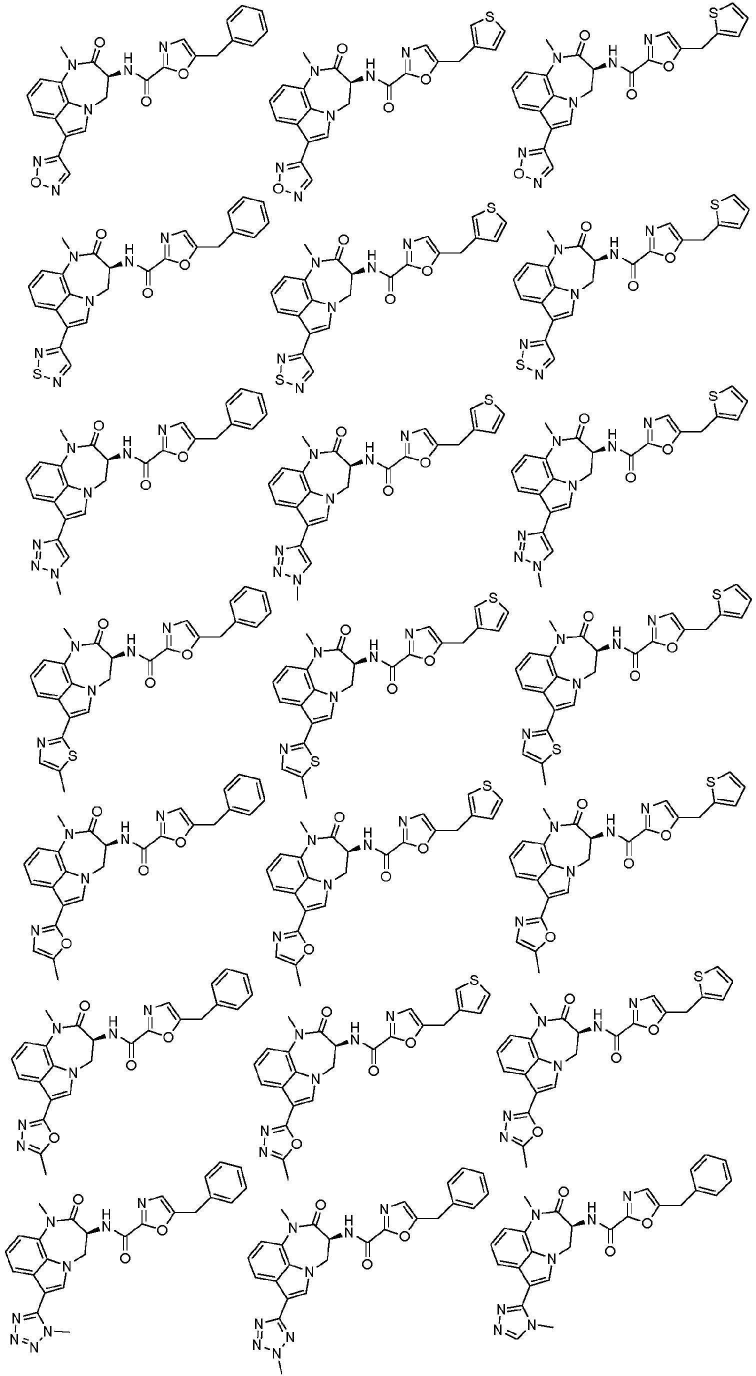

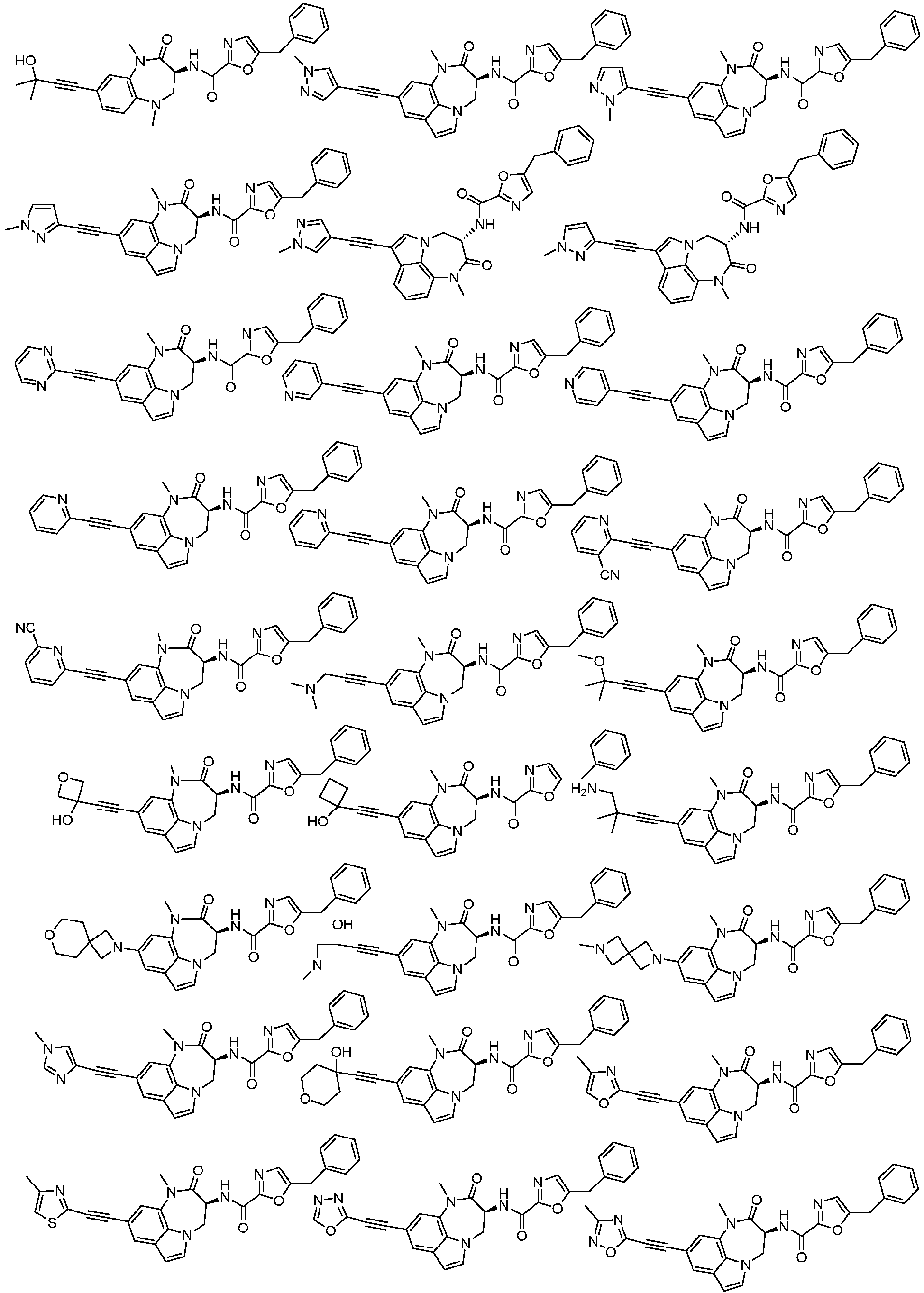

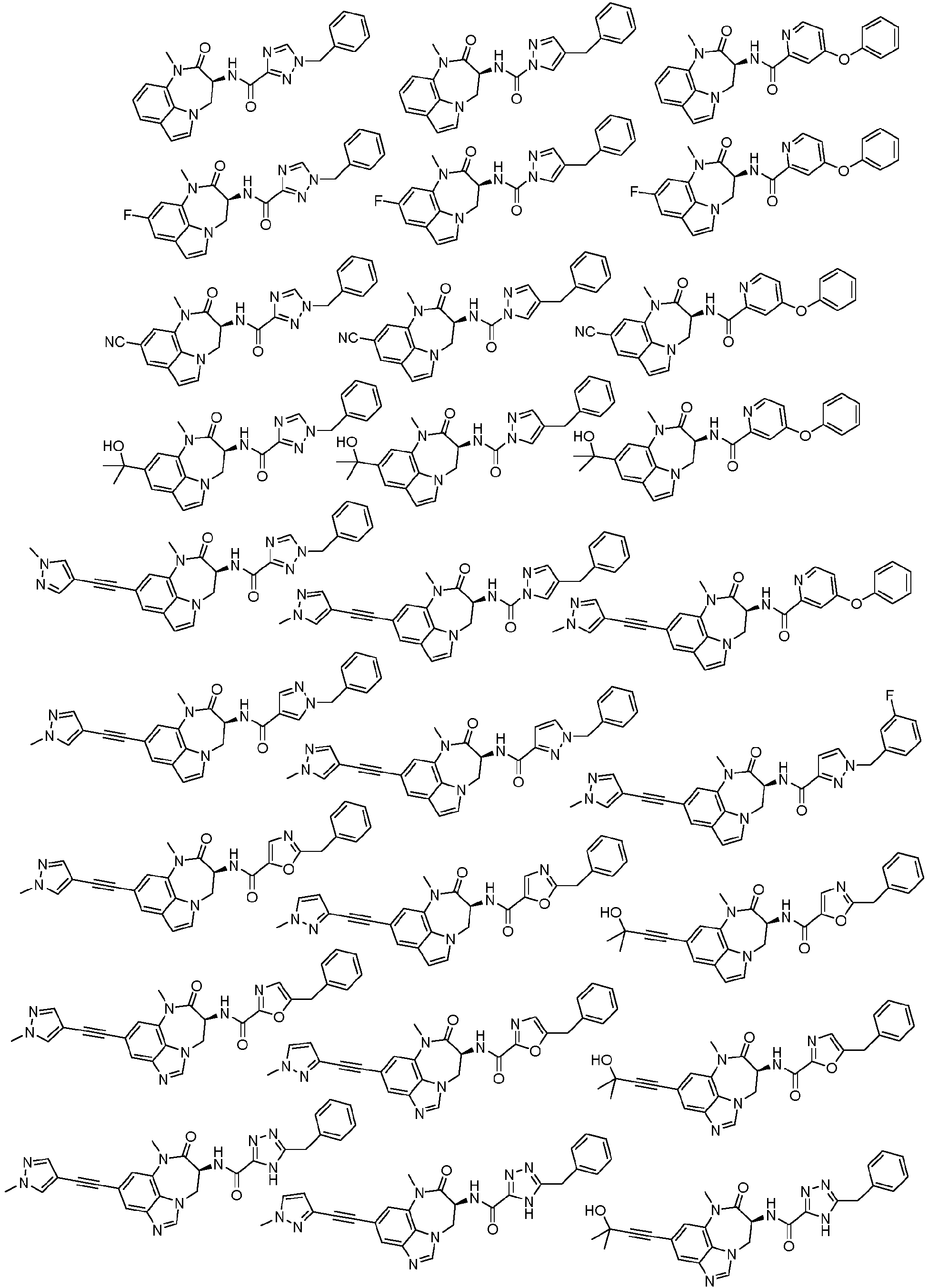

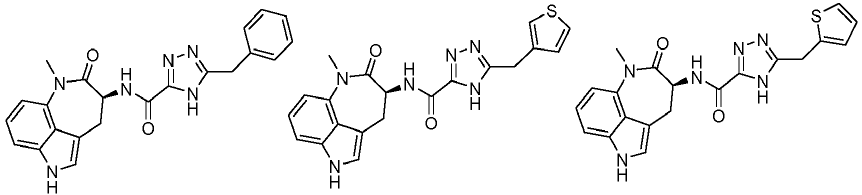

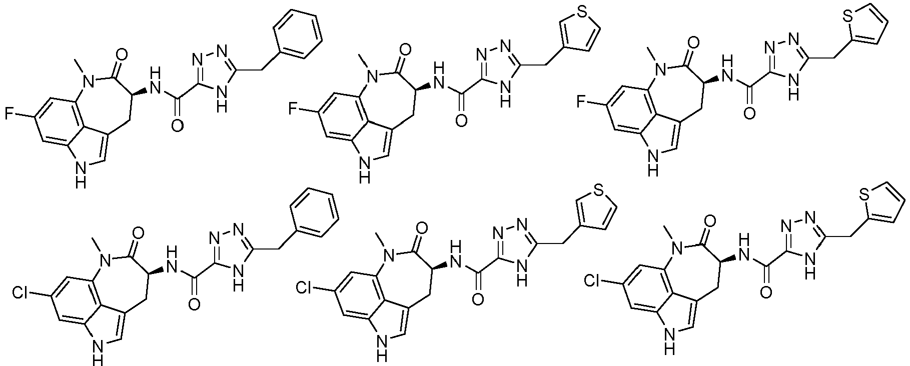

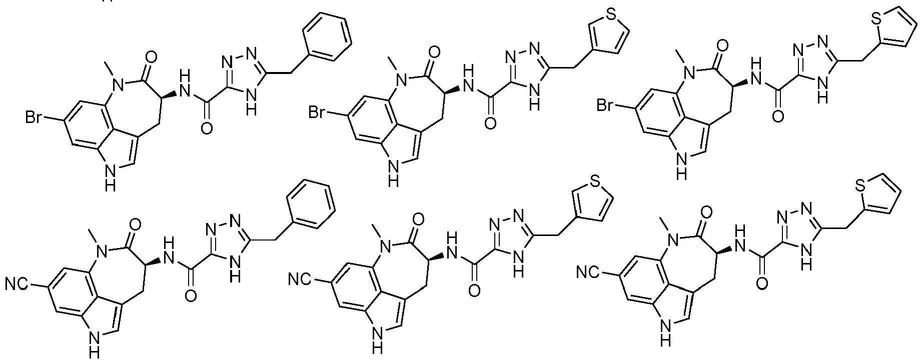

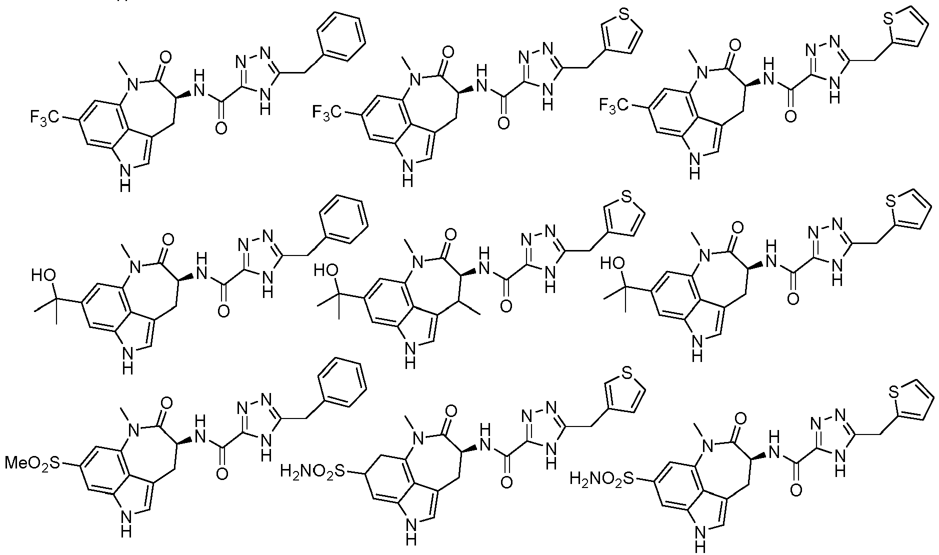

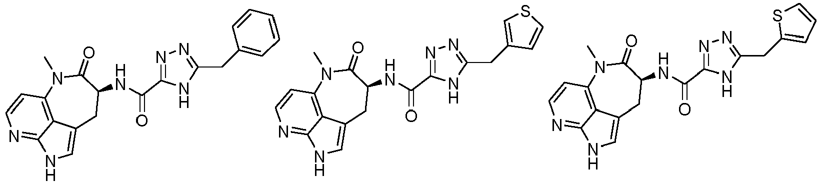

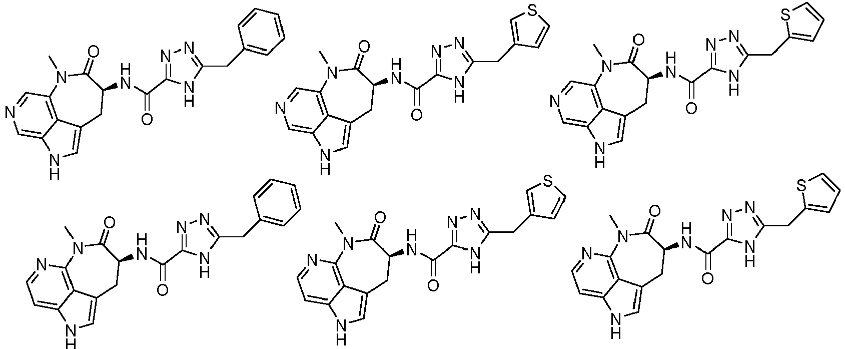

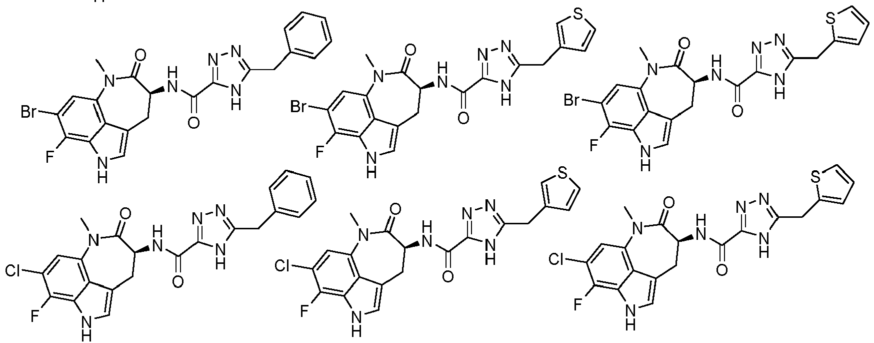

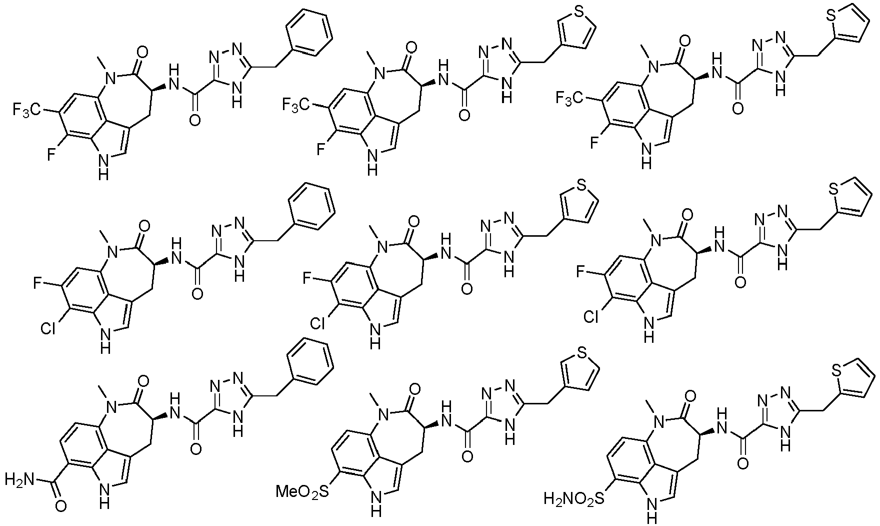

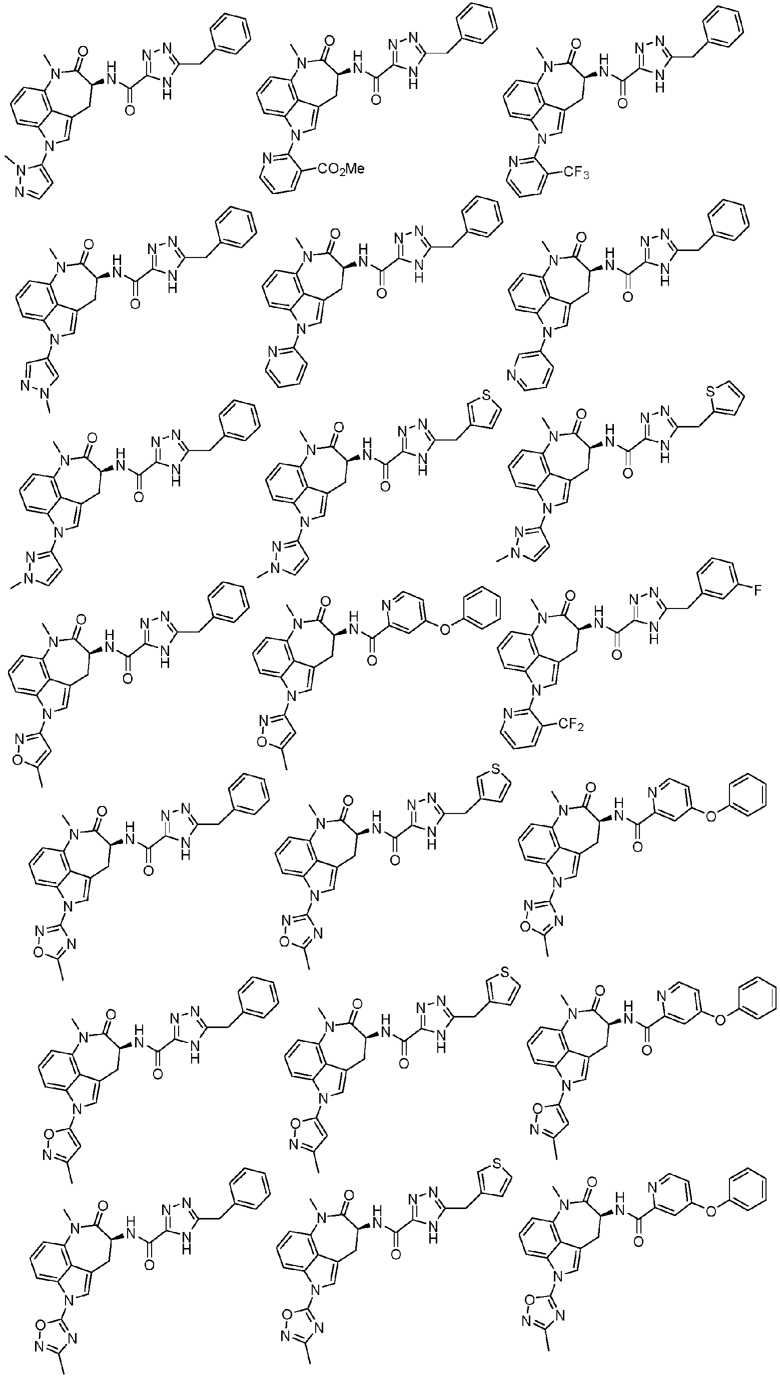

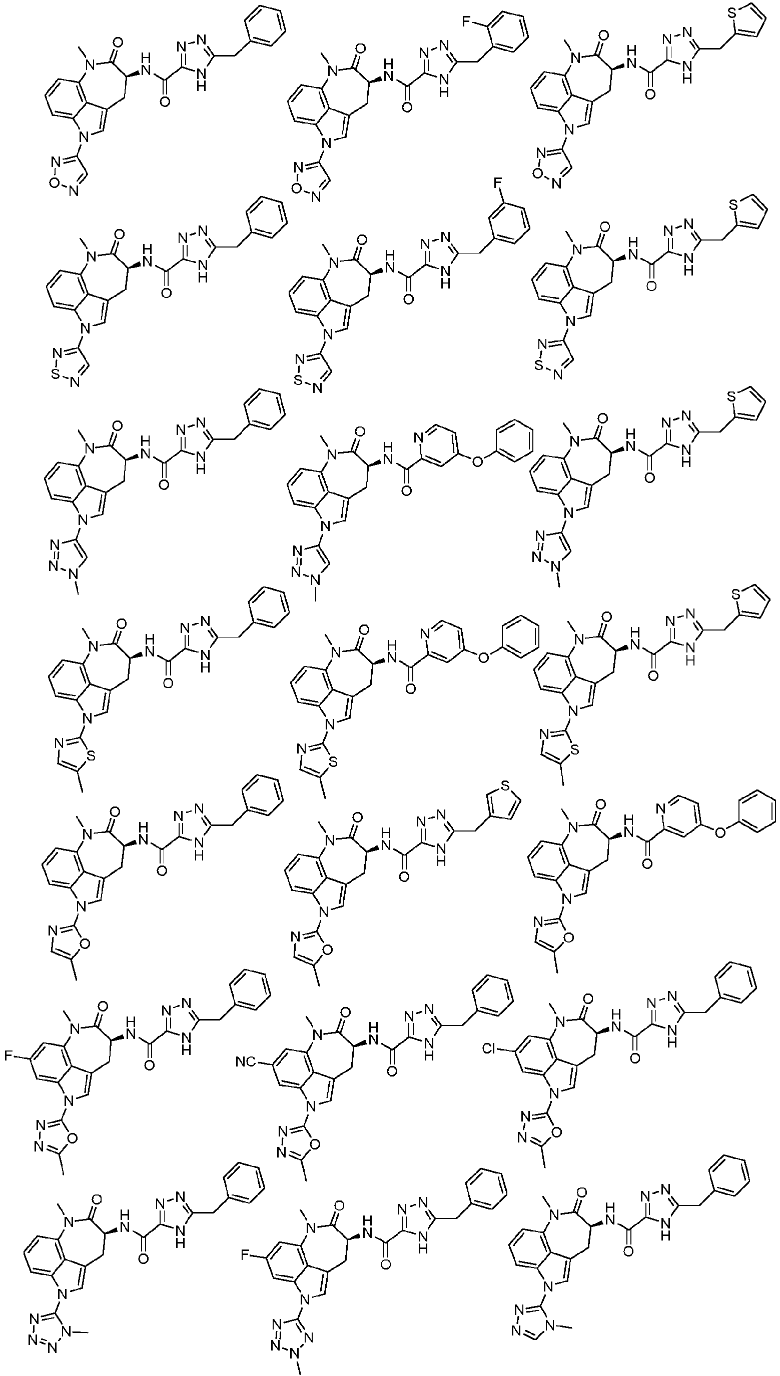

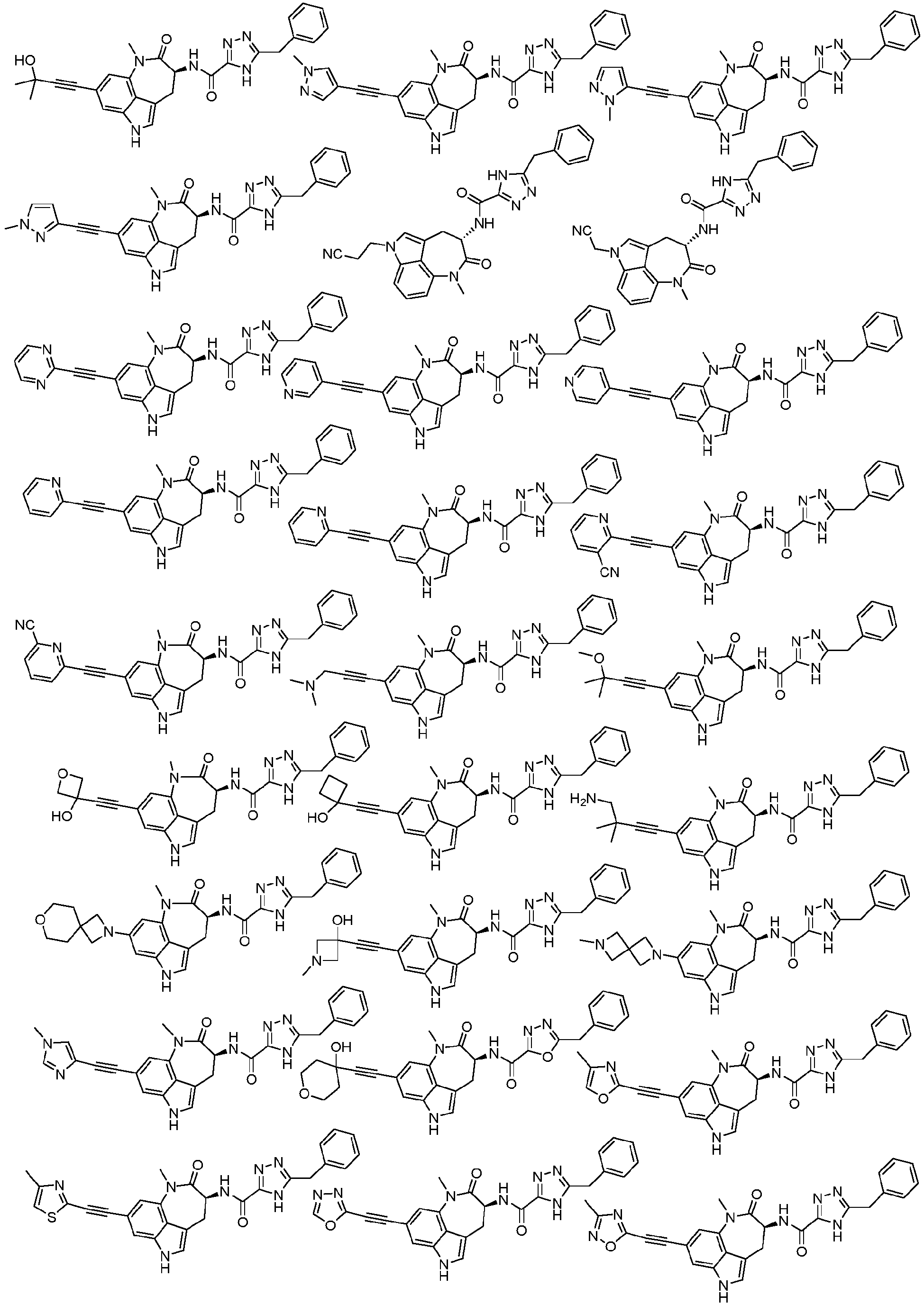

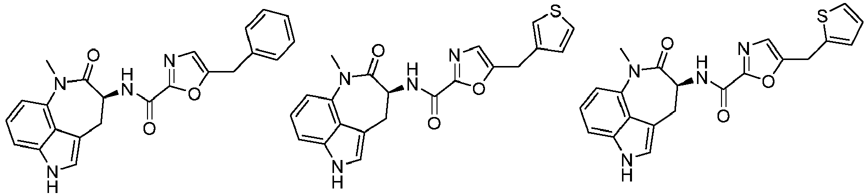

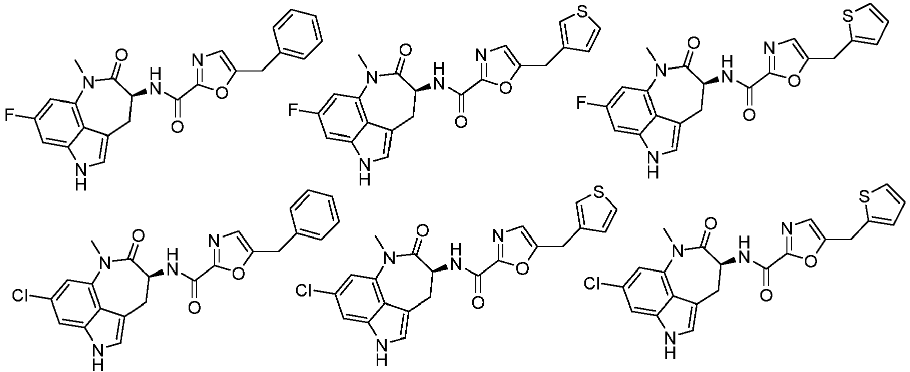

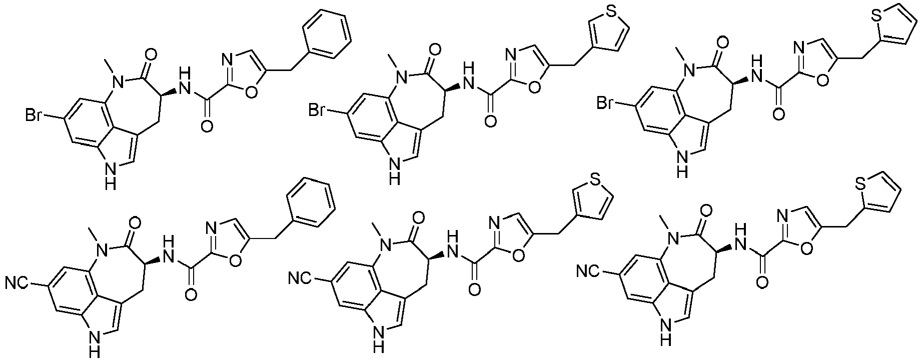

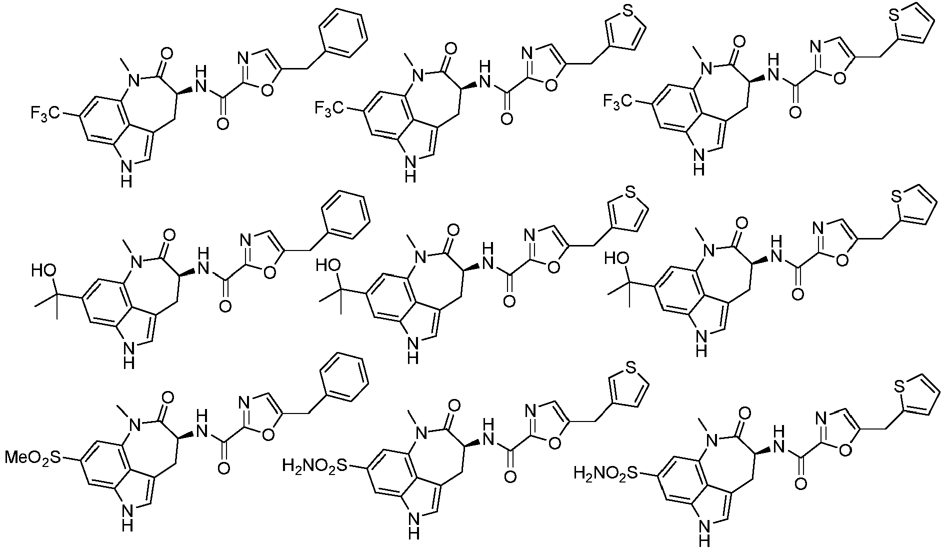

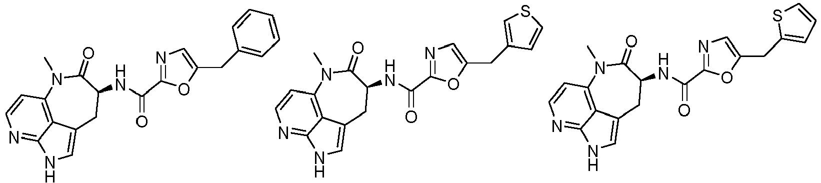

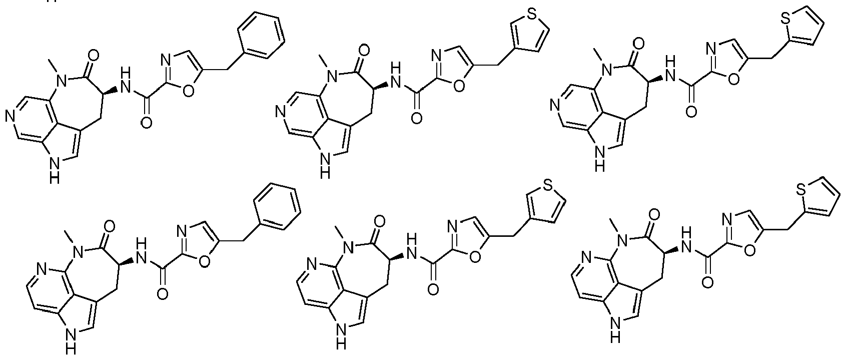

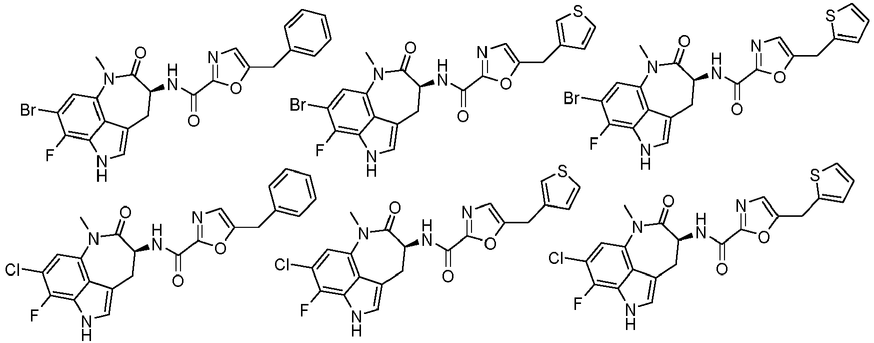

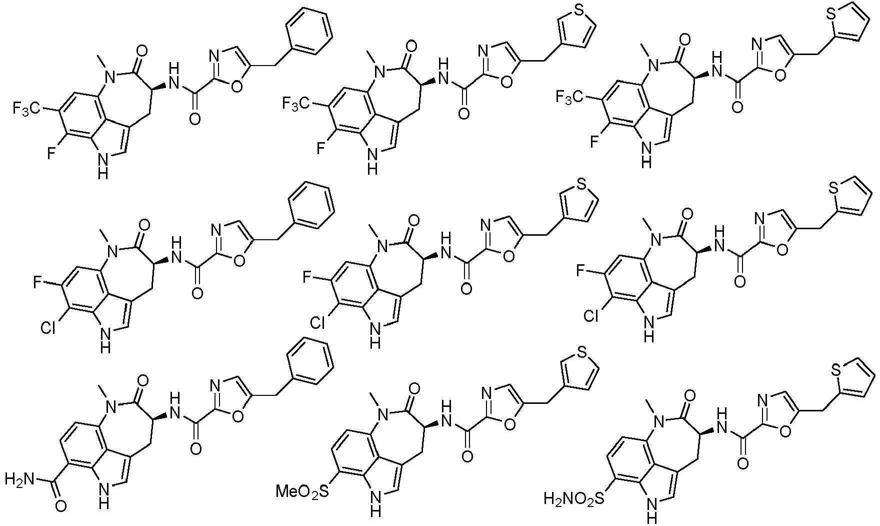

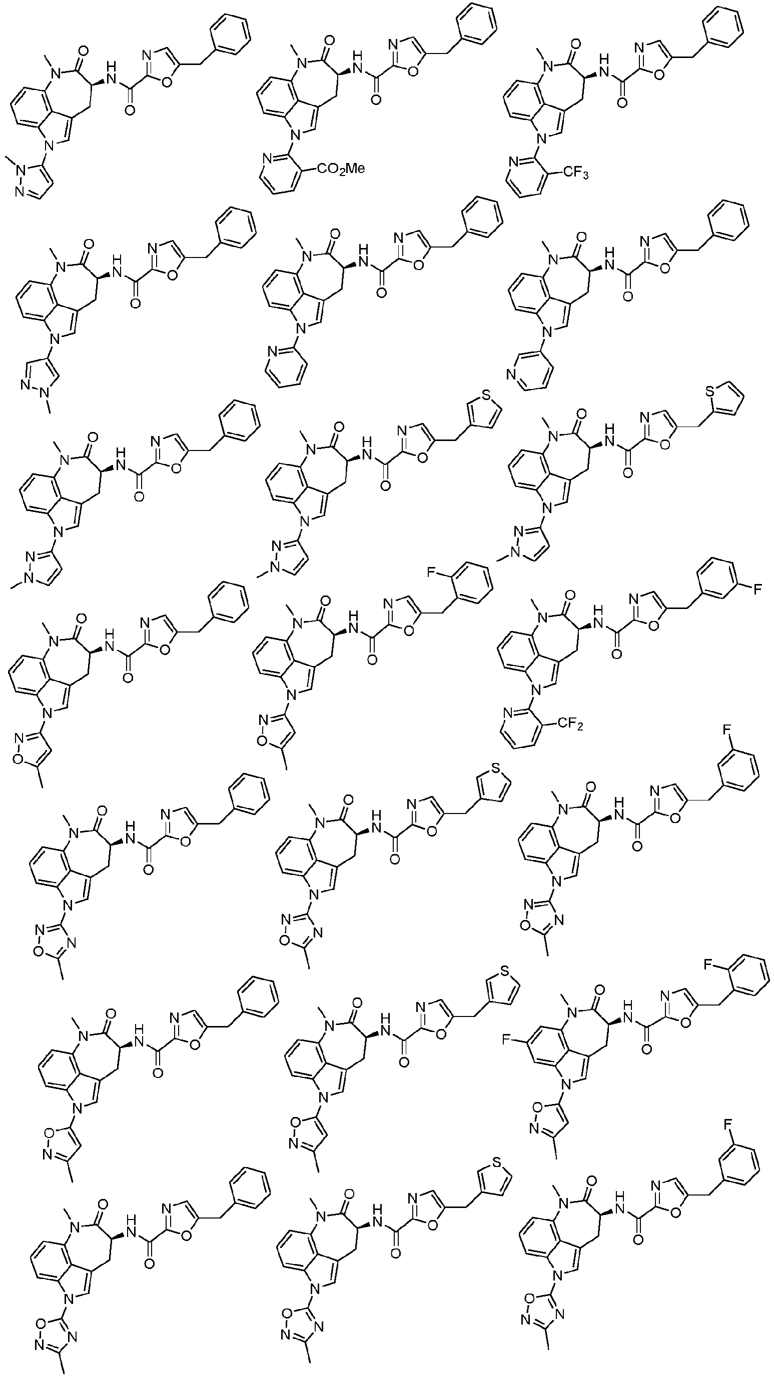

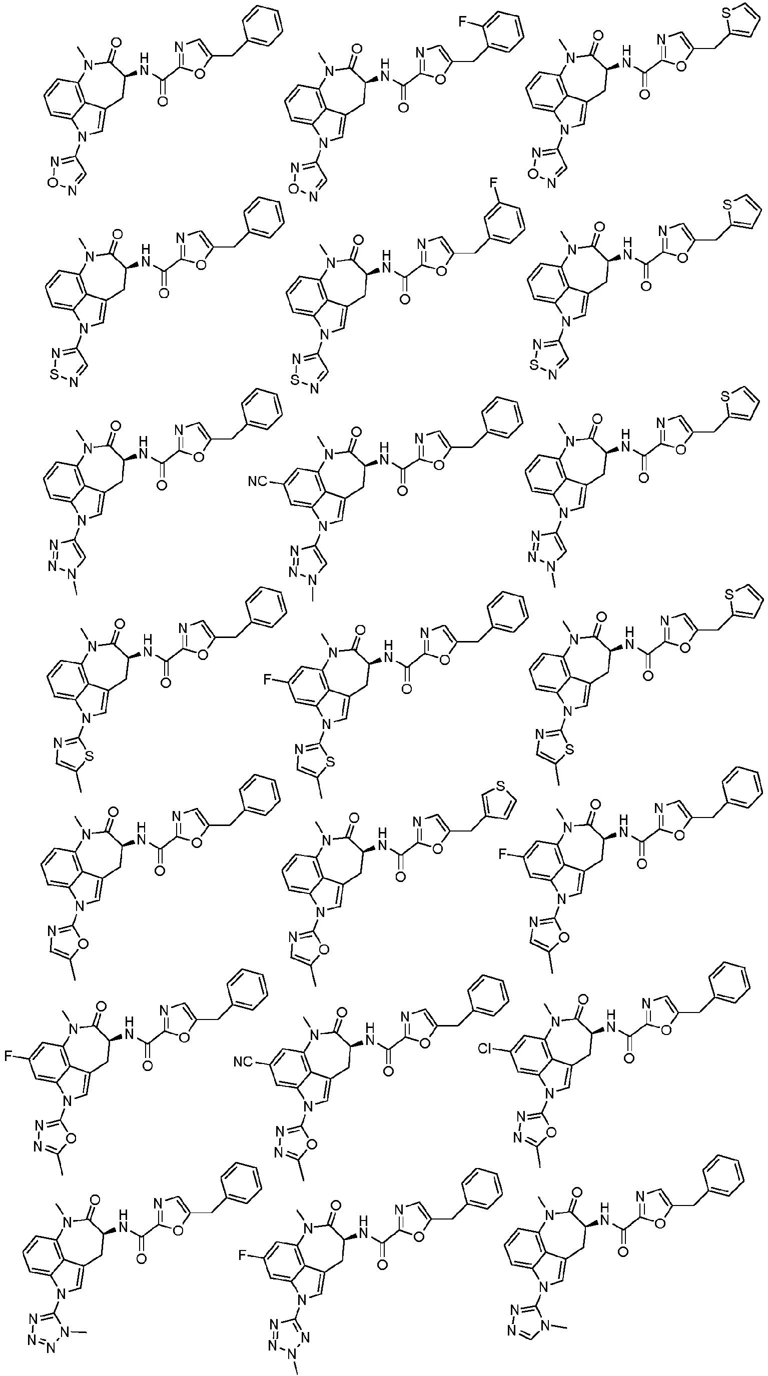

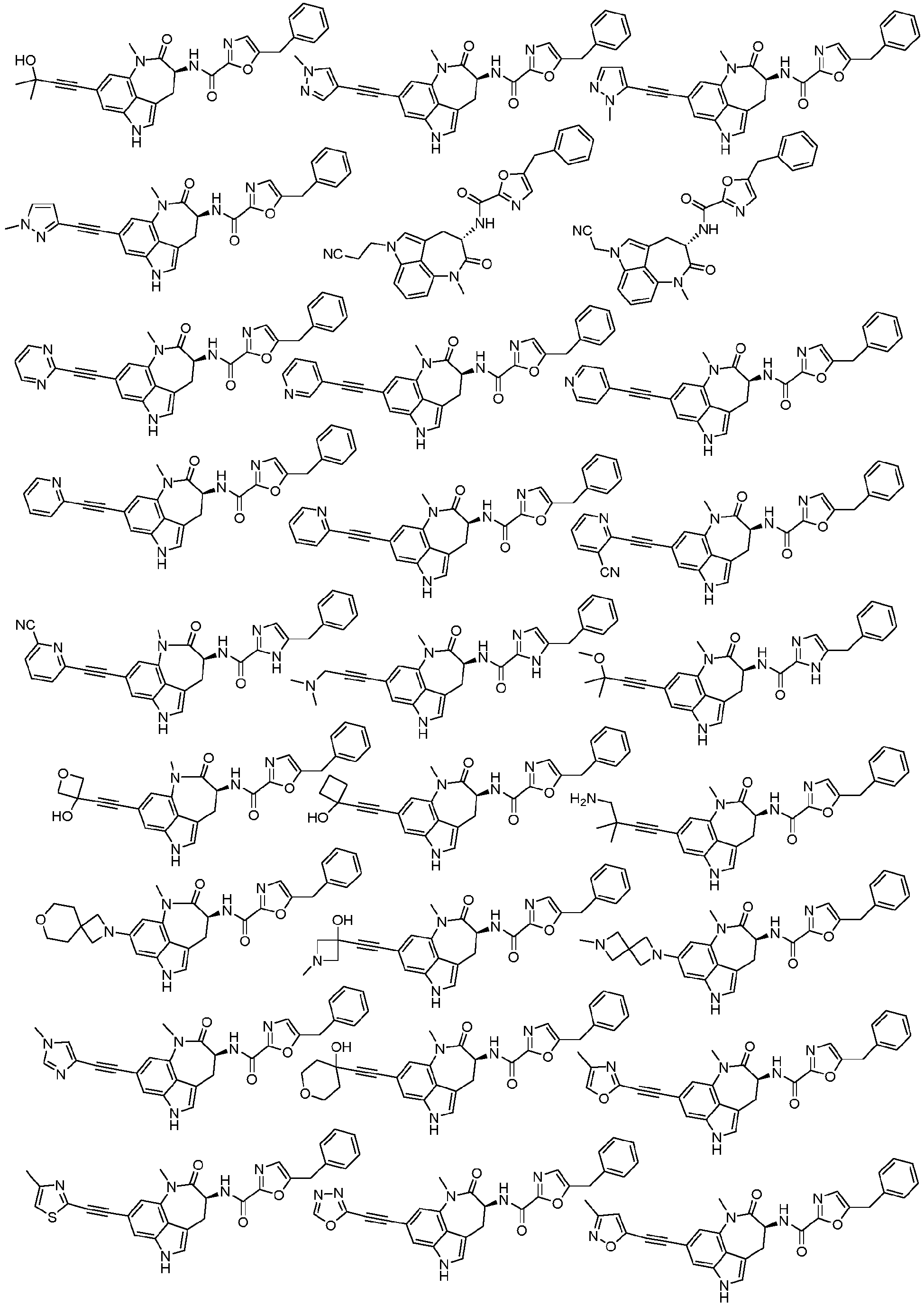

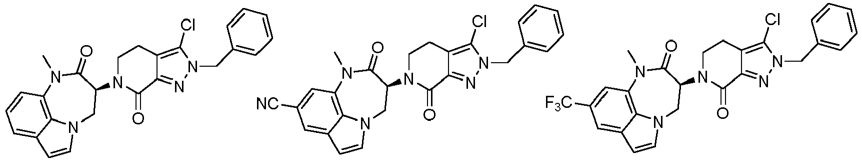

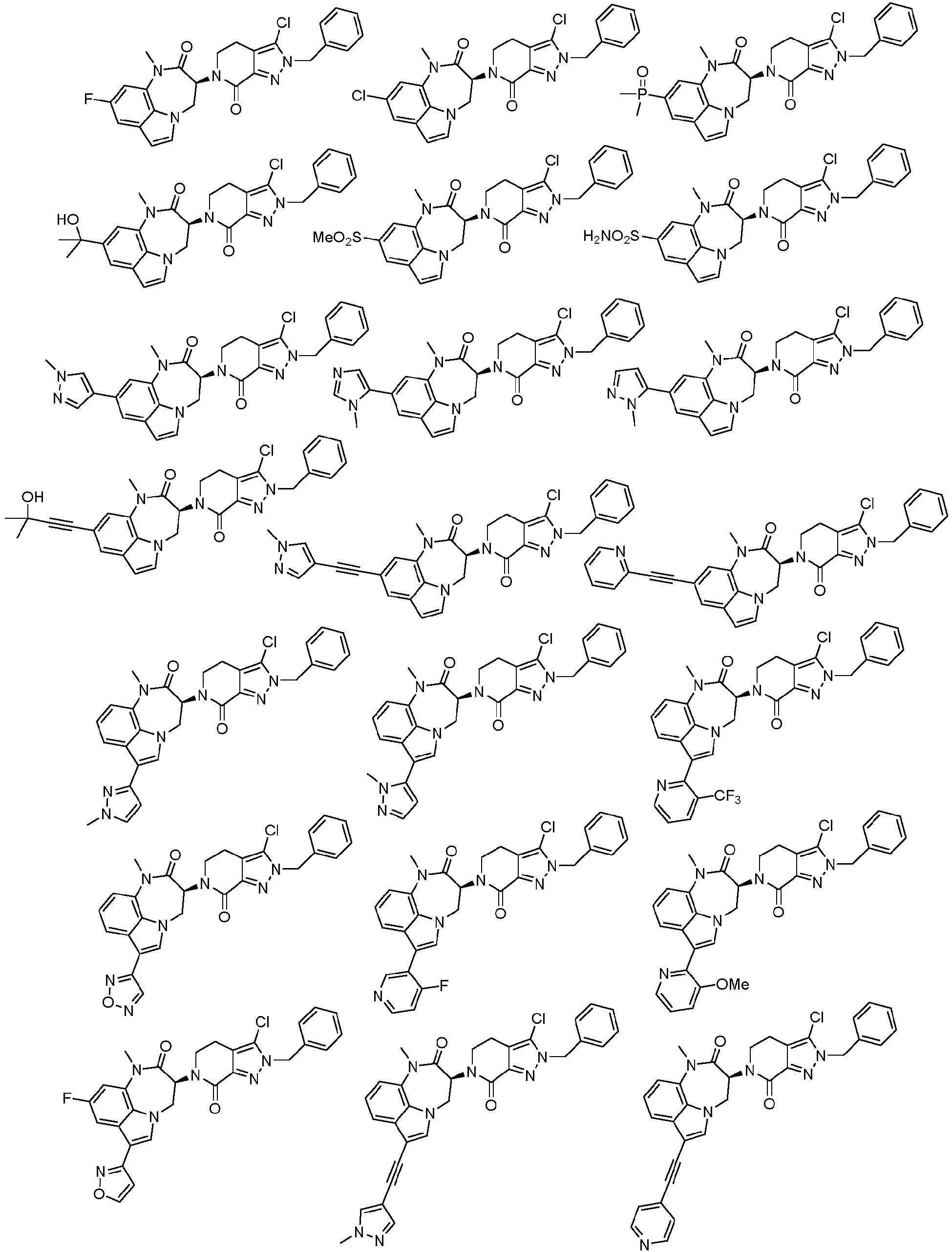

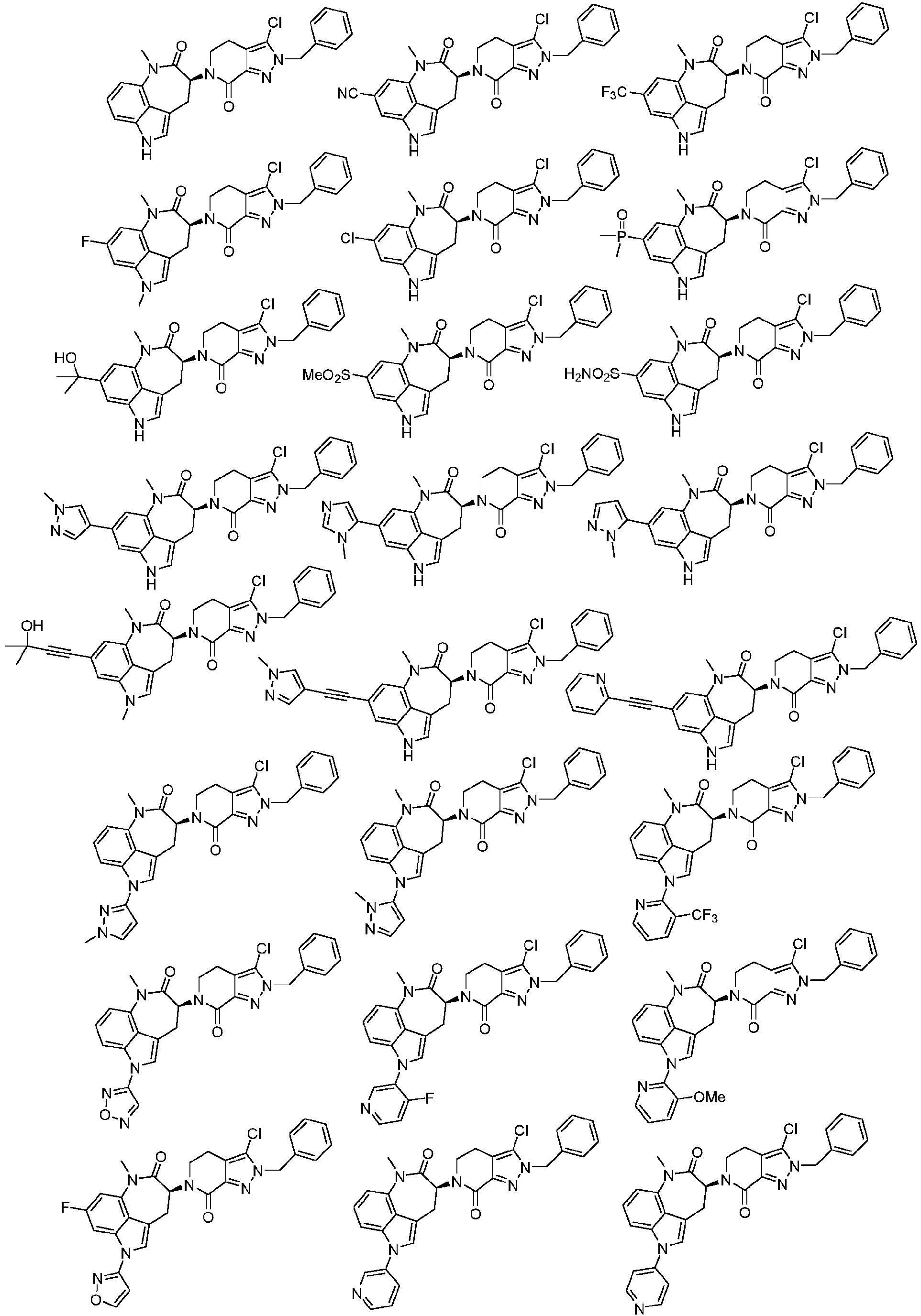

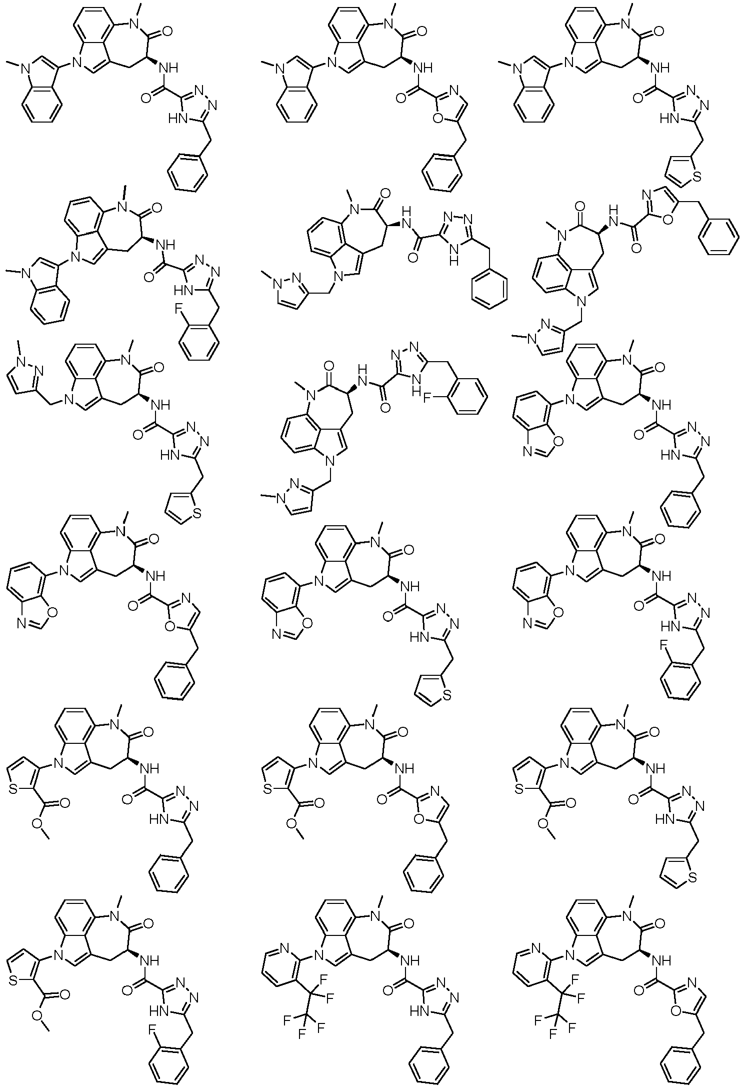

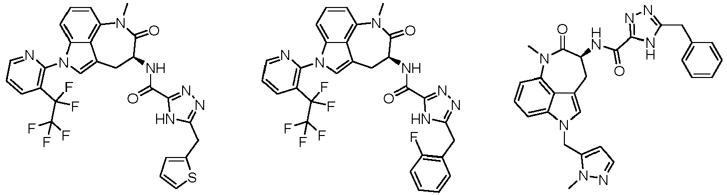

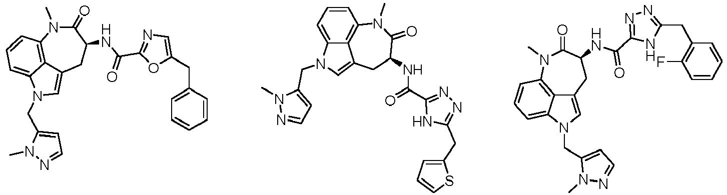

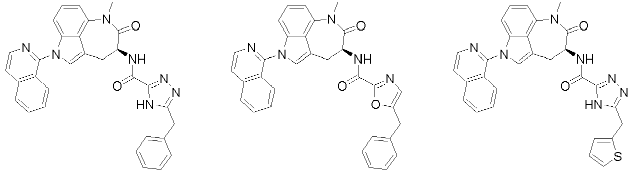

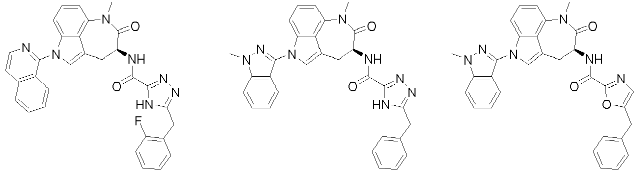

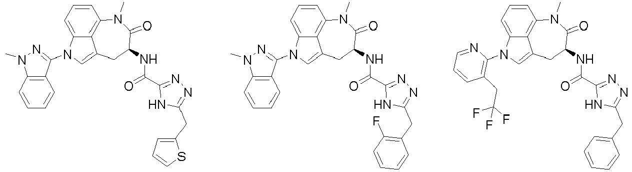

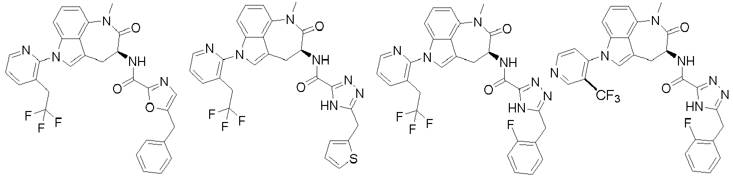

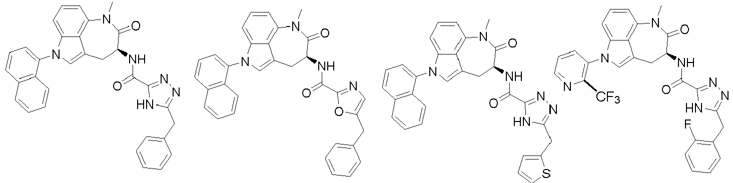

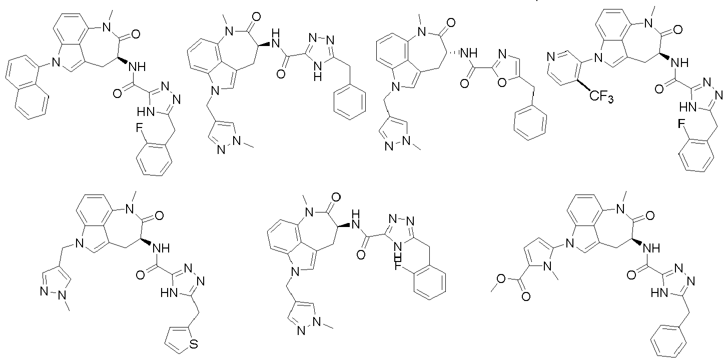

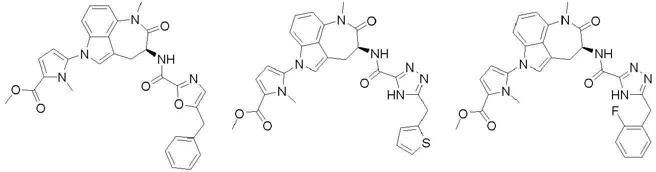

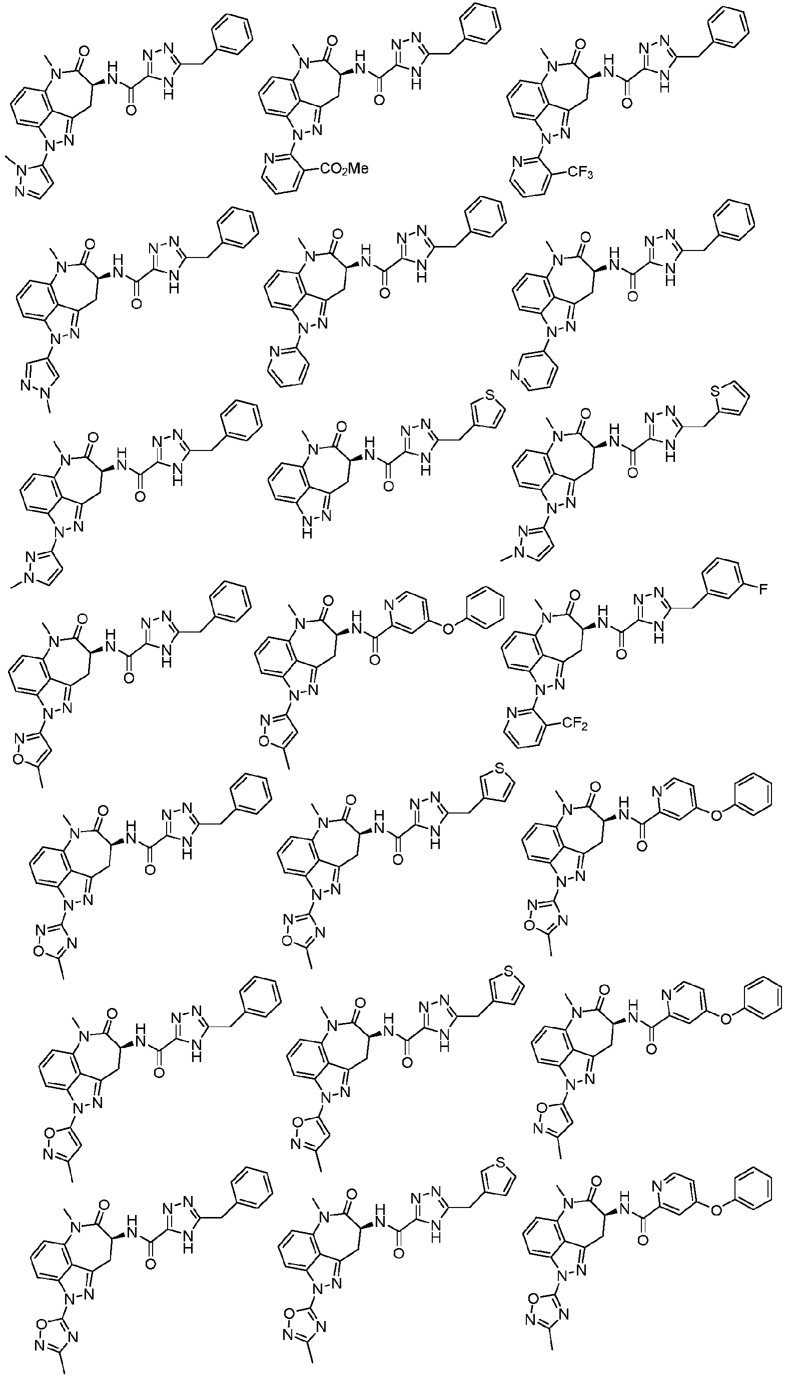

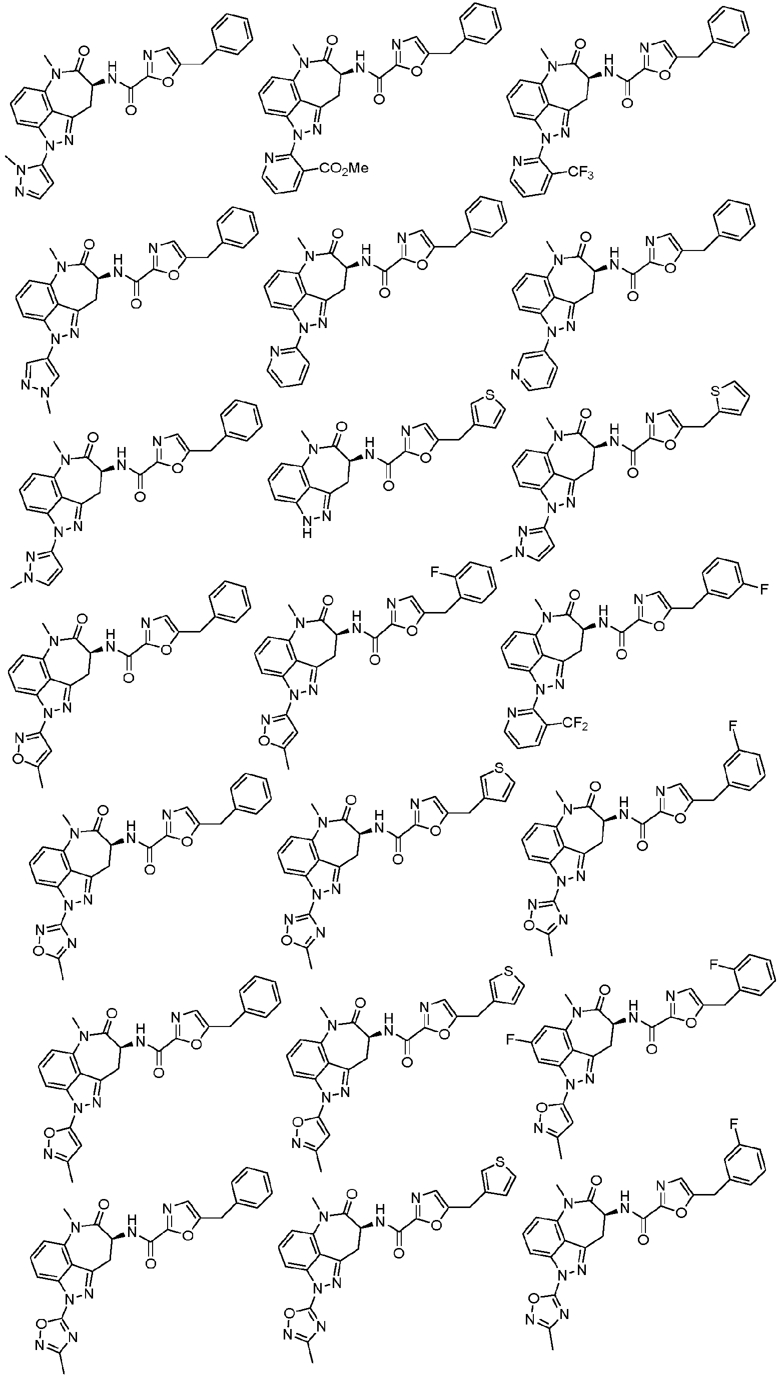

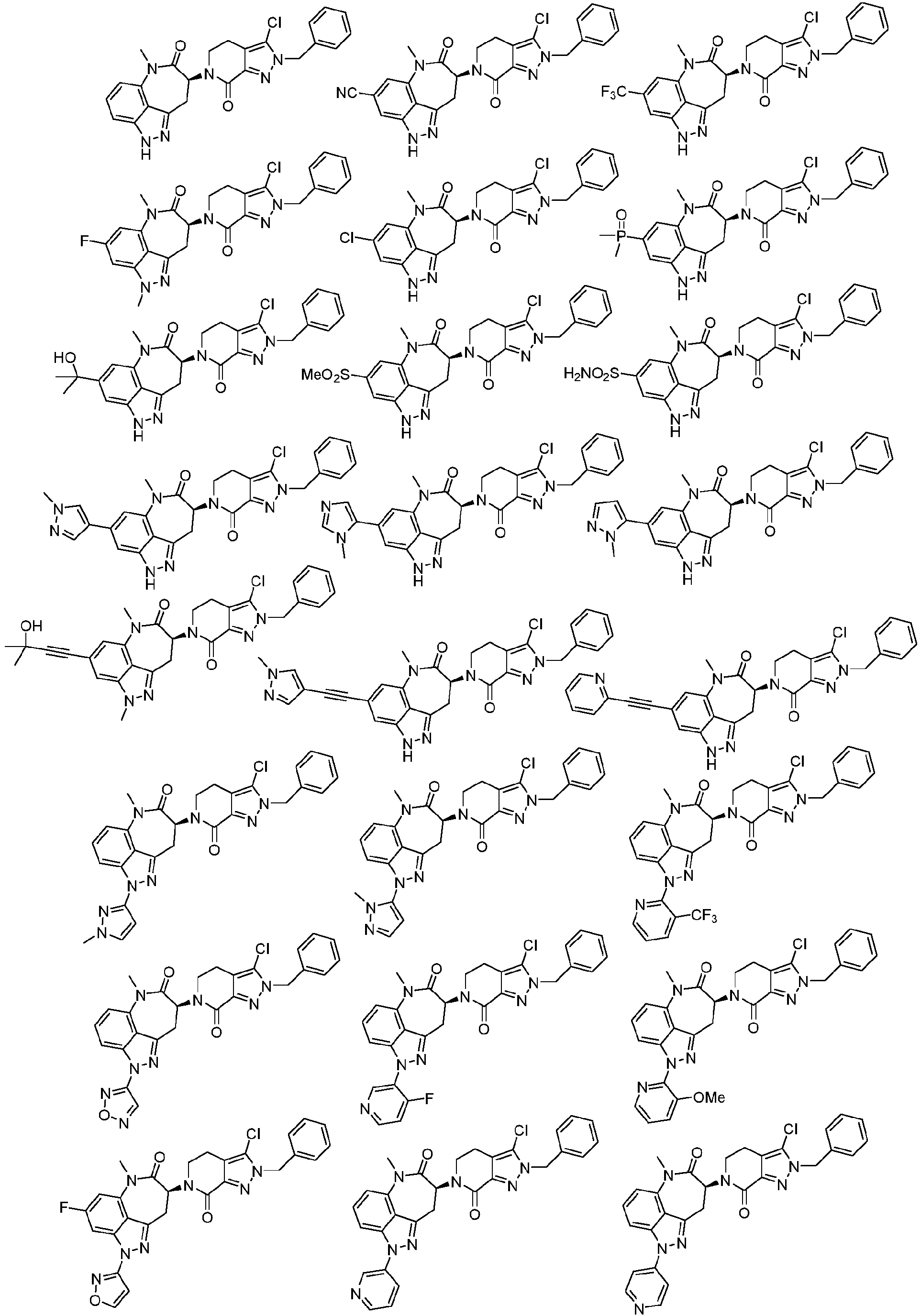

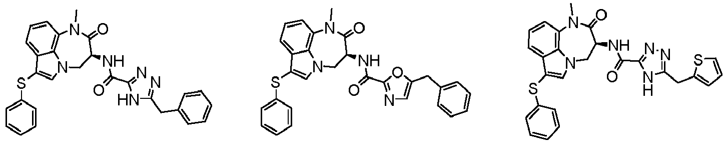

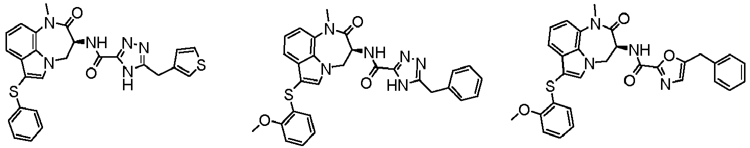

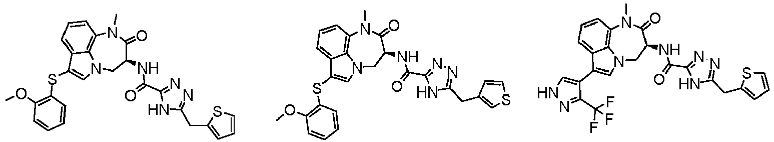

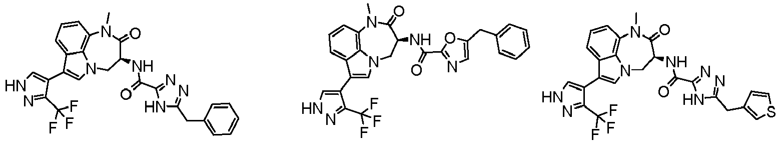

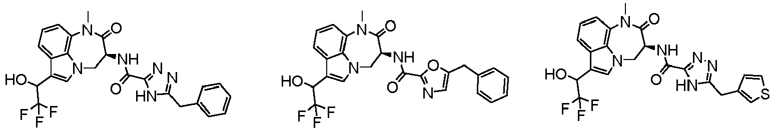

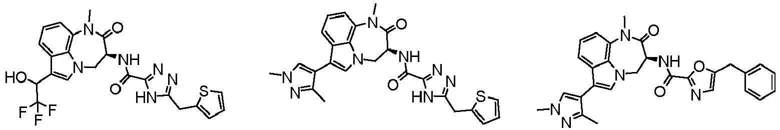

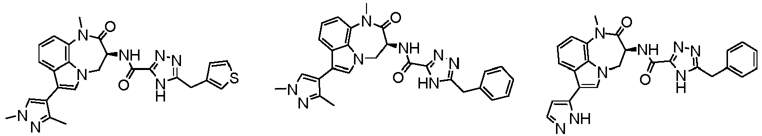

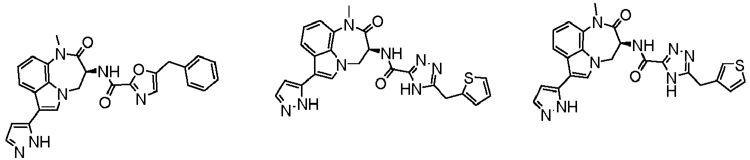

In another preferred embodiment, the said compounds of general formulas I to XII include all stereoisomers.

In another preferred embodiment, the said stereoisomers are cis-trans isomers.

In another preferred embodiment, the said compounds are optically pure isomers.

In another preferred embodiment, the said compounds are racemates.

In another preferred embodiment, the said stereoisomers are enantiomers.

In another preferred embodiment, any one or more hydrogen atoms in the said compounds may be substituted by deuterium atoms.

In another preferred embodiment, the said compounds of general formulas I to XII include their prodrugs.

In another preferred embodiment, the pharmaceutically acceptable salt in the said general formulas I to XII is selected from: hydrochloride, hydrobromate, sulfate, phosphate, mesylate, trifluoromethanesulfonate, benzene sulfonate, p-toluenesulfonate (tosylate), 1-naphthalenesulfonate, 2-naphthalenesulfonate, acetate, trifluoroacetate, malate, tartrate, citrate, lactate, oxalate, succinate, fumarate, maleate, benzoate, salicylate, phenylacetate and mandelate.

The second aspect of the present invention provides the preparation method for the said compounds in general formulas I to XII in the first aspect, wherein the method is selected from one of the following schemes.

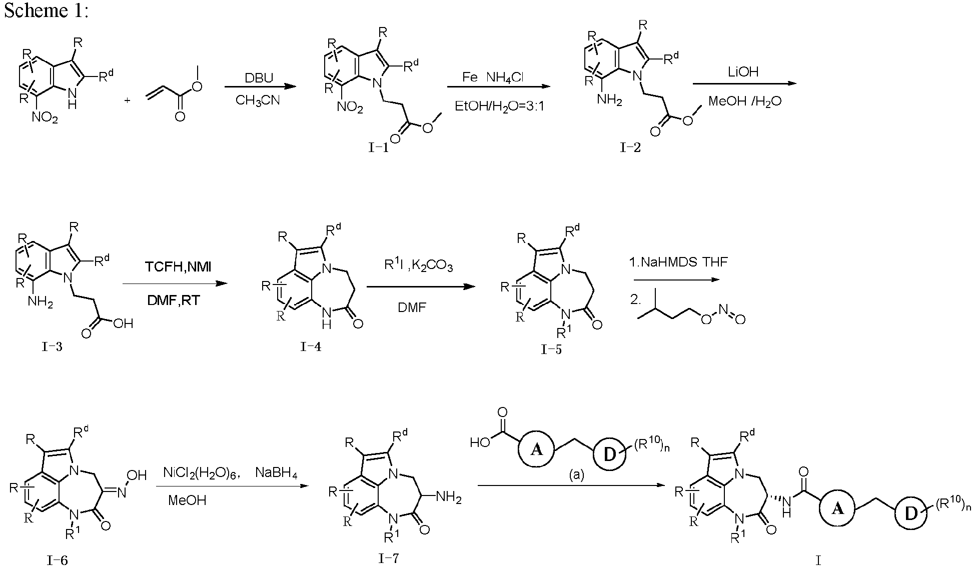

Methyl acrylate is reacted with a suitably substituted 7-nitroindole using a base to obtain I-1, followed by reducing the nitro to amine I-2 (the reduction conditions include but are not limited to Zn/AcOH, Fe/NH₄Cl/EtOH, Zn/NH₄Cl/EtOH, and Pd/C/H₂/MeOH). Using a coupling reagent, the (formula I-4) intermediate is obtained through an intramolecular coupling (the coupling reagent includes but is not limited to HOAT, HOBT, HATU, EDCI, BOP, and TCFH/NMI). Afterward, the compound I-4 is alkylated to obtain I-5, and I-5 is reacted with isopentyl nitrite under basic conditions to obtain the intermediate I-6. Subsequently, the oxime is reduced to amine I-7 (the reduction conditions include but are not limited to Pd/C/H₂/MeOH, Ni/C/H₂/MeOH, and NaBH₄/NiCl₂(H₂O)₆/MeOH). After chiral resolution, the desired enantiomer of I-7 is coupled with acid (a) using the amide coupling reagent (the amide coupling reagent includes but is not limited to HOAT, EDCI, HATU, and TCFH) to obtain the final product I.

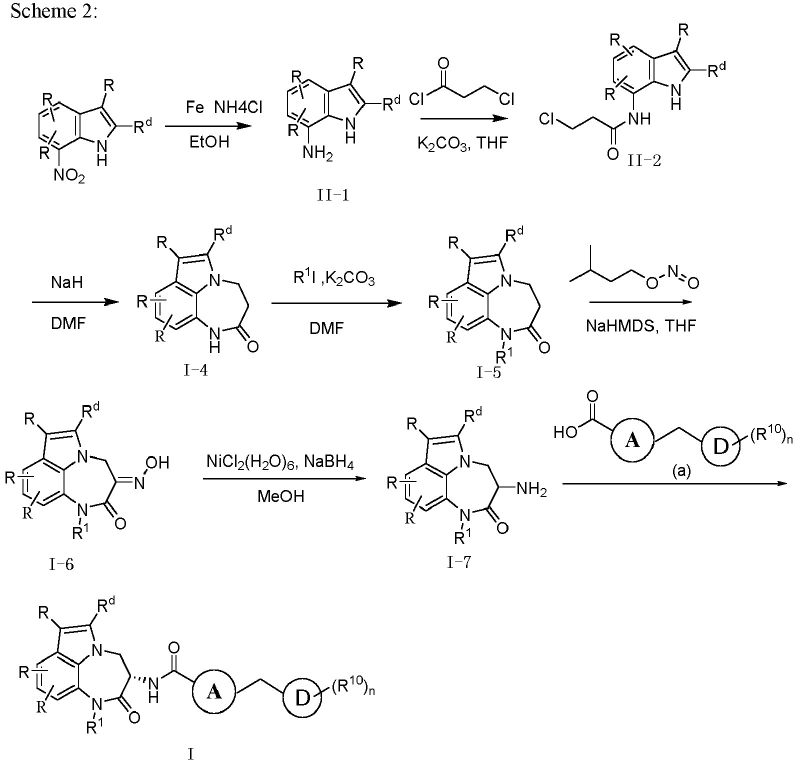

Reduction of a properly substituted 7-nitro-indole gives rise to II-1 (the reduction conditions include but are not limited to Zn/AcOH, Fe/NH₄Cl/EtOH, Zn/NH₄Cl/EtOH, and Pd/C/H₂/MeOH). II-1 then reacts with an acid chloride under basic conditions to obtain the intermediate II-2. Intermediate I-4 is obtained through cyclization of II-2 under basic conditions. The final product I is obtained using the same method as in Scheme 1.

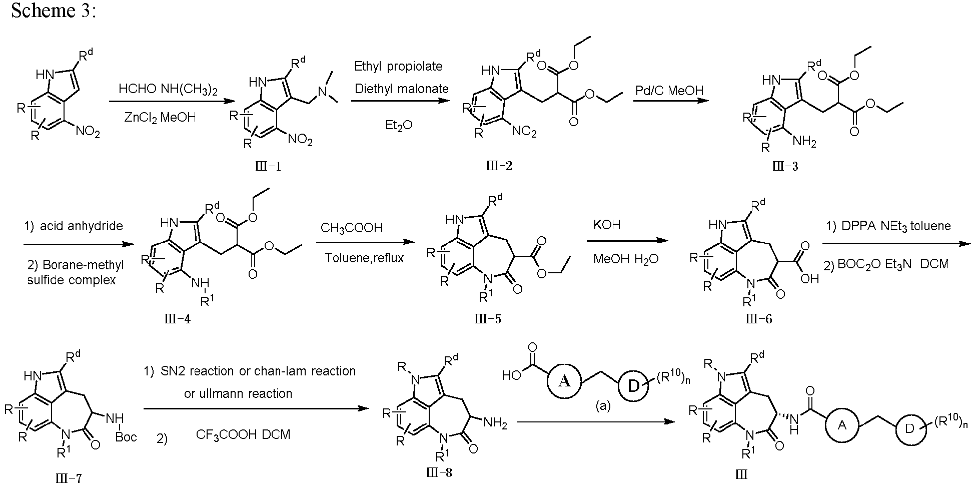

A suitably substituted 4-nitroindole undergoes Mannich reaction with dimethylamine and formaldehyde, catalyzed by a Lewis acid or a Bronsted acid, to provide III-1. III-1 then reacts with diethyl malonate under catalysis of ethyl propiolate or by heating to obtain III-2. Subsequently, reduction of III-2 gives amine III-3 (the reduction conditions include but are not limited to Zn/AcOH, Fe/NH₄Cl/EtOH, Zn/NH₄Cl/EtOH, and Pd/C/H₂/MeOH). III-3 is then reacted with acid anhydride to obtain amide intermediate which is reduced to alkylamine III-4. Refluxing III-4 in toluene with a catalytic amount of acid provide III-5. Hydrolysis of III-5, followed by Curtis reaction and Boc protection gives III-7. Using III-7 as an intermediate, nucleophilic substitutions followed by amide coupling reactions with a coupling reagent (which includes but is not limited to HOAT, EDCI, HATU, and TCFH) after removal of Boc group provide the final product III. Depending on the difficulty of the resolution conditions, chiral resolution can be carried out for III-7, III-8, or the racemic III.

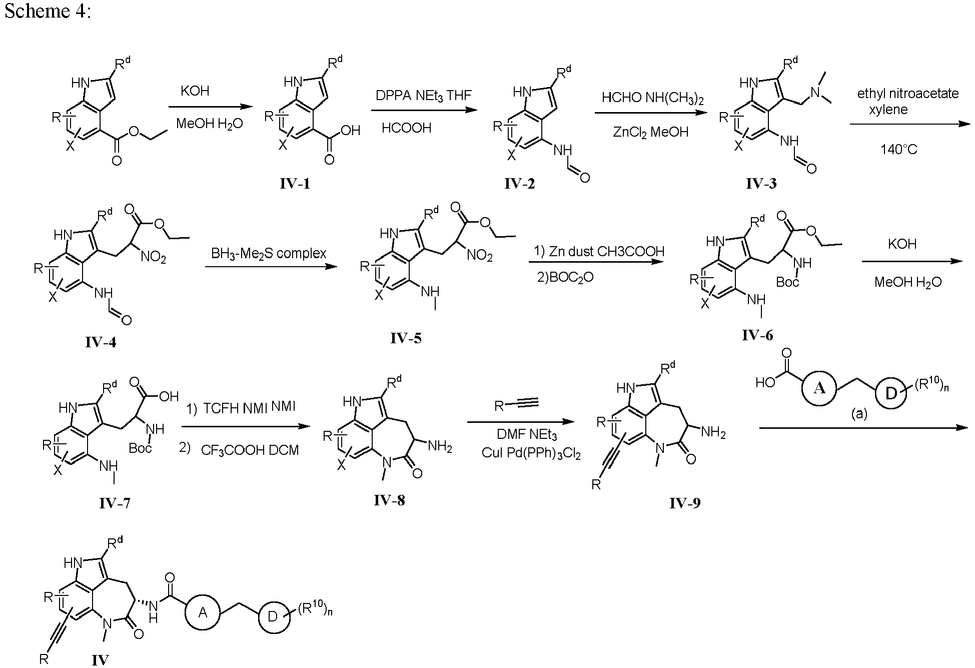

A suitable halogenated ioindole-4-carboxylic acid ester is hydrolyzed to acid IV-1, which is subjected to Curtius rearrangement, followed by quenching with formic acid to obtain IV-2. Mannich reaction of IV-2 provides IV-3, which is reacted with ethyl nitroacetate under heating conditions to obtain IV-4. Subsequently, reduction of the formamide group with borane-methyl sulfide and then reduction of the nitro group (the reduction conditions include but are not limited to Zn/AcOH, Fe/NH₄Cl/EtOH, Zn/NH₄Cl/EtOH, and Raney Ni/H₂/MeOH), followed by Boc protection afford IV-6. After ester hydrolysis of IV-6, the acid intermediate is cyclized using the amide coupling reagent (which includes but is not limited to HOAT, EDCI, HATU, and TCFH), followed by removal of the protecting group to obtain IV-8. Sonogashira reaction of IV-8 obtain IV-9. Resolution of IV-9 and coupling of the desired enantiomer with acid (a) using an amide coupling reagent (which includes but is not limited to HOAT, EDCI, HATU, and TCFH) afford the final product IV

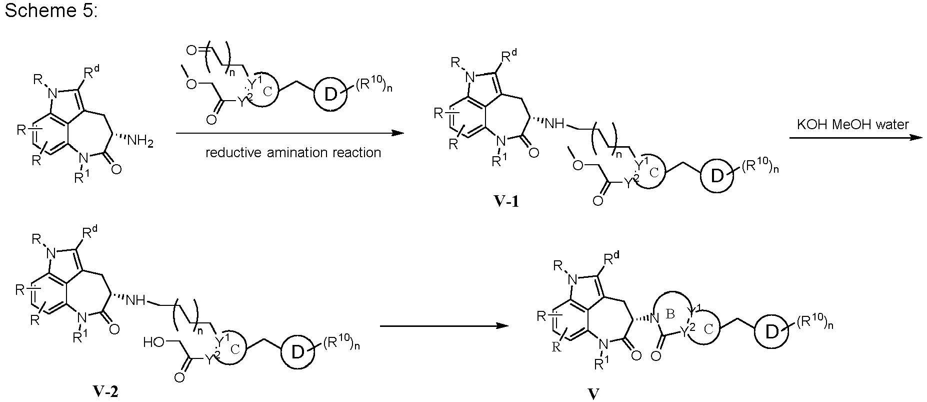

Using an appropriate amine as the starting material, reductive amination followed by hydrolysis affords acid V-2, which is cyclized with an amide coupling reagent (which includes but is not limited to HOAT, EDCI, HATU, and TCFH) to obtain the final product V

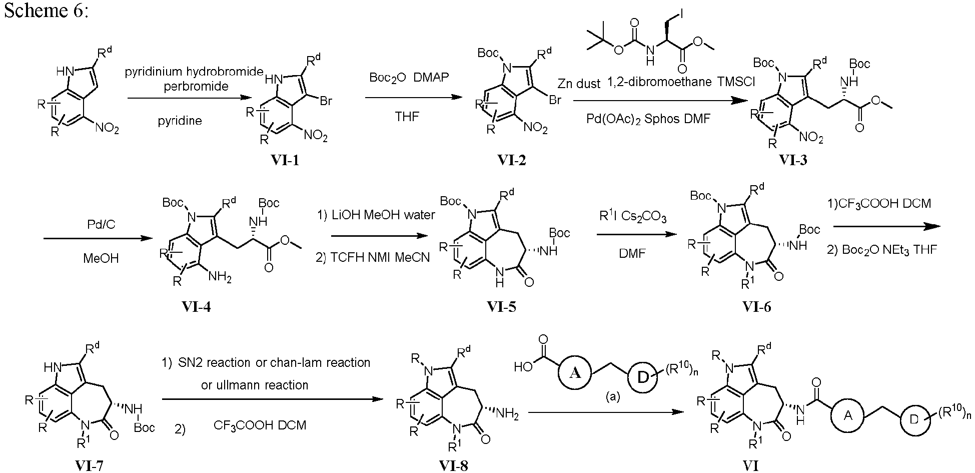

A suitably substituted 4-nitroindole is halogenated at the 3-position to give VI-1 which is then protected with a Boc group to give VI-2. Negishi coupling of VI-2 with (R)-N-(tert-butoxycarbonyl)-3-iodo-L-alanine methyl ester obtain VI-3. Reduction of the nitro group (reduction conditions include but are not limited to Zn/AcOH, Fe/NH₄Cl/EtOH, Zn/NH₄Cl/EtOH, and Ranney Ni/H₂/MeOH), and base hydrolysis followed by cyclization with an amide coupling reagent (coupling reagents include but are not limited to HOAT, EDCI, HATU, and TCFH) obtain VI-5. Reaction of VI-5 with a suitable alkyl iodide to obtain VI-6. Manipulations of Boc protecting groups in VI-6 lead to VI-7. Introduction of different substituents onto the nitrogen atom of the indole in VI-7 by various means, followed by removal of the Boc group afford VI-8, which is coupled with the acid (a) using an amide coupling reagent (coupling reagents include but are not limited to HOAT, EDCI, HATU, and TCFH) to obtain the final product VI.

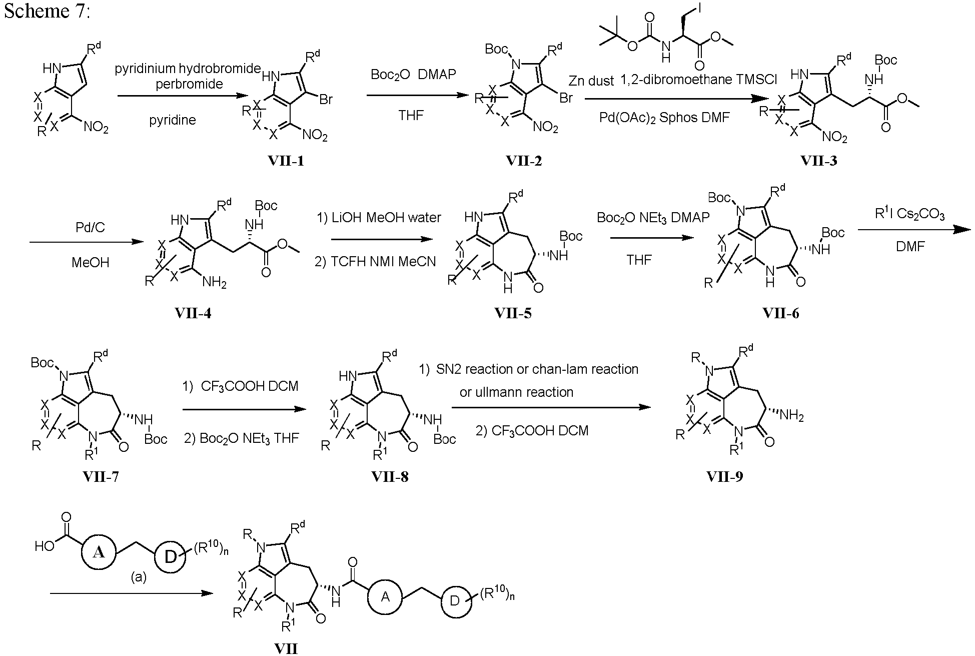

A suitably substituted 4-nitroindole is halogenated at the 3-position to give VII-1 which is then protected with a Boc group to give VII-2. Negishi coupling of VII-2 with (R)-N-(tert-butoxycarbonyl)-3-iodo-L-alanine methyl ester obtain VII-3. Reduction of the nitro group (reduction conditions include but are not limited to Zn/AcOH, Fe/NH₄Cl/EtOH, Zn/NH₄Cl/EtOH, and Ranney Ni/H₂/MeOH), and base hydrolysis followed by cyclization with an amide coupling reagent (coupling reagents include but are not limited to HOAT, EDCI, HATU, and TCFH) obtain VII-5. Boc protection of VII-5 affords VII-6. Reaction of VII-6 with a suitable alkyl iodide to obtain VII-7. Manipulations of Boc protecting groups in VII-7 lead to VII-8. Introduction of different substituents onto the nitrogen atom of the indole in VII-7 by various means, followed by removal of the Boc group afford VII-9, which is coupled with the acid (a) using an amide coupling reagent (coupling reagents include but are not limited to HOAT, EDCI, HATU, and TCFH) to obtain the final product VII.

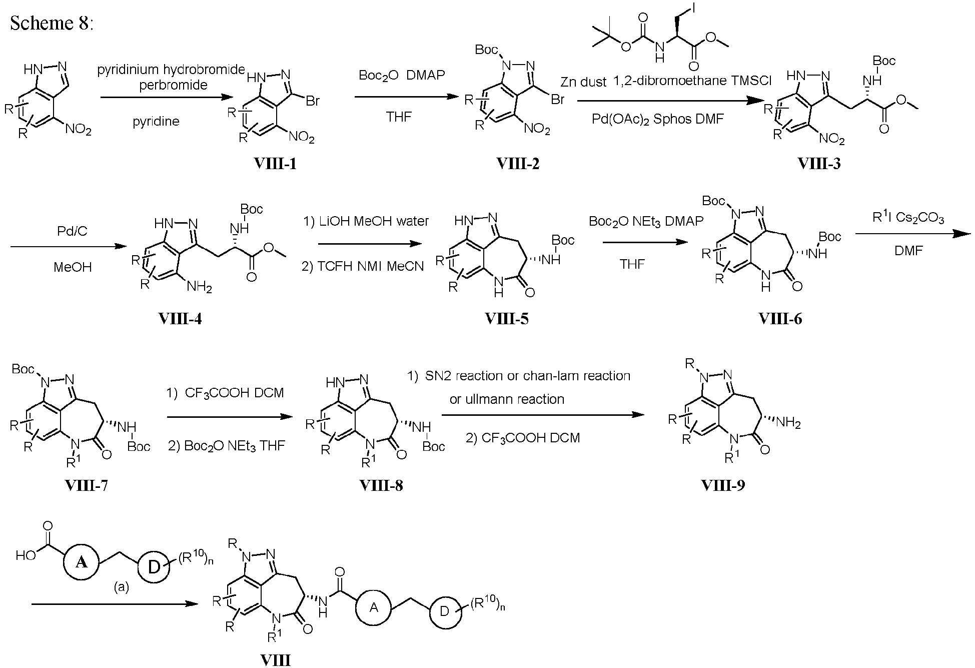

A suitably substituted 4-nitroindole is halogenated at the 3-position to give VIII-1 which is then protected with a Boc group to give VIII-2. Negishi coupling of VIII-2 with (R)-N-(tert-butoxycarbonyl)-3-iodo-L-alanine methyl ester obtain VIII-3. Reduction of the nitro group (reduction conditions include but are not limited to Zn/AcOH, Fe/NH₄Cl/EtOH, Zn/NH₄Cl/EtOH, and Ranney Ni/H₂/MeOH), and base hydrolysis followed by cyclization with an amide coupling reagent (coupling reagents include but are not limited to HOAT, EDCI, HATU, and TCFH) obtain VIII-5. Boc protection of VIII-5 affords VIII-6. Reaction of VIII-6 with a suitable alkyl iodide to obtain VIII-7. Manipulations of Boc protecting groups in VIII-7 lead to VIII-8. Introduction of different substituents onto the nitrogen atom of the indole in VIII-8 by various means, followed by removal of the Boc group afford VIII-9, which is coupled with the acid (a) using an amide coupling reagent (coupling reagents include but are not limited to HOAT, EDCI, HATU, and TCFH) to obtain the final product VIII.

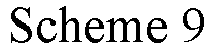

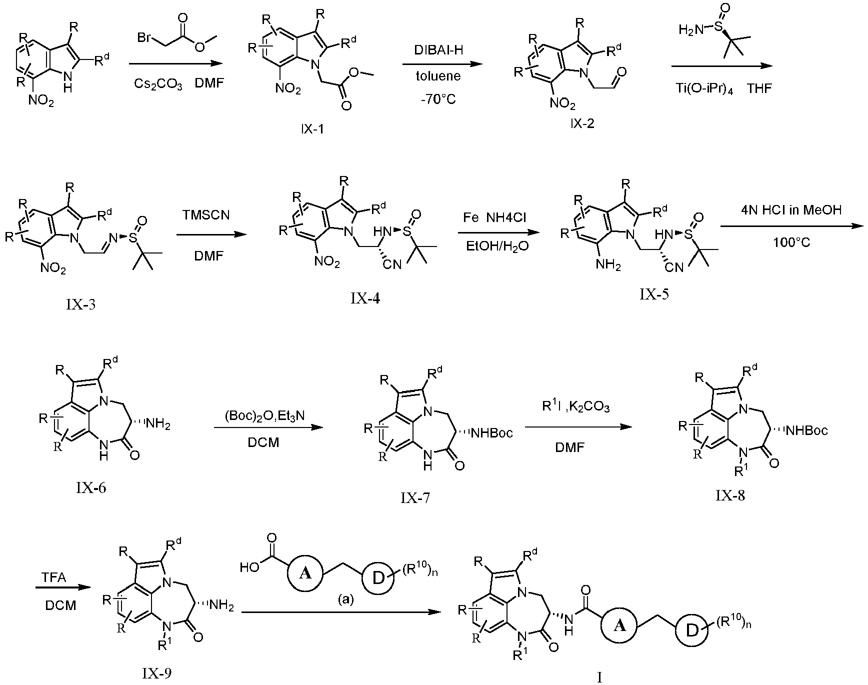

Reaction of methyl bromoacetate react with a suitably substituted 7-nitroindole under basic conditions to obtain IX-1. Reduction of the ester group provides aldehyde IX-2, which is coupled with a suitable chiral tert-butanesulfinamide to afford IX-3. Treatment of IX-3 with trimethylsilyl cyanide to obtain IX-4. Reduction (reduction conditions include but are not limited to Zn/AcOH, Fe/NH₄Cl/EtOH, Zn/NH₄Cl/EtOH, and Pd/C/H₂/MeOH) of the nitro group of IX-4, followed by separation via silica gel column chromatograph to give rise to the desired enantiomer IX-5. Heating IX-5 in a methanol solution of hydrochloric acid to obtain intermediate IX-6. Protecting the amine group in IX-6 with Boc group to obtain IX-7, which is alkylated with a suitable alkyl iodide to obtain IX-8. Removal of the Boc group in IX-8 obtain IX-9. Coupling of IX-9 with acid (a) using an amide coupling reagent (coupling reagents include but are not limited to HOAT, EDCI, HATU, and TCFH) to obtain the final product I.

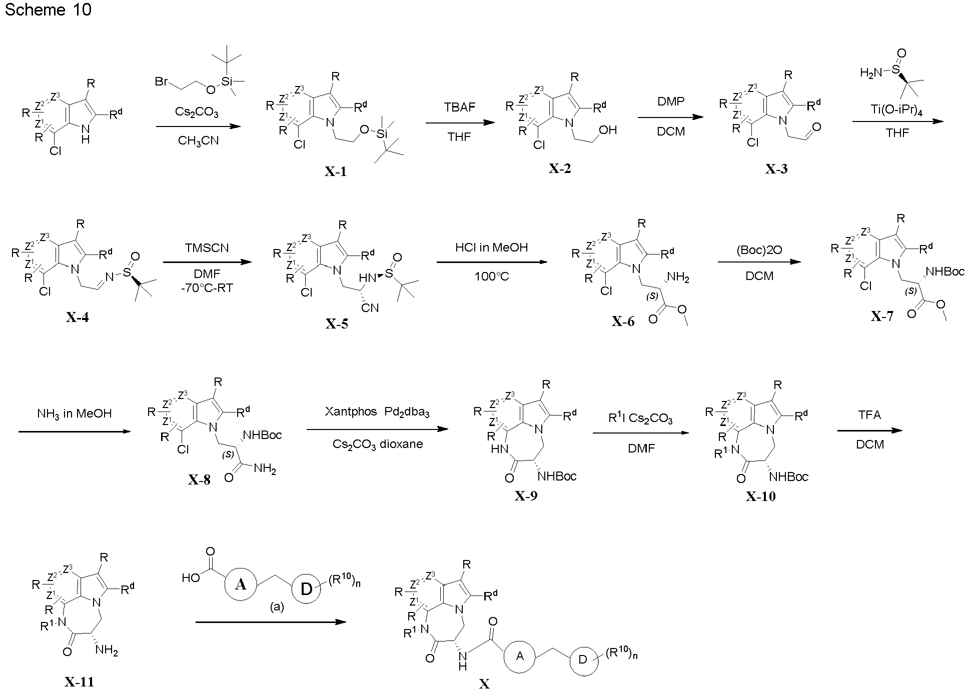

Reaction of (2-bromoethoxy)-tert-butyldimethylsilane with a suitably substituted 7-chloroindole and a base to obtain X-1. Removal of the silyl protecting group by tetrabutylammonium fluoride provide X-2, which is oxidized to aldehyde X-3 using an oxidizing agent (oxidation conditions include but are not limited to Dess-Martin periodinane). Coupling of X-3 with a chiral tert-butanesulfinamide afford X-4. Treatment of X-4 with trimethylsilyl cyanide followed by separation via silica gel column chromatograph to give rise to the desired enantiomer X-5. Heating X-5 in a methanol solution of hydrochloric acid obtains X-6. Protecting the amino group in X-6 with Boc affords X-7, which undergoes aminolysis reaction in the methanol solution of ammonia to obtain X-8. Intramolecular Buchwald coupling (coupling conditions include but are not limited to Pd₂dba₃/Xantphos) to provides X-9. Reaction of X-9 with suitable alkyl iodide and a base gives rise to X-10. Removal of Boc group of X-10 affords X-11, which is coupled with acid (a) using an amide coupling reagent (coupling reagents include but are not limited to HOAT, EDCI, HATU, and TCFH) to obtain the final product X.

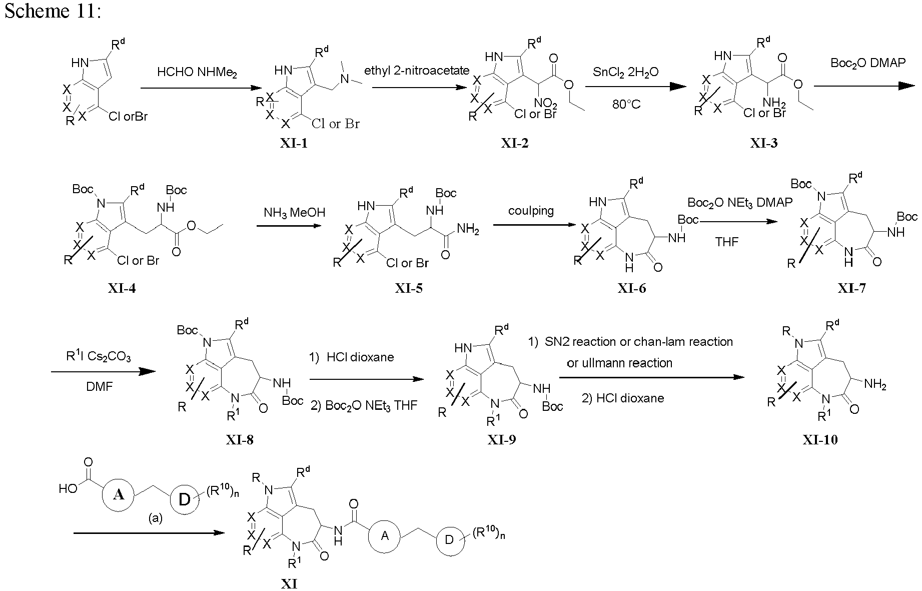

Mannich reaction of a suitable 4-halogenoindole, or a heteroindole, or a substituted indole with dimethylamine, and formaldehyde under the catalysis of a Lewis acid or Bronsted acid provides XI-1. Reaction of XI-1 with ethyl nitroacetate under catalysis of propynoic ester or by heating obtains XI-2. Reduction of the nitro group of XI-2 affords amine XI-3 (reduction conditions include but are not limited to SnCl₂·2H₂O, Zn/AcOH, Fe/NH₄Cl/EtOH, and Zn/NH₄Cl/EtOH). Boc protection of XI-3 gives rise to XI-4. Aminolysis of XI-4 provides amide XI-5. Intramolecular coupling reaction of XI-5 such as Buchwald coupling (coupling conditions include but are not limited to Pd₂dba₃/Xantphos) affords cyclic compound XI-6. Protection of the indole NH of XI-6 with Boc group gives rise to XI-7. Alkylation of XI-7 with alkyl halide and a base provides XI-8. Manipulations of Boc protecting group in XI-8 lead to XI-9. Introduction of different substituents onto the nitrogen atom of the indole in XI-9 by various means, followed by removal of the Boc group afford XI-10, which is coupled with the acid (a) using an amide coupling reagent (coupling reagents include but are not limited to HOAT, EDCI, HATU, and TCFH) to obtain the final product XI.

The third aspect of the present invention provides the purposes of the said compounds of general formulas I-XII in the first aspect, as follows:
(i) preparation of necroptosis inhibitor;
(ii) preparation of a medicine for the prevention and/or treatment of diseases mediated by necroptosis.

In another preferred embodiment, the said diseases mediated by necroptosis include cancer, COVID-19 infection, inflammatory bowel disease, Crohn's disease, ulcerative colitis, psoriasis, retinal detachment, pigmentary retinitis, macular degeneration, pancreatitis, atopic dermatitis, rheumatoid arthritis, spondyloarthritis, gout, systemic lupus erythematosus, Sjögren's syndrome, systemic scleroderma, antiphospholipid syndrome, vasculitis, osteoarthritis, non-alcoholic steatohepatitis, autoimmune hepatitis, autoimmune liver and gallbladder diseases, primary sclerosing cholangitis, nephritis, celiac sprue, primary immunologic thrombocytopenic purpura, transplant rejection, ischemia-reperfusion injury of solid organs, septicemia, systemic inflammatory response syndrome, cerebrovascular accident, myocardial infarction, Huntington's disease, Alzheimer's disease, Parkinson's disease, allergic diseases, asthma, multiple sclerosis, diabetes mellitus type 1, Wegener's granulomatosis, pulmonary sarcoidosis, Behcet's disease, interleukin-1 conversion enzyme-related fever syndrome, chronic obstructive pulmonary disease, tumor necrosis factor receptor-associated periodic syndrome, and periodontitis.

It is noteworthy that, within the scope of the present invention, the technical features as mentioned above and described in detail below (embodiments) can be combined with each other to form new or preferred technical schemes, which will not be expatiated here due to limited space.

### Detailed Description of Embodiments

### Definition

The term "alkyl" refers to monovalent saturated aliphatic hydrocarbyl having 1-10 carbon atoms, including straight-chain hydrocarbyl and branched-chain hydrocarbyl, such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃)(CH₃CH₂)CH-), tert-butyl ((CH₃)₃C-), and n-amyl (CH₃CH₂CH₂CH₂CH₂-), and neopentyl ((CH₃)₃CCH₂-).

As used herein, the term "aryl" refers to the monovalent aromatic carbon ring group with 6-20 (preferably 6-14) carbon atoms having monocyclic (e.g. phenyl) or coupled rings (e.g. naphthyl or anthracyl), and the coupled ring may be nonaromatic (e.g. 2-benzoxazolinone, 2H-1, and 4-benzoxazine-3(4H)-keone-7-yl) if the attachment point is on the aromatic carbon atom. Phenyl and naphthyl are preferred aryls.

As used herein, the term "alkenyl" refers to an alkenyl with 2-10 (e.g. 2-6 or 2-4) carbon atoms and at least 1 (e.g. 1-2) unsaturated olefinic bond (>C = C<). Such groups include ethenyl, allyl, and but-3-enyl. As used herein, the term "cycloalkyl" refers to a cyclic alkyl with 3-10 carbon atoms and single/multiple rings (including coupled, bridged, and spiro systems). In a coupled ring system, one or more rings can be cycloalkyl, heterocyclic, aryl or heteroaryl, only if the connecting sites are rings passing through the cycloalkyl. Examples of suitable cycloalkyls include adamantyl, cyclopropyl, cyclobutyl, cyclopentyl, and cyclooctyl.

As used herein, the term "halogenated"/"halogen" refers to fluorine, chlorine, bromine and iodine.

As used herein, the term "heteroaryl" refers to an aromatic group that has 1 to 10 carbon atoms and 1 to 4 heteroatoms selected from oxygen, nitrogen, and sulfur in the ring. Such a heteroaryl can be monocyclic (such as pyridyl or furyl) or coupled (such as indolizinyl or benzo[b]thienyl), wherein the coupled ring can be non-aromatic and/or contain a heteroatom, as long as the connecting sites are atoms passing through the aromatic heteroaryl. In one embodiment, nitrogen and/or sulfur atoms on the heteroaryl ring are selectively oxidized to N-oxide(N-O), sulfinyl, or sulfonyl. Preferably, the heteroaryl group includes pyridyl, pyrrolil, indolyl, thienyl, and furyl.

As used herein, the term "substituted heteroaryl" refers to a heteroaryl substituted by 1-5, preferably 1-3, more preferably 1-2 substituent that is the same as substituents defined by the substituted aryl.

As used herein, the term "heterocyclic ring"/"heterocyclic"/"heterocyclic alkyl"/"heterocyclyl" refers to a saturated, partially saturated, or unsaturated (but not aromatic) group, with single rings or coupled rings (including the bridged ring system and the spiro system) in which there are 1-10 carbon atoms and 1-4 heteroatoms selected from nitrogen, sulfur or oxygen. In a coupled ring system, one or more rings can be cycloalkyl, aryl or heteroaryl, only if the connecting sites pass through the nonaromatic rings. In one embodiment, nitrogen and/or sulfur atoms of a heterocyclic group are selectively oxidized to provide N-oxide, sulfinyl, and sulfonyl.

As used herein, the term "substituted heterocyclic"/"substituted heterocyclic alkyl"/"substituted heterocyclyl" refers to a heterocyclic group substituted by 1-5 (e.g. 1-3) substituents which are the same as the substituent defined by the substituted cycloalkyl.

The said substituents include but are not limited to the following groups: halogen, -C₁₋₆ alkyl, - C₃₋₈ cycloalkyl, -C₁₋₆ halogenated alkyl, -C₃₋₈ halogenated cycloalkyl, -C₁₋₆ alkoxy, -C₃₋₈ cycloalkyloxy, -C₁₋₆ alkylthio, -C₀₋₆ alkylene-OH, nitro, aldehyde, -SF₅, -C₀₋₆ alkylene-NR^{a}R^{b}, -C₀₋₆ alkylene-carboxyl, -C₀₋₆ alkylene-COR^{a}, -C₀₋₆ alkylene-CO₂R^{a}, -C₀₋₆ alkylene-CONR^{d}R^{e}, -C₀₋₆ alkylene-SO₂R^{a}, -C₀₋₆ alkylene-SO₂NR^{d}R^{e}, carbonyl, -C₀₋₆ alkylene-CN, -C₃₋₈ cycloalkyl-OH, -C₂₋₆ alkenyl, C₂₋₆ alkynyl, -C₀₋₆ alkylene-S(O)(NH)C₁₋₆alkyl, -C₀₋₆ alkylene-S(O)(NCN)C₁₋₆alkyl, -C₀₋₆ alkylene-NR^{c}S(O)₂R^{b}, -C₀₋₆ alkylene-NR^{c}S(O)₂NR^{c}R^{b}, -C₀₋₆ alkylene-NR^{c}C(O)NH₂, -C₀₋₆ alkylene-NR^{c}C(O)R^{b}, -C₀₋₆ alkylene-NR^{c}C(O)NR^{d}R^{e}, -C₀₋₆ alkylene-NR^{c}C(O)OR^{b}, -C₀₋₆ alkylene-NRSO₂R^{b}C(O)-R^{b}, -C₀₋₆ alkylene-P(O)R^{c}R^{b}, -C₀₋₆ alkylene-P(O)(OR^{c})(OR^{b}), -C₀₋₆ alkylene-C(O)C₁₋₆ alkylene amino, C₁₋₆ heteroalkyl, C₅₋₁₀ carbocycle, C₅₋₁₀ aryl, C₂₋₁₀ heterocyclic ring, C₂₋₁₀ heteroaromatic ring, -C₁₋₆ halogenated alkoxy.

In the present invention, C₀₋₆ alkylene refers to no alkylene or C₁₋₆ alkylene.

As used herein, the term "stereoisomer" refers to compounds with different chirality in one or several stereocenters. Stereoisomers include enantiomers and diastereomers.

As used herein, the term "tautomer" refers to alternative forms of compounds with different proton locations, such as tautomers of enol-ketone and imine-enamine, or tautomeric forms of heteroaryl which contains ring atoms connected to the -NH- and =N- parts of the ring, such as pyrazol, imidazole, benzimidazole, triazole, and tetrazole.

"Prodrug" refers to any derivative of the embodiment compound, which can directly or indirectly provide the embodiment compound, its active metabolite or residue when being applied to a subject. Particularly preferred derivatives and prodrugs are those that improve the bioavailability of the embodiment compound (e.g. the compound administered orally tends to be absorbed in the blood more easily) or the delivery of the parent compound to a biological compartment (such as brain or lymphocytic system) when being applied to a subject. Prodrugs include ester forms in the compounds of the present invention.

Where there are stereoisomers of the compounds as mentioned in the present invention, the present invention shall include all the stereoisomers of such compounds.

Where there are tautomers of the compounds as mentioned in the present invention, the present invention shall include all the tautomers of such compounds.

The present invention also includes the deuterated compounds generated from the substitution of any one/more hydrogen atoms in the said compounds with its/their stable isotope deuterium.

The present invention also provides the active ingredients within the safe and effective dosage of compounds of general formulas I-XII, as well as their pharmaceutically acceptable carriers.

The said "active ingredients" in the present invention refer to the compounds of general formulas I-XII.

The said "active ingredients" and pharmaceutical composition of the present invention can be used as inhibitors for diseases mediated by necroptosis. In another preferred embodiment, it is used to prepare drugs for the prevention and/or treatment of necroptosis mediated diseases.

"Safe and effective dosage" refers to the dosage of active ingredients is sufficient to improve the condition without serious side effects significantly. Generally, a pharmaceutical composition contains 1-2,000mg of active ingredients/agents; preferably, it contains 10-200mg of active ingredients/agents. More preferably, the said "one dosage" is contained in a tablet or a capsule.

"Pharmaceutically acceptable carrier" refers to one or more compatible solid/liquid fillers or gel substances, which are suitable for human use, and shall be of sufficient purity and low toxicity. "Compatibility" here refers to the fact that each component of the composition can be blended with and among the active ingredients in the present invention without significant reduction of the active ingredient's efficacy.

In general, the compounds of preferred embodiment will be administered in a therapeutically effective dosage and any acceptable mode via any medicament of a similar effect. The actual dosages of the compounds (i.e. active ingredients) in the preferred embodiments are determined based on numerous factors, such as the severity of diseases to be treated, age and relative health of the patient, efficacy of the compounds used, and route & form of application. The drug may be administered for times a day (once or twice preferably a day). All of such factors are taken into account by the attending physician.

For the purpose of the preferred embodiment, the therapeutically effective dosage can be a daily total dosage generally, for example, from 0.001-1,000mg/kg for one time or times (preferably 1.0-30mg/kg per day for a patient). Dosage unit composition may include its dosage factors to form a daily dosage. The dosage forms depend on various factors, such as administration mode and bioavailability of drug substances. In general, the compounds of the preferred embodiment can be administered as a pharmaceutical composition through any of the routes as follows: oral, systemic (e.g. transdermal, intranasal or suppository), or parenteral (e.g. intramuscular, intravenous or subcutaneous). The preferred method of administration is oral, whose appropriate daily dosage can be adjusted as per the bitterness. The composition may be made in the forms of tablet, pill, capsule, semi-solid, powder, sustained-release preparation, solution, suspension, elixir, aerosol or any other appropriate composition. Another preferred administration mode of compounds in the preferred embodiment is inhalation, which is an effective mode to deliver therapeutic agents directly to the respiratory tract (refer to U.S. Patent No. 5,607,915 for example).

Pharmaceutically acceptable carriers or excipients include treatment agents, drug delivery modifiers, and accelerators, such as calcium phosphate, magnesium stearate, talc, monosaccharide, disaccharide, starch, gelatin, cellulose, sodium methylcellulose, carboxymethyl cellulose, glucose, hydroxypropyl-B-cyclodextrin, polyvinylpyrrolidone, low-melting-point wax, ion exchange resin, and any combination of two or more of them. Liquid and semi-solid excipients can be selected from glycerol, propylene glycol, water, ethanol, and various oils (including petroleum, animal oil, vegetable oil or synthetic oils, such as peanut oil, soybean oil, mineral oil and sesame oil). The preferred liquid carriers (in particular those for injectable solutions) include water, brine, glucose aqueous solution, and ethylene glycol. Other pharmaceutically acceptable excipients are described in Remington's Pharmaceutical Sciences, Mack pub. Co., New Jersey (1991) and incorporated by reference.

As used herein, the term "pharmaceutically acceptable salt" refers to non-toxic acid or alkaline-earth metal salts of compounds in general formulas I-XII. Such salts can be prepared in situ during the final separation and purification of compounds in general formulas I-XII, or via the reaction among proper organic/inorganic acids, alkalis and alkali/acidic functional groups. Representative salts include, but are not limited to acetate, adipate, alginate, citrate, aspartate, benzoate, benzene sulfonate, disulfate, butyrate, camphorate, camphorsulfonate, digluconate, cypionate, lauryl sulfate, esilate, glucose heptanate, glycerophosphate, hemisulphate, enanthate, hexanoate, fumarate, hydrochloride, hydrobromate, hydriodate, 2-hydroxyethyl sulfonate, lactate, maleate, mesylate, nicotinate, 2-naphthyl sulfonate, oxalate, pamoate, pectate, thiocyanate, 3-phenyl propionate, picrate, pivalate, propionate, succinate, sulfate, tartrate, thiocyanate, p-toluenesulfonate, and undecanoate. In addition, the basic groups containing nitrogen can be quaternary-ammonium salted with the agents as follows: alkyl halides such as chlorides, bromides, and iodides of methyl, ethyl, propyl and butyl; dialkyl sulfates such as dimethyl, diethyl, dibutyl, and diamyl sulfate; long-chain halides such as chlorides, bromides and iodides of decyl, lauryl, myristyl, and stearyl; aromatic alkyl halides such as benzyl and phenylethyl bromide. Water soluble, oil soluble or dispersible products are obtained. Examples of acids that can be used to form pharmaceutically acceptable acid-addition salts include inorganic acids of hydrochloride, sulfuric acid, phosphoric acid, as well as the organic acids of oxalic acid, maleic acid, methanesulfonic acid, succinic acid, and citric acid. Alkali-addition salts can be prepared in situ while final separation and purification of compounds in general formulas I-XII, or via the reaction of carboxylic acid portion with proper alkali (such as pharmaceutically acceptable hydroxides of metal cations, carbonate or bicarbonate), ammonia, organic primary, secondary or tertiary amines, respectively. Pharmaceutically acceptable salts include, but are not limited to, alkali metal and alkaline-earth metal based cations, such as sodium, lithium, potassium, calcium, magnesium, aluminum salts, as well as non-toxic ammonium, quaternary ammonium, and amine cations, including but not limited to: ammonium, tetramethyl-ammonium, tetraethylammonium, methylamine, dimethylamine, trimethylamine, triethylamine, and ethylamine. Other representative organic amines used to produce alkali-addition salts include diethylamine, ethylenediamine, ethanolamine, diethanolamine, and piperazine.

The following abbreviations have the meanings indicated: EA: ethyl acetate. DBU: 1,8-diazabicyclo[5.4.0]undec-7-ene; DCM: dichloromethane; DIBAL: diisobutylaluminum hydride; DIEA: diisopropylamine; DMAP: N,N-dimethylaminopyridine; DME: 1,2-dimethoxyethane; DMF: N, N-dimethylformamide; DMPE: 1,2-bis(dimethylphosphino)ethane; DMSO: dimethyl sulfoxide; DPPB: 1,4-bis(diphenylphosphino)butane; DPPE: 1,2-bis(diphenylphosphino)ethane; DPPF: 1,1'-bis(diphenylphosphino)ferrocene; DPPM: 1,1'-bis(diphenylphosphino)methane; DIAD: diisopropyl azodicarboxylate; EDCI: 1-(3-dimethylaminopropyl)-3-ethylcarbodiimide methiodide; HATU: O-(7-azabenzotriazol-1-yl)-1,1,3,3-tetramethyluronium Hexafluorophosphate; HMPA: hexamethyl phosphoramide; HOAT: N-hydroxy-7-azabenzotriazole; IPA: isopropanol; LDA: lithium diisopropylamide; LHMDS: lithium bis(trimethylsilyl)amide; LAH: lithium aluminium hydride; NCS: N-chlorosuccinimide; PE: petroleum ether; PyBOP: benzotriazol-1-yloxytripyrrolidinophosphonium hexafluorophosphate; TDA: tris(2-(2-methoxyethoxy)ethyl)amine; DCM: dichloromethane; TEA: triethylamine; TFA: trifluoroacetic acid; THF: tetrahydrofuran; NCS: N-chlorosuccinimide; NMM: N-methyl morpholine; NMP: N-methyl pyrrolidinone; PPh₃: triphenylphosphine; rt: room temperature; T3P: propylphosphonic anhydride; TCFH: N,N,N',N'-tetramethylchloroformamidinium hexafluorophosphate; SGCC: silica gel column chromatography.

HPLC purification conditions: The Waters micromass ZQ 4000 (MAA050 model) is used as the mass detector and the Waters 2487 UV as the detector, and the HPLC-MS analysis is carried on the Waters HPLC 2790. The chromatographic column used is Phenomenex OOB-4605-E0 (5U-XB-C18-100A, 50*4.6mm). The mobile phase is eluent A (water, 0.05% TFA) and eluent B (CH₃CN, 0.05% TFA), with an elution rate of 1mL/minute. The initial condition is 90% A acting for 1 minute, and the 90% A linearly decreases to 10% A in 5 minutes and rises back to 90% A in 1 minute, with a total operation time of 7 minutes. Depending on the properties of compounds, the gradient of the mobile phase and the operation time can be adjusted.

HPLC chiral resolution conditions: an Agilent Technologies 1200 Infinity LC system is used, with a chiral chromatographic column from Daicel CHIRALPAK IG (product number: IG00EE-AT002). The mobile phase is eluent A (n-hexane) and eluent B (ethanol), with an elution rate of 3mL/min, and the elution consumes a constant proportion of ethanol. Depending on the properties of compounds and the types of chiral chromatographic columns, ethanol proportion in the mobile phase and the operation time can be adjusted.

The following examples are given to make the present invention more readily comprehensible, instead of limiting its scope. Unless otherwise specified, all percentages and parts are calculated by weight, with units of parts by weight.

Unless otherwise specified, all materials and reagents used in the examples of the present invention are commercially available.

Unless otherwise specified, all the professional and scientific terms referred herein share the same meaning as those familiar to the skilled in this field. In addition, any method and material similar to the content described herein can be applied to the methods in the present invention. The preferable implementation methods and materials described herein are only for demonstration.

### Example 1: preparation of intermediate Z-7

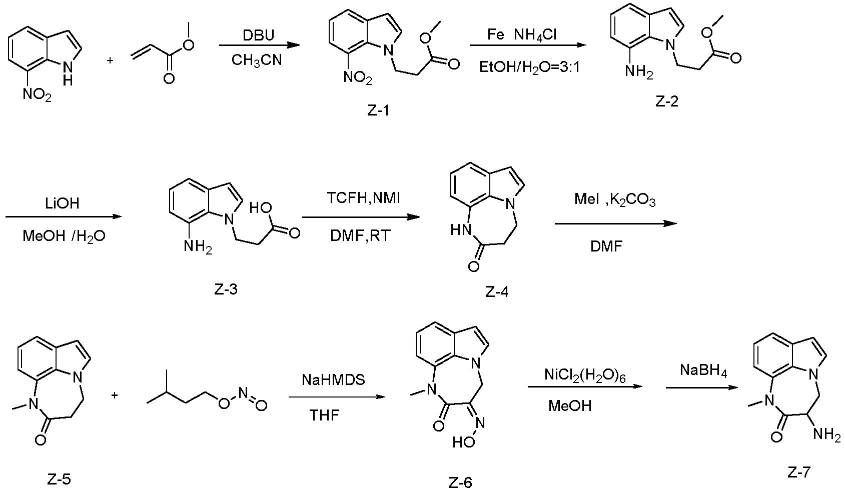

### Step 1: preparation of Z-1

To a solution of 500mg of 7-nitroindole in 10mL of acetonitrile, were added methyl acrylate (670.4mg) and DBU (234.9mg) and the reaction mixture was stirred for reaction at 50°C for 4 hours. The reaction mixture was cooled to room temperature and quenched with water. Extraction was conducted with ethyl acetate. The extract was dried over anhydrous sodium sulfate, and concentrated to obtain the intermediate Z-1 which was purified by SGCC.

MS ESI: m/z=249.4, [M+H]⁺.

### Step 2: preparation of Z-2

Z-1 (330mg), NH₄Cl (290.4mg), and iron powder (744.9mg) were dissolved in ethanol (12mL) and water (4mL) and the mixture was stirred for reaction at rt for 1 hour. Extraction was conducted with ethyl acetate. The extract was dried over anhydrous sodium sulfate, and concentrated to obtain the intermediate Z-2 which was purified by SGCC.

MS ESI: m/z=218.2, [M+H]⁺.

### Step 3: preparation of Z-3

Z-2 (250mg) and lithium hydroxide (140mg) were dissolved in methanol (10mL) and water (3mL) and the mixture was stirred for reaction at rt for 1 hour. The reaction mixture was adjusted to pH 5 with hydrochloric acid, and extracted with ethyl acetate. The extract was dried over anhydrous sodium sulfate and concentrated to obtain the crude product Z-3.

MS ESI: m/z=205, [M+H]⁺.

### Step 4: preparation of Z-4

A mixture of Z-3, NMI (N-methylimidazole), and TCFH in DMF (10mL) was stirred at rt for 3 hours and then poured into water and extracted with ethyl acetate. The extract was dried over anhydrous sodium sulfate, and concentrated to obtain the intermediate Z-4 which was purified by SGCC.

MS ESI: m/z=187.2, [M+H]⁺.

### Step 5: preparation of Z-5

A mixture of Z-4 (100mg), iodomethane (233.5mg), and cesium carbonate (185.7mg) in DMF (5mL) stirred at room temperature for 3 hours and then poured into water and extracted with ethyl acetate. The extract was dried over anhydrous sodium sulfate, concentrated to obtain the intermediate Z-5 which was purified by SGCC.

MS ESI: m/z=201.2, [M+H]⁺.

### Step 6: preparation of Z-6

Z-5 (100mg), NaHMDS, and isopentyl nitrite were dissolved in THF (5mL). The mixture was stirred in an ice bath for 3 hours and then poured into water and extracted with ethyl acetate. The extract was dried over anhydrous sodium sulfate, and concentrated to give the intermediate Z-6 which was purified by SGCC.

MS ESI: m/z=230.2, [M+H]⁺.

### Step 7: preparation of Z-7

A solution of Z-6 (200mg) and nickel chloride hexahydrate (423.5mg) in MeOH (8mL). After was stirred at 0 °C for 30 minutes, and was then treated with sodium borohydride (198.2mg. After the reaction mixture was stirred at rt for 3 hours, the reaction mixture was concentrated and the residue was swished from dichloromethane, and the solid was collected by filtration to obtain the intermediate Z-7.

MS ESI: m/z=216.2, [M+H]⁺.

### Example 2: T-1

### (t)-5-benzyl-N-(1-methyl-2-oxy-1,2,3,4-tetrahydro-[1,4] diazepino [3,2,1-hi] indol-3-yl)-4H-1,2,4-triazole-3-carboxamide

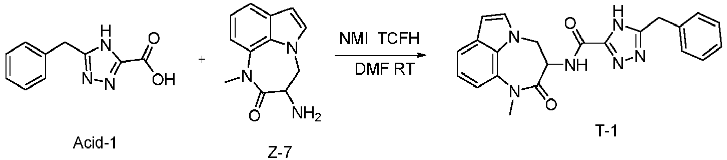

Acid-1 (73.6mg), NMI (34.3mg), and TCFH (119.7mg) were dissolved in DMF (1mL). The mixture was stirred under ice bath conditions for 10 minutes before Z-7 (60mg) was added. After stirring at rt for 3 hours, the reaction was quenched with ice water. The mixture was extracted with EA three times, and the organic phase was combined. The combined organic phase was washed three times with water, three times with saturated brine, dried over anhydrous sodium sulfate, and concentrated. The residue was purified by HPLC [mobile phase: A: H₂O (+0.1% CF₃COOH); B: MeCN; separation condition: 50%B; flow rate: 10mL/min; chromatographic column: Waters Xterra^{®} Prep MSC18, 19*250mm, 10µm] to provide the product 5mg of white solid (T-1).

¹H NMR (400 MHz, CDCl₃) δ 8.63 (s, 1H), 7.49 (d, J = 7.8 Hz, 1H), 7.09-7.32 (m, 7H), 7.06 (d, J = 7.7 Hz, 1H), 6.58 (m,1H), 4.92 (m,1H), 4.67 (m,1H), 4.33 (m, 1H), 4.19 (s, 2H), 3.64 (s, 3H).

MS ESI: m/z=401.1, [M+H]⁺.

### Example 3: T-2

### (t)-5-benzyl-N-(1-methyl-2-oxy-1,2,3,4-tetrahydro-[1,4] diazepino [3,2,1-hi] indol-3-yl) oxazole-2-carboxamide

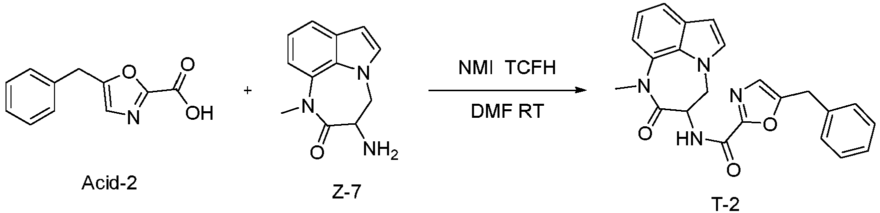

Using Acid-2 (56.6mg) and Z-7 as starting materials, T-2 was prepared under the same conditions as in Example 2.

¹H NMR (400 MHz, CD₃OD) δ 7.45- 7.12 (m,10H), 7.02 (s, 1H), 6.55 (m,1H), 4.76 (m, 1H), 4.63 (m, 1H), 4.58 (s, 1H), 4.39 (m,1H), 4.11 (s, 2H), 3.59 (s, 3H).

MS ESI: m/z= 401.1, [M+H]⁺.

### Example 4: T-3

### (t)-5-benzyl-N-(7-iodo-1-methyl-2-oxo-1,2,3,4-tetrahydro-[1,4] diazepino [3,2,1-hi] indol-3-yl)-4H-1,2,4-triazole-3-carboxamide

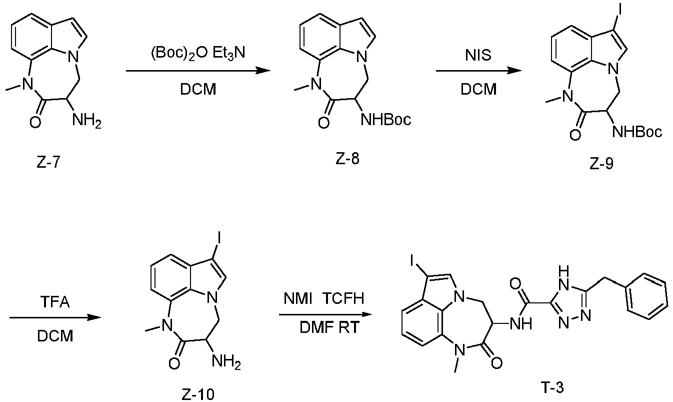

### Step 1: preparation of Z-8

The intermediate Z-7 (1eq), di-tert-butyl dicarbonate (1.1eq), and triethylamine (1.5eq) were dissolved in dichloromethane. After stirring at rt for 2 hours, the reaction was quenched with ice water. Extraction was conducted three times with ethyl acetate, and the organic phase was combined. The combined organic phase was washed three times with water and three times with saturated brine respectively, dried over anhydrous sodium sulfate, and concentrated. The residue was purified by SGCC to give the intermediate Z-8.

### Step 2: preparation of Z-9

The intermediate Z-8 (1eq) and N-iodosuccinimide (1.1eq) were dissolved in dichloromethane. The mixture was stirred at room temperature for 30 minutes, and then quenched with ice water. Extraction was conducted three times with ethyl acetate, the extracted mixture was washed with saturated solution of sodium thiosulfate pentahydrate, and the organic phase was combined. The combined organic phase was washed three times with water and three times with saturated brine respectively, dried over anhydrous sodium sulfate, and concentrated. The residue was purified by SGCC to give intermediate Z-9.

### Step 3: preparation of Z-10

The intermediate Z-9 (1eq) and trifluoroacetic acid (10eq) were dissolved in dichloromethane. The mixture was stirred at rt for 3 hours and then concentrated. The residue was purified by SGCC to give intermediate Z-10.

MS ESI: m/z= 341.6, [M+H]⁺.

### Step 4: preparation of T-3

Using Acid-1 (1.5eq) and Z-9 (1eq) as starting materials, T-3 was prepared under the same conditions as in Example 2.

¹H NMR (400 MHz, acetone-d₆) δ 8.55 (s, 1H), 7.62 (s, 1H), 7.43 - 7.21 (m, 8H), 4.88 - 4.70 (m, 2H), 4.45 (m,1H), 4.21 (s, 2H), 3.60 (s, 3H).

MS ESI: m/z= 527, [M+H]⁺.

### Example 5: T-4

### (t)-5-benzyl-N-(1-methyl-7-(methylthio)-2-carbonyl-1,2,3,4-tetrahydro-[1,4] diazepino [3,2,1-hi] indol-3-yl)-4H-1,2,4-triazole-3-carboxamide

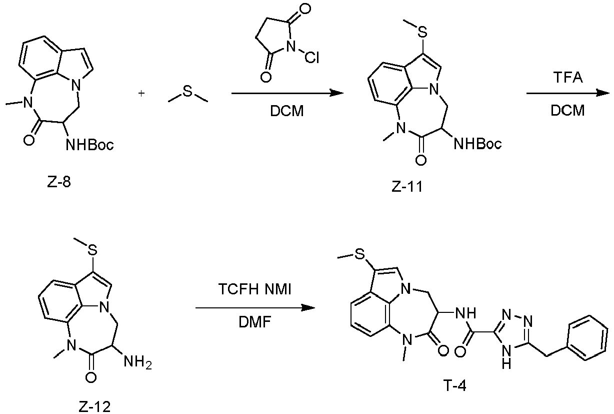

### Step 1: preparation of Z-11

The intermediate Z-8 (1eq), dimethyl sulfide (1.3eq), and N-chlorosuccinimide (1.3eq) were dissolved in dichloromethane. The mixture was stirred at rt for 2 hours, and then quenched with ice water. Extraction was conducted three times with ethyl acetate, and the organic phase was combined. The combined organic phase was washed three times with water and three times with saturated brine respectively, dried over anhydrous sodium sulfate, and concentrated. The residue was purified by SGCC to give intermediate Z-11.

### Step 2: preparation of Z-12

The intermediate Z-11 (1eq) and trifluoroacetic acid (10eq) were dissolved in dichloromethane, and then the mixture was stirred at rt for 3 hours. The reaction mixture was concentrated, and the residue was purified by SGCC to give intermediate Z-12.

MS ESI: m/z=262 , [M+H]⁺.

### Step 3: preparation of T-4

Using Acid-1 (1.5eq) and Z-2 (1eq) as starting materials, T-4 was prepared under the same conditions as in Example 2.

¹H NMR (400 MHz, CD₃OD) δ 7.54 (m, 1H), 7.34 -7.17 (m, 7H), 4.77 (m, 1H), 4.65 - 4.59 (m, 1H), 4.37 - 4.30 (m, 1H), 4.16 (s, 2H), 3.57 (s, 3H), 2.32 (s, 3H).

MS ESI: m/z=447, [M+H]⁺.

### Example 6: T-5

### (t)-5-benzyl-N-(9-chloro-1-methyl-2-oxo-1,2,3,4-tetrahydro-[1,4] diazepino [3,2,1-hi] indol-3-yl)-4H-1,2,4-triazole-3-carboxamide

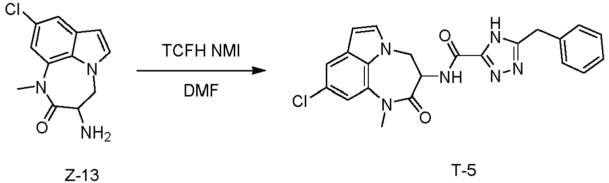

Using 5-chloro-7-nitroindole as starting material, Z-13 was prepared under the same conditions as in Example 1; Starting with acid-1 (1.5eq) and Z-13 (1eq) as starting materials, T-5 was prepared under the same conditions as in Example 2.

¹H NMR (400 MHz, CDCl₃) δ 8.60 (m, 1H), 7.45 (d, J = 1.8 Hz, 1H), 7.33 - 7.27 (m, 5H), 7.08 (d, J = 3.2 Hz, 1H), 7.03 (d, J = 1.8 Hz, 1H), 6.51 (d, J = 3.1 Hz, 1H), 4.91 - 4.86 (m, 1H), 4.65 (m, 1H), 4.30 (m, 1H), 4.19 (s, 2H), 3.61 (s, 3H).

MS ESI: m/z=435, [M+H]⁺.

### Example 7: T-6

### (t)-5-benzyl-N-(9-bromo-1-methyl-2-oxo-1,2,3,4-tetrahydro-[1,4] diazepino [3,2,1-hi] indol-3-yl)-4H-1,2,4-triazole-3-carboxamide

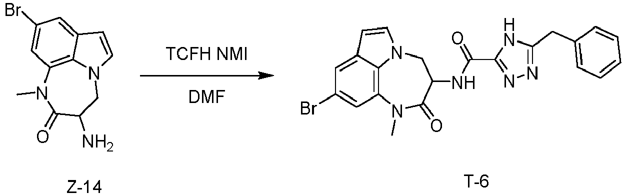

Using 5-bromo-7-nitroindole as starting material, Z-14 was prepared under the same conditions as in Example 1; Starting with acid-1 (1.5eq) and Z-14 (1eq) as starting materials, T-6 was prepared under the same conditions as in Example 2.

¹H NMR (400 MHz, CDCl₃) δ 8.59 (m, 1H), 7.61 (d, J = 1.6 Hz, 1H), 7.33-7.27 (m,5H), 7.15 (d, J = 1.6 Hz, 1H), 7.06 (d, J = 3.1 Hz, 1H), 6.51 (d, J = 3.2 Hz, 1H), 4.91 - 4.84 (m, 1H), 4.65 (m, 1H), 4.29 (m, 1H), 4.19 (s, 2H), 3.61 (s, 3H).

MS ESI: m/z=481, [M+H]⁺.

### Example 8: T-7

### (t)-5-benzyl-N-(9-fluoro-1-methyl-2-oxo-1,2,3,4-tetrahydro-[1,4] diazepino [3,2,1-hi] indol-3-yl)-4H-1,2,4-triazole-3-carboxamide

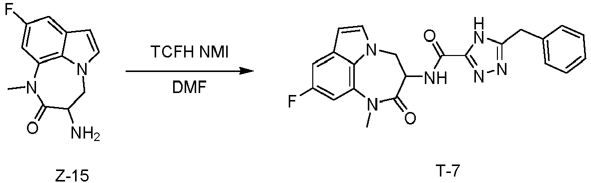

Using 5-fluoro-7-nitroindole as starting material, Z-15 was prepared under the same conditions as in Example 1; Starting with acid-1 (1.5eq) and Z-15 (1eq) as starting materials, T-7 was prepared under the same conditions as in Example 2.

¹H NMR (400 MHz, DMSO-d₆) δ 8.66 (s, 1H), 7.52 (d, J = 3.1 Hz, 1H), 7.40 - 7.19 (m, 6H), 7.09 (m, 1H), 6.55 (d, J = 3.1 Hz, 1H), 4.70 (m, 1H), 4.59 (m, 1H), 4.41 (m, 2H), 4.13 (s, 2H), 3.50 (s, 3H).

MS ESI: m/z= 419, [M+H]⁺.

### Example 9: T-8

### (±)-5-benzyl-N-(1-methyl-7-((1-methyl-1H-pyrazol-4-yl)ethynyl)-2-oxy-1,2,3,4-tetrahydro-[1,4] diazepino [3,2,1-hi] indol-3-yl)-4H-1,2,4-triazole-3-carboxamide

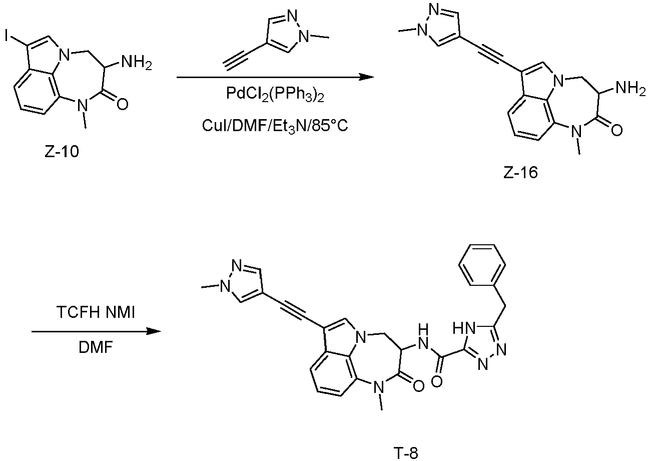

### Step 1: preparation of Z-16

4-Ethynyl-1-methyl-1-H-pyrazole (1.3eq), intermediate Z-10 (1eq), CuI (0.05eq) and PdCl₂(PPh₃)₂ (0.1eq) were dissolved in a mixed of triethylamine and DMF. The reaction mixture was stirred at 85°C for 0.5 hour, cooled to rt and poured in to water. Extraction was conducted with EA, and the extract was washed three times with water and concentrated. The residue was purified by SGCC to give intermediate Z-16.

MS-ESI: m/z =420, [M+H]⁺.

### Step 2: preparation of T-8

Using Acid-1 (1.5eq) and Z-16 (1eq) as starting materials, T-8 was prepared under the same conditions as in Example 2.

¹H NMR (400 MHz, acetone-d₆) δ7.88 (s, 1H), 7.68 (s, 1H), 7.60 (s, 1H), 7.58 - 7.55 (m, 1H), 7.34-7.27 (m, 7H), 4.86 - 4.73 (m, 2H), 4.44 (m, 1H), 4.21 (s, 2H), 3.92 (s, 3H), 3.62 (s, 3H).

MS ESI: m/z=505, [M+H]⁺.

### Example 10: T-9

### (t)-5-benzyl-N-(1-methyl-9-((1-methyl-1H-pyrazol-4-yl)ethynyl)-2-oxy-1,2,3,4-tetrahydro-[1,4] diazepino [3,2,1-hi] indol-3-yl)-4H-1,2,4-triazole-3-carboxamide

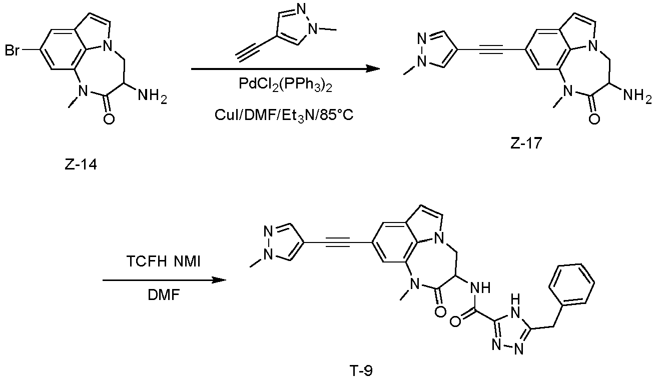

### Step 1: preparation of Z-17

4-Ethynyl-1-methyl-1-H-pyrazole (1.3eq), intermediate Z-14 (1eq), CuI (0.05eq) and PdCl₂(PPh₃)₂ (0.1eq) were dissolved in a mixture of triethylamine and DMF. The reaction mixture was stirred at 85°C for 3 hours and cooled to rt. Water was added and the mixture was extracted with EA. The extract was washed three times with water, the organic phase was concentrated. The residue was purified by SGCC to give intermediate Z-17.

MS-ESI: m/z =420, [M+H]⁺.

### Step 2: preparation of T-9

Using Acid-1 (1.5eq) and Z-17 (1eq) as starting materials, T-9 was prepared under the same conditions as in Example 2.

¹H NMR (400 MHz, acetone-d₆) δ 8.56 (s, 1H), 7.87 (s, 1H), 7.58 (s, 1H), 7.45 (d, J = 3.2 Hz, 1H), 7.34 -7.24 (m, 6H), 6.62 (d, J = 3.2 Hz, 1H), 4.82 (m, 1H), 4.75 (m, 1H), 4.42 (m, 1H), 4.21 (s, 2H), 3.91 (s, 3H), 3.63 (s, 3H).

MS ESI: m/z=505, [M+H]⁺.

### Example 11: T-10

### (t)-5-benzyl-N-(1-ethyl-2-oxy-1,2,3,4-tetrahydro-[1,4] diazepino [3,2,1-hi] indol-3-yl)-4H-1,2,4-triazole-3-carboxamide

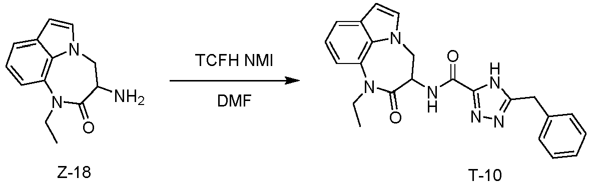

### Step 1: preparation of Z-18

Using Iodoethane and intermediate Z-4 as starting materials, Z-18 was prepared under the same conditions as in Example 1.

MS-ESI: m/z =230, [M+H]⁺.

### Step 2: preparation of T-10

Using Acid-1 (1.5eq) and Z-18 (1eq) as starting materials, T-10 under was prepared the same conditions as in Example 2.

¹H NMR (400 MHz, CD₃OD) δ 7.47 (m, 1H), 7.35 - 7.21 (m, 7H), 7.14 (m, 1H), 6.56 (m, 1H), 4.77 (m, 1H), 4.65 (m, 1H), 4.32 (m, 1H), 4.18 (q, J = 6.6 Hz, 4H), 1.28 (t, J = 7.1 Hz, 3H).

MS ESI: m/z= 415, [M+H]⁺.

### Example 12: T-11

### (t)-5-benzyl-N-(7-bromo-1-methyl-2-oxo-1,2,3,4-tetrahydro-[1,4] diazepino [3,2,1-hi] indol-3-yl)-4H-1,2,4-triazole-3-carboxamide

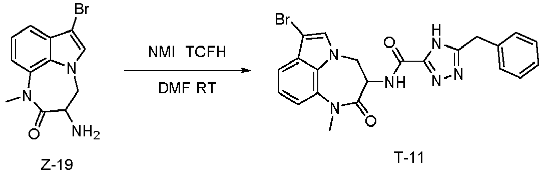

### Step 1: preparation of Z-19

Using NBS and intermediate Z-8 as starting materials, Z-19 was prepared under the same conditions as in Example 4.

MS-ESI: m/z = 296, [M+H]⁺.

### Step 2: preparation of T-11

Using Acid-1 (1.5eq) and Z-19 (1eq) as starting materials, T-11 was prepared under the same conditions as in Example 2.

¹H NMR (400 MHz, CD₃OD) δ 7.37 - 7.20 (m, 9H), 4.80 - 4.75 (m, 1H), 4.65 - 4.59 (m, 1H), 4.35 (m,1H), 4.16 (s, 2H), 3.56 (s, 3H).

MS ESI: m/z=481, [M+H]⁺.

### Example 13: T-12

### (±)-3-(5-benzyl-4H-1,2,4-triazole-3-carboxamido)-1-methyl-2-oxy-1,2,3,4-tetrahydro-[1,4] diazepino [3,2,1-hi] indole-6-carboxylate

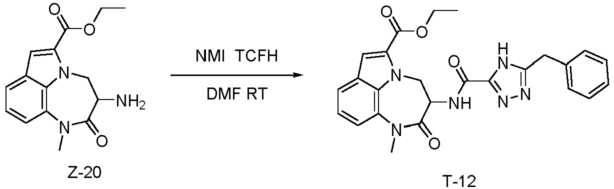

7-nitroindole-2-carboxylate was used as the starting material and Z-20 was prepared under the same conditions as in Example 1; Starting acid-1 (1.5eq) and Z-20 (1eq) as starting materials, T-12 was prepared under the same conditions as in Example 2.

¹H NMR (400 MHz, acetone-d₆) δ 8.55 (s, 1H), 7.62 (m, 1H), 7.42 (s, 1H), 7.38 -7.26 (m, 7H), 5.37 (m, 1H), 4.86 - 4.80 (m, 1H), 4.43 (m,1H), 4.39 - 4.32 (q, J = 7.1 Hz ,2H), 4.21 (s, 2H), 3.60 (s, 3H), 1.37 (t, J = 7.1 Hz, 3H).

MS ESI: m/z= 473, [M+H]⁺.

### Example 14: T-13

### (±)-3-(5-benzyl-4H-1,2,4-triazole-3-carboxamido)-1-methyl-2-oxy-1,2,3,4-tetrahydro-[1,4] diazepino [3,2,1-hi] indole-6-carboxylic acid

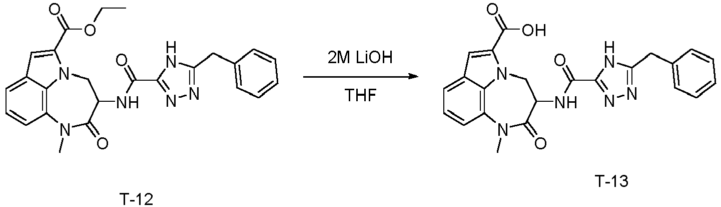

T-12 (1eq) and 2M aqueous solution of lithium hydroxide were dissolved in THF, and the mixture was stirred at 60°C for three hours. The reaction mixture was then neutralized with 2N HCl, extracted with EA three times, and the organic phase was combined. The combined organic phase was washed three times with water and three times with saturated brine respectively, dried over anhydrous sodium sulfate, and concentrated. The residue was purified by HPLC to give intermediate T-13.

¹H NMR (400 MHz, acetone-d₆) δ 8.55 (s, 1H), 7.63 (m, 1H), 7.45 (s, 1H), 7.38 - 7.30 (m, 5H), 7.25 (m, 2H), 5.44 (m, 1H), 4.83 (m, 1H), 4.40 (m, 1H), 4.21 (s, 2H),3.61 (s, 3H).

MS ESI: m/z= 445, [M+H]⁺.

### Example 15: T-14

### (t)-5-benzyl-N-(1-methyl-2-oxo-1,2,3,4,6,7-hexahydro-[1,4] diazepino [3,2,1-hi] indol-3-yl)-4H-1,2,4-triazole-3-carboxamide

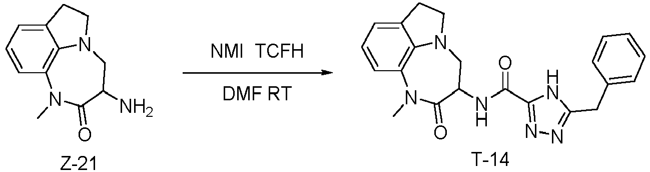

Z-8 (1eq) and sodium cyanoborohydride (3eq) were dissolved in acetic acid, and the reaction mixture was stirred for 3 hours under ice bath conditions and poured into water. Extraction was conducted with EA three times, and the organic phase was combined and dried over anhydrous sodium sulfate. The organic phase was concentrated, and the intermediate Z-21 was obtained under the same conditions as in the Step 3 of Example 4. Using acid-1 (1.5eq) and Z-21 (1eq) as starting materials, T-14 was prepared under the same conditions as in Example 2.

¹H NMR (400 MHz, CDCl₃) δ 8.34 (s, 1H), 7.39 - 7.29 (m, 4H), 6.96- 6.90 (m, 2H), 6.75 (m, 2H), 4.87 (m, 1H), 4.17 (s, 2H), 3.78 (m, 2H), 3.62 (m, 2H), 3.44 (s, 3H), 3.27 - 3.18 (m, 2H).

MS ESI: m/z= 403, [M+H]⁺.

### Example 16: T-15

### (±)-5-benzyl-N-(1-methyl-7-(1-methyl-1H-pyrazol-4-yl)-2-oxy-1,2,3,4-tetrahydro-[1,4] diazepino [3,2,1-hi] indol-3-yl)-4H-1,2,4-triazole-3-carboxamide

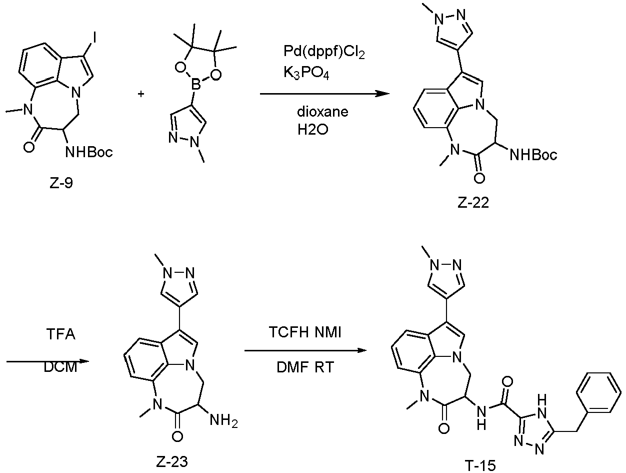

### Step 1: preparation of Z-22

1-methyl-1H-pyrazol-4-boronic acid pinacol ester (1.5eq), Z-9 (1eq), Pd(dppf)Cl₂ (0.1eq), and K₃PO₄ were dissolved in a mixture of dioxane and water. The reaction mixture was stirred under argon protection at 95°C overnight. After cooling to rt, the mixture was poured into water and extracted with EA. The extract was washed three times with water and concentrated. The residue was purified by SGCC to give intermediate Z-22.

### Step 2: preparation of Z-23

Z-22 (1eq) and trifluoroacetic acid (10eq) were dissolved in dichloromethane, and then the mixture was stirred at room temperature for 3 hours. The reaction mixture was concentrated and the residue was purified by SGCC to give intermediate Z-23.

MS ESI: m/z=296, [M+H]⁺.

### Step 3: preparation of T-15

Using Acid-1 (1.5eq) and Z-23 (1eq) as starting materials, T-15 was prepared under the same conditions as in Example 2.

¹H NMR (400 MHz, CDCl₃) δ 7.73 (s, 1H), 7.60 (m, 2H), 7.32 (m, 4H), 7.23 - 7.07 (m, 4H), 4.91 (m, 1H), 4.65 (m, 1H), 4.34 (m, 1H), 4.20 (s, 2H), 3.98 (s, 3H), 3.63 (s, 3H).

MS ESI: m/z= 481, [M+H]⁺.

### Example 17: T-16

### (±)-5-benzyl-N-(1-methyl-7-(1-methyl-1H-pyrazol-5-yl)-2-oxy-1,2,3,4-tetrahydro-[1,4] diazepino [3,2,1-hi] indol-3-yl)-4H-1,2,4-triazole-3-carboxamide

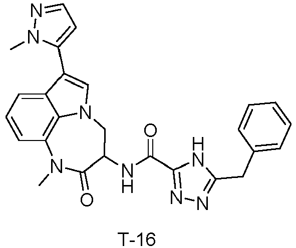

Using 1-methyl-1H-pyrazol-5-boronic acid pinacol ester and Z-9 as starting materials, T-16 was prepared under the same conditions as in Example 16.

¹H NMR (400 MHz, CDCl₃) δ 8.65 (s, 1H), 7.62 (s, 1H), 7.43 (m, 1H), 7.22-7.14 (m,8H), 6.41 (m, 1H), 4.97 - 4.91 (m, 1H), 4.71 (m, 1H), 4.40 - 4.33 (m, 1H), 4.19 (s, 2H), 3.87 (s, 3H), 3.64 (s, 3H).

MS ESI: m/z=481, [M+H]⁺.

### Example 18: T-17

### (t)-5-benzyl-N-(1-methyl-2-oxo-7-(pyridin-3-yl)-1,2,3,4-tetrahydro-[1,4] diazepino [3,2,1-hi] indol-3-yl)-4H-1,2,4-triazole-3-carboxamide

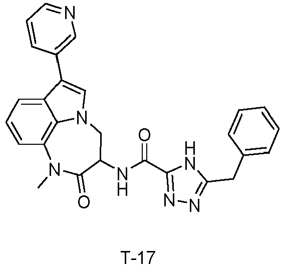

Using 3-pyridineboronic acid pinacol ester and Z-9 as starting materials, T-17 was prepared under the same conditions as in Example 16.

¹H NMR (400 MHz, CDCl₃) δ 8.88 (m, 1H), 8.64 (s, 1H), 8.56 (m, 1H), 7.93 (d, J = 7.9 Hz, 1H), 7.71 (m, 1H), 7.42 - 7.30 (m, 7H), 7.14 (d, J = 7.9 Hz, 1H), 4.99 (m, 1H), 4.75 (m, 1H), 4.45 - 4.36 (m, 1H), 4.20 (s, 2H), 3.66 (s, 3H).

MS ESI: m/z=478, [M+H]⁺.

### Example 19: T-18

### (±)-5-benzyl-N-(1-methyl-7-(1-methyl-3-(trifluoromethyl)-1H-pyrazol-5-yl)-2-oxo-1,2,3,4-tetrahydro-[1,4] diazepino [3,2,1-hi] indol-3-yl)-4H-1,2,4-triazole-3-carboxamide

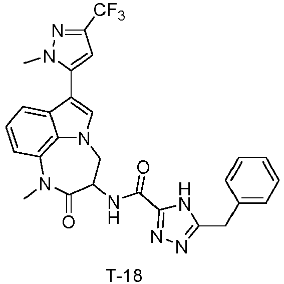

Starting from 1-methyl-3-trifluoromethylpyrazole-5-boronic acid pinacol ester and Z-9, T-18 was prepared under the same conditions as in example 16.

¹H NMR (400 MHz, CDCl₃) δ 8.65 (s,1H), 7.42 (d, J = 7.9 Hz, 1H), 7.38 - 7.29 (m, 5H), 7.24 (s, 1H), 7.17 (d, J = 7.8 Hz, 1H), 6.65 (s, 1H), 5.01 (m, 1H), 4.75 (m, 1H), 4.40 (m, 1H), 4.21 (s, 2H), 3.93 (s, 3H), 3.67 (s, 3H).

MS ESI: m/z= 549, [M+H]⁺.

### Example 20: preparation of intermediate Y-8

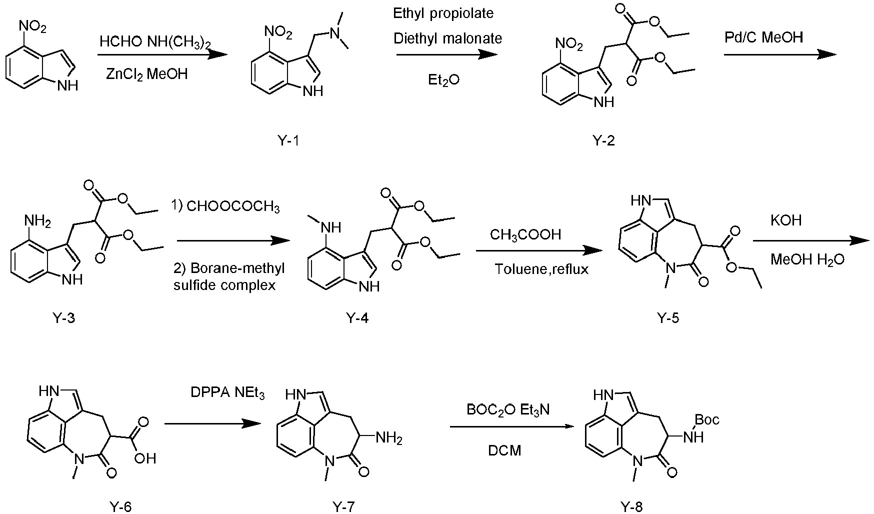

### Step 1: preparation of Y-1

A mixture of 4-nitroindole (4,000mg), NH(CH₃)₂ (13.5 8mL, 2mol/l in MeOH), HCHO (2.2mL, 37%), ZnCl₂ (5,048mg) in 10mL methanol was stirred at rt for 2 hours, and additional NH(CH₃)₂ (2.46mL) and HCHO (0.5mL) was added. After stirring for 4 hours, NaOH solution was added to adjust the pH to ~ 13. EA was then added and filtered. The filtrate was extracted using EA, the extract was dried over anhydrous sodium sulfate, and concentrated to obtain a yellow solid which was swished from 10:1 of PE/EA to obtain 4,2g of Y-1.

MS ESI: m/z=220, [M+1]⁺.

### Step 2: preparation of Y-2

Y-1 (4,250mg), diethyl malonate (2.94mL), ethyl propiolate (1.96mL), and anhydrous ether (97mL) were mixed at 0 °C under N₂. After stirring at rt for 3 hours, additional diethyl malonate (0.59mL) and ethyl propiolate (1.96mL) were added. The mixture was stirred for additional 4 hours, and was quenched with 1N HCl. Extraction was conducted with EA and the extract was dried over anhydrous sodium sulfate and concentrated. The residue was purified by SGCC to give 5.47g of Y-2 as a yellow solid.

MS ESI: m/z=335, [M+1]⁺.

### Step 3: preparation of Y-3

A solution of Y-2 (5,470mg), Pd/C (3,530mg, 5%) in MeOH (78mL) was stirred at rt under hydrogen atmosphere for 2 hours. The reaction mixture was then filtered through celite and concentrated to give 5.04g of Y-3 as a yellow-brown oil.

MS ESI: m/z=305, [M+1]⁺.

### Step 4: preparation of Y-4

Formic acid (3.1mL) and acetic anhydride (1.9mL) were mixed and stirred at rt for 20 minutes to prepare formic acetic mixed anhydride, which was then added into a solution of Y-3 (4,900mg) in dichloromethane (16mL) at 0 °C. After the mixture was stirred at rt for 3 hours, additional of the mixed anhydride prepared from 1mL of formic acid and 0.6mL of acetic anhydride was added. The mixture was then stirred for another 2 hours and concentrated to give a solid which was dissolved in 64 mL of THF and treated with a solution of borane-methyl sulfide complex (16.2mL, 2mol/l in THF) at 0 °C. The mixture was stirred at rt overnight and then quenched with dilute hydrochloric acid. Extraction was conducted with EA, the extract was dried over Na₂SO₄ and concentrated. The residue was purified by SGCC to give 4.87g of Y-4 as a pale-green oil.

MS ESI: m/z=319, [M+1]⁺.

### Step 5: preparation of Y-5

A mixture of Y-4 (2 g), TFA (10mL), and toluene (52mL) was refluxed for 26 hours under nitrogen atmosphere. The reaction mixture was then concentrated, and the residue was purified by SGCC to give 1.2g of Y-5.

MS ESI: m/z=273, [M+1]⁺.

### Step 6: preparation of Y-6

A mixture of Y-5 (1,225mg), KOH (1,010mg), water (1.6mL), and MeOH (10.5mL) was stirred at rt for 2.5 hours. The reaction mixture was acidified with 1N HCl and extracted with EA. The organic phase was dried over anhydrous sodium sulfate, and concentrated to give 1.2 g of Y-6 as a yellowish-brown soli.

MS ESI: m/z=245, [M+1]⁺.

### Step 7: preparation of Y-7

A mixture of Y-6 (500mg), DPPA (845.3mg), DIEA (399.6mg), and toluene (10mL) was stirred at 85°C under N₂ for 2 hour. A solution of 1.25N NaOH (10mL) was added, and was then stirred overnight. The reaction mixture was acidified with hydrochloric acid, and washed away with EA. The aqueous phase was basified with NaOH solution, extracted with EA. The extract was dried over Na₂SO₄ and concentrated to give 274.2mg of Y-7 as a pale-yellow solid.

MS ESI: m/z=216, [M+1]⁺.

### Step 8: preparation of Y-8

A mixture of Y-7 (224mg), BoczO (340.7mg), NEt₃ (315.1mg), and THF (5mL) was stirred at rt for 6 hours. The mixture was concentrated and a small amount of EA was added to dissolve the solid. PE was then added to precipitate the product, and the solid was collected by filtration to give 285.7mg of Y-8 as a yellow solid. Using Daicel CHIRALPAK IG column, the product was eluted with the ratio of ethanol/n-hexane =25%. The result showed that the first peak corresponded to the S configuration, while the second peak corresponded to the R configuration.

MS ESI: m/z=314, [M-1]⁻.

### Example 21: T-19

### (t)-5-benzyl-N-(1-methyl-2-oxo-2,3,4,6-tetrahydro-1H-azacyclo [4,3,2-cd] indol-3-yl)-4H-1,2,4-triazole-3-carboxamide

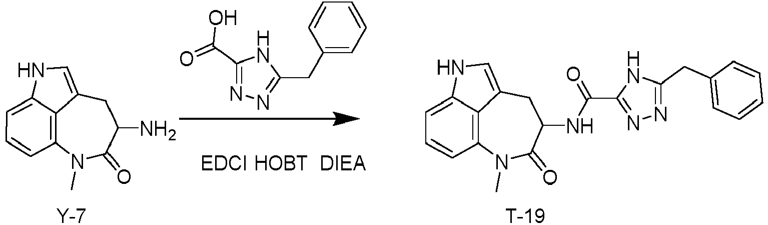

A mixture of Y-7 (20mg), 5-benzyl-4H-1,2,4-triazole-3-carboxylic acid (18.9mg), EDCI (23.4mg), HOBT (16.6mg), DIEA (22.8ul), and anhydrous DCM (2mL) were sealed in a 10mL reaction tube under nitrogen, and stirred at rt overnight. The reaction was quenched with saturated NaHCO₃ solution, extracted with EA. The extract was concentrated and the residue was purified by SGCC to give the crude product which was purified by HPLC [mobile phase: A: H₂O (+0.1% TFA); B: MeCN; separation condition: 50%B; flow rate: 18mL/min; chromatographic column: Waters Xterra^{®} Prep MSC18, 19*250mm, 10µm] to obtain 5mg of Y-7 as a white solid. Starting from optically pure Y-7, S-T-19 and R-T-19 were prepared using the same method.

¹H NMR (400 MHz, DMSO-d₆) δ 14.37 (s, 1H), 11.19 (s, 1H), 8.67 (s, 1H), 7.39 - 7.08 (m, 8H), 6.95 (d, J = 7.6 Hz, 1H), 4.65 - 4.54 (m, 1H), 4.13 (s, 2H), 3.50 (s, 3H), 3.24 (m, 1H), 3.09 - 2.93 (m, 1H).

MS ESI: m/z=401, [M+1]⁺.

### Example 22: T-20

### (t)-5-benzyl-N-(1,6-dimethyl-2-oxo-2,3,4,6-tetrahydro-1H-azacyclo [4,3,2-cd] indol-3-yl)-4H-1,2,4-triazole-3-carboxamide

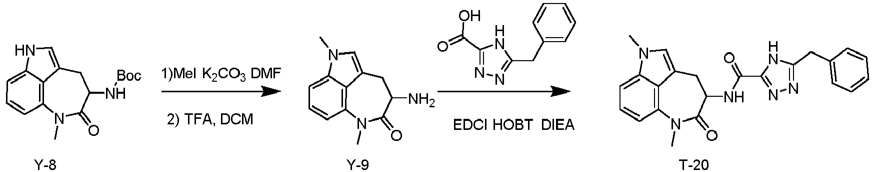

### Step 1: preparation of Y-9

A mixture of Y-8 (29mg), K₂CO₃ (15.2mg), MeI and DMF (1mL) was stirred under N₂ at rt overnight. Additional Mel and K₂CO₃ ware added until the reaction was complete. TFA was added to quench the reaction, and the solvent was evaporated. DCM (2mL) and TFA (0.4mL) were added into the residue, and the mixture was stirred overnight to remove the BOC. Then the mixture was concentrated to give the crude Y-9, which was directly used in the next step.

MS ESI: m/z=230, [M+1]⁺.

### Step 2: preparation of T-20

Y-9 was used as the starting material to obtain 10mg of T-20 as a white solid under the same conditions as in Example 21.

¹H NMR (400 MHz, DMSO-d₆) δ 14.59 (s, 1H), 8.71 (m, 1H), 7.43 - 7.07 (m, 8H), 6.99 (d, J = 7.5 Hz, 1H), 4.57 (m,1H), 4.16 (s, 2H), 3.78 (s, 3H), 3.47 (s, 3H), 3.22 (m,1H), 3.01 (m, 1H).

MS ESI: m/z=415, [M+1]⁺.

### Example 23: T-21

### (t)-5-benzyl-N-(1-methyl-2-oxo-6-(benzenesulfonyl)-2,3,4,6-tetrahydro-1H-aza [4,3,2-cd] indol-3-yl)-4H-1,2,4-triazole-3-carboxamide

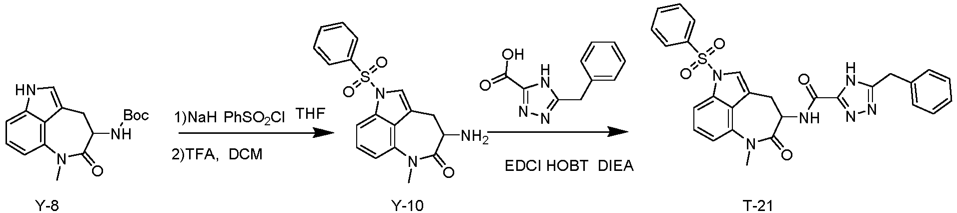

### Step 1: preparation of Y-10

A mixture of Y-8 (29mg), NaH (4.4mg, 60%) and THF (1mL) was stirred for a few minutes, and then treated with PhSO₂Cl (35.52mg). After stirring for 2 hours, additional NaH (10mg) was added and the mixture was stirred for 1 hour. The mixture was washed with saturated NaHCO₃ solution and NH₄Cl solution twice respectively, and the organic phase was dried with anhydrous Na₂SO₄ and concentrated. The residue was dissolved in DCM (1.6mL) and treated with TFA (0.4mL). After stirring for 2 hours, the reaction mixture was concentrated to obtain Y-10.

MS ESI: m/z=356, [M+1]⁺.

### Step 2: preparation of T-21

Y-10 was used as the starting material to obtain 15.7mg of white solid under the same conditions as in Example 21.

¹H NMR (400 MHz, DMSO-d₆) δ 14.39 (s, 1H), 8.69 (s, 1H), 8.06 - 7.96 (m, 2H), 7.83 - 7.76 (m, 2H), 7.72 m, 1H), 7.62 m, 2H), 7.44 (m, 1H), 7.38 - 7.12 (m, 6H), 4.54 (m,1H), 4.13 (s, 2H), 3.43 (s, 3H), 3.23 (m,1H), 3.13 - 2.78 (m, 1H).

MS ESI: m/z=541, [M+1]⁺.

### Example 24: T-22

### (±)-5-benzyl-N-(6-(cyclopropylmethyl)-1-methyl-2-oxo-2,3,4,6-tetrahydro-1H-azacyclo [4,3,2-cd] indol-3-yl)-4H-1,2,4-triazole-3-carboxamide

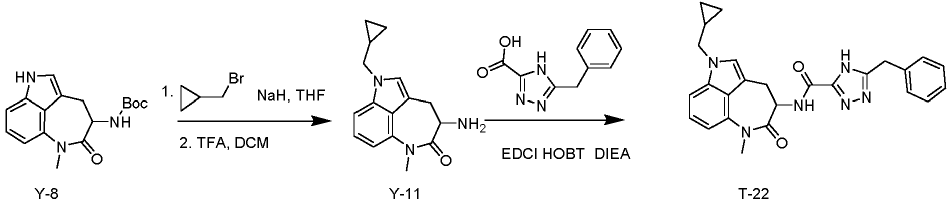

### Step 1: preparation of Y-11

A mixture of Y-8 (29mg), NaH (7.4mg, 60%) and DMF (1mL) was stirred for a few minutes, and then treated with (bromomethyl)cyclopropane (14.9mg). After stirring for 3 hours, the reaction mixture was washed with saturated NH₄Cl solution twice, and the organic phase was dried with anhydrous Na₂SO₄, concentrated to obtain 44.5mg of an intermediate which was dissolved in DCM (0.9mL) and TFA (0.3mL. After the mixture was stirred at rt for 4 hours, the mixture was then concentrated to obtain Y-11.

MS ESI: m/z=270, [M+1]⁺.

### Step 2: preparation of T-22

Y-11 was used as the starting material to obtain 17.8mg of pale-yellow solid under the same conditions as in Example 21.

¹H NMR (400 MHz, CDCl₃) 8.75 (d, *J* = 7.0 Hz, 1H), 7.38 - 7.12 (m, 7H), 7.04 (s, 1H), 6.90 (d, *J* = 7.5 Hz, 2H), 4.98 - 4.75 (m, 1H), 4.16 (s,2H), 3.99 (dd, *J* = 14.3, 6.7 Hz, 1H), 3.87 (dd, *J* = 14.3, 6.7 Hz, 1H), 3.61 (s, 3H), 3.39 (m, 1H), 3.18 - 3.00 (m, 1H), 1.33 - 1.11 (m, 1H), 0.64 (m, 2H), 0.37 (m, 2H).

MS ESI: m/z=455, [M+1]⁺.

### Example 25: T-23

### (t)-5-benzyl-N-(6-cyclopropyl-1-methyl-2-oxo-2,3,4,6-tetrahydro-1H-azacyclo [4,3,2-cd] indol-3-yl)-4H-1,2,4-triazole-3-carboxamide

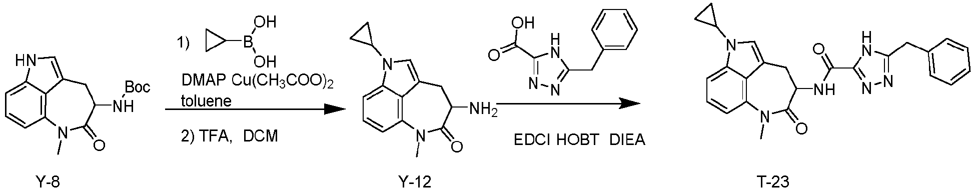

### Step 1: preparation of Y-12

A mixture of Y-8 (29mg), cyclopropylboronic acid (9.5mg), DMAP (33mg), Cu(AcO)₂ (20mg), and toluene (1mL) was stirred at 100°C under N₂ for 19 hours. Then, additional cyclopropylboronic acid (15mg) and Cu(AcO)₂ (20mg) were added and the mixture was stirred for another 17 hours. The mixture was washed with saturated NaHCO₃ solution, and the organic phase was dried over anhydrous Na₂SO₄, and concentrated. The residue was purified by SGCC to give 20mg of intermediate, which was dissolved in DCM (0.9mL) and TFA (0.3mL). The mixture was stirred at rt for 4 hours and then concentrated to obtain Y-12.

MS ESI: m/z=256, [M+1]⁺.

### Step 2: preparation of T-23

Y-12 was used as the starting material to obtain 10.8mg of T-23 as a white solid under the same conditions as in Example 21.

¹H NMR (400 MHz, CDCl₃) δ 11.75 (s, 1H), 8.72 (d, J = 6.6 Hz, 1H), 7.36 (m, 1H), 7.32 - 7.29 (m, 4H), 7.25 - 7.20 (m, 2H), 6.94 (s, 1H), 6.91 (m, 1H), 4.96 - 4.76 (m, 1H), 4.17 (s, 2H), 3.60 (s, 3H), 3.36 m,1H), 3.33 - 3.27 (m, 1H), 3.08 (m, 1H), 1.06 (m,2H), 1.02 - 0.95 (m, 2H).

MS ESI: m/z=441, [M+1]⁺.

### Example 26: T-24

### (t)-5-benzyl-N-(1-methyl-2-oxo-6-phenyl-2,3,4,6-tetrahydro-1H-azacyclo [4,3,2-cd] indol-3-yl)-4H-1,2,4-triazole-3-carboxamide

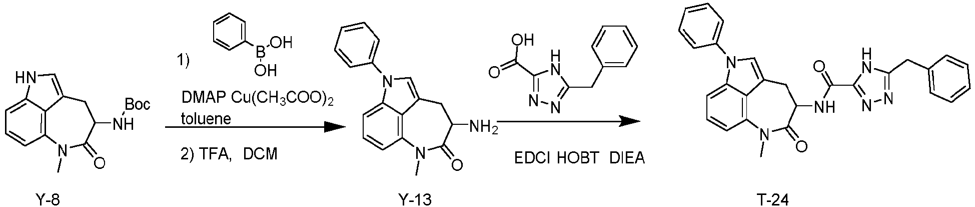

### Step 1: preparation of Y-13

A mixture ofY-8 (29mg), phenylboronic acid (13.4mg), DMAP (33mg), Cu(AcO)₂ (20mg) in toluene (1mL) was stirred at 100°C under N₂ for 6 hours. Additional phenylboronic acid (13.4mg) and Cu(AcO)₂ (20mg) were added. After stirring for 17 hours, the mixture was washed with saturated NaHCO₃ solution, and the organic phase was dried over anhydrous Na₂SO₄, concentrated. The residue was purified by SGCC to give 10mg of intermediate which was dissolved in DCM (0.9mL) and TFA (0.3mL). After stirring at room temperature for 4 hours, the reaction mixture was concentrated to obtain Y-13.

MS ESI: m/z=292, [M+1]⁺.

### Step 2: preparation of T-24

Y-13 was used as the starting material to obtain 6.2mg of white solid under the same conditions as in Example 21.

¹H NMR (400 MHz, CDCl₃) δ 8.78 (d, J = 5.7 Hz, 1H), 7.51-7.. 10 (m, 13H), 6.98 (dd, J = 15.8, 6.4 Hz, 1H), 5.02 - 4.84 (m, 1H), 4.20 (s,2H), 3.64 (s, 3H), 3.47 (m, 1H), 3.10 m, 1H).

MS ESI: m/z=477, [M+1]⁺.

### Example 27: T-25

### (t)-5-benzyl-N-(6-(cyanomethyl)-1-methyl-2-oxo-2,3,4,6-tetrahydro-1H-azacyclo [4,3,2-cd] indol-3-yl)-4H-1,2,4-triazole-3-carboxamide

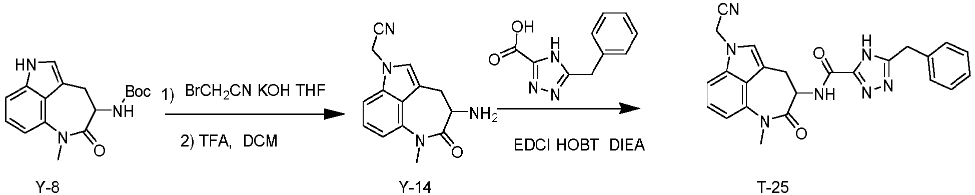

### Step 1: preparation of Y-14

A mixture of Y-8 (15mg), KOH (5.1mg), and THF (0.5mL) was stirred at room temperature for several minutes and then treated with BrCH₂CN (7.2mg). After stirring for 2 hours, additional KOH and BrCH₂CN were added until TLC indicated that the reaction was complete. The reaction was quenched with saturated NH₄Cl solution, and extracted with EA. The organic phase was dried over anhydrous Na₂SO₄ and concentrated. The residue was purified by SGCC to give 14mg of intermediate which was dissolved in DCM (0.9mL) and TFA (0.3mL). After stirring for 2 hours, the mixture was concentrated to obtain Y-14.

MS ESI: m/z=255, [M+1]⁺.

### Step 2: preparation of T-25

Y-14 was used as the starting material to obtain 3.9mg of white solid under the same conditions as in example 21.

¹H NMR (400 MHz, acetone-d₆) δ 13.36 (s, 1H), 8.66 (d, *J* = 5.0 Hz, 1H), 7.46 - 7.20 (m, 8H), 7.11 (d, *J* = 7.7 Hz, 1H), 5.55 - 5.37 (m, 2H), 4.86 - 4.62 (m, 1H), 4.20 (s, 2H), 3.60 (s, 3H), 3.41 m, 1H), 3.01 (m, 1H).

MS ESI: m/z=440, [M+1]⁺.

### Example 28: T-26

### (t)-5-benzyl-N-(6-(2-fluoroethyl)-1-methyl-2-oxo-2,3,4,6-tetrahydro-1H-azacyclo [4,3,2-cd] indol-3-yl)-4H-1,2,4-triazole-3-carboxamide

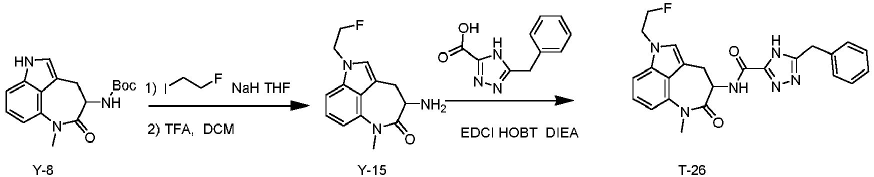

### Step 1: preparation of Y-15

A mixture of Y-8 (30mg), 1-fluoro-2-iodoethane (62.6mg), NaH (14.4mg, 60%), and THF (1mL) was stirred at room temperature under N₂ for 13 hours. Additional NaH (20mg) was added and the mixture was stirred for another 4 hours. The mixture was quenched with saturated NH₄Cl solution, and extracted with EA. The organic phase was dried over anhydrous Na₂SO₄, and concentrated. The residue was purified by SGCC to give 22.5mg of intermediate which was dissolved in DCM (2mL) and TFA (0.5mL). After stirring at room temperature for 2 hours, the mixture was then concentrated to obtain Y-15.

MS ESI: m/z=262, [M+1]⁺.

### Step 2: preparation of T-26

Y-15 was used as the starting material to obtain 8.8mg of white solid under the same conditions as in Example 21.

¹H NMR (400 MHz, CDCl₃) δ 12.62 (s, 1H), 8.74 (d, *J* = 6.7 Hz, 1H), 7.33- 7.07 (m, 6H), 6.91 (d, *J* = 7.7 Hz, 1H), 6.88 (s, 1H), 4.95 - 4.80 (m, 1H), 4.72 (m, 1H), 4.60 (m, 1H), 4.33 (m, 2H), 4.17 (s, 2H), 3.61 (s, 3H), 3.45 - 3.31 (m, 1H), 3.22 - 2.97 (m, 1H).

MS ESI: m/z=447, [M+1]⁺.

### Example 29: T-27

### (t)-5-benzyl-N-(6-(2-cyanopropyl)-1-methyl-2-oxo-2,3,4,6-tetrahydro-1H-azacyclo [4,3,2-cd] indol-3-yl)-4H-1,2,4-triazole-3-carboxamide

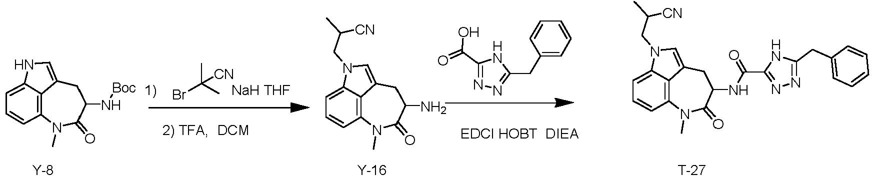

### Step 1: preparation of Y-16

A mixture ofY-8 (30mg), 2-bromo-2-methylpropanenitrile (140.6mg), NaH (16mg, 60%), and THF (2mL) was stirred at 70°C for 2 hours. Additional 2-bromo-2-methylpropionitrile (109mg) was added and the mixture was stirred for another 5 hours. The mixture was washed with saturated NH₄Cl solution, and extracted with EA. The organic phase was dried over anhydrous Na₂SO₄, and concentrated. The residue was purified by SGCC to give 21mg of intermediate which was dissolved in DCM (2mL) and TFA(0.5mL). After stirring at room temperature for 2 hours, the reaction mixture was then concentrated to obtain Y-16.

MS ESI: m/z=283, [M+1]⁺.

### Step 2: preparation of T-27

Y-16 was used as the starting material to obtain 4mg of white solid under the same conditions as in Example 21.

¹H NMR (400 MHz, CDCl₃) δ 8.72 (d, *J* = 6.3 Hz, 1H), 7.34 -.24 (6H), 7.12 (m, 1H), 6.93 (m, 2H), 4.94 - 4.77 (m, 1H), 4.43 (m, 0.5H), 4.26 (m 1H), 4.17 (s, 2H), 4.15 - 4.05 (m, 0.5H), 3.61 (s, 3H), 3.38 (m, 1H), 3.20 - 2.97 (m, 2H), 1.33 (dd, *J* = 22.6, 7.1 Hz, 3H).

MS ESI: m/z=468, [M+1]⁺.

### Example 30: T-28

### (t)-5-benzyl-N-(6-(2-methoxyethyl)-1-methyl-2-oxo-2,3,4,6-tetrahydro-1H-azacyclo [4,3,2-cd] indol-3-yl)-4H-1,2,4-triazole-3-carboxamide

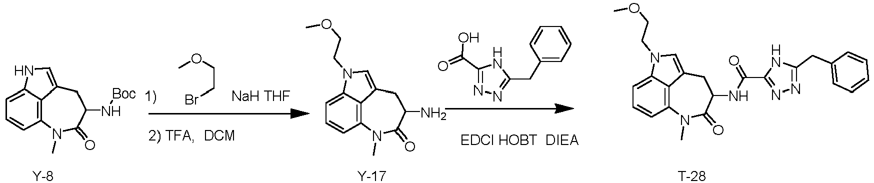

### Step 1: preparation of Y-17

A mixture of Y-8 (30mg), 1-bromo-2-methoxyethane (36ul), NaH (15.2mg, 60%), and THF (1mL) was stirred at room temperature overnight. Additional 1-bromo-2-methoxyethane (40ul) was added and mixture was stirred for another 20 hours. The reaction mixture was then quenched with saturated NH₄Cl solution and extracted with EA. The organic phase was dried over anhydrous Na₂SO₄, and concentrated. The residue was purified by SGCC to give 13mg of intermediate. The reaction was repeated under similar conditions again to obtain a total of 26mg of intermediate. The combined intermediate was dissolved in DCM (2mL) and TFA (0.5mL. After stirring at room temperature for 2 hours, the reaction mixture was then concentrated to obtain Y-17.

MS ESI: m/z=274, [M+1]⁺.

### Step 2: preparation of T-28

Y-17 was used as the starting material to obtain 5mg of white solid under the same conditions as in Example 21.

¹H NMR (400 MHz, CDCl₃) δ 8.73 (d, *J=* 6.0 Hz, 1H), 7.25 - 7.09 (m, 7H), 6.91 (s, 1H), 6.87 (d, *J* = 7.6 Hz, 1H), 4.87 - 4.75 (m, 1H), 4.30 - 4.17 (m, 2H), 4.14 (s, 2H), 3.66 (t, *J* = 5.4 Hz, 2H), 3.58 (s, 3H), 3.32 (m, 1H), 3.30 (s, 3H), 3.12 - 2.98 (m, 1H).

MS ESI: m/z=459, [M+1]⁺.

### Example 31: T-29

### (±)-methyl2-(3-(5-benzyl-4H-1,2,4-triazole-3-carboxamide)-1-methyl-2-oxo-1,2,3,4-tetrahydro-6H-azacyclo [4,3,2-cd] indol-6-yl)acetate

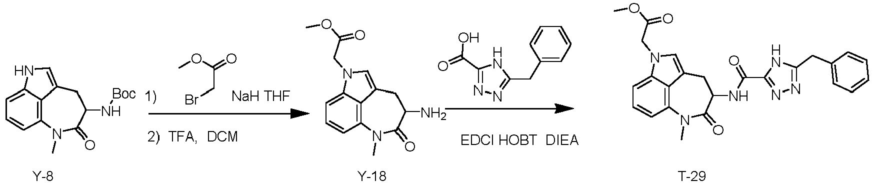

### Step 1: preparation of Y-18

A mixture of Y-8 (63mg), NaH (32mg, 60%), and THF (1mL) was stirred for a few minutes and then treated with methyl 2-bromoacetate (306mg). After stirring at room temperature overnight, the reaction mixture was quenched with saturated NH₄Cl solution and extracted with EA. The organic phase was dried over anhydrous Na₂SO₄, and concentrated. The residue was purified by SGCC to give 51mg of intermediate which was dissolved in DCM (2mL) and TFA (0.5mL). After stirring at room temperature for 2 hours, the reaction mixture was then concentrated to obtain Y-18.

MS ESI: m/z=288, [M+1]⁺.

### Step 2: preparation of T-29

Y-18 was used as the starting material to obtain 30mg of white solid under the same conditions as in Example 21.

¹H NMR (400 MHz, CDCl₃) δ 13.47 (s, 1H), 8.75 (d, *J* = 6.7 Hz, 1H), 7.25- 7.16 (m,6H), 7.02 (d, *J* = 8 Hz, 1H), 6.91 (d, *J* = 8 Hz, 1H), 6.69 (m, 1H), 4.85 (m, 1H), 4.79 (d, *J* = 17.7 Hz, 1H), 4.65 (d, *J* = 17.7 Hz, 1H), 4.17 (s, 2H), 3.73 (s, 3H), 3.59 (s, 3H), 3.43 - 3.30 (m, 1H), 3.15 - 2.97 (m, 1H).

MS ESI: m/z=473, [M+1]⁺.

### Example 32: T-30

### (t)-2-(3-(5-benzyl-4H-1,2,4-triazole-3-carboxamide)-1-methyl-2-oxo-1,2,3,4-tetrahydro-6H-azacyclo [4,3,2-cd] indol-6-yl)acetic acid

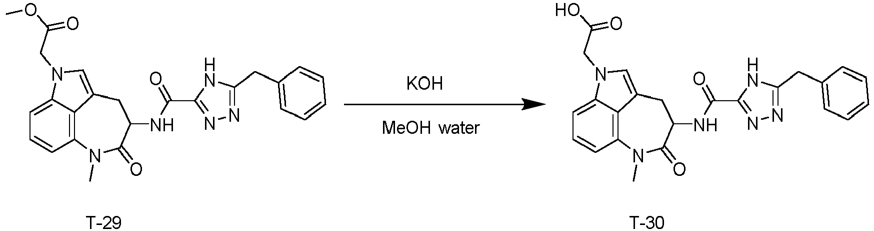

A mixture of T-29 (8mg), KOH (4.9mg), MeOH (0.5mL) and water (0.1mL) was stirred at room temperature for about 18 hours, and then quenched with dilute HCl until it was acidic. The mixture was concentrated and the residue was purified by HPLC (mobile phase: A: H₂O (+0.1% TFA); B: MeCN; separation condition: 50%B; flow rate: 18mL/min; chromatographic column: Waters Xterra^{®} Prep MSC18, 19*250mm, 10µm) to obtain 5mg of T-30 as a white solid.

¹H NMR (400 MHz, DMSO-d₆) δ 14.49 (s, 1H), 8.68 (s, 1H), 7.40 - 7.13 (m, 8H), 6.99 (d, *J=* 7.2 Hz, 1H), 4.95 (s, 2H), 4.69 - 4.52 (m, 1H), 4.13 (s, 2H), 3.50 (s, 3H), 3.22 (m, 1H), 3.11 - 2.93 (m, 1H).

MS ESI: m/z=459, [M+1]⁺.

### Example 33: T-31

### (t)-N-(6-(2-amino-2-oxoethyl)-1-methyl-2-oxo-2,3,4,6-tetrahydro-1H-azacyclo [4,3,2-cd] indol-3-yl)-5-benzyl-4H-1,2,4-triazole-3-carboxamide

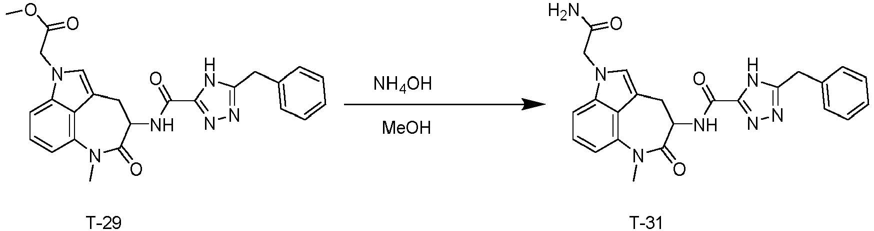

A mixture of T-29 (6mg), ammonium hydroxide (0.5mL) and MeOH (0.2mL) was stirred at room temperature for 18 hours, and then concentrated. The residue was purified by for HPLC (mobile phase: A: H₂O (+0.1% TFA); B: MeCN; separation condition: 50%B; flow rate: 18mL/min; chromatographic column: Waters Xterra^{®} Prep MSC18, 19*250mm, 10µm) to obtain 3mg of T-31 as a white solid.

¹H NMR (400 MHz, DMSO-d₆) δ 14.36 (s, 1H), 8.66 (s, 2H), 7.58 (s, 1H), 7.39 - 7.15 (m, 8H), 7.00 (dd, J = 6.8, 1.5 Hz, 1H), 4.79 (s, 2H), 4.66 - 4.57 (m, 1H), 4.13 (s, 2H), 3.50 (s, 3H), 3.22 (m, 1H), 3.10 - 2.93 (m, 1H).

MS ESI: m/z=458, [M+1]⁺.

### Example 34: T-32

### (t)-3-(2-benzyl-3-chloro-7-oxo-2,4,5,7-tetrahydro-6H-pyrazole [3,4-c] pyridin-6-yl)-1-methyl-1,3,4,6-tetrahydro-2H-azacyclo [4,3,2-cd] indole-2-one

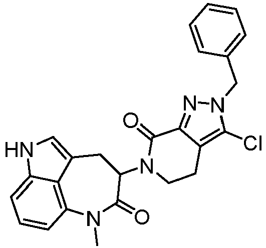

### Step 1: preparation of Y-20

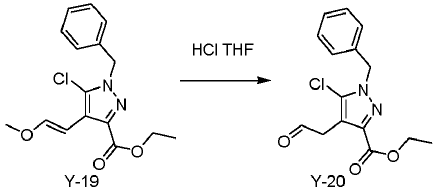

A mixture of Y-19 (32mg), THF (1mL) and HCl (1mL, 6N) was stirred at room temperature overnight. The mixture was then concentrated to give the crude Y-20.

MS ESI: m/z=307, [M+1]⁺.

### Step 2: preparation of Y-21

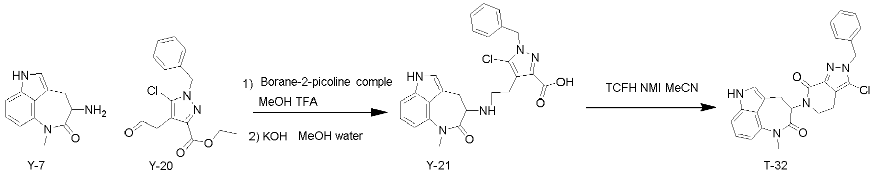

A mixture of Y-7 (18mg), Y-20 (30mg), borane-2-picoline complex (11.1mg), MeOH (1mL) and TFA (0.1mL) was stirred at room temperature for 1 day. The reaction was quenched with saturated NH₄Cl solution, extracted with EA. The organic phase was dried over anhydrous Na₂SO₄, and concentrated. The residue was purified by SGCC to give 22mg of intermediate which was dissolved in MeOH (1mL) and water (0.5mL) and treated with KOH (100mg). The mixture was then stirred at room temperature overnight, and quenched with HCl (1N), and extracted with EA. The extract was concentrated to obtain 20mg of Y-21 as a solid.

MS ESI: m/z=478, [M+1]⁺.

### Step 3: preparation of T-32

A mixture of Y-21 (20mg), TCFH (17mg), NMI (6.6mg) and MeCN (1mL) was stirred at room temperature overnight. The reaction was then quenched with saturated NH₄Cl solution, and extracted with EA. The EA extract wad concentrated and the residue was purified by HPLC (mobile phase: A: H₂O (+0.1% TFA); B: MeCN; separation condition: 50%B; flow rate: 18mL/min; chromatographic column: Waters Xterra^{®} Prep MSC18, 19*250mm, 10µm) to obtain 6mg of T-32 as a white solid.

¹H NMR (400 MHz, acetone-d₆) δ 10.33 (s, 1H), 7.40 - 7.26 (m, 5H), 7.27 - 7.13 (m, 3H), 6.95 (dd, J = 7.6, 0.7 Hz, 1H), 5.51 (dd, J = 11.5, 1.5 Hz, 1H), 5.43 (s, 2H), 4.18 - 4.05 (m, 1H), 3.85 - 3.73 (m, 1H), 3.57 (dd, J = 14.0, 7.0 Hz, 2H), 3.52 (s, 3H), 3.46 - 3.37 (m, 1H), 3.25 (m, 1H).

MS ESI: m/z=460, [M+1]⁺.

### Example 35: T-33

### (±)-5-benzyl-N-(6-(2-hydroxyethyl)-1-methyl-2-oxo-2,3,4,6-tetrahydro-1H-azacyclo [4,3,2-cd] indol-3-yl)-4H-1,2,4-triazole-3-carboxamide

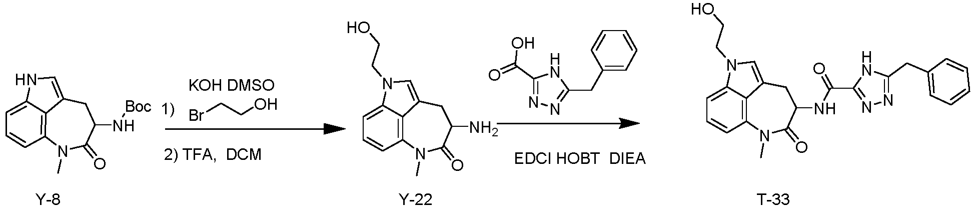

### Step 1: preparation of Y-22

A mixture of Y-8 (30mg), KOH (10mg) and DMSO (1mL) was stirred for several minutes under N₂ and treated with 2-bromoethanol (17.5mg). The mixture was then stirred at room temperature overnight and quenched with saturated NH₄Cl solution, extracted with EA. The organic phase was dried over anhydrous Na₂SO₄, and concentrated. The residue was purified by SGCC to give 19.6mg of intermediate which was dissolved in DCM (2mL) and TFA (0.5mL. After stirring at room temperature for 2 hours, the reaction mixture was then concentrated to obtain Y-22.

MS ESI: m/z=260, [M+1]⁺.

### Step 2: preparation of T-33

Y-22 was used as the starting material to obtain 2.4mg of T-33 as a white solid under the same conditions as in Example 21.

¹H NMR (400 MHz, CDCl₃) δ 8.74 (d, J = 6.0 Hz, 1H), 7.26 (m, 7H), 6.95 (s, 1H), 6.89 (d, J = 7.4 Hz, 1H), 4.82 (m, 1H), 4.20 (m, 2H), 4.14 (s, 2H), 3.91 (m, 2H), 3.60 (s, 3H), 3.34 (m, 1H), 3.14 - 2.99 (m, 1H).

MS ESI: m/z=445, [M+1]⁺.

### Example 36: T-34

### (±)-5-benzyl-N-(1-methyl-6-(1-methyl-1H-pyrazol-4-yl)-2-oxo-2,3,4,6-tetrahydro-1H-azacyclo [4,3,2-cd] indol-3-yl)-4H-1,2,4-triazole-3-carboxamide

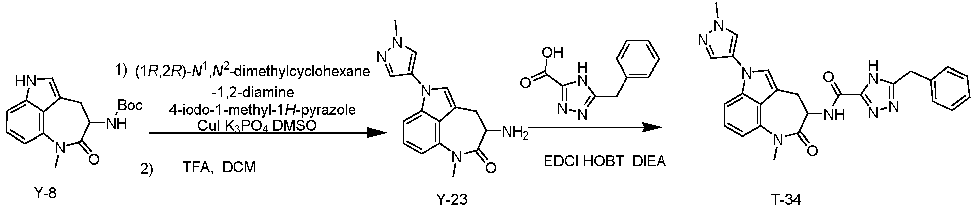

### Step 1: preparation of Y-23

A mixture of Y-8 (30mg), 1-methyl-4-iodo-pyrazole (29.7mg), trans-(1R,2R)-N,N'-dimethylcyclohexane-1,2-diamine (7.1mg), CuI (4mg), K₃PO₄ (64mg) and DMSO (1mL) was stirred at 90°C under N₂ overnight. The mixture was quenched with saturated NH₄Cl solution and extracted with EA. The organic phase was dried over and concentrated. The residue was purified by SGCC to give 25.4mg of intermediate which was dissolved in DCM (2mL) and TFA (0.5mL). After stirring at room temperature for 2 hours, the reaction mixture was then concentrated to obtain Y-23.

MS ESI: m/z=296, [M+1]⁺.

### Step 2: preparation of T-34

Y-23 was used as the starting material to obtain 4mg ofT-34 as a white solid under the same conditions as in Example 21.

¹H NMR (400 MHz, DMSO-d₆) δ 14.33 (s, 1H), 8.72 (s, 1H), 8.24 (s, 1H), 7.82 (s, 1H), 7.46 (s, 1H), 7.41 - 7.17 (m, 7H), 7.08 (d, J = 6.9 Hz, 1H), 4..68- 4.64 (m, 1H), 4.14 (s, 2H), 3.93 (s, 3H), 3.52 (s, 3H), 3.26 (m, 1H), 3.15 - 2.92 (m, 1H).

MS ESI: m/z=481, [M+1]⁺.

### Example 37: T-35

### (±)-5-benzyl-N-(1-methyl-6-((1-methyl-1H-pyrazol-4-yl)methyl)-2-oxo-2,3,4,6-tetrahydro-1H-azacyclo [4,3,2-cd] indol-3-yl)-4H-1,2,4-triazole-3-carboxamide

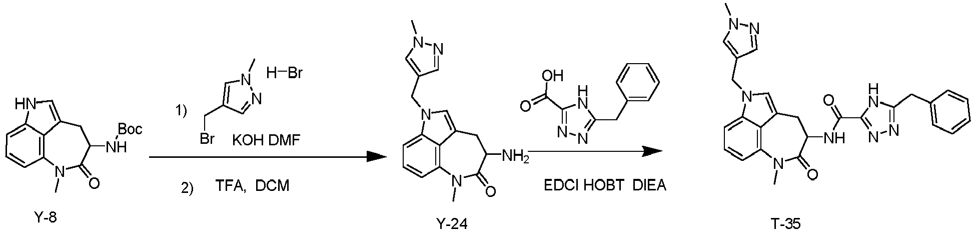

### Step 1: preparation of Y-24

A mixture of 4-(bromomethyl)-1-methyl-1H-pyrazole hydrobromide (29.3mg), KOH (16mg) and DMF (2mL) was stirred at room temperature for several minutes and then treated with Y-8 (30mg) under N₂. The mixture was stirred at room temperature for 20 hours, and additional 4-(bromomethyl)-1-methyl-1H-pyrazole hydrobromide (29mg) was then added. After the mixture was stirred at room temperature overnight, the reaction was quenched with saturated NH₄Cl solution, and extracted with EA. The organic phase was dried over and concentrated. The residue was purified by SGCC to give 15mg of intermediate which was dissolved in DCM (2mL) and TFA (0.5mL). After the mixture was stirred at room temperature for 2 hours, the reaction mixture was then concentrated to obtain Y-24.

MS ESI: m/z=310, [M+1]⁺.

### Step 2: preparation of T-35

Y-24 was used as the starting material to obtain 18mg of T-35 as a white solid under the same conditions as in Example 21.

¹H NMR (400 MHz, CDCl₃) δ 8.71 (d, J = 6.1 Hz, 1H), 7.44 (s, 1H), 7.31 (m, 4H), 7.21 (m 3H), 7.16 (m, 1H), 6.90 (m, 2H), 5.12 (m,2H), 4.92 - 4.83 (m, 1H), 4.17 (s, 2H), 3.84 (s, 3H), 3.61 (s, 3H), 3.36 (m, 1H), 3.24 - 2.77 (m, 1H).

MS ESI: m/z=495, [M+1]⁺.

### Example 38: T-36

### (±)-5-benzyl-N-(1-methyl-6-(1-methyl-1H-pyrazol-5-yl)-2-oxo-2,3,4,6-tetrahydro-1H-azacyclo [4,3,2-cd] indol-3-yl)-4H-1,2,4-triazole-3-carboxamide

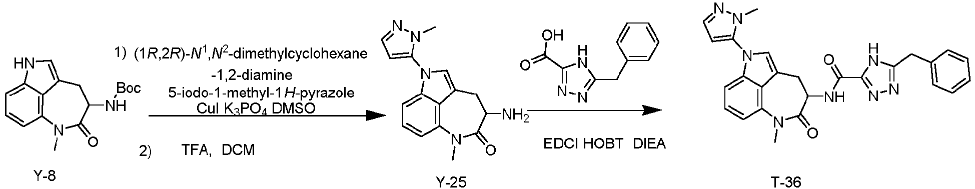

### Step 1: preparation of Y-25

Y-8 (100mg) and 5-iodo-1-methyl-1H-pyrazole were used as starting materials to obtain 22mg of solid Y-25 under the same conditions as in the Step 1 of Example 36.

MS ESI: m/z=296, [M+1]⁺.

### Step 2: preparation of T-36

Y-25 was used as the starting material to obtain 10mg of T-36 as a white solid under the same conditions as in Example 21.

¹H NMR (400 MHz, CDCl₃) δ 8.74 (d, J = 6.7 Hz, 1H), 7.62 (d, J = 1.7 Hz, 1H), 7.34 - 7.21 (m, 6H), 7.01 (d, J = 8.0Hz, 1H), 6.96 (s, 1H), 6.92 (d, J =8.0 Hz, 1H), 6.35 (d, J = 1.7 Hz, 1H), 5.03 - 4.91 (m, 1H), 4.19 (s, 2H), 3.64 (s, 6H), 3.45 (m, 1H), 3.23 - 2.95 (m, 1H).

MS ESI: m/z=481, [M+1]⁺.

### Example 39: T-37

### (±)-5-benzyl-N-(1-methyl-6-(1-methyl-1H-pyrazol-3-yl)-2-oxo-2,3,4,6-tetrahydro-1H-azacyclo [4,3,2-cd] indol-3-yl)-4H-1,2,4-triazole-3-carboxamide

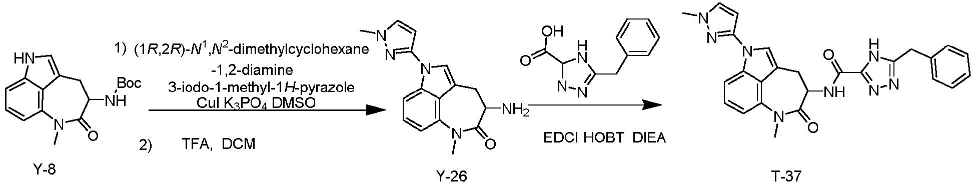

### Step 1: preparation of Y-26

Y-8 (100mg) and 3-iodo-1-methyl-1H-pyrazole were used as starting materials to obtain 13mg of Y-26 as a sold under the same conditions as in the Step 1 of Example 36.

MS ESI: m/z=296, [M+1]⁺.

### Step 2: preparation of T-37

Y-26 was used as the starting material to obtain 7.3mg of T-37 as a pale-yellow solid under the same conditions as in Example 21.

¹H NMR (400 MHz, CDCl₃) δ 8.72 (d, J = 6.8 Hz, 1H), 7.78 (d, J = 8.0 Hz, 1H), 7.41 (d, J = 1.7 Hz, 1H), 7.26~7.35(m, 7H), 6.97 (d, J = 8.0Hz, 1H), 6.31 (d, J = 1.9 Hz, 1H), 4.93 (m, 1H), 4.17 (s, 2H), 3.95 (s, 3H), 3.63 (s, 3H), 3.44 (m, 1H), 3.21 - 2.99 (m, 1H).

MS ESI: m/z=481, [M+1]⁺.

### Example 40: T-38

### (±)-5-benzyl-N-(1-methyl-6-(2-morpholinoethyl)-2-oxo-2,3,4,6-tetrahydro-1H-azacyclo [4,3,2-cd] indol-3-yl)-4H-1,2,4-triazole-3-carboxamide

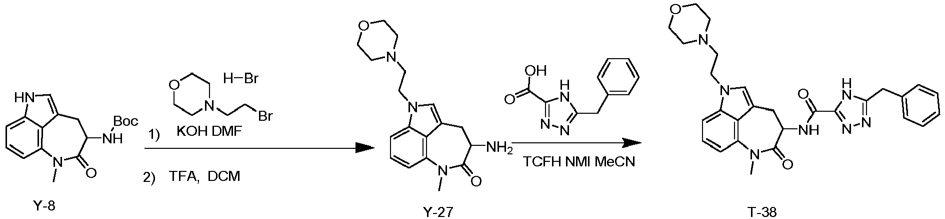

### Step 1: preparation of Y-27

Y-8 (50mg) and 4-(2-bromoethyl)morpholine hydrobromate (48mg) were used as starting materials to obtain 52mg of solid Y-27 under the same conditions as in the Step 1 of Example 37.

MS ESI: m/z=329, [M+1]⁺.

### Step 2: preparation of T-38

A mixture of Y-27 (52mg), 5-benzyl-4H-1,2,4-triazole-3-carboxylic acid (48.7mg), TCFH (65mg), NMI (40ul) and anhydrous DCM (2mL) was stirred at room temperature for 5 hours. The reaction was quenched with saturated NH₄Cl solution, and extracted with EA. The extract was concentrated and the residue was purified by SGCC. The product was further purified by HPLC (mobile phase: A: H2O (+0.1% TFA); B: MeCN; separation condition: 60%B; flow rate: 18 mL/min; chromatographic column: Waters Xterra^{®} Prep MSC18, 19*250mm, 10µm) to obtain 20mg ofT-38 as a white solid.

¹H NMR (400 MHz, CDCl₃) δ 8.75 (d, J = 6.8 Hz, 1H), 7.26 - 7.09 (m, 7H), 6.90-6088 (m,2H), 4.93 - 4.72 (m, 1H), 4.32 - 3.98 (m, 4H), 3.78 - 3.66 (m, 4H), 3.60 (s, 3H), 3.41 (m, 1H), 3.23 - 2.99 (m, 1H), 2.70 (t, J = 7.0 Hz, 2H), 2.48 (m, 4H).

MS ESI: m/z=514, [M+1]⁺.

### Example 41: T-39

### (±)-5-benzyl-N-(1-methyl-2-oxo-6-pivaloyl-2,3,4,6-tetrahydro-1H-azacyclo [4,3,2-cd] indol-3-yl)-4H-1,2,4-triazole-3-carboxamide

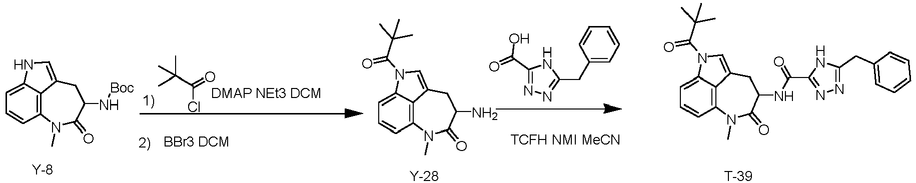

### Step 1: preparation of Y-28

A mixture of Y-8 (50mg), DCM (2mL), pivaloyl chloride (100ul), DMAP (12.5mg) and triethylamine (144ul) was stirred at room temperature overnight, The reaction was quenched with NaHCOs, and extracted with EA. The organic phase was then dried over anhydrous Na₂SO₄, and concentrated. The residue was purified by SGCC to give 64 mg of an oily intermediate. 24mg of intermediate was dissolved in DCM (2mL) and treated with BBr₃ (0.12mL, 1N). After stirring at room temperature for 5 hours, the reaction was quenched with NaHCOs and extracted with EA. The organic phase was then dried over Na₂SO₄ and concentrated to obtain Y-28.

MS ESI: m/z=300, [M+1]⁺.

### Step 2: preparation of T-39

Y-28 was used as the starting material to obtain 3mg of T-39 as a white solid under the same conditions as in the Step 2 of Example 40.

¹H NMR (400 MHz, CDCl₃) δ 8.70 (d, J = 6.6 Hz, 1H), 8.36 (d, J = 8.0 Hz, 1H), 7.56 (s 1H), 7.39 (t, J = 8.2 Hz, 1H), 7.30 (m, 5H), 7.09 (d, J = 8.0 Hz, 1H), 4.98 - 4.80 (m, 1H), 4.18 (s, 2H), 3.60 (s, 3H), 3.39 (m 1H), 3.14 - 2.97 (m, 1H), 1.50 (s, 9H).

MS ESI: m/z=485, [M+1]⁺.

### Example 42: T-40

### (±)-5-benzyl-N-(1-methyl-2-oxo-6-(3-(trifluoromethyl)pyridin-2-yl)-2,3,4,6-tetrahydro-1H-azacyclo [4,3,2-cd] indol-3-yl)-4H-1,2,4-triazole-3-carboxamide

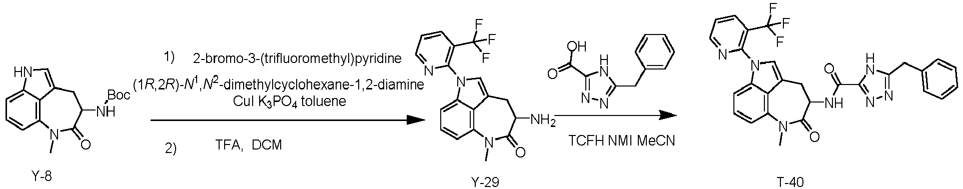

### Step 1: preparation of Y-29

A mixture ofY-8 (50mg), 2-bromo-3-(trifluoromethyl)pyridine (66.6mg), trans-(1R,2R)-N,N'-dimethyl-cyclohexane-1,2-diamine (11.4mg), CuI (15.2mg), K₃PO₄ (101.9mg) and toluene (2mL) was stirred at 110°C under N₂ for 21 hours. The mixture was quenched with saturated NH₄Cl solution and extracted with EA. The organic phase was dried over anhydrous Na₂SO₄, and concentrated. The residue was purified by SGCC to give 13.1mg of intermediate which was dissolved in DCM (2mL) and TFA (0.5mL). After the mixture was stirred at room temperature for 2 hours, the mixture was then concentrated to obtain Y-29.

MS ESI: m/z=361, [M+1]⁺.

### Step 2: preparation of T-40

Y-29 was used as the starting material to obtain 6mg of T-40 as a pale-yellow solid under the same conditions as in the Step 2 of Example 40.

¹H NMR (400 MHz, CDCl₃) δ 8.82 (d, J = 3.6 Hz, 1H), 8.70 (d, J = 6.4 Hz, 1H), 8.25 (d, J = 7.9 Hz, 1H), 7.54 (dd, J = 12.9, 5.2 Hz, 1H), 7.35~7.25 (m,5H), 7.19 (d, J = 7.9 Hz, 2H), 7.01 (d, J = 7.6 Hz, 1H), 5.10 - 4.78 (m, 1H), 4.17 (s, 2H), 3.64 (s, 3H), 3.45 (m, 1H), 3.19 (m, 1H).

MS ESI: m/z=546, [M+1]⁺.

### Example 43: T-41

### (±)-5-benzyl-N-(6-(3-methoxypyridin-2-yl)-1-methyl-2-oxo-2,3,4,6-tetrahydro-1H-azacyclo [4,3,2-cd] indol-3-yl)-4H-1,2,4-triazole-3-carboxamide

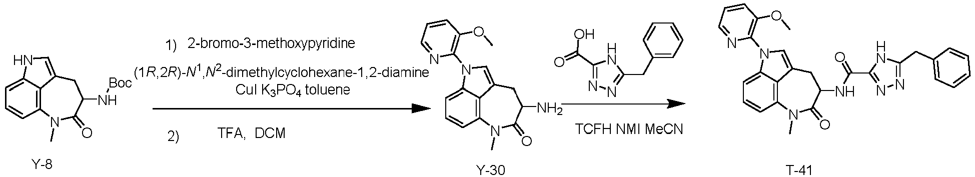

### Step 1: preparation of Y-30

Y-8 (50mg) and 2-bromo-3-methoxypyridine were used as starting materials to obtain 68.3mg of an intermediate under the same conditions as in the Step 1 of Example 42 at 120°C. 20mg of the intermediate was dissolved in DCM (2mL) and TFA (0.5mL). After the mixture was stirred at room temperature for 2 hours, the reaction mixture was then concentrated to obtain Y-30, which was directly used in the next step.

MS ESI: m/z=323, [M+1]⁺.

### Step 2: preparation of T-41

Y-30 was used as the starting material to obtain 8mg of T-41 as a white solid under the same conditions as in the Step 2 of Example 40.

¹H NMR (400 MHz, acetone-d₆) δ 8.55 (m, 1H), 8.07 - 8.03 (m, 1H), 7.61 (d, J = 8.3 Hz, 1H), 7.49 (s, 1H), 7.35 (d, J = 8.0Hz, 1H), 7.31 (dd, J = 8.3, 4.7 Hz, 1H), 7.28 - 7.17 (m, 4H), 7.14 (m 2H), 6.98 (d, J = 8.0Hz, 1H), 4.72 (m, 1H), 4.07 (s, 2H), 3.84 (s, 3H), 3.50 (s, 3H), 3.35 (m, 1H), 3.07 - 2.87 (m, 1H).

MS ESI: m/z=508, [M+1]⁺.

### Example 44: T-42

### (±)-5-benzyl-N-(6-(4-(methoxymethyl)pyridin-3-yl)-1-methyl-2-oxo-2,3,4,6-tetrahydro-1H-azacyclo [4,3,2-cd] indol-3-yl)-4H-1,2,4-triazole-3-carboxamide

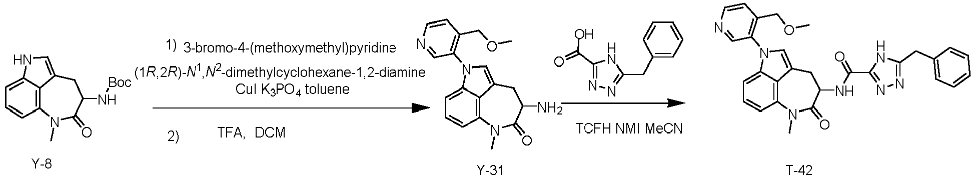

### Step 1: preparation of Y-31

Y-8 (100mg) and 3-bromo-4-(methoxymethyl)pyridine were used as starting materials to obtain 100mg of solid Y-31 under the same conditions as in the Step 1 of Example 43.

MS ESI: m/z=337, [M+1]⁺.

### Step 2: preparation of T-42

Y-31 was used as the starting material to obtain 40mg of T-42 as a white solid under the same conditions as in the Step 2 of Example 40.

¹H NMR (400 MHz, acetone-d₆) δ 13.54 (s, 1H), 8.72 (d, J = 5.0 Hz, 1H), 8.69 (d, J = 6.2 Hz, 1H), 8.63 (s, 1H), 7.68 (d, J = 4.8 Hz, 1H), 7.44 - 7.18 (m, 7H), 7.12 (d, J = 7.8 Hz, 1H), 6.89 (d, J = 8.2 Hz, 1H), 4.86 (m, 1H), 4.35 - 4.23 (m, 2H), 4.20 (s, 2H), 3.63 (s, 3H), 3.49 (m, 1H), 3.29 (s, 3H), 3.20 - 3.02 (m, 1H).

MS ESI: m/z=522, [M+1]⁺.

### Example 45: T-43

### (±)-2-(3-(5-benzyl-4H-1,2,4-triazole-3-carboxamide)-1-methyl-2-oxo-1,2,3,4-tetrahydro-6H-azacyclo [4,3,2-cd] indol-6-yl)nicotinate

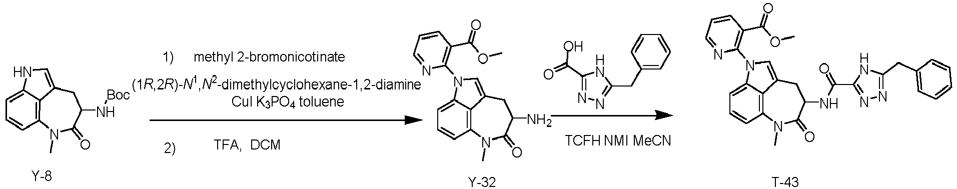

### Step 1: preparation of Y-32

Y-8 (50mg) and methyl 2-bromonicotinate were used as starting materials to obtain 56mg of solid Y-32 under the same conditions as in the Step 1 of Example 43.

MS ESI: m/z=351, [M+1]⁺.

### Step 2: preparation of T-43

Y-32 was used as the starting material to obtain 30mg of T-43 as a white solid under the same conditions as in the Step 2 of Example 40.

¹H NMR (400 MHz, acetone-d₆) δ 8.67 (dd, J = 4.8, 1.8 Hz, 1H), 8.55 (d, J = 5.6 Hz, 1H), 8.27 (dd, J = 7.8, 1.8 Hz, 1H), 7.48 (dd, J = 7.7, 4.8 Hz, 1H), 7.31 (s, 1H), 7.28 - 7.07 (m, 7H), 7.01 (d, J = 7.8 Hz, 1H), 4.71 (m, 1H), 4.07 (s, 2H), 3.53 (s, 3H), 3.50 (s, 3H), 3.37 (m, 1H), 3.05 - 2.89 (m, 1H).

MS ESI: m/z=536, [M+1]⁺.

### Example 46: T-44

### (±)-5-benzyl-N-(1-methyl-6-(3-methylpyridin-2-yl)-2-oxo-2,3,4,6-tetrahydro-1H-azacyclo [4,3,2-cd] indol-3-yl)-4H-1,2,4-triazole-3-carboxamide

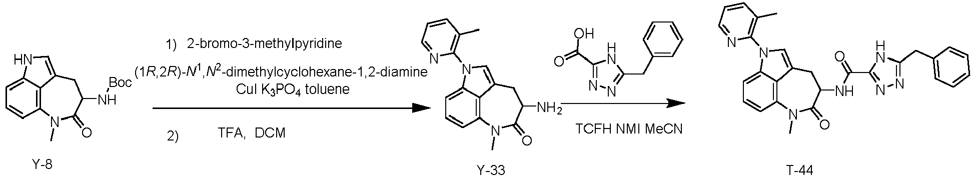

### Step 1: preparation of Y-33

Y-8 (50mg) and 2-bromo-3-methylpyridine were used as starting materials to obtain 46.3mg of Y-33 under the same conditions as in the Step 1 of Example 43.

MS ESI: m/z=307, [M+1]⁺.

### Step 2: preparation of T-44

Y-33 was used as the starting material to obtain 52.9mg of T-44 as a white solid under the same conditions as in the Step 2 of Example 40.

¹H NMR (400 MHz, acetone-d₆) δ 8.68 (d, J = 4.8 Hz, 1H), 8.47 (d, J = 3.5 Hz, 1H), 7.94 (d, J = 7.5 Hz, 2H), 7.48 (s, 1H), 7.44 (dd, J = 7.5, 4.8 Hz, 1H), 7.39 - 7.20 (m, 6H), 7.11 (d, J = 2.9 Hz, 1H), 7.09 (d, J = 3.4 Hz, 1H), 4.86 (m, 1H), 4.19 (s, 2H), 3.63 (s, 3H), 3.50 (m, 1H), 3.24 - 3.03 (m, 1H), 2.24 (s, 3H).

MS ESI: m/z=492, [M+1]⁺.

### Example 47: T-45

### (±)-5-benzyl-N-(1-methyl-8-((1-methyl-1H-pyrazol-4-yl)ethynyl)-2-oxo-2,3,4,6-tetrahydro-1H-aza [4,3,2-cd] indol-3-yl)-4H-1,2,4-triazole-3-carboxamide

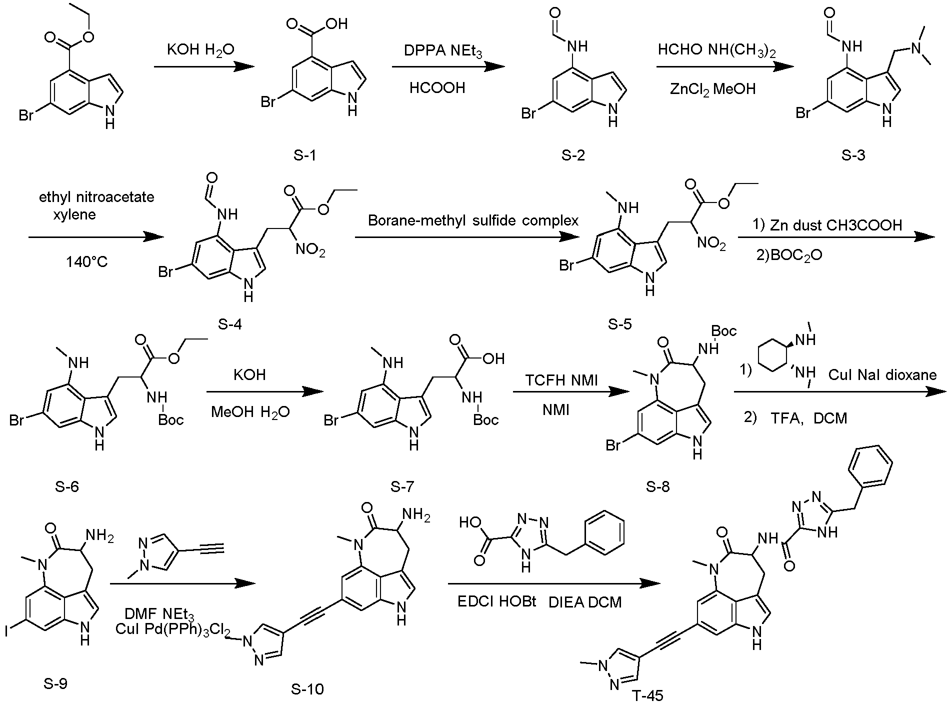

### Step 1: preparation of S-1

A mixture of 6-bromo-4-indolecarboxylic acid methyl ester (25g), KOH (22.05g), water (28mL) and MeOH (169mL) was stirred at room temperature for 3 hours. The pH of the mixture was adjusted to about 2 with dilute hydrochloric acid and a large amount of solid was precipitated. The mixture was filtrated with the Buchner funnel, and the filter cake was dried in a vacuum drying oven. The filtrate was further extracted with EA. The extract was then dried over anhydrous Na₂SO₄, and concentrated to obtain a combined amount of 23g of S-1 as a light red solid.

MS ESI: m/z=238, [M-1]⁻.

### Step 2: preparation of S-2

A mixture of S-1 (8.62g), DPPA (10.86g), TEA (7.47mL) and THF (180mL) was heated at 70°C for about 2 hours, and was cooled to room temperature. HCOOH (100mL) was added and the mixture was heated for another 2 hours. Part of the solvent was removed under reduced pressure before EA was added. The mixture was washed with water, and the organic phase was dried over anhydrous Na₂SO₄, and then concentrated. The residue was purified by SGCC to give 3.65g of of S-2 as a yellowish-brown solid.

MS ESI: m/z=239, [M+1]⁺.

### Step 3: preparation of S-3

A mixture of S-2 (3.65g), dimethylamine (8.4mL, 2N in THF), HCHO (1.36mL, 37% aqueous solution), ZnCl₂ (3,122.6mg) and MeOH (76mL) was stirred at room temperature overnight. The mixture was then treated with NaOH (2N) until the pH is basic. The mixture was filtered, and the filtrate was extracted with EA. The extract was then dried with anhydrous Na₂SO₄ and concentrated to obtain 1.7 g of S-3 as a yellowish-brown solid.

MS ESI: m/z=296, [M+1]⁺.

### Step 4: preparation of S-4

A mixture of S-3 (1,676mg), ethyl nitroacetate (3,781mg) and anhydrous xylene (28mL) was heated at 140°C for about 16 hours. The reaction mixture was concentrated and the residue was dissolved in EA. The EA solution was treated with activated carbon for decolorization. The mixture was then filtered, and the activated carbon was washed with ethanol. The filtrate was concentrated and the residue was purified by SGCC to give 561mg of S-4 as a white solid.

MS ESI: m/z=384, [M+1]⁺.

### Step 5: preparation of S-5

A mixture of S-4 (775mg), borane-methyl sulfide complex (2.02mL, 2N in THF) and THF (10mL) was stirred at room temperature for about 20 hours. The reaction was then quenched with saturated NH₄Cl solution, and extracted with EA. The organic phase was dried with anhydrous Na₂SO₄, and concentrated. The residue was purified by SGCC to give 330mg of S-5 as a solid.

MS ESI: m/z=370, [M+1]⁺.

### Step 6: preparation of S-6

A mixture of S-5 (330mg), zinc powder (1, 169mg), AcOH (4mL) and THF (4mL) was stirred at room temperature for 12 hour. EA was added into the reaction mixture and the mixture was filtered. The filtrate was washed with saturated NaHCO₃ solution. The organic phase was dried over anhydrous Na₂SO₄ and concentrated to obtain 120mg of solid intermediate which was dissolved in THF (4mL) before (Boc)₂O (85.1mg) and TEA (54ul). After the mixture had been stirred at room temperature for 6 hours, the reaction mixture was concentrated. The residue was purified by SGCC to give 130mg of S-6 as a solid.

MS ESI: m/z=440, [M+1]⁺.

### Step 7: preparation of S-7

A mixture of S-6 (130mg), MeOH (2mL), water (0.5mL) and KOH (67.2mg) was stirred at room temperature for about 6 hours. The mixture was acidified with Dilute HCl, and extracted with EA. The organic phase was dried with Na₂SO₄ and concentrated to obtain 121mg of S-7 as a solid.

MS ESI: m/z=412, [M+1]⁺.

### Step 8: preparation of S-8

A mixture of S-7 (125mg) was dissolved in MeCN (3mL) cooled at 0 °C was treated with TCFH (102mg) and NMI (36ul). After the mixture was stirred at room temperature for 2 hours, additional TCFH (10mg) and NMI (5ul) were added. After stirring for another 3 hours, The mixture was quenched with saturated NH₄Cl solution and extracted with EA. The organic phase was dried and concentrated over Na₂SO₄, and concentrated. The residue was purified by SGCC to give 66mg of S-8 as a white solid.

MS ESI: m/z=392, [M-1]⁻.

### Step 9: preparation of S-9

A mixture S-8 (60mg), trans-(1R,2R)-N,N'-dimethyl-cyclohexane-1,2-diamine (22.8mg), CuI (30.5mg), NaI (48mg) and 1,4-dioxane (2mL) was heated at 110°C under N₂ for 3 days. The reaction was quenched with saturated NH₄Cl solution, and extracted with EA. The organic phase was dried over anhydrous Na₂SO₄, and concentrated. The residue was purified by SGCC to give 53mg of a white solid (the ratio of starting materials to products was about 1:1). The sold was dissolved in DCM (2mL) and treated with CF₃COOH (0.4mL). After the mixture had been stirred at room temperature for about 3 hours, the reaction mixture was then concentrated to obtain 38mg of a mixture, which was directly used for the next step.

MS ESI: m/z=342, [M+1]⁺.

### Step 10: preparation of S-10

A mixture of S-9 (38mg, containing bromides), 4-ethynyl-1-methyl-1-H-pyrazole (14mg), Pd(PPh)₃Cl₂ (8.4mg), CuI (2.3mg), NEt₃ (1mL) and DMF (1mL) was heated at 70°C under N₂ for 2 hours. The reaction was quenched with saturated NH₄Cl solution and extracted with EA. The organic phase was dried over anhydrous Na₂SO₄, and concentrated. The residue was purified by SGCC to give 22.4mg of S-10 as a solid (containing a small amount of bromide).

MS ESI: m/z=320, [M+1]⁺.

### Step 11: preparation of T-45

S-10 was used as the starting material to obtain 2mg of T-45 as a white solid under the same conditions as in Example 21.

¹H NMR (400 MHz, DMSO-d₆) δ 11.36 (s, 1H), 8.69 (s, 1H), 8.06 (s, 1H), 7.69 (s, 1H), 7.28 (m, 7H), 7.02 (s, 1H), 4.68 - 4.47 (m, 1H), 4.13 (s, 2H), 3.86 (s, 3H), 3.23 (m, 1H), 3.10 - 2.93 (m, 1H).

MS ESI: m/z=505, [M+1]⁺.

### Example 48: T-46

### (±)-2-(3-(5-benzyl-4H-1,2,4-triazole-3-carboxamide)-1-methyl-2-oxo-1,2,3,4-tetrahydro-6H-azacyclo [4,3,2-cd] indol-6-yl)nicotinic acid

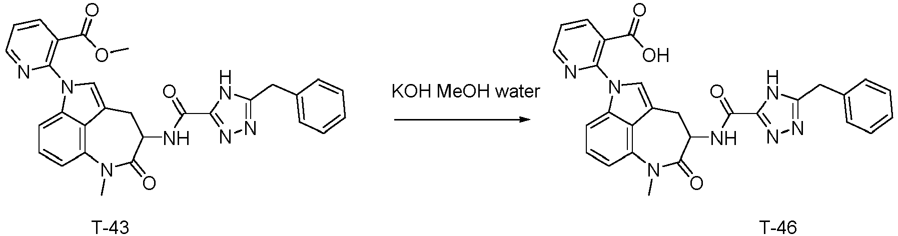

T-43 (10mg) was used as the starting material to obtain 3.2mg of T-46 as a white solid under the same conditions as in Embodiment 32.

¹H NMR (400 MHz, acetone-d₆) δ 8.77 (d, J = 2.7 Hz, 1H), 8.67 (d,J=5.6Hz,1H), 7.56 m, 1H), 7.47 (s, 1H), 7.32 (m, 7H), 7.10 (d, J = 7.5 Hz, 1H), 4.83 (m, 1H), 4.20 (s, 2H), 3.61 (s, 3H), 3.59 - 3.53 (m, 1H), 3.51 - 3.42 (m, 1H).

MS ESI: m/z=522, [M+1]⁺.

### Example 49: T-47

### (±)-5-benzyl-N-(6-(3-hydroxypyridin-2-yl)-1-methyl-2-oxo-2,3,4,6-tetrahydro-1H-azacyclo [4,3,2-cd] indol-3-yl)-4H-1,2,4-triazole-3-carboxamide

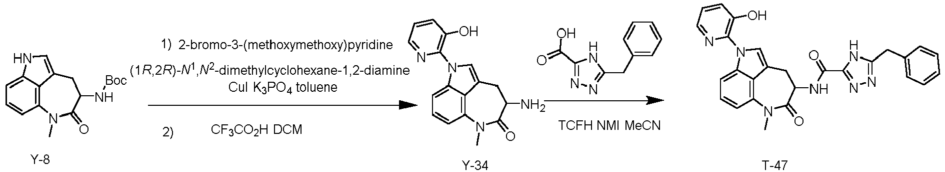

### Step 1: preparation of Y-34

Y-8 (30mg) and 2-bromo-3-(methoxymethoxy)pyridine were used as starting materials to obtain 28.6mg of Y-34 as a solid under the same conditions as in the Step 1 of Example 43.

MS ESI: m/z=309, [M+1]⁺.

### Step 2: preparation of T-47

Y-34 was used as the starting material to obtain 6.9 mg of T-47 as a white solid under the same conditions as in the Step 2 of Example 40.

¹H NMR (400 MHz, acetone-d₆) δ 8.69 (d, J = 6.1 Hz, 1H), 8.12 (dd, J = 4.8 Hz, J = 1.2 Hz, 1H), 7.66 (s, 1H), 7.58 (d, J = 8.0 Hz, 1H), 7.53 (d, J = 8.3 Hz, 1H), 7.41 - 7.19 (m, 7H), 7.09 (d, J = 7.8 Hz, 1H), 4.91 - 4.74 (m, 1H), 4.19 (s, 3H), 3.62 (s, 3H), 3.49 (m, 1H), 3.08 m, 1H).

MS ESI: m/z=494, [M+1]⁺.

### Example 50: T-48

### (±)-5-benzyl-N-(6-(4-(hydroxymethyl)pyridin-3-yl)-1-methyl-2-oxo-2,3,4,6-tetrahydro-1H-azacyclo [4,3,2-cd] indol-3-yl)-4H-1,2,4-triazole-3-carboxamide

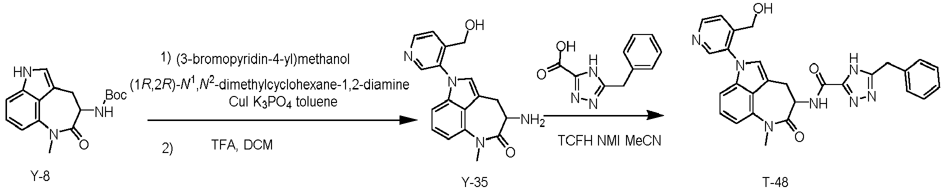

### Step 1: preparation of Y-35

Y-8 (30mg) and (3-bromopyridin-4-yl) methanol were used as starting materials to obtain 21mg ofY-35 under the same conditions as in the Step 1 of Example 43. A longer reaction time was required for this Ullmann reaction.

MS ESI: m/z=323, [M+1]⁺.

### Step 2: preparation of T-48

Subjecting Y-35 to the same conditions as in the Step 2 of Example 40, both the amino and hydroxyl groups on Y-35 reacted with the triazole carboxylic acid. After the product was isolated, the ester group was hydrolyzed using lithium hydroxide monohydrate (20mg), MeOH (2mL) in water (0.3mL). Subsequently, the product was purified under the same conditions as in the Step 2 of Example 40 to obtain 6.7mg of T-48 as a white solid.

¹H NMR (400 MHz, Acetone-d₆) δ 8.72 (d, J = 4.8 Hz, 1H), 8.69 (s, 1H), 8.59 (m, 1H), 7.81 (d, J = 4.8Hz, 1H), 7.42 - 7.20 (m, 7H), 7.12 (d, J = 7.8 Hz, 1H), 6.87 (d, J = 8.0 Hz, 1H), 4.87 (s, 1H), 4.50 s, 2H), 4.20 (s, 2H), 3.63 (s, 3H), 3.49 (m, 1H), 3.23 - 3.01 (m, 1H).

MS ESI: m/z=508, [M+1]⁺.

### Example 51: T-49

### (±)-5-benzyl-N-(6-(3-(hydroxymethyl)pyridin-2-yl)-1-methyl-2-oxo-2,3,4,6-tetrahydro-1H-azacyclo [4,3,2-cd] indol-3-yl)-4H-1,2,4-triazole-3-carboxamide

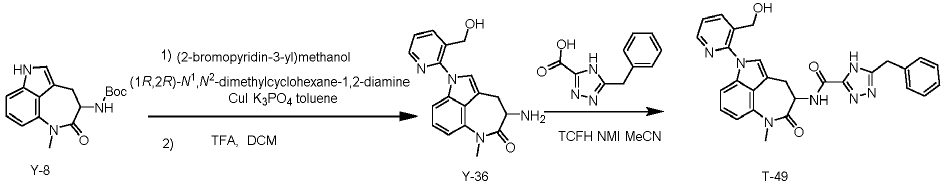

### Step 1: preparation of Y-36

Y-8 (30mg) and (2-bromopyridin-3-yl) methanol were used as starting materials to obtain 24mg of Y-36 as a solid under the same conditions as in the Step 1 of Example 43. A longer reaction time was required for this Ullmann reaction.

MS ESI: m/z=323, [M+1]⁺.

### Step 2: preparation of T-49

Y-36 was used as the starting material to obtain 5.3mg of T-49 as a white solid under the same conditions as in Example 21.

¹H NMR (400 MHz, acetone-d₆) δ 8.68 (m, 1H), 8.54 (d, J = 2.9 Hz, 1H), 8.23 (d, J = 7.0 Hz, 1H), 7.59 - 7.48 (m, 2H), 7.40 - 7.16 (m, 7H), 7.11 (d, J = 7.4 Hz, 1H), 4.84 (m, 1H), 4.68 - 4.43 (s, 3H), 4.20 (s, 2H), 3.63 (s, 3H), 3.48 (m, 1H), 3.07 (m, 1H).

MS ESI: m/z=508, [M+1]⁺.

### Example 52: T-50

### (±)-3-(3-(5-benzyl-4H-1,2,4-triazole-3-carboxamide)-1-methyl-2-oxo-1,2,3,4-tetrahydro-6H-azacyclo [4,3,2-cd] indol-6-yl)methyl pyrazine-2-carboxylate

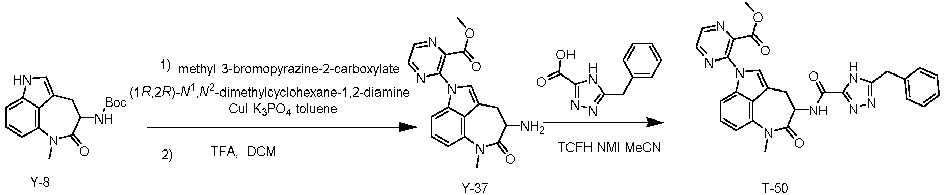

### Step 1: preparation of Y-37

Y-8 (50mg) and 3-bromopyrazine-2-carboxylate were used as starting materials to obtain 58.3mg of Y-37 as a solid under the same conditions as in the Step 1 of Example 43.

MS ESI: m/z=352, [M+1]⁺.

### Step 2: preparation of T-50

Y-37 was used as the starting material to obtain 53.6mg of T-50 as a white solid under the same conditions as in the Step 2 of Embodiment 40.

¹H NMR (400 MHz, DMSO-d₆) δ 14.38 (s, 1H), 8.91 (d, J = 2.2 Hz, 1H), 8.82 (d, J = 2.2 Hz, 1H), 8.69 (bs, 1H), 7.52 (s, 1H), 7.42 (d, J = 8.2 Hz, 1H), 7.38 - 7.22 (m, 6H), 7.17 (d, J = 7.8 Hz, 1H), 4.75 - 4.63 (m, 1H), 4.14 (s, 2H), 3.72 (s, 3H), 3.53 (s, 3H), 3.29 (m, 1H), 3.19 - 2.95 (m, 1H).

MS ESI: m/z=537, [M+1]⁺.

### Example 53: T-51

### (±)-5-(3-(5-benzyl-4H-1,2,4-triazole-3-carboxamide)-1-methyl-2-oxo-1,2,3,4-tetrahydro-6H-azacyclo [4,3,2-cd] indol-6-yl)pyrimidine-4-carboxylic acid methyl ester

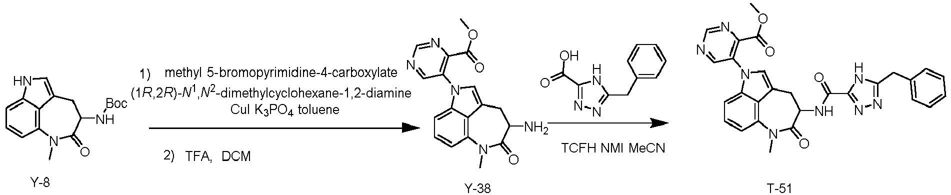

### Step 1: preparation of Y-38

Y-8 (50mg) and 5-bromo-4-pyrimidinecarboxylate were used as starting materials to obtain 26.5mg of Y-38 as a solid under the same conditions as in the Step 1 of Example 43.

MS ESI: m/z=352, [M+1]⁺.

### Step 2: preparation of T-51

Y-38 was used as the starting material to obtain 19mg of T-51 as a pale-yellow solid under the same conditions as in the Step 2 of Example 40.

¹H NMR (400 MHz, acetone-d₆) δ 9.36 (s, 1H), 9.26 (s, 1H), 8.67 (bs, 1H), 7.40 (s, 1H), 7.32 (m, 5H), 7.26 (m, 1H), 7.16 (d, J = 7.8 Hz, 1H), 7.09 (d, J = 8.1 Hz, 1H), 4.84 (m, 1H), 4.20 (s, 2H), 3.68 (s, 3H), 3.63 (s, 3H), 3.48 (m, 1H), 3.20 - 3.02 (m, 1H).

MS ESI: m/z=537, [M+1]⁺.

### Example 54: T-52

### (±)-5-benzyl-N-(6-(3-cyclopropylpyridin-2-yl)-1-methyl-2-oxo-2,3,4,6-tetrahydro-1H-azacyclo [4,3,2-cd] indol-3-yl)-4H-1,2,4-triazole-3-carboxamide

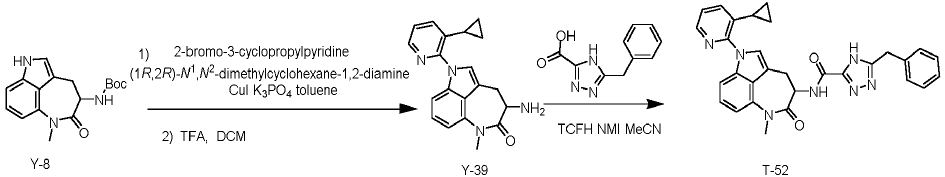

### Step 1: preparation of Y-39

Y-8 (50mg) and 2-bromo-3-cyclopropylpyridine were used as starting materials to obtain 57.6mg of Y-39 as a solid under the same conditions as in the Step 1 of Example 43.

MS ESI: m/z=333, [M+1]⁺.

### Step 2: preparation of T-52

Y-39 was used as the starting material to obtain 56mg of T-52 as a white solid under the same conditions as in the Step 2 of Example 40.

¹H NMR (400 MHz, acetone-d₆) δ 8.67 (d,J = 4.6 Hz,1H), 8.43 - 8.40 (m, 1H), 7.61 (d, J = 8.0 Hz, 1H), 7.57 (s, 1H), 7.42 (dd, J = 7.6, 4.6 Hz, 1H), 7.28 (m, 8H), 7.11 (d, J = 7.6 Hz, 1H), 4.87 (s, 1H), 4.19 (s, 2H), 3.63 (s, 3H), 3.51 (m, 1H), 3.19 - 3.02 (m, 1H), 1.77 (m, 1H), 1.08 - 0.89 (m, 2H), 0.80 (m, 2H).

MS ESI: m/z=518, [M+1]⁺.

### Example 55: T-53

### (±)-5-benzyl-N-(6-(3-(difluoromethyl)pyridin-2-yl)-1-methyl-2-oxo-2,3,4,6-tetrahydro-1H-azacyclo [4,3,2-cd] indol-3-yl)-4H-1,2,4-triazole-3-carboxamide

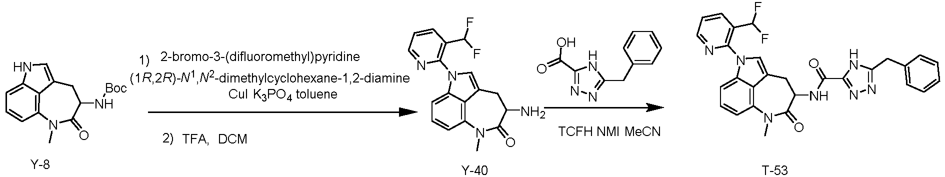

### Step 1: preparation of Y-40

Y-8 (50mg) and 2-bromo-3-(difluoromethyl)pyridine were used as starting materials to obtain 58mg of Y-40 as a solid under the same conditions as in the Step 1 of Example 43.

MS ESI: m/z=343, [M+1]⁺.

### Step 2: preparation of T-53

Y-40 was used as the starting material to obtain 80mg of T-53 as a white solid under the same conditions as in the Step 2 of Example 40.

¹H NMR (400 MHz, acetone-d₆) δ 8.82 (d, *J* = 4.8Hz 1H), 8.66 (d, *J* =5.2 Hz,1H), 8.38 (d, *J* = 7.7 Hz, 2H), 7.74 (dd, *J* = 7.6, 4.8 Hz, 1H), 7.48 (s, 1H), 7.41 - 7.19 (m, 7H), 7.16 (d, *J* = 7.4 Hz, 1H), 6.92 (t, *J* = 54.1 Hz, 1H), 4.87 (s, 1H), 4.20 (s, 2H), 3.63 (s, 3H), 3.50 (m, 1H), 3.20 - 3.02 (m, 1H).

MS ESI: m/z=528, [M+1]⁺.

### Example 56: T-54

### (±)-5-benzyl-N-(6-(3-(fluoromethyl)pyridin-2-yl)-1-methyl-2-oxo-2,3,4,6-tetrahydro-1H-azacyclo [4,3,2-cd] indol-3-yl)-4H-1,2,4-triazole-3-carboxamide

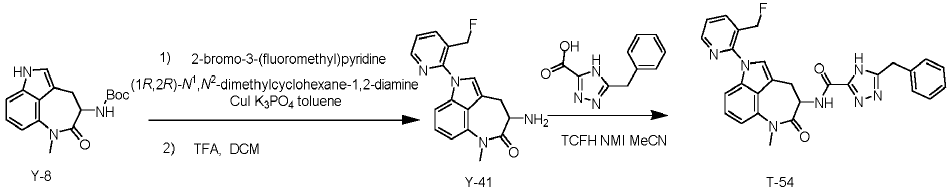

### Step 1: preparation of Y-41

Y-8 (50mg) and 2-bromo-3-(fluoromethyl)pyridine were used as starting materials to obtain 51.2mg of Y-41 as a solid under the same conditions as in the Step 1 of Example 43.

MS ESI: m/z=325, [M+1]⁺.

### Step 2: preparation of T-54

Y-41 was used as the starting material to obtain 73mg of T-54 as a white solid under the same conditions as in the Step 2 of Example 40.

¹H NMR (400 MHz, acetone-d₆) δ 8.67 (m, 2H), 8.22 (d, J = 7.0 Hz, 1H), 7.70 - 7.56 (m, 1H), 7.49 (s, 1H), 7.40 - 7.17 (m, 7H), 7.14 (m,1H), 5.46 (d, J = 47.4 Hz, 2H), 4.86 (m, 1H), 4.19 (s, 2H), 3.63 (s, 3H), 3.49 (m, 1H), 3.20 - 3.01 (m, 1H).

MS ESI: m/z=510, [M+1]⁺.

### Example 57: T-55

### (±)-5-benzyl-N-(6-(3-(2-hydroxypropan-2-yl)pyridin-2-yl)-1-methyl-2-oxy-2,3,4,6-tetrahydro-1H-azacyclo [4,3,2-cd] indol-3-yl)-4H-1,2,4-triazole-3-carboxamide

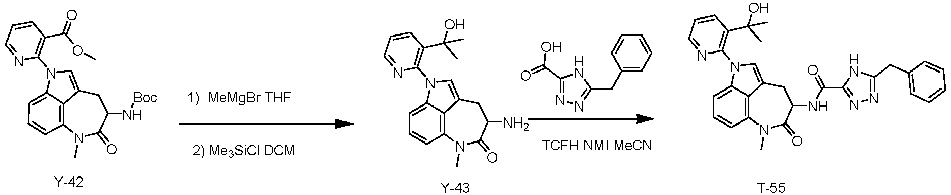

### Step 1: preparation of Y-43

To a solution of Y-42 (30mg) and THF (1mL) was added MeMgBr (0.07mL, 3M in THF) under ice bath conditions. After the mixture had been stirred at room temperature for 1 hour, additional MeMgBr (0.2mL, 3M in THF) was added. After stirring for another 2 hours, the reaction mixture was quenched with saturated NH₄Cl solution, and extracted with EA. The organic phase was dried over Na₂SO₄ and concentrated to obtain 22mg of crude product, which was dissolved in DCM (2mL) and treated with Me₃SiCl. After stirring at room temperature for 1 hour, the reaction was quenched with saturated NH₄Cl solution, and extracted with EA. The organic phase was dried with anhydrous Na₂SO₄ and concentrated to obtain 17mg of Y-43, which was directly used in the next step.

MS ESI: m/z=351, [M+1]⁺.

### Step 2: preparation of T-55

Y-43 was used as the starting material to obtain 7mg of T-55 as a white solid under the same conditions as in the Step 2 of Example 40.

¹H NMR (400 MHz, acetone-d₆) δ 8.32 - 8.28 (m, 1H), 8.19 (s, 1H), 8.01 (d, J = 8.0 Hz, 1H), 7.68 (d, J = 7.6 Hz, 1H), 7.35 (m ,5H), 7.24 (m, 1H), 6.97 (dd, J = 7.6, 4.9 Hz, 1H), 6.90 (d, J = 8.0 Hz, 1H), 5.57 (d, J = 3.6 Hz, 1H), 4.80 (m, 1H), 4.17 (s, 2H), 3.59 (m, 1H), 3.43 (s, 3H), 2.62 (m, 1H), 1.70 (s, 3H), 1.52 (s, 3H).

MS ESI: m/z=536, [M+1]⁺.

### Example 58: Intermediate Y-45

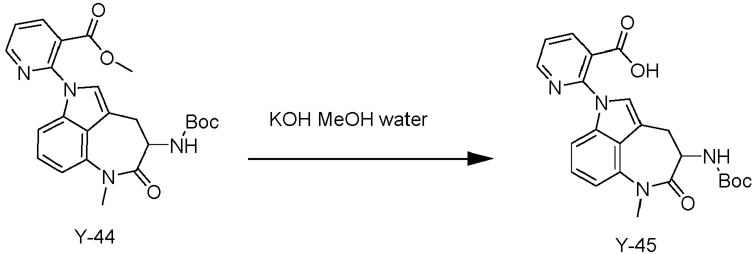

A mixture ofY-44 (40mg), KOH (37.3mg), MeOH (1mL) and water (0.2mL) was stirred at room temperature overnight, and then treated with saturated NH₄Cl solution, followed by adjusting the the pH to acidic with 2N HCl. The mixture was extracted with EA, and the extract was dried and concentrated to obtain 58mg of Y-45.

MS ESI: m/z=435, [M-1]⁻.

### Example 59: T-56

### (±)-2-(3-(5-benzyl-4H-1,2,4-triazole-3-carboxamide)-1-methyl-2-oxy-1,2,3,4-tetrahydro-6H-azacyclo [4,3,2-cd] indol-6-yl)-N-methylnicotinamide

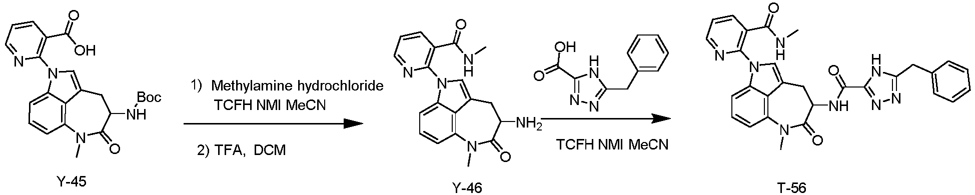

### Step 1: preparation of Y-46

Y-45 (32mg) and methylamine hydrochloride were used as the starting materials to prepare Y-46 (26.4mg, solid) under the same conditions as in the Step 2 of Example 40.

MS ESI: m/z=350, [M+1]⁺.

### Step 2: preparation of T-56

Y-46 was used as the starting material to obtain 27mg of T-56 as a white solid under the same conditions as in the Step 2 of Example 40.

¹H NMR (400 MHz, acetone-d₆) δ 8.66 (d, J = 3.3 Hz, 1H), 8.11 (d, J = 7.1 Hz, 1H), 7.60 (d, J = 8.2 Hz, 1H), 7.54 - 7.45 (m, 2H), 7.42 (s, 1H), 7.39 - 7.19 (m, 6H), 7.12 (d, J = 7.6 Hz, 1H), 4.81 (m, 1H), 4.19 (s, 2H), 3.62 (s, 3H), 3.46 (m, 1H), 3.05 (mz, 1H), 2.72 (s, 3H).

MS ESI: m/z=535, [M+1]⁺.

### Example 60: T-57

### (±)-2-(3-(5-benzyl-4H-1,2,4-triazole-3-carboxamide)-1-methyl-2-oxy-1,2,3,4-tetrahydro-6H-azacyclo [4,3,2-cd] indol-6-yl)-N,N-dimethlnicotinamide

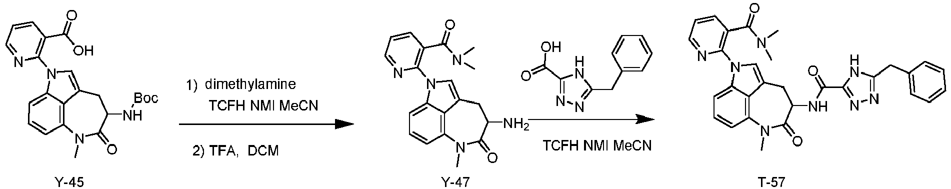

### Step 1: preparation of Y-47

Y-45 (26mg) and dimethylamine were used as the starting materials to prepare Y-47 (13.3mg, solid) under the same conditions as in the Step 2 of Example 40.

MS ESI: m/z=364, [M+1]⁺.

### Step 2: preparation of T-57

Y-47 was used as the starting material to obtain 10.9 mg of T-67 as a white solid under the same conditions as in the Step 2 of Example 40.

¹H NMR (400 MHz, DMSO-d₆) δ 14.34 (s, 1H), 8.71 (d, J = 4.6 Hz, 1H), 8.04 (d, J = 7.6 Hz, 1H), 7.60 (d, J = 8.4 Hz, 1H), 7.58 - 7.52 (m, 1H), 7.40 - 7.20 (m, 8H), 7.14 (d, J = 7.8 Hz, 1H), 4.65 (m, 1H), 4.14 (s, 2H), 3.53 (s, 3H), 3.26 (s, 1H), 3.15 - 2.95 (m, 1H), 2.84 (s, 3H), 2.49 - 2.40 (m, 3H).

MS ESI: m/z=549, [M+1]⁺.

### Example 61: T-58

### (±)-5-benzyl-N-(6-(3-(1-hydroxyethyl)pyridin-2-yl)-1-methyl-2-oxo-2,3,4,6-tetrahydro-1H-azacyclo [4,3,2-cd] indol-3-yl)-4H-1,2,4-triazole-3-carboxamide

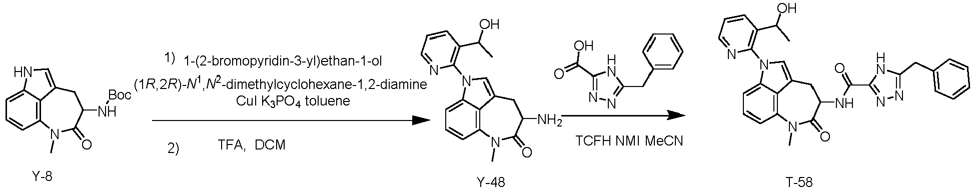

### Step 1: preparation of Y-48

Y-8 (50mg) and 1-(2-bromopyridin-3-yl)ethan-1-ol were used as starting materials to prepare Y-48 under the same conditions as in the Step 1 of Example 43. Since crude Y-48 was used, the next step was carried out according to the theoretical yield.

MS ESI: m/z=325, [M+1]⁺.

### Step 2: preparation of T-58

Y-48 was used as the starting material to obtain 10.4 mg of T-58 as a white solid under the same conditions as in Example 21.

¹H NMR (400 MHz, acetone-d₆) δ 8.68 (m, 1H), 8.58 - 8.47 (m, 1H), 8.30 (d, J = 7.9 Hz, 1H), 7.58 (m, 1H), 7.46 (s, 1H), 7.42 - 7.30 (m, 4H), 7.27 - 7.19 (m, 2H), 7.08 (m 2H), 4.95 (m, 1H), 4.88 (m, 1H), 4.20 (s, 2H), 3.63 (s, 3H), 3.49 (m, 1H), 3.14 (m, 1H), 1.26 (dd, J = 22.0, 6.3 Hz, 3H).

MS ESI: m/z=522, [M+1]⁺.

### Example 62: T-59

### (±)-N-(6-(3-acetylpyridin-2-yl)-1-methyl-2-oxo-2,3,4,6-tetrahydro-1H-azacyclo [4,3,2-cd] indol-3-yl)-5-benzyl-4H-1,2,4-triazole-3-carboxamide

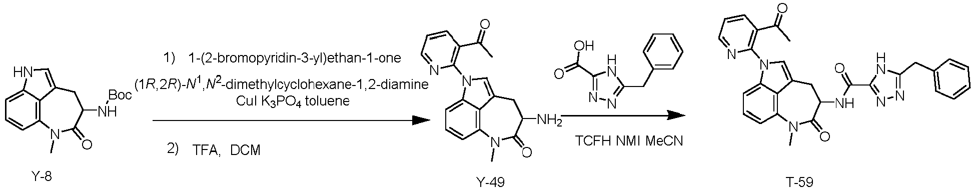

### Step 1: preparation of Y-49

Y-8 (50mg) and 1-(2-bromopyridin-3-yl)ethan-1-one were used as starting materials to prepare 42.3mg of Y-49 as a solid under the same conditions as in the Step 1 of Example 43.

MS ESI: m/z=335, [M+1]⁺.

### Step 2: preparation of T-59

Y-49 was used as the starting material to obtain 37mg of T-59 as a white solid under the same conditions as in the Step 2 of Example 40.

¹H NMR (400 MHz, DMSO-d₆) δ 14.33 (s, 1H), 8.77 (d, *J* = 3.2 Hz, 1H), 8.67 (bs, 1H), 8.29 (d, *J* = 6.3 Hz, 1H), 7.64 (dd, *J* = 7.7, 4.8 Hz, 1H), 7.47 (s, 1H), 7.40 - 7.22 (m, 7H), 7.14 (d, *J* = 7.0 Hz, 1H), 4.67 (m, 1H), 4.15 (s, 2H), 3.53 (s, 3H), 3.28 (m, 1H), 3.10 (m, 1H), 2.13 (s, 3H).

MS ESI: m/z=520, [M+1]⁺.

### Example 63: T-60

### (±)-3-(2-benzyl-3-chloro-7-oxo-2,4,5,7-tetrahydro-6H-pyrazole [3,4-c] pyridin-6-yl)-1-methyl-6(3-(trifluoromethyl)pyridin-2-yl)-1,3,4,6-tetrahydro-2H-azepine [4,3,2-cd] indole-2-one

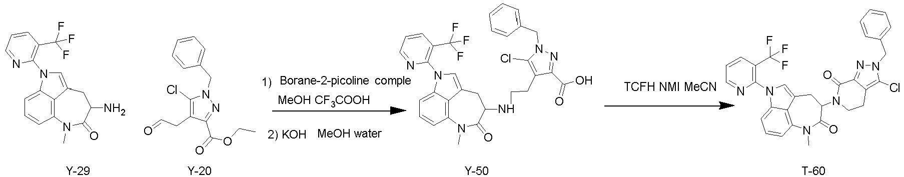

### Step 1: preparation of Y-50

Y-29 (50mg) was used as the starting material to prepare 31.5mg of Y-50 as a solid under the same conditions as in the Step 2 of Example 34.

MS ESI: m/z=623, [M+1]⁺.

### Step 2: preparation of T-60

Y-50 was used as the starting material to obtain 20mg of T-60 as a white solid under the same conditions as in the Step 3 of Example 34.

¹H NMR (400 MHz, acetone-d₆) δ 8.90 (d, *J* = 4.0 Hz, 1H), 8.48 (d, *J* = 7.9 Hz, 1H), 7.84 - 7.78 (m, 1H), 7.31 (m, 7H), 7.10 (dd, *J* = 8.0, 4.2 Hz, 2H), 5.59 (d, *J* = 11.5 Hz, 1H), 5.44 (s, 2H), 4.22 - 4.03 (m, 1H), 3.80 (m, 1H), 3.55 (s, 3H), 3.53 - 3.44 (m, 1H), 3.33 (d, *J* = 14.5 Hz, 1H), 3.00 - 2.89 (m, 1H), 2.89 - 2.82 (m, 1H).

MS ESI: m/z=605, [M+1]⁺.

### Example 64: T-61

### (±)-2-(3-(5-benzyl-4H-1,2,4-triazole-3-carboxamide)-1-methyl-2-oxy-1,2,3,4-tetrahydro-6H-azacyclo [4,3,2-cd] indol-6-yl)nicotinamide

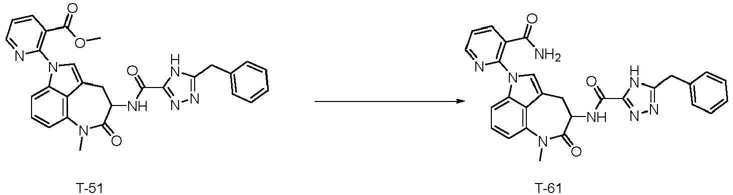

A mixture of T-51 (30mg) and ammonia-methanol solution (2mL, 7N) was stirred at 50°C for about 3 days. The mixture was then concentrated and the residue was purified by HPLC (mobile phase: A: H₂O (+0.1% TFA); B: MeCN; separation condition: 50%B; flow rate: 18mL/min; chromatographic column: Waters Xterra^{®} Prep MSC18, 19*250mm, 10µm) to obtain 23mg of T-61 as a white solid.

¹H NMR (400 MHz, acetone-d₆) δ 8.65 (d, *J* = 2.8 Hz, 1H), 8.17 (d, *J* = 7.4 Hz, 1H), 7.56 (d, *J* = 8.2 Hz, 1H), 7.49 (dd, *J* = 6.4, 4.2 Hz, 1H), 7.47 (s, 1H), 7.40 - 7.18 (m, 7H), 7.10 (d, *J* = 7.7 Hz, 1H), 6.97 (s, 1H), 4.80 (m, 1H), 4.19 (s, 2H), 3.60 (s, 2H), 3.43 (m, 1H), 3.15 - 2.93 (m, 1H).

MS ESI: m/z=521, [M+1]⁺.

### Example 65: T-62

### (±)-5-benzyl-N-(6-(3-chloropyridin-2-yl)-1-methyl-2-oxo-2,3,4,6-tetrahydro-1H-azacyclo [4,3,2-cd] indol-3-yl)-4H-1,2,4-triazole-3-carboxamide

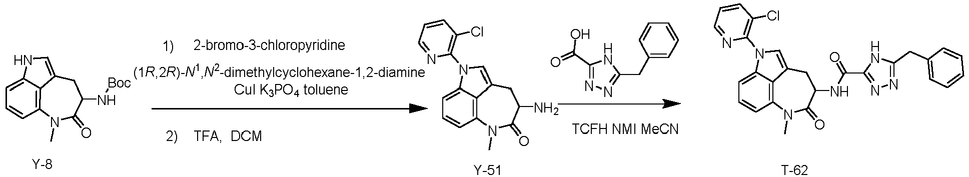

### Step 1: preparation of Y-51

Y-8 (30mg) and 2-bromo-3-chloropyridine were used as starting materials to obtain 25mg of Y-51 as a solid under the same conditions as in the Step 1 of Example 43.

MS ESI: m/z=327, [M+1]⁺.

### Step 2: preparation of T-62

Y-51 was used as the starting material to obtain 38mg of T-62 as a white solid under the same conditions as in the Step 2 of Example 40.

¹H NMR (400 MHz, acetone-d₆) δ 8.68 (d, *J* = 5.6 Hz, 1H), 8.60 (dd, *J* = 4.6, 1.5 Hz, 1H), 8.19 (dd, *J* = 8.0, 1.5 Hz, 1H), 7.59 - 7.56 (m, 1H), 7.55 (d, *J* = 4.7 Hz, 1H), 7.41 - 7.18 (m, 7H), 7.13 (d, *J* = 7.4 Hz, 1H), 4.86 (m, 1H), 4.11 (s, 2H), 3.63 (s, 3H), 3.48 (m, 1H), 3.09 (m, 1H).

MS ESI: m/z=512, [M+1]⁺.

### Example 66: T-63

### (±)-5-benzyl-N-(1-methyl-2-oxo-6-(3-(trifluoromethyl)pyridin-2-yl)-2,3,4,6-tetrahydro-1H-azacyclo [4,3,2-cd] indol-3-yl) oxazole-2-carboxamide

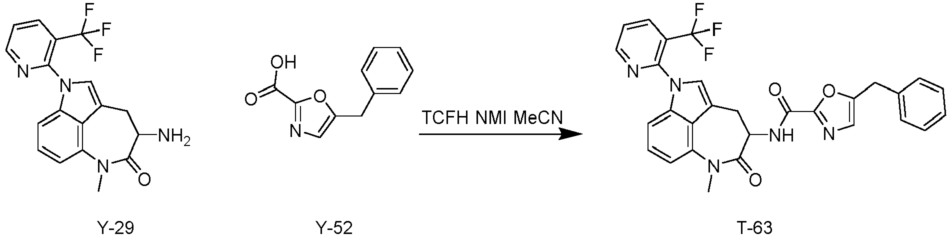

Y-29 (23.5mg) and the newly prepared Y-52 were used as starting materials to prepare 17mg of T-63 as a white solid under the same conditions as in the Step 2 of Example 40.

¹H NMR (400 MHz, acetone-d₆) δ 8.91 (d, *J* = 3.3 Hz, 1H), 8.57 (bs, 1H), 8.49 (d, *J* = 7.9 Hz, 1H), 7.82 (dd, *J* = 7.2, 5.0 Hz, 1H), 7.42 (s, 1H), 7.40 - 7.21 (m, 6H), 7.18 - 7.08 (m, 2H), 7.06 (s, 1H), 4.96 - 4.65 (m, 1H), 4.15 (s, 2H), 3.62 (s, 3H), 3.47 (m, 1H), 3.12 (m, 1H).

MS ESI: m/z=546, [M+1]⁺.

### Example 67: T-64

### (±)-5-benzyl-N-(6-(3-methoxy-6-methylpyridin-2-yl)-1-methyl-2-oxo-2,3,4,6-tetrahydro-1H-azacyclo [4,3,2-cd] indol-3-yl)-4H-1,2,4-triazole-3-carboxamide

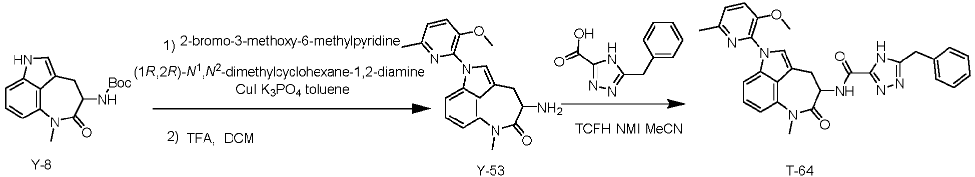

### Step 1: preparation of Y-53

Y-8 (30mg) and 2-bromo-3-methoxy-6-methylpyridine were used as starting materials to obtain 31mg of Y-53 as a solid under the same conditions as in the Step 1 of Example 43.

MS ESI: m/z=337, [M+1]⁺.

### Step 2: preparation of T-64

Y-53 was used as the starting material to obtain 38mg of T-64 as a white solid under the same conditions as in the Step 2 of Example 40.

¹H NMR (400 MHz, acetone-d₆) δ 8.68 (d, J = 5.8 Hz, 1H), 7.63 (d, J = 8.3 Hz, 1H), 7.60 (s, 1H), 7.49 (d, J = 8.2 Hz, 1H), 7.34 (m, 4H), 7.30 - 7.21 (m, 3H), 7.09 (d, J = 7.8 Hz, 1H), 4.89 - 4.81 (m, 1H), 4.20 (s, 2H), 3.91 (s, 3H), 3.62 (s, 3H), 3.47 (m, 1H), 3.07 (m, 1H), 2.49 (s, 3H).

MS ESI: m/z=522, [M+1]⁺.

### Example 68: T-65

### (±)-5-benzyl-N-(6-(3-isopropylpyridin-2-yl)-1-methyl-2-oxo-2,3,4,6-tetrahydro-1H-azacyclo [4,3,2-cd] indol-3-yl)-4H-1,2,4-triazole-3-carboxamide

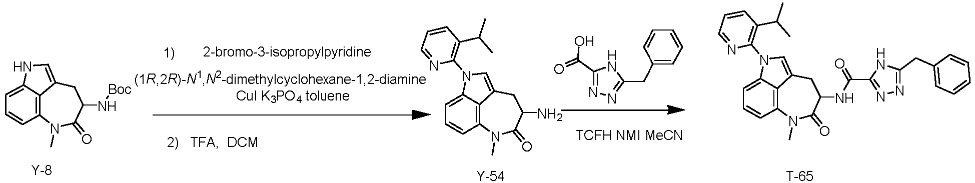

### Step 1: preparation of Y-54

Y-8 (30mg) and 2-bromo-3-isopropylpyridine were used as starting materials to obtain 30mg of Y-54 as a solid under the same conditions as in the Step 1 of Example 43.

MS ESI: m/z=335, [M+1]⁺.

### Step 2: preparation of T-65

Y-54 was used as the starting material to obtain 41mg of T-65 as a white solid under the same conditions as in the Step 2 of Example 40.

¹H NMR (400 MHz, acetone-d₆) δ 8.69 (d, *J* = 5.3 Hz, 1H), 8.55 - 8.42 (m, 1H), 8.08 (d, *J* = 7.9 Hz, 1H), 7.55 (dd, *J* = 7.8, 4.6 Hz, 1H), 7.42 (s, 1H), 7.34 (m, 4H), 7.25 (m, 2H), 7.09 (d, *J* = 7.8 Hz, 1H), 6.97 (d, *J* = 8.2 Hz, 1H), 4.88 (m, 1H), 4.25 (s, 2H), 3.63 (s, 3H), 3.50 (m, 1H), 3.13 (m, 1H), 2.90 (m, 1H), 1.21 (d, *J* = 6.8 Hz, 3H), 1.17 (d, *J* = 6.8 Hz, 3H).

MS ESI: m/z=520, [M+1]⁺.

### Example 69: T-66

### (±)-2-(3-(5-benzyl-4H-1,2,4-triazole-3-carboxamide)-1-methyl-2-oxo-1,2,3,4-tetrahydro-6H-azacyclo [4,3,2-cd] indol-6-yl)nicotinic acid cyclopropane

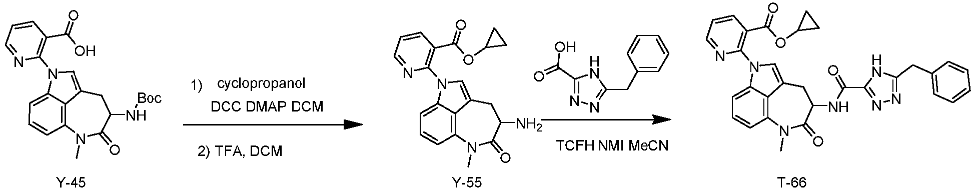

### Step 1: preparation of Y-55

A mixture of Y-55 (30mg), cyclopropanol (20.3mg), DCC (21.6mg), DMAP (4.3mg) and DCM (2mL) was stirred at room temperature overnight. The reaction was quenched with saturated NH₄Cl solution, and the mixture was extracted with EA, and the organic phase was dried and concentrated. The residue was purified by SGCC to obtain 30mg of Y-55 as a white solid. The white solid was dissolved in DCM (2mL) and TFA(0.5mL). The mixture was stirred at room temperature for 2 hours, and concentrated to obtain 23.7mg of Y-55 as a solid.

MS ESI: m/z=377, [M+1]⁺.

### Step 2: preparation of T-66

Y-55 was used as the starting material to obtain 16.6mg of T-66 as a white solid under the same conditions as in the Step 2 of Example 40.

¹H NMR (400 MHz, acetone-d₆) δ 8.81 - 8.78 (m, 1H), 8.67 (bs, 1H), 8.40 (dd, J = 7.8, 1.8 Hz, 1H), 7.63 (dd, J = 7.8, 4.8 Hz, 1H), 7.47 (s, 1H), 7.40 - 7.21 (m, 6H), 7.16 (m, 2H), 4.85 (m, 1H), 4.20 (s, 2H), 4.06 (m, 1H), 3.64 (s, 3H), 3.51 (m, 1H), 3.07 (m, 1H), 0.56 - 0.47 (m, 2H), 0.19 (m, 1H), 0.08 m, 1H).

MS ESI: m/z=562, [M+1]⁺.

### Example 70: T-67

### (±)-5-benzyl-N-(6-(3-ethoxypyridin-2-yl)-1-methyl-2-oxy-2,3,4,6-tetrahydro-1H-azacyclo [4,3,2-cd] indol-3-yl)-4H-1,2,4- triazole-3-carboxamide

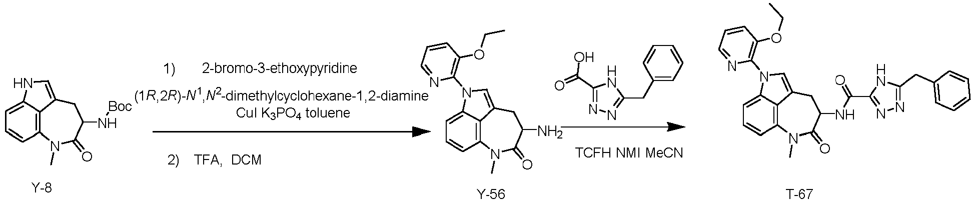

### Step 1: preparation of Y-56

Y-8 (30mg) and 2-bromo-3-ethoxypyridine were used as starting materials to obtain 26.2mg of Y-56 as a solid under the same conditions as in the Step 1 of Example 43.

MS ESI: m/z=337, [M+1]⁺.

### Step 2: preparation of T-67

Y-56 was used as the starting material to obtain 21.8mg of T-67 as a white solid under the same conditions as in the Step 2 of Example 40.

¹H NMR (400 MHz, acetone-d₆) δ 8.66 (d, *J* = 5.6 Hz,, 1H), 8.16 (d, *J* = 4.7 Hz, 1H), 7.70 (d, *J* = 8.0 Hz, 1H), 7.66 (s, 1H), 7.51 (d, *J* = 8.1 Hz, 1H), 7.43 - 7.20 (m, 7H), 7.10 (d, *J* = 7.8 Hz, 1H), 4.85 (m, 1H), 4.31 - 4.20 (m, 2H), 4.19 (s, 2H), 3.62 (s, 3H), 3.49 (m, 1H), 3.07 (m, 1H), 1.37 (t*, J* = 6.9 Hz, 3H).

MS ESI: m/z=522, [M+1]⁺.

### Example 71: T-68

### (±)-5-benzyl-N-(1-methyl-6-(3-(methylsulfonyl)pyridin-2-yl)-2-oxo-2,3,4,6-tetrahydro-1H-azepine [4,3,2-cd] indol-3-yl)-4H-1,2,4-triazole-3-carboxamide

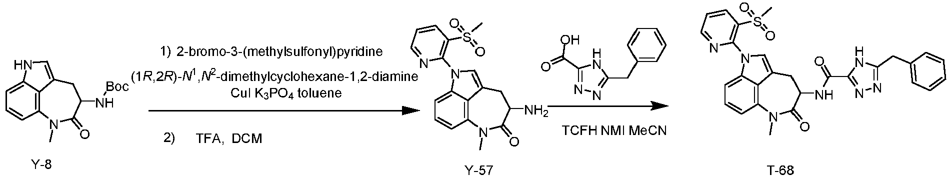

### Step 1: preparation of Y-57

Y-8 (30mg) and 2-bromo-3-(methylsulfonyl)pyridine were used as starting materials to obtain 15.7mg of Y-57 as a solid under the same conditions as in the Step 1 of Example 43.

MS ESI: m/z=371, [M+1]⁺.

### Step 2: preparation of T-68

Y-57 was used as the starting material to obtain 21.6mg of T-68 as a white solid under the same conditions as in the Step 2 of Example 40.

¹H NMR (400 MHz, acetone-d₆) δ 8.91 (d, *J* = 2.9 Hz, 1H), 8.68 (d, *J* = 7.9 Hz, 2H), 7.87 (dd, *J* = 7.9, 4.8 Hz, 1H), 7.60 (s, 1H), 7.44 - 7.19 (m, 6H), 7.15 (d, *J* = 8.1 Hz, 2H), 4.87 (m, 1H), 4.23 (s, 2H), 3.63 (s, 3H), 3.49 (m, 1H), 3.14 (m, 1H), 2.86 (s, 3H).

MS ESI: m/z=556, [M+1]⁺.

### Example 72: T-69

### (±)-5-benzyl-N-(1-methyl-2-oxo-6-(pyridin-2-yl)-2,3,4,6-tetrahydro-1H-azacyclo [4,3,2-cd] indol-3-yl)-4H-1,2,4-triazole-3-carboxamide

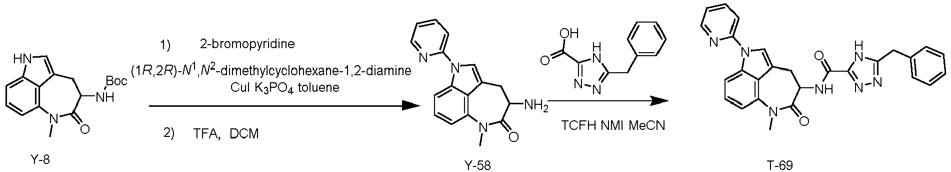

### Step 1: preparation of Y-58

Y-8 (30mg) and 2-bromopyridine were used as starting materials to obtain 26mg of Y-58 as a solid under the same conditions as in the Step 1 of Example 43.

MS ESI: m/z=293, [M+1]⁺.

### Step 2: preparation of T-69

Y-58 was used as the starting material to obtain 30.4mg of T-89 as a white solid under the same conditions as in the Step 2 of Embodiment 40.

¹H NMR (400 MHz, acetone-d₆) δ 8.67 (bs, 1H), 8.60 (d, *J* = 3.0 Hz, 1H), 8.28 (d, *J =* 8.4 Hz, 1H), 8.00 (t, *J* = 7.5 Hz, 1H), 7.87 (s, 1H), 7.75 (d, *J* = 8.3 Hz, 1H), 7.42 - 7.28 (m, 6H), 7.25 (d, *J* = 6.1 Hz, 1H), 7.17 (d, *J* = 7.8 Hz, 1H), 4.83 (m, 1H), 4.20 (s, 2H), 3.62 (s, 3H), 3.49 (m, 1H), 3.06 (m, 1H).

MS ESI: m/z=478, [M+1]⁺.

### Example 73: T-70

### (±)-N-(1-methyl-2-oxo-6-(3-(trifluoromethyl) pyridin-2-yl)-2,3,4,6-tetrahydro-1H-azacyclo[4,3,2-cd]indol-3-yl)-4-phenoxy pyridinamide

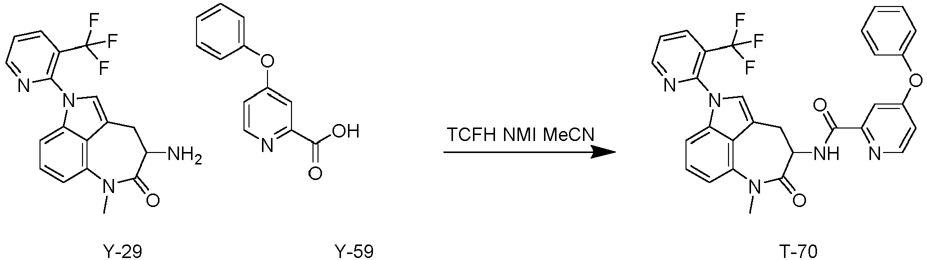

Y-29 (14mg) and Y-59 were used as starting materials to prepare 17mg of T-70 as a white solid under the same conditions as in the Step 2 of Example 40.

¹H NMR (400 MHz, acetone-d₆) δ 9.41 (d, J = 5.2 Hz, 1H), 8.91 (d, J = 3.5 Hz, 1H), 8.58 (d, J = 5.5 Hz, 1H), 8.49 (d, J = 6.7 Hz, 1H), 7.82 (dd, J = 7.3, 4.9 Hz, 1H), 7.59 - 7.47 (m, 3H), 7.41 (s, 1H), 7.33 (t, J = 7.5 Hz, 1H), 7.27 (t, J = 8.0 Hz, 1H), 7.22 (m, 2H), 7.12 (m, 3H), 4.94 - 4.80 (m, 1H), 3.64 (s, 3H), 3.48 (m 1H), 3.08 (m, 1H).

MS ESI: m/z=558, [M+1]⁺.

### Example 74: preparation of intermediate R-7

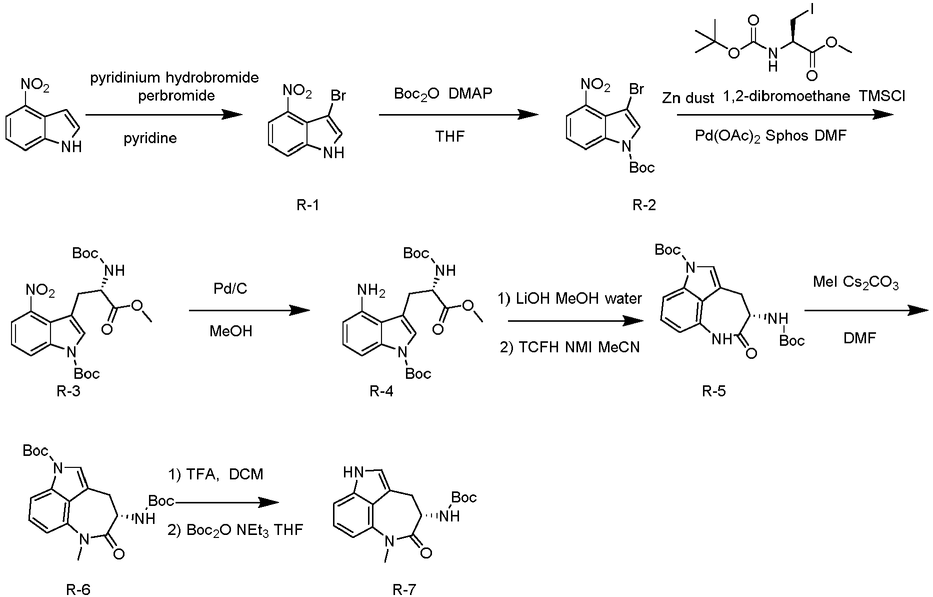

### Step 1: preparation of R-1

To a solution of 4-nitro-indole (11g) and pyridine (150mL) was added a solution of pyridine tribromide in pyridine (50mL) at 0 °C. After stirring at room temperature overnight, the reaction mixture was concentrated. Water was added and the mixture was extracted with EA. The organic phase was concentrated and the residue was purified by SGCC to obtain 15.62g of R-1 as a yellow solid.

MS ESI: m/z=241, [M+1]⁺.

### Step 2: preparation of R-2

A mixture of R-1 (15.62g), (Boc)₂O (15.5g), DMAP (0.79g) and THF (100mL) was stirred at room temperature for 2 hours. The reaction mixture was concentrated and the residue was purified by SGCC to give 21.23g of R-2 as a yellow solid.

### Step 3: preparation of R-3

A mixture of zinc powder (43.56g), DMF (50mL) and 1,2-dibromoethane (1.9mL) was stirred at 50°C for 30 minutes and then was cooled to room temperature. TMSCl (0.68mL) was added and the mixture was stirred at room temperature for 30 minutes. A solution of (R)-N-(tert-butoxycarbonyl)-3-iodo-L-alanine methyl ester (43.84g) in DMF (50mL) was added to the mixture. The mixture was stirred at room temperature for 2 hours and stood for 30 minutes. After the excess zinc powder settled down, the supernatant was taken to a 500mL flask containing R-2 (22.72g), Pd (OAc)₂ (1.50g), and Sphos (2.74g). The mixture was stirred for 3 hours at 35°C. The reaction was quenched with NH₄Cl and the mixture was extracted with EA. The organic phase was concentrated and the residue was purified by SGCC to obtain 10.21g of R-3 as a solid.

MS ESI: m/z=364, [(M-Boc)+1]⁺.

### Step 4: preparation of R-4

A mixture of R-3 (4,000mg), Pd/C (1,839mg, 10% of active ingredient, and 55% of water), and MeOH (43mL) was stirred at room temperature overnight under H₂ atmosphere. The mixture was filtered through celite. The filtrate was was concentrated and the residue was purified by SGCC to give 2.995g of R-4 as a light pale-yellow.

MS ESI: m/z=424, [M+1]⁺.

### Step 5: preparation of R-5

A mixture of R-4 (2,998 mg) was dissolved in MeOH (30mL) and water (5mL) and lithium hydroxide monohydrate (1,162.6 mg) was stirred at room temperature for about 3 hours, and then diluted with the saturated solution of NH₄Cl. The mixture was adjusted to acidic with 2N ofHCl, and extracted with EA. The organic phase was dried with anhydrous Na₂SO₄ and then concentrated. The residue was dissolved in MeCN (30mL), and treated with TCFH (2,912.4mg) and NMI (2.76mL). The mixture was stirred at room temperature for 2 hours, and then quenched with saturated NH₄Cl solution, extracted with EA. The organic phase was concentrated and the residue was purified by SGCC to give 2,588.6mg of R-5 as a solid.

MS ESI: m/z=400, [M-1]⁻.

### Step 6: preparation of R-6

A mixture of R-5 (2,807mg), Cs₂CO₃ (2,738.4 mg) and MeI (1,192.8 mg) in DMF (30mL) was stirred at room temperature for 5 hours. The reaction was quenched with saturated NH₄Cl solution and the mixture was extracted with EA. The organic phase was concentrated and the residue was purified by SGCC to give 2.9g of solid R-6.

MS ESI: m/z=316, [(M-Boc)+1]⁺.

### Step 7: preparation of R-7

A solution of R-6 (2,900mg) in DCM (35mL) was treated with TFA (8mL). The mixture was stirred at room temperature for 2 hours, and the reaction mixture was then concentrated. The residue was dissolved in THF (30mL), and treated with (Boc)₂O (1.6 g), and the mixture was stirred at room temperature for 20 minutes. The reaction mixture was concentrated and the residue was purified by SGCC to give 1,876 mg of colorless solid R-7. The S-isomer with the same structure could be obtained using enantiopure R-7 as a starting material under the same conditions as in Example 72.

MS ESI: m/z=314, [M-1]⁻.

### Example 75: preparation of intermediate L-7

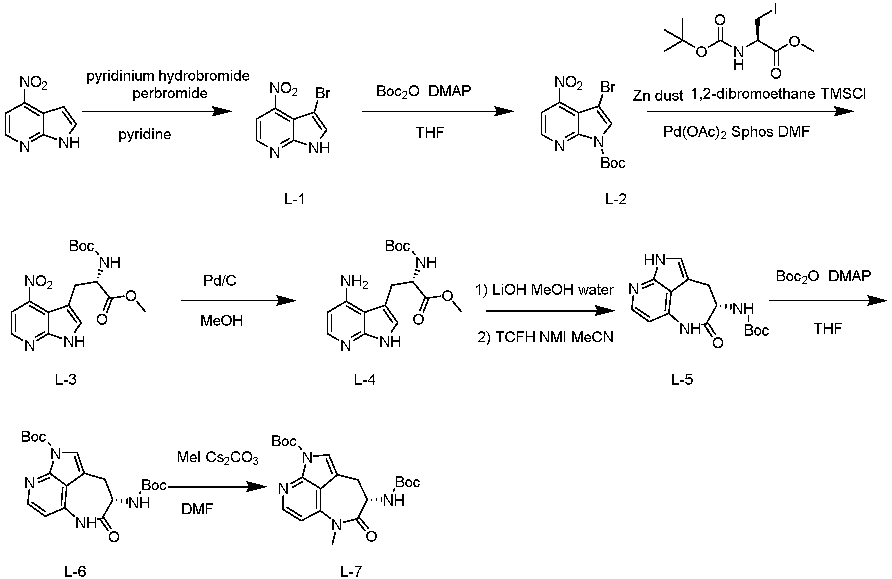

### Step 1: preparation of L-1

To a solution of 4-nitro-7-azindole (1g) and pyridine (10mL) was added a solution of pyridine tribromide in pyridine (5mL) at 0 °C. After the mixture was stirred at room temperature for 3 hours. The mixture was concentrated and water and EA were added to extract the product. The organic phase was concentrated and the residue was purified by SGCC to give 1.09g of L-1 as a yellow solid.

MS ESI: m/z=242, [M+1]⁺.

### Step 2: preparation of L-2

A mixture ofL-1 (1,090mg), (Boc)₂O (1,800.2mg), DMAP (55mg), and THF (10mL) was stirred at room temperature overnight. The reaction mixture concentrated and the residue was purified by SGCC to give 1.197g of L-2 as a yellow solid.

### Step 3: preparation of L-3

A mixture of Zinc powder (2,288.3mg), DMF (8mL) and 1, 2-dibromoethane (O.lmL) was stirred at 50°C for 30 minutes. The mixture was cooled to room temperature and TMSC1 (35ul) was added. The mixture was stirred at room temperature for 30 minutes. A solution of (R)-N-(tert-butoxycarbonyl)-3-iodo-L-alanine methyl ester (2,533.3mg) in DMF (8mL) was added to the mixture. The mixture was stirred at room temperature for 2 hours and stood for 30 minutes. After the excess zinc powder settled down, the supernatant was taken to a 50mL flask containing L-2 (1,197mg), Pd (OAc)₂ (78mg), and Sphos (144mg). The mixture was stirred for 4 hours at 35°C and then quenched with NH₄Cl. The mixture was extracted with EA. The organic phase was concentrated and the residue was purified by SGCC to give 404mg of yellow solid L-3.

MS ESI: m/z=365, [M+1]⁺.

### Step 4: preparation of L-4

A mixture of L-3 (404mg), Pd/C (236.9mg, 10% of active ingredient, and 55% of water) in MeOH (5mL) was stirred at room temperature overnight in H₂ atmosphere, and then filtrated with celite. The filtrate was concentrated to obtain L-4, which was directly used in the next step.

MS ESI: m/z=335, [M+1]⁺.

### Step 5: preparation of L-5

L-4 was dissolved in MeOH (20mL) and water (0.5mL), followed by addition of lithium hydroxide monohydrate (186.5mg). The mixture was stirred at room temperature for 3 hours, and diluted with the saturated solution of NH₄Cl. The mixture was adjusted to acidic with 2N ofHCl, and extracted with EA. The organic phase was dried with anhydrous Na₂SO₄ and concentrated. The residue was dissolved in MeCN (10mL), and treated with TCFH (467.2mg) and NMI (0.44mL). The mixture was stirred at room temperature for 2 hours, quenched with saturated NH₄Cl solution, and extracted with EA. The organic phase was concentrated and the residue was purified by SGCC to give 75mg of solid L-5.

MS ESI: m/z=303, [M+1]⁺.

### Step 6: preparation of L-6

A mixture of L-5 (75mg), (Boc)₂O (266.2mg) and DMAP (13.5mg) in THF (5mL) was stirred overnight. NH₄Cl saturated solution was added to quench the reaction and the mixture was extracted with EA. The organic phase was concentrated and the residue was purified by SGCC to give 100mg of solid L-6.

MS ESI: m/z=403, [M+1]⁺.

### Step 7: preparation of L-7

L-6 was dissolved in DMF (2mL), and Cs₂CO₃ (162.2mg) and MeI (42.4mg) were added under ice bath conditions. After the mixture had been stirred at room temperature for 1 hour, the reaction mixture was quenched with NH₄Cl and extracted with EA. The organic phase was concentrated and the residue was purified by SGCC to give 93mg of L-7 as a pale-yellow solid.

MS ESI: m/z=417, [M+1]⁺.

### Example 76: T-71

### (S)-5-benzyl-N-(6-methyl-7-oxo-6,7,8,9-tetrahydro-2H-2, 3, 6-triazabenzene[cd]azulene-8-yl)-4H-1,2,4-triazole-3-carboxamide

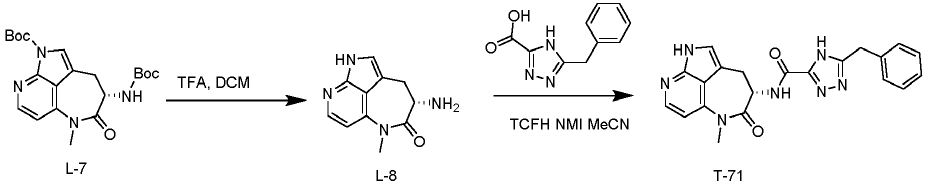

### Step 1: preparation of L-8

A solution of L-7 (30mg) in DCM (2mL) was treated with TFA (0.5mL). The mixture was stirred at room temperature for 2 hours, and then was concentrated to obtain L-8, which was directly used in the next step.

MS ESI: m/z=217, [M+1]⁺.

### Step 2: preparation of T-71

L-8 was used as the starting material to obtain 43.9mg of T-71 as a white solid under the same conditions as in the Step 2 of Example 40.

¹H NMR (400 MHz, acetone-d₆) δ 13.36 (s, 1H), 10.69 (s, 1H), 8.61 (s, 1H), 8.25 (d, *J* = 5.4 Hz, 1H), 7.38 - 7.29 (m, 5H), 7.24 (m, 1H), 6.97 (d, *J* = 5.4 Hz, 1H), 4.84 (m, 1H), 4.19 (s, 2H), 3.61 (s, 3H), 3.43 (m, 1H), 3.24 - 2.94 (m, 1H).

MS ESI: m/z=402, [M+1]⁺.

### Example 77: preparation of intermediate S-7

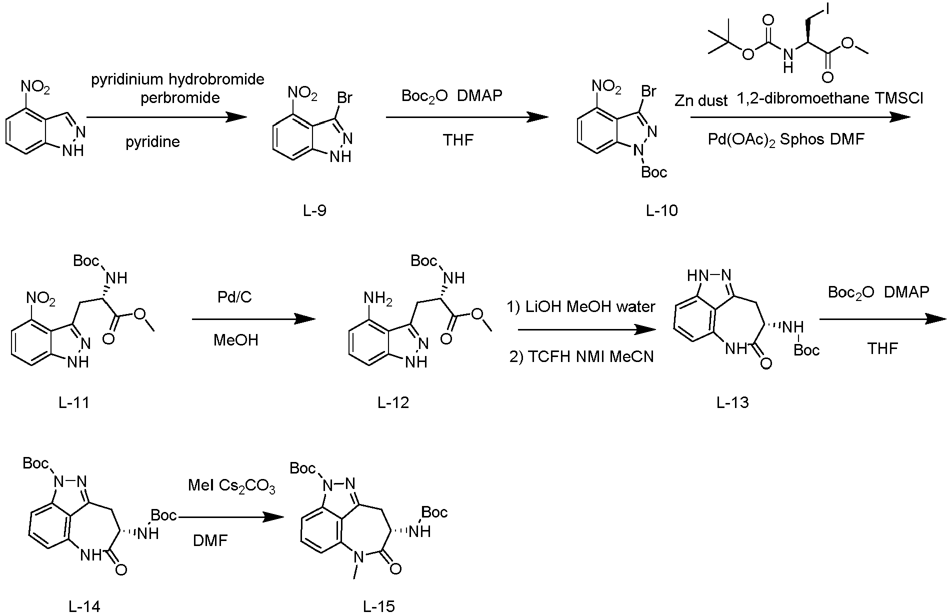

4-nitro-1H-indazole (1g) was used as the starting material to prepare L-15 (33mg) under the same conditions as in Example 75.

### Example 78: T-72

### (S)-5-benzyl-N-(6-methyl-7-oxo-6,7,8,9-tetrahydro-2H-azacyclo [4,3,2-cd] indazol-8-yl)-4H-1,2,4-triazole-3-carboxamide

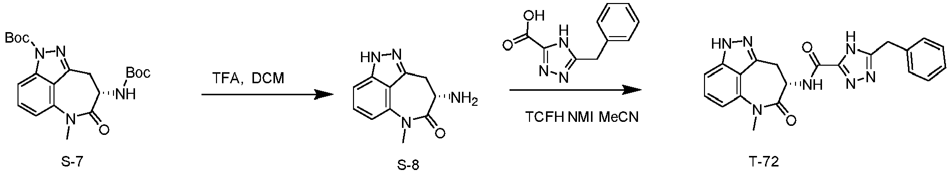

S-7 (33mg) was used as the starting material to prepare 20.8mg of T-72 as a white solid under the same conditions as in Example 76.

¹H NMR (400 MHz, acetone-d₆) δ 12.19 (s, 2H), 8.66 (s, 1H), 7.51 - 7.39 (m, 1H), 7.34 (m, 5H), 7.25 (m, 1H), 7.02 (d, J = 7.5 Hz, 1H), 4.86 (m, 1H), 4.20 (s, 2H), 3.66 - 3.59 (m, 1H), 3.62 (s, 3H), 3.37 - 3.15 (m, 1H).

MS ESI: m/z=402, [M+1]⁺.

### Example 79: T-73

### (±)-5-benzyl-N-(1-methyl-6-(1-methyl -1H-1,2,4-triazole-3-yl)-2-oxo-2,3,4,6-tetrahydro-1H-azacyclo [4,3,2-cd] indol-3-yl)-4H-1,2,4-triazole-3-carboxamide

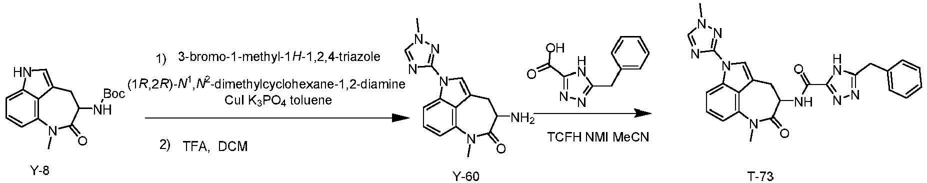

### Step 1: preparation of Y-60

Y-8 (30mg) and 3-bromo-1-methyl-1,2,4-thiazole were used as starting materials to obtain 15. 1 mg of solid Y-60 under the same conditions as in the Step 1 of Example 43 (4N of HCl dioxane solution was used to remove the Boc group).

MS ESI: m/z=297, [M+1]⁺.

### Step 2: preparation of T-73

Y-60 was used as the starting material to obtain 10mg of T-73 as a white solid under the same conditions as in the Step 2 of Example 40.

¹H NMR (400 MHz, acetone-d₆) δ 13.31 (s, 1H), 8.65 (bs, 1H), 8.36 (s, 1H), 8.27 (d, *J* = 8.2 Hz, 1H), 7.84 (s, 1H), 7.41 (d, *J* = 8.1 Hz, 1H), 7.35 (m, 4H), 7.22 (d, *J* = 21.3 Hz, 1H), 7.16 (d, *J* = 7.8 Hz, 1H), 4.81 (m, 1H), 4.20 (s, 2H), 4.03 (s, 3H), 3.62 (s, 3H), 3.49 (m, 1H), 3.04 (m, 1H).

MS ESI: m/z=482, [M+1]⁺.

### Example 80: T-74

### (±)-5-benzyl-N-(1-methyl-6-(1-methyl-1H-imidazol-5-yl)-2-oxo-2,3,4,6-tetrahydro-1H-azacyclo [4,3,2-cd] indol-3-yl)-4H-1,2,4-triazole-3-carboxamide

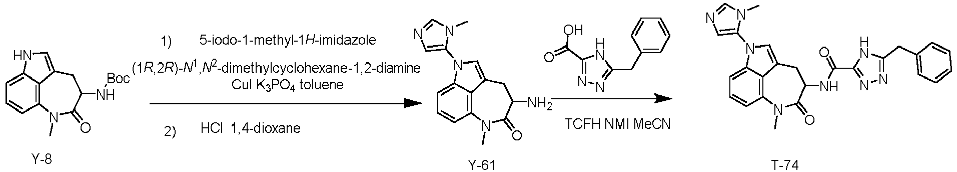

### Step 1: preparation of Y-61

Y-8 (30mg) and 5-iodo-1-methyl-1H-imidazole were used as starting materials to obtain 18.6mg of solid Y-61 under the same conditions as in the Step 1 of Example 43 (4N of HCl dioxane solution was used to remove the Boc group).

MS ESI: m/z=296, [M+1]⁺.

### Step 2: preparation of T-74

Y-61 was used as the starting material to obtain 12mg of T-74 as a white solid under the same conditions as in the Step 2 of Example 40.

¹H NMR (400 MHz, acetone-d₆) δ 8.67 (bs, 1H), 7.69 (s, 1H), 7.40 - 7.28 (m, 6H), 7.25 (m, 1H), 7.13 (m, 1H), 6.96 (d, *J* = 8.0 Hz, 1H), 4.84 (m, 1H), 4.20 (s, 2H), 3.63 (s, 3H), 3.48 (m, 1H), 3.45 (s, 3H), 3.18 - 3.00 (m, 1H).

MS ESI: m/z=481, [M+1]⁺.

### Example 81: T-75

### (±)-5-benzyl-N-(1-methyl-6-(3-(N-methylsulfamoyl)pyridin-2-yl)-2-oxo-2,3,4,6-tetrahydro-1H-azacyclo [4,3,2-cd] indol-3-yl)-4H-1,2,4-triazole-3-carboxamide

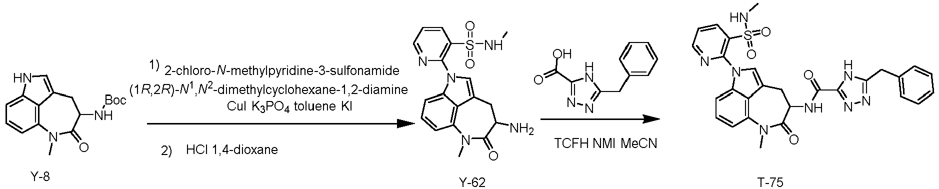

### Step 1: preparation of Y-62

Y-8 (30mg), 2-chloro-N-methylpyridine-3-sulfonamide and KI (1eq) were used as starting materials to obtain15.8 mg of solid Y-62 under the same conditions as in the Step 1 of Example 80.

MS ESI: m/z=386, [M+1]⁺.

### Step 2: preparation of T-75

Y-62 was used as the starting material to obtain 8mg of T-75 as a white solid under the same conditions as in the Step 2 of Example 40.

¹H NMR (400 MHz, acetone-d₆) δ 8.83 (dd, J = 4.7, 1.7 Hz, 1H), 8.65 (d, J = 5.0 Hz, 1H), 8.61 (dd, J = 7.9, 1.7 Hz, 1H), 7.77 (dd, J = 7.9, 4.7 Hz, 1H), 7.55 (s, 1H), 7.34 (m, 4H), 7.26 (m, 2H), 7.18 (d, J = 8.0 Hz, 1H), 7.12 (d, J = 7.7 Hz, 1H), 6.14 (s, 1H), 4.85 (m, 1H), 4.21 (s, 2H), 3.62 (s, 3H), 3.46 (m, 1H), 3.05 (m, 1H), 2.47 (d, J = 4.4 Hz, 3H).

MS ESI: m/z=571, [M+1]⁺.

### Example 82: T-76

### (±)-5-benzyl-N-(7-cyano-1-methyl-2-oxo-1,2,3,4-tetrahydro-[1,4] diazepino [3,2,1-hi] indol-3-yl)-4H-1,2,4-triazole-3-carboxamide

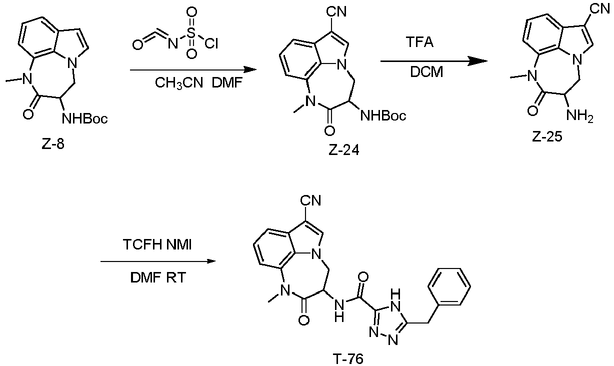

### Step 1: preparation of Z-24

A mixture of Z-8 (1eq) and chlorosulfonate isocyanate (1.1eq) in acetonitrile was stirred for 2 hours of reaction under ice bath conditions. DMF (25eq) was added to continue the reaction mixture and the mixture was stirred for another 2 hours. The mixture was then diluted with EA, washed with water three times, and the organic phase was concentrated and the residue was purified by SGCC to give the intermediate Z-24.

### Step 2: preparation of Z-25

A solution of Z-24 (1eq) and trifluoroacetic acid (10eq) in dichloromethane was stirred at room temperature for 3 hours. The mixture was concentrated and the residue was purified by SGCC to give Z-25.

MS ESI: m/z=241, [M+H]⁺ .

### Step 3: preparation of T-76

Acid-1 (1.5eq) and Z-25 (1eq) were used as starting materials under the same conditions as in Example 2 to obtain T-76.

¹H NMR (400 MHz, acetone-*d*₆) δ 8.23 (s, 1H), 7.59 - 7.51 (m, 2H), 7.44 - 7.30 (m, 6H), 7.26 (m, 2H), 4.88-4.83 (m, 2H), 4.59 - 4.50 (m, 1H), 4.21 (s, 2H), 3.62 (s, 3H).

MS ESI: m/z=426 ,[M+H]⁺ .

### Example 83: T-77

### (t)-5-benzyl-N-(1-methyl-2-oxo-7-(2-(trifluoromethyl) pyridin-4-yl)-1,2,3,4-tetrahydro-[1,4] diazepino [3,2,1-hi] indol-3-yl)-4H-1,2,4-triazole-3-carboxamide

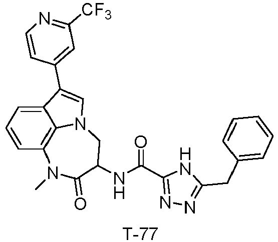

2-Trifluoromethylpyridine-4-boronic acid pinacol ester and Z-9 were used to obtain T-77 under the same conditions as in Example 16.

¹H NMR (400 MHz, acetone-*d*₆) δ 8.76 (d, *J* = 5.2 Hz, 1H), 8.58 (s, 1H), 8.19 (s, 1H), 8.14 (s, 1H), 8.05 (d, *J* = 5.3Hz, 1H), 7.94 (m, 1H), 7.41 - 7.30 (m, 6H), 7.26 (m, 2H), 4.88-4.84 (m, 2H), 4.58 - 4.51 (m, 1H), 4.22 (s, 2H), 3.64 (s, 3H).

MS ESI: m/z=546,[M+H]⁺ .

### Example 84: T-78

### 5-benzyl-N-(7-(2-methoxypyridin-3-yl)-1-methyl-2-oxo-1,2,3,4-tetrahydro-[1,4]diazepino[3,2,1-hi]indol-3-yl)-4H-1,2,4-triazole-3-carboxamide

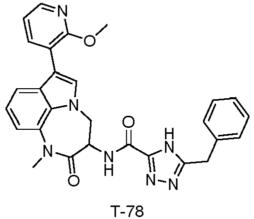

2-methoxy-3-(4,4,5,5-tetramethyl-1,3,2-dioxaborolan-2-yl) pyridine and intermediate Z-9 were used as starting materials to obtain T-78 under the same conditions as in Example 16.

¹H NMR (400 MHz, acetone-d₆) δ 8.58 (s, 1H), 8.12 (dd, *J* = 4.9, 1.7 Hz, 1H), 7.99 (dd, *J* = 7.3, 1.7 Hz, 1H), 7.77 (s, 1H), 7.62 (m, 1H), 7.34 (m, 4H), 7.28 - 7.19 (m, 3H), 7.08 (dd, *J* = 7.3, 5.0 Hz, 1H), 4.89 (m, 1H), 4.84 - 4.76 (m, 1H), 4.47 (m, 1H), 4.21 (s, 2H), 3.97 (s, 3H), 3.63 (s, 3H).

MS ESI: m/z=508, [M+H]⁺ .

### Example 85: T-79

### (±)-5-benzyl-N-(7,10-dibromo-1-methyl-2-oxo-1,2,3,4-tetrahydro-[1,4] diazepino [3,2,1-hi] indol-3-1-4H-1,2,4-triazole-3-carboxamide

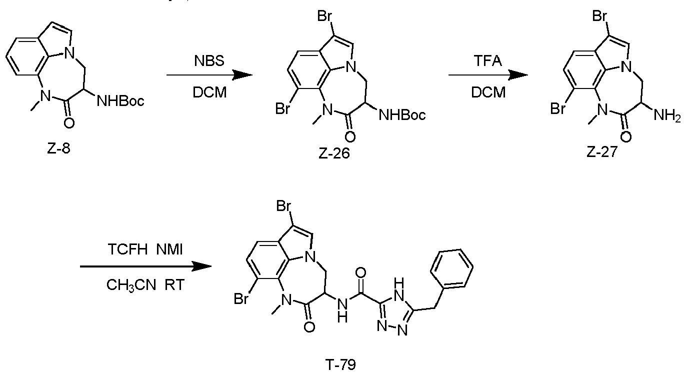

### Step 1: preparation of Z-26

A solution of Z-8 (1eq) and N-bromosuccinimide (1.1eq) in dichloromethane was stirred for 1 hour at room temperature and then was quenched with saturated sodium thiosulfate solution. The mixture was extracted with EA, and the extract was washed with water three times. The organic phase was concentrated and the residue was purified by SGCC to give Z-26 was obtained.

### Step 2: preparation of Z-27

A solution of Z-26 (1eq) and trifluoroacetic acid (10eq) in dichloromethane was stirred at room temperature for 3 hours. The mixture was concentrated and the residue was purified by SGCC to give Z-27.

MS ESI: m/z=374, [M+H]⁺ .

### Step 3: preparation of T-79

Acid-1 (1.5eq) and Z-27 (1eq) were used as starting materials to obtain T-79 under the same conditions as in Example 2.

¹H NMR (400 MHz, acetone-d₆) δ 8.51 (s, 1H), 7.70 (s, 1H), 7.41 (d, *J* = 8.4 Hz, 1H), 7.34 (m, 4H), 7.26 (m, 1H), 7.17 (d, *J* = 8.4 Hz, 1H), 4.85 (m, 1H), 4.77 - 4.70 (m, 1H), 4.45 (m, 1H), 4.21 (s, 2H), 3.58 (s, 3H).

MS ESI: m/z=560, [M+H]⁺ .

### Example 86: T-80

### (±)-5-benzyl-N-(7-(3-(methoxymethyl) phenyl)-1-methyl-2-oxo-1,2,3,4-tetrahydro-[1,4] diazepino [3,2,1-hi] indol-3-yl)-4H-1,2,4-triazole-3-carboxamide

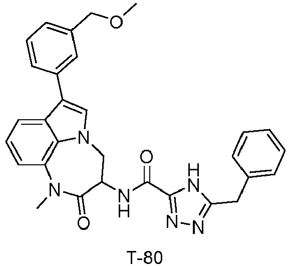

2-(3-(methoxymethyl) phenyl)-4,4,5,5-tetramethyl-1,3,2-dioxaborolan and the intermediate Z-9 were used to obtain T-80 under the same conditions as in Example 16.

¹H NMR (400 MHz, acetone-d₆) δ 8.58 (s, 1H), 7.78 (m, 1H), 7.68 (s, 2H), 7.63 (m, 1H), 7.45 (m, 1H), 7.39 - 7.30 (m, 4H), 7.29 - 7.21 (m, 4H), 4.87 (m, 1H), 4.81 (m, 1H), 4.53 (s, 2H), 4.47 (m, 1H), 4.21 (s, 2H), 3.63 (s, 3H), 3.39 (s, 3H).

MS ESI: m/z=521, [M+H]⁺ .

### Example 87: T-81

### 5-benzyl-N-(7-(1,4-dimethyl-1H-pyrazol-5-yl)-1-methyl-2-oxo-1,2,3,4-tetrahydro-[1,4]diazepino[3,2,1-hi]indol-3-yl)-4H-1,2,4-triazole-3-carboxamide

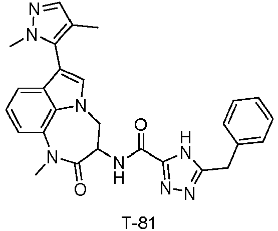

1,4-dimethylpyrazole-5-boronic acid pinacol ester and the intermediate Z-9 were used to obtain T-81 under the same conditions as in Example 16.

¹H NMR (400 MHz, acetone-d₆) δ 8.59 (s, 1H), 7.59 (s, 1H), 7.34 (m, 5H), 7.29 - 7.21 (m, 4H), 4.91 (m, 1H), 4.88 - 4.81 (m, 1H), 4.51 (m, 1H), 4.21 (s, 2H), 3.72 (s, 3H), 3.64 (s, 3H).

MS ESI: m/z=495, [M+H]⁺ .

### Example 88: T-82

### 5-benzyl-N-(1-methyl-2-oxo-7-(3-(trifluoromethyl) pyridin-2-yl)-1,2,3,4-tetrahydro-[1,4] diazepino[3,2,1-hi]indol-3-yl)-4H-1,2,4-triazole-3-carboxamide

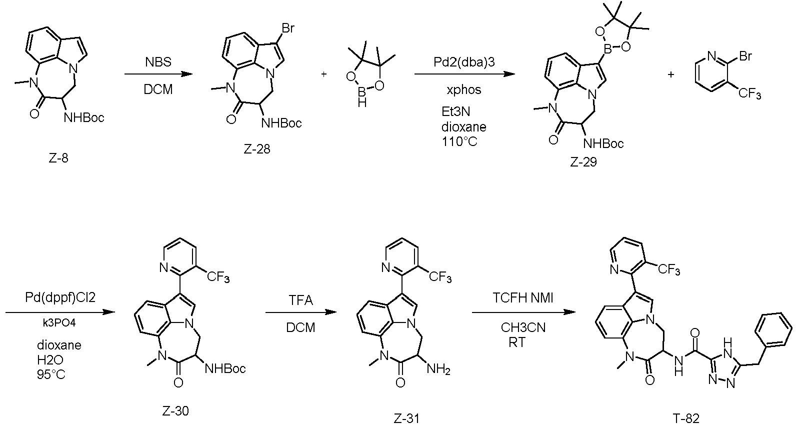

### Step 1: preparation of Z-28

A solution of Z-8 (1eq) and N-bromosuccinimide (1.1eq) in dichloromethane was stirred for 1 hour at room temperature, and then the reaction was quenched with saturated sodium thiosulfate solution. The mixture was extracted with EA, and the extract was washed with water three times concentrated. The residue was purified by SGCC to give Z-28.

MS ESI: m/z=396, [M+H]⁺ .

### Step 2: preparation of Z-29

A mixture of Z-28 (1eq), pinacolborane (2eq), Pd₂(dba)₃(0.02eq), Xphos (0.02eq) and triethylamine (3eq) in anhydrous 1,4-dioxane solution was stirred for 3 hours under nitrogen at 110°C. The reaction mixture was cooled to room temperature, poured into water and extracted with EA. The extract was washed with water three times, concentrate. The residue was purified by SGCC to give Z-29.

### Step 3: preparation of Z-30

A mixture of 2-bromo-3-trifluoromethylpyridine (1.5eq), the intermediate Z-29 (1eq), Pd (dppf)Cl₂ (O.leq), and K₃PO₄ in a mixed solvent of dioxane and water was stirred under argon protection at 95°C overnight. The reaction mixture was cooled to room temperature, poured into water and extracted with EA. The extract was washed with water three times, concentrate. The residue was purified by SGCC to give Z-30.

### Step 4: preparation of Z-31

A mixture of Z-30 (1eq) and TFA(lOeq) in dichloromethane was stirred at room temperature for 3 hours. The mixture was concentrated, and the residue was purified by SGCC to give Z-31.

MS ESI: m/z=361, [M+H]⁺ .

### Step 5: preparation of T-82

Acid-1 (1.5eq) and Z-31 (1eq) were used as starting materials to obtain T-82 under the same conditions as in Example 2.

¹H NMR (400 MHz, acetone-d₆) δ 8.96 (m, 1H), 8.56 (s, 1H), 8.26 (m, 1H), 7.81 (m, 1H), 7.73 (s, 1H), 7.54 (m, 1H), 7.34 (m, 4H), 7.29 - 7.21 (m, 3H), 4.94 - 4.83 (m, 2H), 4.55 - 4.46 (m, 1H), 4.21 (s, 2H), 3.64 (s, 3H).

MS ESI: m/z=546, [M+H]⁺.

### Example 89: T-83

### (±)-5-benzyl-N-(1-methyl-7-(1-methyl-1H-pyrazol-5-yl)-2-oxo-1,2,3,4-tetrahydro-[1,4] diazepino[3,2,1-hi]indol-3-yl)-oxazole-2-carboxamide

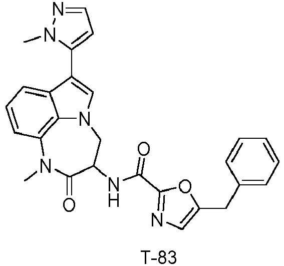

1-methyl-1H-pyrazole-5-boronic acid pinacol ester, the intermediate Z-9, and acid-2 were used to obtain T-83 under the same conditions as in Example 16.

¹H NMR (400 MHz, acetone-d₆) δ 8.46 (s, 1H), 7.66 (s, 1H), 7.49 (m, 2H), 7.32 (m, 4H), 7.25 (m, 3H), 7.08 (s, 1H), 6.45 (m, 1H), 4.88 - 4.75 (m, 2H), 4.48 (m, 1H), 4.15 (s, 2H), 3.89 (s, 3H), 3.61 (s, 3H).

MS ESI: m/z=481, [M+H]⁺.

### Example 90: T-84

### (±)-5-benzyl-N-(1-methyl-2-oxo-7-(2-(trifluoromethyl) phenyl)-1,2,3,4-tetrahydro-[1,4] diazepino [3,2,1-hi] indol-3-yl)-4H-1,2,4-triazole-3-carboxamide

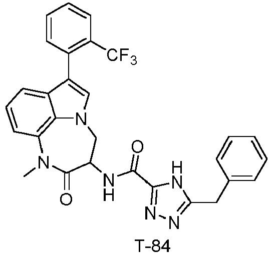

2-trifluoromethylphenylboronic acid pinacol ester and the intermediate Z-9 were used to obtain T-84 under the same conditions as in Example 16.

¹H NMR (400 MHz, acetone-d₆) δ 8.58 (s, 1H), 7.89 (d, *J* = 8.1 Hz, 1H), 7.76 (t, *J* = 7.4 Hz, 1H), 7.68 - 7.57 (m, 2H), 7.48 (s, 1H), 7.34 (q, *J* = 8.0 Hz, 4H), 7.27 - 7.22 (m, 3H), 7.22 - 7.16 (m, 1H), 4.89 (d, *J* = 6.7 Hz, 1H), 4.83 (d, *J* = 12.5 Hz, 1H), 4.53 - 4.45 (m, 1H), 4.21 (s, 2H), 3.64 (s, 3H).

MS ESI: m/z=555, [M+H]⁺.

### Example 91: T-85

### (±)-5-benzyl-N-(7-(2,6-dimethoxyphenyl)-1-methyl-2-oxo-1,2,3,4-tetrahydro-[1,4] diazepino [3,2,1-hi] indol-3-yl)-4H-1,2,4-triazole-3-carboxamide

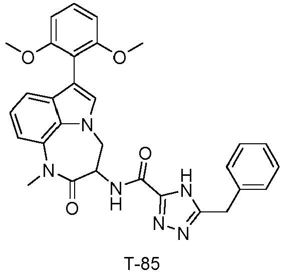

2,6-dimethoxyphenylborate pinacol ester and the intermediate Z-9 were used to obtain T-85 under the same conditions as in Example 16.

¹H NMR (400 MHz, acetone-d₆) δ 8.45 (s, 1H), 7.49 - 7.44 (m, 1H), 7.41 - 7.34 (m, 1H), 7.30 (m, 4H), 7.23 (m, 1H), 7.14 (m, 2H), 6.78 (m, 1H), 6.73 (m, 1H), 6.55 (s, 1H), 4.81 (m, 1H), 4.21 (m, 1H), 4.15 (s, 2H), 4.09 (m, 1H), 3.78 (s, 3H), 3.75 (s, 3H), 3.62 (s, 3H).

MS ESI: m/z=537, [M+H]⁺.

### Example 92: T-86

### 5-benzyl-N-(7-(3,5-dimethylisoxazol-4-yl)-1-methyl-2-oxo-1,2,3,4-tetrahydro-[1,4]diazepine[3,2,1-hi]indol-3-yl)-4H-1,2,4-triazole-3-carboxamide

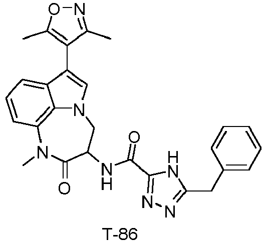

3,5-dimethylisoxazole-4-boronic acid pinacol ester and the intermediate Z-9 were used to obtain T-86 under the same conditions as in Example 16.

¹H NMR (400 MHz, acetone-d₆) δ 8.58 (s, 1H), 7.51 (s, 1H), 7.38 - 7.19 (m, 8H), 4.92 - 4.84 (m, 1H), 4.81 (m, 1H), 4.52 - 4.43 (m, 1H), 4.21 (s, 2H), 3.63 (s, 3H), 2.37 (s, 3H), 2.20 (s, 3H).

MS ESI: m/z=496, [M+H]⁺.

### Example 93: T-87

### (t)-5-benzyl-N-(7-(2-methoxyphenyl)-1-methyl-2-oxo-1,2,3,4-tetrahydro-[1,4] diazepine [3,2,1-hi] indol-3-yl)-4H-1,2,4-triazole-3-carboxamide

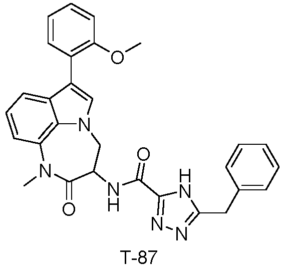

2-methoxyphenylboronic acid pinacol ester and the intermediate Z-9 were used to obtain T-87 under the same conditions as in Example 16.

¹H NMR (400 MHz, acetone-d₆) δ 8.58 (s, 1H), 7.60 (s, 1H), 7.59 - 7.52 (m, 2H), 7.38 - 7.28 (m, 5H), 7.27 - 7.11 (m, 4H), 7.05 (m, 1H), 4.93 - 4.86 (m, 1H), 4.79 (m, 1H), 4.49 - 4.40 (m, 1H), 4.21 (s, 2H), 3.85 (s, 3H), 3.63 (s, 3H).

MS ESI: m/z=507, [M+H]⁺.

### Example 94: T-88

### (±)-5-benzyl-N-(7-(isoxazol-4-yl)-1-methyl-2-oxo-1,2,3,4-tetrahydro-[1,4] diazepine [3,2,1-hi] indol-3-yl)-4H-1,2,4-triazole-3-carboxamide

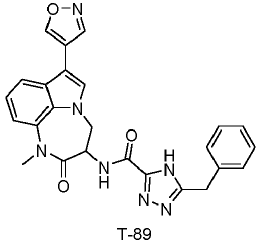

4-isoxazoleboronic acid pinacol ester and the intermediate Z-9 were used to obtain T-88 under the same conditions as in Example 16.

¹H NMR (400 MHz, acetone-*d*₆) δ 9.21 (s, 1H), 8.91 (s, 1H), 8.56 (s, 1H), 7.79 (s, 1H), 7.72 (m, 1H), 7.40 - 7.30 (m, 4H), 7.30 - 7.21 (m, 3H), 4.85 (m, 1H), 4.78 (m, 1H), 4.46 (m, 1H), 4.21 (s, 2H), 3.63 (s, 3H).

MS ESI: m/z=468, [M+H]⁺.

### Example 95: T-89

### (±)-3-(2-benzyl-3-chloro-7-oxo-2,4,5,7-tetrahydro-6H-pyrazole [3,4-c] pyridin-6-yl)-1-methyl-7-(1-methyl-1H-pyrazol-5-yl)-3,4-dihydro-[1,4] diazepine [3,2,1-hi] indole-2(1H)-one

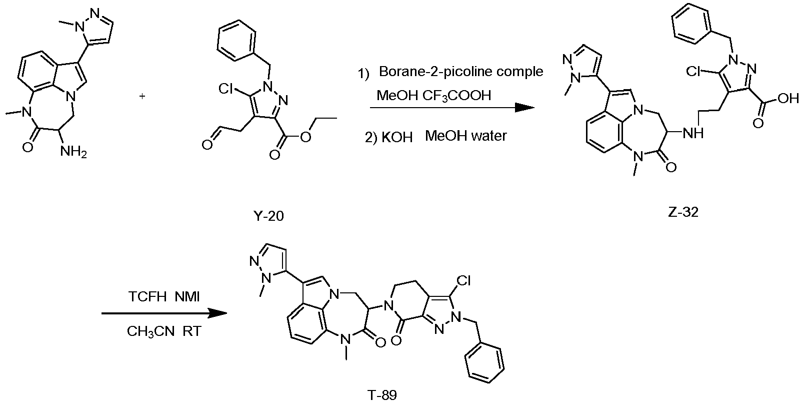

The amine intermediate of Example 89 was used as the starting material together with Y-20 to obtain T-89 under the same conditions as in Example 34.

¹H NMR (400 MHz, acetone-d₆) δ 7.62 (s, 1H), 7.50 - 7.44 (m, 2H), 7.39 - 7.28 (m, 5H), 7.25 (m, 2H), 6.44 (m, 1H), 5.52 - 5.46 (m, 1H), 5.45(s, 2H),5.06 - 4.99 (m, 1H), 4.90 (m, 1H), 4.08 - 4.01 (m, 1H), 3.88 (s, 3H), 3.77 - 3.70 (m, 1H), 3.56 (s, 3H), 2.83 (m, 2H).

MS ESI: m/z=541, [M+H]⁺.

### Example 96: T-90

### (±)-5-benzyl-N-(7-(1-isopropyl-1H-pyrazol-5-yl)-1-methyl-2-oxo-1,2,3,4-tetrahydro-1,4-diazepine [3,2,1-hi] indol-3-yl)-4H-1,2,4-triazole-3-carboxamide

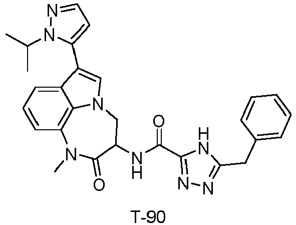

1-Isopropylpyrazole-5-boronic acid pinacol ester and Z-9 were used to obtain T-90 under the same conditions as in Example 16.

¹H NMR (400 MHz, acetone-*d*₆) δ 8.57 (s, 1H), 7.62 (s, 1H), 7.56 (d, *J* = 1.8 Hz, 1H), 7.43 - 7.27 (m, 6H), 7.27 - 7.20 (m, 2H), 6.35 (d, *J* = 1.8 Hz, 1H), 4.94 - 4.79 (m, 2H), 4.68 (p, *J* = 6.7 Hz, 1H), 4.48 (m, 1H), 4.25 (s, 2H), 3.63 (s, 3H), 1.43 (t, *J* = 6.8 Hz, 6H).

MS ESI: m/z=509, [M+H]⁺.

### Example 97: T-91

### (±)-5-benzyl-N-(1-methyl-7-(3-methylthiophen-2-yl)-2-oxo-1,2,3,4-tetrahydro-[1,4] diazepine [3,2,1-hi] indol-3-yl)-4H-1,2,4-triazole-3-carboxamide

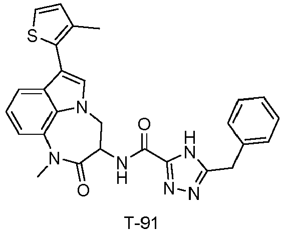

3-methylthiophene-2-boronic acid pinacol ester and Z-9 were used to obtain T-90 under the same conditions as in Example 16.

¹H NMR (400 MHz, DMSO-*d*₆) δ 7.60 (s, 1H), 7.47 (m, 1H), 7.38 (d, *J* = 5.1 Hz, 1H), 7.31 - 7.11 (m, 7H), 6.97 (d, *J* = 5.1 Hz, 1H), 4.71 (m, 1H), 4.64 - 4.56 (m, 1H), 4.41 (m, 1H), 4.08 (s, 2H), 3.45 (s, 3H), 2.19 (s, 3H).

MS ESI: m/z=497, [M+H]⁺.

### Example 98: preparation of intermediate Z-40

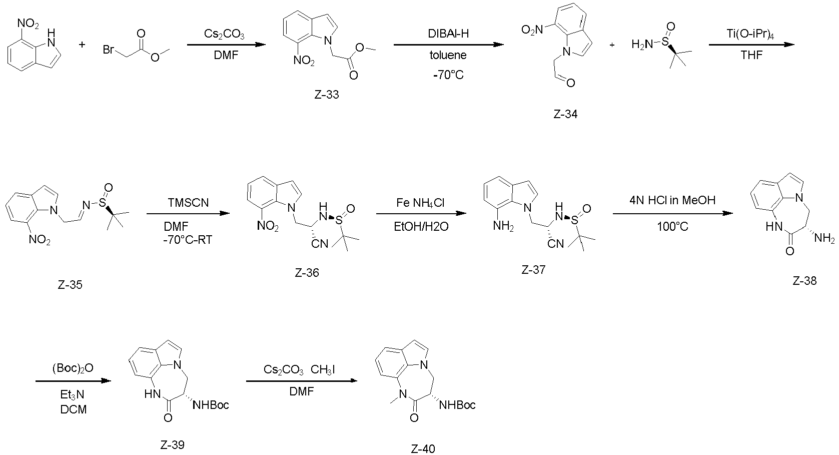

### Step 1: preparation of Z-33

A mixture 7-nitroindole (1eq), methyl bromoacetate (1.1eq), and cesium carbonate (1.5eq) in DMF was stirred for 2 hours at 0 °C. The reaction was quenched with water, and the mixture was extracted with EA. The extract was washed with water three times, and concentrated. The residue was purified by SGCC to give Z-33.

MS ESI: m/z=235, [M+H]⁺.

### Step 2: preparation of Z-34

A mixture of Z-33 (1eq) in anhydrous toluene was treated slowly with diisobutylaluminium hydride (1.5eq) at -70°C. After stirring at -70°C for 4 hours, methanol was added. After stirring for another 0.5 hour, the reaction mixture was quenched with 2M of sodium hydroxide aqueous solution, extracted with EA. The ectract was washed with water three times, and concentrated. The residue was purified by SGCC to give Z-34.

MS ESI: m/z=205, [M+H]⁺.

### Step 3: preparation of Z-35

A mixture of Z-34 (1eq), Ti(O-iPr)₄ (2.5eq), and R-(+)-tert-butylsulfinamide (1.2eq) in anhydrous THF was stirred at room temperature for 6 hours. The mixture was poured into water and extracted with EA. The extract was washed with water three times, and concentrated. The residue was purified by SGCC to give Z-35.

MS ESI: m/z=308, [M+H]⁺.

### Step 4: preparation of Z-36

To a solution of Z-35 (1eq) in anhydrous DMF was added trimethylsilyl cyanide (3eq) at -70°C. After stirring at -70°C to room temperature for 12 hours, the mixture was treated with water and extracted with EA. The extract was washed with water three times, and the organic phase was concentrated. The residue was purified by SGCC to give Z-36.

MS ESI: m/z=335, [M+H]⁺.

### Step 5: preparation of Z-37

A mixture of Z-36 (1eq) and iron powder (8eq) in ethanol solution was treated with aqueous solution of ammonium chloride (4eq) at room temperature. The mixture was stirred for 4 hours at room temperature, filtrated with celite, extracted with EA. The extract was washed with water three times. The organic phase was concentrated and the residue was purified by SGCC to give Z-37 was obtained.

MS ESI: m/z=305, [M+H]⁺.

### Step 6: preparation of Z-38

A solution of Z-37 (1eq) in 4N of hydrochloric methanol solution was stirred at 100°C for 1 hour. The mixture was cooled to room temperature, extracted with EA. The extract was washed with water three times, was concentrated. The residue was purified by SGCC to give intermediate Z-38.

MS ESI: m/z=202, [M+H]⁺.

### Step 7: preparation of Z-39

To a solution of Z-38 (1eq) in dichloromethane were added triethylamine (1eq) and di-tert-butyl dicarbonate (1.2eq). After stirring at room temperature for 3 hours, the mixture was diluted with EA and washed with water three times. The organic phase was concentrated and the residue was purified by SGCC to give Z-39.

### Step 8: preparation of Z-40

A mixture of Z-39 (1eq), cesium carbonate (1.5eq) and iodomethane (1.2eq) in anhydrous DMF, was stirred at room temperature for 3 hours. The mixture was diluted with EA, and washed with water three times. The organic phase was concentrated and the residue was purified by SGCC to give Z-40.

MS ESI: m/z=216, [M+H]⁺.

### Example 99: T-92

### Methyl(S)-2-(3-(5-benzyl-4H-1,2,4-triazole-3-carboxamide)-1-methyl-2-oxo-1,2,3,4-tetrahydro-[1,4] diazepine [3,2,1-hi] indol-7-yl)-2-oxoacetic acid

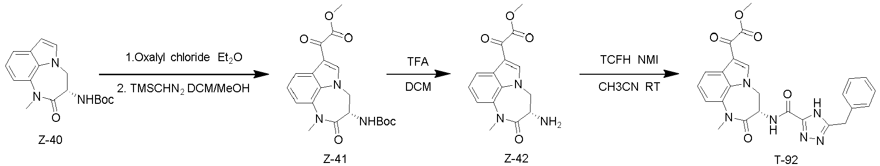

### Step 1: preparation of Z-41

A solution ofZ-40 (1eq) and oxalyl chloride (1.1eq) in anhydrous ether was stirred for 2.5 hours at 0 °C. The reaction mixture was concentrated. The resulting material was directly dissolved in a mixture of dichloromethane and methanol, followed by addition of trimethylsilyldiazomethane (2eq) . The mixture was stirred at room temperature for 3 hours, diluted with EA, washed with water three times. The organic phase was concentrated and the residue was purified by SGCC to give Z-41.

### Step 2: preparation of Z-42

A solution of Z-41 (1eq) and TFA (10eq) in dichloromethane was stirred at room temperature for 3 hours. The mixture was concentrated and the residue was purified by SGCC to give Z-42.

MS ESI: m/z=302, [M+H]⁺ .

### Step 3: preparation of T-92

Acid-1 (1.5eq) and Z-42 (1eq) were used as starting materials to obtain T-92 under the same conditions as in Example 2.

¹H NMR (400 MHz, acetone-d₆) δ 8.56 (s, 2H), 8.21 (m, 1H), 7.45 - 7.25 (m, 7H), 4.90 (m, 2H), 4.56 (m, 1H), 4.22 (s, 2H), 3.93 (s, 3H), 3.62 (s, 3H).

MS ESI: m/z=487, [M+H]⁺.

### Example 100: T-93

### (S)-5-benzyl-N-(1-methyl-2-oxo-7-(2,2,2-trifluoroacetyl)-1,2,3,4-tetrahydro-[1,4] diazepine [3,2,1-hi] indol-3-yl)-4H-1,2,4-triazole-3-carboxamide

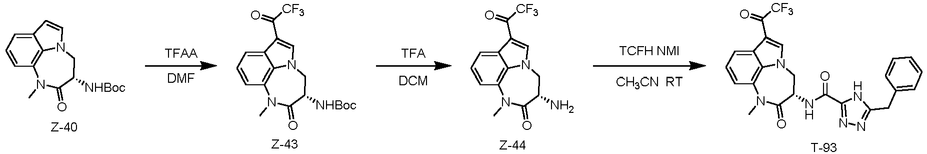

### Step 1: preparation of Z-43

A mixture of Z-40 (1eq) and TFAA (1. 5eq) in DMF was stirred at room temperature for 2 hours, and quenched with water. The mixture was extracted with EA, and the extract was washed with water three times. The organic phase was concentrated and the residue was purified by SGCC to give e Z-43.

### Step 2: preparation of Z-44

A solution of Z-43 (1eq) and trifluoroacetic acid (10eq) in dichloromethane was stirred at room temperature for 3 hours. The mixture was concentrated, and the residue was purified by SGCC to give Z-44.

MS ESI: m/z=312, [M+H]⁺ .

### Step 3: preparation of T-93

Acid-1 (1.5eq) and Z-44 (1eq) were used as starting materials to obtain T-93 under the same conditions as in Example 2.

¹H NMR (400 MHz, acetone-*d*₆) δ 8.57 (s, 2H), 8.21 (m, 1H), 7.52 - 7.39 (m, 2H), 7.34 (m, 4H), 7.26 (m, 1H), 4.97 (m, 2H), 4.62 (m, 1H), 4.23 (s, 2H), 3.63 (s, 3H).

MS ESI: m/z=497, [M+H]⁺.

### Example 101: T-94

### (S)-3-(5-benzyl-4H-1,2,4-triazole-3-carboxamide)-1-methyl-2-oxo-1,2,3,4-tetrahydro-[1,4] diazabicyclo [3,2,1-hi] indole-7-carboxylic acid

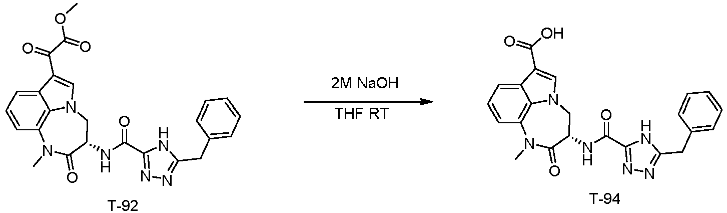

To a solution of T-92 (1eq) in THF solution was added 2M sodium hydroxide aqueous solution (3eq). The mixture was stirred at room temperature for 3 hours, quenched with water, and extracted with EA. The extract was washed with water three times and concentrated. The residue was purified by SGCC and HPLC to give T-94.

¹H NMR (400 MHz, acetone-*d*₆) δ 8.56 (s, 1H), 8.13 (s, 1H), 8.06 (m, 1H), 7.39 - 7.22 (m, 7H), 4.90 - 4.85 (m, 1H), 4.83 (m, 1H), 4.55 - 4.44 (m, 1H), 4.21 (s, 2H), 3.62 (s, 3H).

MS ESI: m/z=445, [M+H]⁺.

### Example 102: T-95

### Methyl (S)-3-(5-benzyl-4H-1,2,4-triazole-3-carboxamide)-1-methyl-2-oxo-1,2,3,4-tetrahydro-[1,4] diazepine [3,2,1-hi] indole-7-carboxylate

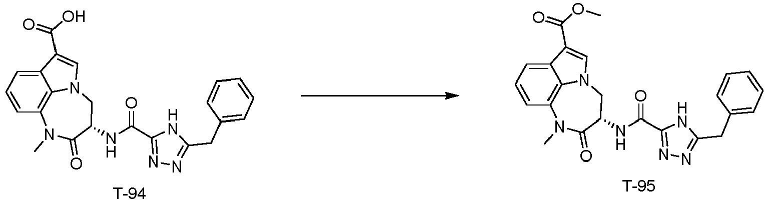

To a solution of T-94 (1eq) was directly dissolved in a mixed solvent of dichloromethane and methanol was added trimethylsilyldiazomethane (2eq). The mixture was stirred at room temperature for 3 hours, diluted with EA, washed with water three times. The organic phase was concentrated and the residue was purified by SGCC and HPLC to give T-95.

¹H NMR (400 MHz, acetone-*d*₆) δ 8.55 (s, 1H), 8.13 (s, 1H), 8.03 (m, 1H), 7.40 - 7.22 (m, 7H), 4.90 - 4.80 (m, 2H), 4.21 (m, 1H), 3.86 (s, 3H), 3.61 (s, 3H).

MS ESI: m/z=459, [M+H]⁺.

### Example 103: T-96

### (S)-5-benzyl-N-(1-methyl-2-oxo-6-(3-(trifluoromethyl)pyridin-2-yl)-2,3,4,6-tetrahydro-1H-azacyclo [4,3,2-cd] indol-3-yl)-4H-1,2,4-triazole-3-carboxamide

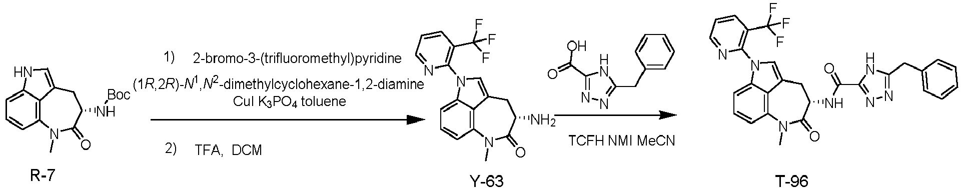

R-7 (400mg) was used as the starting material to obtain 520mg of T-96 as a white solid under the same conditions as in Example 42.

¹H NMR (400 MHz, acetone-d₆) δ 8.92 (d, J = 4.5 Hz, 1H), 8.67 (d, J = 5.7 Hz, 1H), 8.50 (d, J = 7.9 Hz, 1H), 7.83 (dd, J = 7.6, 4.9 Hz, 1H), 7.43 (s, 1H), 7.30 (m, 6H), 7.17 - 7.07 (m, 2H), 4.86 (m, 1H), 4.19 (s, 2H), 3.63 (s, 3H), 3.49 (m,1H), 3.20 - 3.05 (m, 1H).

MS ESI: m/z=546, [M+1]⁺.

### Example 104: T-97

### (±)-5-benzyl-N-(1-methyl-2-oxo-6-(3-(2,2,2-trifluoroethyl)pyridin-2-yl)-2,3,4,6-tetrahydro-1H-azacyclo [4,3,2-cd] indol-3-yl)-4H-1,2,4-triazole-3-carboxamide

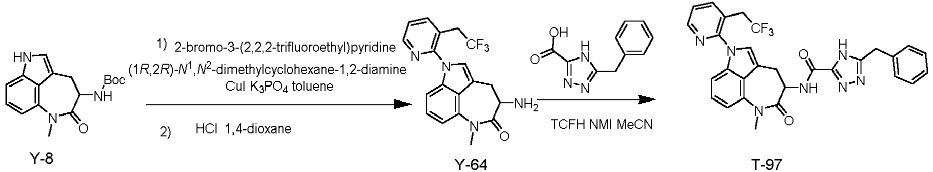

### Step 1: preparation of Y-63

Y-8 (30mg) and 2-bromo-3-(2,2,2-trifluoroethyl)pyridine were used as starting materials to obtain 33.9mg of Y-64 as a solid under the same conditions as in the Step 1 of Example 80.

MS ESI: m/z=375, [M+1]⁺.

### Step 2: preparation of T-97

Y-64 was used as the starting material to obtain 40mg of T-97 as a white solid under the same conditions as in the Step 2 of Example 40.

¹H NMR (400 MHz, acetone-d₆) δ 8.67 (m, 2H), 8.19 (d, J = 7.9 Hz, 1H), 7.63 (dd, J = 7.6, 4.6 Hz, 1H), 7.48 (s, 1H), 7.40 - 7.18 (m, 6H), 7.13 (d, J = 7.8 Hz, 1H), 7.02 (d, J = 8.2 Hz, 1H), 4.87 (m, 1H), 4.19 (s, 2H), 3.82 - 3.65 (m, 2H), 3.63 (s, 3H), 3.50 (m, 1H), 3.21 - 3.08 (m, 1H).

MS ESI: m/z=560, [M+1]⁺.

### Example 105: T-98

### (±)-5-(3-(5-benzyl-4H-1,2,4-triazole-3-carboxamido)-1-methyl-2-oxo-1,2,3,4-tetrahydro-6H-azacyclo [4,3,2-cd] indol-6-yl)oxazole-4-carboxylate

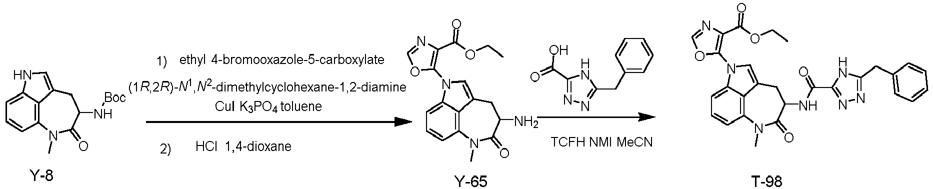

### Step 1: preparation of Y-65

Y-8 (30mg) and ethyl 4-bromoxazole-5-carboxylate were used as starting materials to obtain 31 mg of Y-65 as a solid under the same conditions as in the Step 1 of Example 80.

MS ESI: m/z=355, [M+1]⁺.

### Step 2: preparation of T-98

Y-65 was used as the starting material to obtain 4mg of T-98 as a white solid under the same conditions as in the Step 2 of Embodiment 40.

¹H NMR (400 MHz, acetone-d₆) δ 8.65 (m, 1H), 8.55 (s, 1H), 7.79 (s, 1H), 7.70 (d, J = 8.3 Hz, 1H), 7.34 (m, 5H), 7.24 (m, 1H), 7.18 (d, J = 7.9 Hz, 1H), 4.84 (m, 1H), 4.35 (q, J = 7.1 Hz, 2H), 4.22 (s, 2H), 3.62 (s, 3H), 3.48 (m, 1H), 3.11 (m, 1H), 1.30 (t, J = 7.1 Hz, 3H).

MS ESI: m/z=540, [M+1]⁺.

### Example 106: T-99

### (±)-5-benzyl-N-(6-(isoquinolin-1-yl)-1-methyl-2-oxo-2,3,4,6-tetrahydro-1H-aza [4,3,2-cd] indol-3-yl)-4H-1,2,4-triazole-3-carboxamide

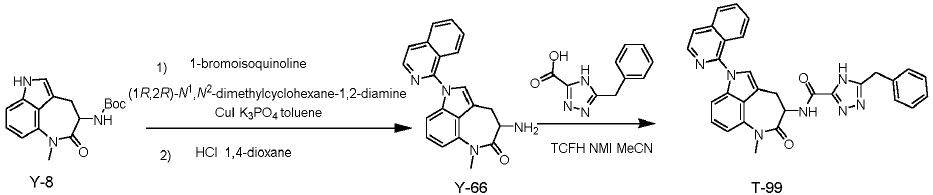

### Step 1: preparation of Y-66

Y-8 (30mg) and 1-bromoisoquinoline were used as starting materials to obtain 27mg of Y-66 as a solid under the same conditions as in the Step 1 of Example 80.

MS ESI: m/z=355, [M+1]⁺.

### Step 2: preparation of T-99

Y-66 was used as the starting material to obtain 25.7mg of T-99 as a pale-yellow solid under the same conditions as in the Step 2 of Example 40.

¹H NMR (400 MHz, acetone-d₆) δ 8.70 (m, 1H), 8.53 (d, J = 5.3 Hz, 1H), 8.16 (d, J = 8.1 Hz, 1H), 7.94 (m, 2H), 7.89 (t, J = 7.4 Hz, 1H), 7.73 (d, J = 7.3 Hz, 1H), 7.69 (s, 1H), 7.41 - 7.20 (m, 5H), 7.17 (t, J = 8.6 Hz, 3H), 4.93 (m, 1H), 4.20 (s, 2H), 3.66 (s, 3H), 3.56 (m, 1H), 3.25 - 3.13 (m, 1H).

MS ESI: m/z=528, [M+1]⁺.

### Example 107: T-100

### (±)-methyl4-(3-(5-benzyl-4H-1,2,4-triazole-3-carboxamide)-1-methyl-2-oxo-1,2,3,4-tetrahydro-6H-azacyclo [4,3,2-cd] indol-6-yl)-1-methyl-1H-pyrrole-2-carboxylate

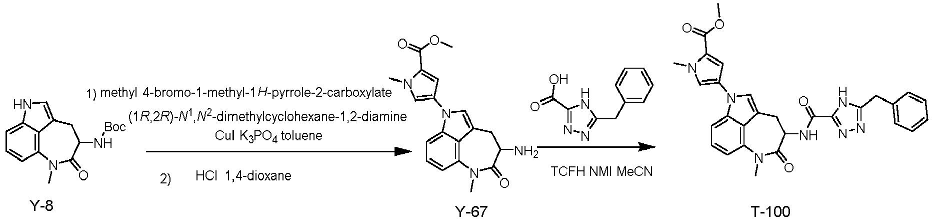

### Step 1: preparation of Y-67

Y-8 (50mg) and 1-bromoisoquinoline were used as starting materials to obtain 38.9mg of Y-67 as solid under the same conditions as in the Step 1 of Example 80.

MS ESI: m/z=353, [M+1]⁺.

### Step 2: preparation of T-100

Y-67 was used as the starting material to obtain 28mg of T-100 as a white solid under the same conditions as in the Step 2 of Example 40.

¹H NMR (400 MHz, acetone-d₆) δ 8.68 (m, 1H), 7.44 (m, 1H), 7.40 - 7.16 (m, 8H), 7.08 (m, 2H), 4.80 (m, 1H), 4.20 (s, 2H), 4.05 (s, 3H), 3.83 (s, 3H), 3.61 (s, 3H), 3.45 (m, 1H), 3.18 - 2.98 (m, 1H).

MS ESI: m/z=538, [M+1]⁺.

### Example 108: T-101

### (±)-5-benzyl-N-(1-methyl-2-oxo-6-(pyridin-3-yl)-2,3,4,6-tetrahydro-1H-azacyclo [4,3,2-cd] indol-3-yl)-4H-1,2,4-triazole-3-carboxamide

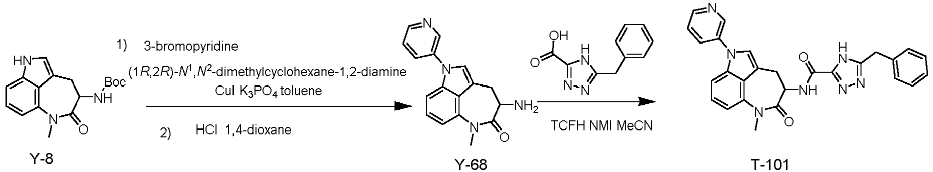

### Step 1: preparation of Y-68

Y-8 (30mg) and 3-bromopyridine were used as starting materials to obtain 29.8mg of Y-68 as a solid under the same conditions as in the Step 1 of Example 80.

MS ESI: m/z=293, [M+1]⁺.

### Step 2: preparation of T-101

Y-68 was used as the starting material to obtain 12.4mg of T-101 as a white solid under the same conditions as in the Step 2 of Example 40.

¹H NMR (400 MHz, DMSO-d₆) δ 8.86 (m, 1H), 8.75 (m, 1H), 8.63 (d, J = 4.0 Hz, 1H), 8.09 (d, J = 8.3 Hz, 1H), 7.69 (s, 1H), 7.64 (dd, J = 7.9, 4.8 Hz, 1H), 7.45 - 7.20 (m, 7H), 7.14 (d, J = 7.6 Hz, 1H), 4.69 (m, 1H), 4.14 (s, 2H), 3.53 (s, 3H), 3.49 (s, 1H), 3.18 - 3.05 (m, 1H).

MS ESI: m/z=478, [M+1]⁺.

### Example 109: T-102

### (±)-5-benzyl-N-(1-methyl-2-oxo-6-(pyridin-4-yl)-2,3,4,6-tetrahydro-1H-azacyclo [4,3,2-cd] indol-3-yl)-4H-1,2,4-triazole-3-carboxamide

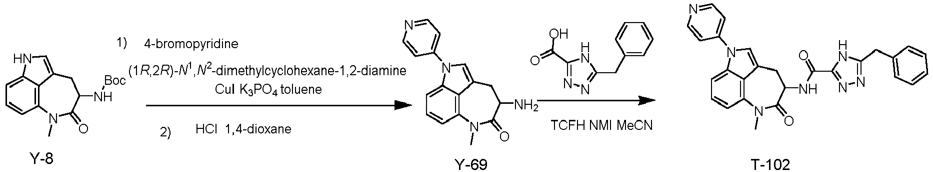

### Step 1: preparation of Y-69

Y-8 (30mg) and 4-bromopyridine were used as starting materials to obtain 27.6mg of slid Y-69 as a solid under the same conditions as in the Step 1 of Example 80.

MS ESI: m/z=293, [M+1]⁺.

### Step 2: preparation of T-102

Y-69 was used as the starting material to obtain 10.5mg of T-102 as a white solid under the same conditions as in the Step 2 of Example 40.

¹H NMR (400 MHz, acetone-d₆) δ 8.76 (s, 2H), 8.67 (m, 1H), 7.71 (m, 2H), 7.65 (m, 2H), 7.49 - 7.29 (m, 5H), 7.25 (m, 1H), 7.19 (d, J = 7.8 Hz, 1H), 4.82 (m, 1H), 4.26 (s, 2H), 3.62 (s, 3H), 3.49 (m, 1H), 3.13 (m, 1H).

MS ESI: m/z=478, [M+1]⁺.

### Example 110: T-103

### (±)-2-(3-(5-benzyl-4H-1,2,4-triazole-3-carboxamide)-1-methyl-2-oxo-1,2,3,4-tetrahydro-6H-azacyclo [4,3,2-cd] indol-6-yl)ethyl nicotinate

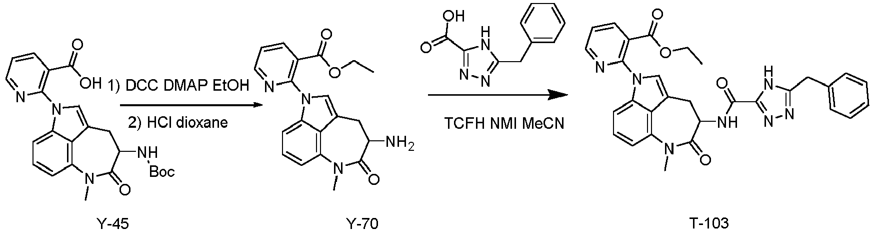

### Step 1: preparation of Y-70

A mixture of DCC (21.6mg), DMAP (4.3mg), EtOH (40ul), and Y-45 (30mg) in DCM (2mL) was stirred overnight, quenched with a saturated NH₄Cl solution. The mixture was extracted with ethyl acetate. The extract was concentrated and the residue was purified by SGCC to give 41.8mg of crude intermediate which was dissolved in a 4N HCl dioxane solution, and stirred at room temperature for 2 hours. The mixture was then concentrated to give Y-70.

MS ESI: m/z=365, [M+1]⁺.

### Step 2: preparation of T-103

Y-70 was used as the starting material to obtain 12.4mg of T-103 as a white solid under the same conditions as in the Step 2 of Example 40.

¹H NMR (400 MHz, acetone-d₆) δ 8.79 (m, 1H), 8.67 (m, 1H), 8.40 (d, J = 6.0 Hz, 1H), 7.61 (dd, J = 7.6, 4.8 Hz, 1H), 7.47 (s, 1H), 7.32 (m, 7H), 7.13 (m, 1H), 4.86 (m, 1H), 4.20 (s, 2H), 4.06 (q, J = 7.1 Hz, 2H), 3.63 (s, 2H), 3.50 (d, J = 14.7 Hz, 1H), 3.20 - 3.03 (m, 2H), 0.93 (t, J = 7.1 Hz, 3H).

MS ESI: m/z=550, [M+1]⁺.

### Example 111: T-104

### (±)-2-(3-(5-benzyl-4H-1,2,4-triazole-3-carboxamide)-1-methyl-2-oxo-1,2,3,4-tetrahydro-6H-azacyclo [4,3,2-cd] indol-6-yl)isopropyl nicotinate

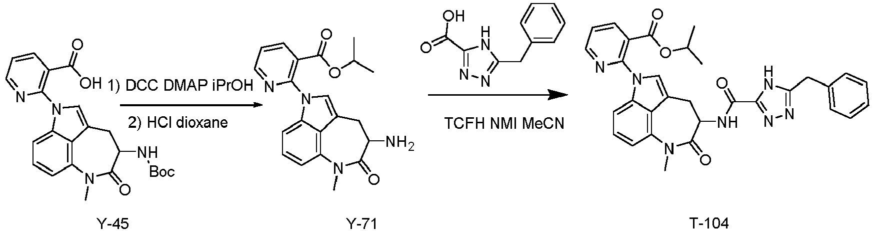

### Step 1: preparation of Y-71

Y-45 (30mg) and i-PrOH were used as starting materials to obtain 31.6mg of Y-71 as a solid under the same conditions as in the Step 1 of Example 110.

MS ESI: m/z=379, [M+1]⁺.

### Step 2: preparation of T-104

Y-71 was used as the starting material to obtain 16.9mg of T-104 as a white solid under the same conditions as in the Step 2 of Example 40.

¹H NMR (400 MHz, DMSO-d₆) δ 8.84 - 8.76 (m, 1H), 8.69 (m, 1H), 8.38 (dd, J = 7.7, 1.6 Hz, 1H), 7.64 (dd, J = 7.7, 4.8 Hz, 1H), 7.56 (s, 1H), 7.40 - 7.17 (m, 6H), 7.11 (dd, J = 7.9, 4.6 Hz, 2H), 4.80 (q, 6.2 Hz, 1H), 4.73 - 4.64 (m, 1H), 4.14 (s, 2H), 3.52 (s, 3H), 3.29 (m, 1H), 3.19 - 3.02 (m, 1H), 0.91 (d, J = 6.2 Hz, 3H), 0.84 (d, J = 6.2 Hz, 3H).

MS ESI: m/z=564, [M+1]⁺.

### Example 112: T-105

### (±)-5-benzyl-N-(1-methyl-2-oxo-6-(3-(trifluoromethoxy) pyridin-2-yl)-2,3,4,6-tetrahydro-1H-azacyclo [4,3,2-cd] indol-3-yl)-4H-1,2,4-triazole-3-carboxamide

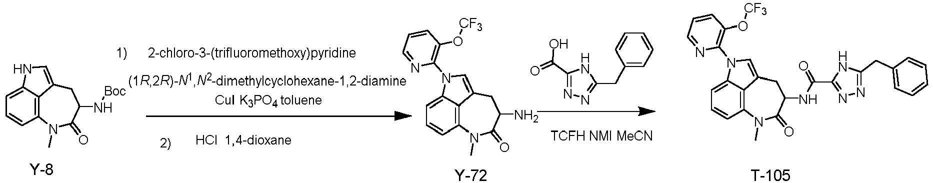

### Step 1: preparation of Y-72

Y-8 (30mg) and 2-bromo-3-(trifluoromethoxy)pyridine were used as starting materials to obtain 22mg of Y-72 as a solid under the same conditions as in the Step 1 of Example 80.

MS ESI: m/z=377, [M+1]⁺.

### Step 2: preparation of T-105

Y-72 was used as the starting material to obtain 17.8mg of T-105 as a white solid under the same conditions as in the Step 2 of Example 40.

¹H NMR (400 MHz, acetone-d₆) δ 8.66 (d, J = 3.6 Hz, 1H), 8.15 (d, J = 7.9 Hz, 1H), 7.64 (dd, J = 8.5, 5.0 Hz, 1H), 7.61 (s, 1H), 7.54 (d, J = 8.1 Hz, 1H), 7.42 - 7.29 (m, 5H), 7.25 (m, 1H), 7.17 (d, J = 7.8 Hz, 1H), 4.85 (m, 1H), 4.19 (s, 2H), 3.63 (s, 3H), 3.50 (m 1H), 3.18 - 3.05 (m, 1H).

MS ESI: m/z=562, [M+1]⁺.

### Example 113: T-106

### (±)-5-benzyl-N-(1-methyl-2-oxo-6-(pyridazin-3-yl)-2,3,4,6-tetrahydro-1H-aza [4,3,2-cd] indol-3-yl)-4H-1,2,4-triazole-3-carboxamide

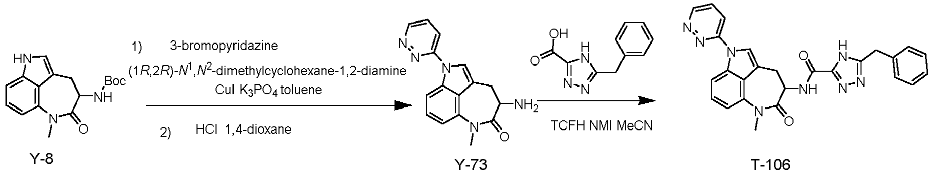

### Step 1: preparation of Y-73

Y-8 (30mg) and 3-bromopyridazine were used as starting materials to obtain 30.6mg of Y-73 as a solid under the same conditions as in the Step 1 of Example 80.

MS ESI: m/z=294, [M+1]⁺.

### Step 2: preparation of T-106

Y-73 was used as the starting material to obtain 13.8mg of T-106 as a white solid under the same conditions as in the Step 2 of Example 40.

¹H NMR (400 MHz, acetone-d₆) δ 9.16 (m, 1H), 8.66 (m, 1H), 8.39 (m, 1H), 8.10 (m, 1H), 7.92 (s, 1H), 7.88 (dd, J = 9.1, 4.2 Hz, 1H), 7.35 (m, 7H), 4.82 (m, 1H), 4.20 (s, 2H), 3.63 (s, 3H), 3.51 (m, 1H), 3.14 (m,1H).

MS ESI: m/z=479, [M+1]⁺.

### Example 114: T-107

### (±)-5-benzyl-N-(6-(3-(difluoromethoxy)pyridin-2-yl)-1-methyl-2-oxy-2,3,4,6-tetrahydro-1H-azacyclo [4,3,2-cd] indol-3-yl)-4H-1,2,4-triazole-3-carboxamide

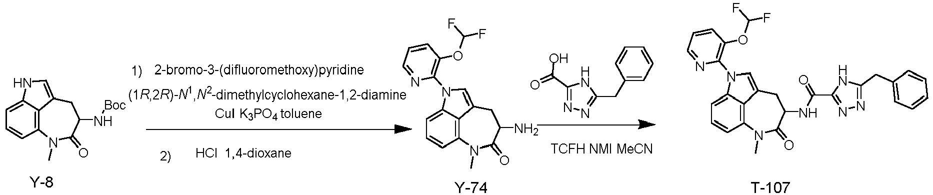

### Step 1: preparation of Y-74

Y-8 (30mg) and 2-bromo-3-(difluoromethoxy)pyridine were used as starting materials to obtain 15.6mg of Y-74 as a solid under the same conditions as in the Step 1 of Example 80.

MS ESI: m/z=359, [M+1]⁺.

### Step 2: preparation of T-107

Y-74 was used as the starting material to obtain 17mg of T-107 as a white solid under the same conditions as in the Step 2 of Example 40.

¹H NMR (400 MHz, acetone-d₆) δ 8.67 (m, 1H), 8.50 (m, 1H), 7.98 (d, J = 8.0 Hz, 1H), 7.61 (s, 1H), 7.55 (m, 2H), 7.40 - 7.24 (m, 6H), 7.14 (d, J = 7.8 Hz, 1H), 7.00 (t, J = 72.7 Hz, 1H), 4.84 (m ,1H), 4.19 (s, 2H), 3.62 (s, 3H), 3.49 (m, 1H), 3.19 - 3.01 (m, 1H).

MS ESI: m/z=544, [M+1]⁺.

### Example 115: T-108

### (±)-5-benzyl-N-(6-(3-(ethylsulfonyl)pyridin-2-yl)-1-methyl-2-oxo-2,3,4,6-tetrahydro-1H-aza [4,3,2-cd] indol-3-yl)-4H-1,2,4-triazole-3-carboxamide

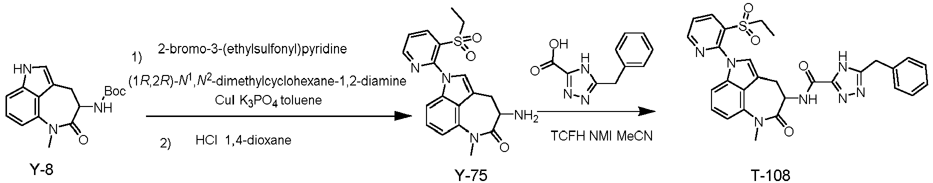

### Step 1: preparation of Y-75

Y-8 (30mg) and 2-bromo-3-(ethylsulfonyl chloride)pyridine were used as starting materials to obtain 37.7mg of Y-75 as a solid under the same conditions as in the Step 1 of Example 80.

MS ESI: m/z=385, [M+1]⁺.

### Step 2: preparation of T-108

Y-75 was used as the starting material to obtain 34.8mg of T-108 as a white solid under the same conditions as in the Step 2 of Example 40.

¹H NMR (400 MHz, acetone-d₆) δ 8.92 (m, 1H), 8.68 (m,2H), 7.88 (dd, J = 7.8, 4.7 Hz, 1H), 7.59 (s, 1H), 7.41 - 7.19 (m, 6H), 7.19 - 7.06 (m, 2H), 4.87 (m, 1H), 4.19 (s, 2H), 3.63 (s, 3H), 3.48 (m, 1H), 3.27 - 3.02 (m, 1H), 2.88 (q, J = 7.1Hz, 2H), 1.05 (t, J = 7.1 Hz, 3H).

MS ESI: m/z=570, [M+1]⁺.

### Example 116: T-109

### (±)-5-benzyl-N-(6-(3-(cyclopropylsulfonyl)pyridin-2-yl)-1-methyl-2-oxo-2,3,4,6-tetrahydro-1H-azacyclo [4,3,2-cd] indol-3-yl)-4H-1,2,4-triazole-3-carboxamide

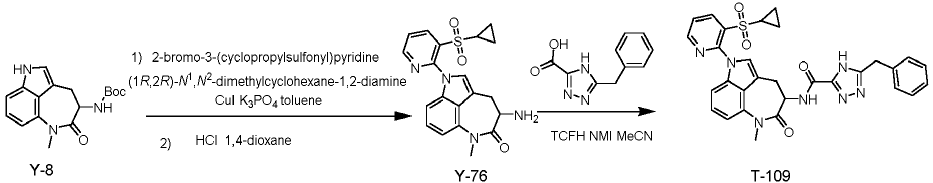

### Step 1: preparation of Y-76

Y-8 (30mg) and 2-bromo-3-(cyclopropylsulfonyl)pyridine were used as starting materials to obtain 27.1mg of Y-76 as a solid under the same conditions as in the Step 1 of Example 80.

MS ESI: m/z=397, [M+1]⁺.

### Step 2: preparation of T-109

Y-76 was used as the starting material to obtain 26.6mg of T-109 as a white solid under the same conditions as in the Step 2 of Example 40.

¹H NMR (400 MHz, acetone-d₆) δ 8.90 (m, 1H), 8.64 (m, 2H), 7.86 (m, 1H), 7.57 (s, 1H), 7.30 (m, 6H), 7.12 (m, 2H), 4.87 (m, 1H), 4.23 (s, 2H), 3.62 (s, 3H), 3.49 (m, 1H), 3.14 (m, 1H), 2.21 (m, 1H), 0.93 (m, 2H), 0.81 (m, 2H).

MS ESI: m/z=582, [M+1]⁺.

### Example 117: T-110

### (±)-5-benzyl-N-(6-(3-(dimethylphosphoryl)pyridin-2-yl)-1-methyl-2-oxo-2,3,4,6-tetrahydro-1H-aza [4,3,2-cd] indol-3-yl)-4H-1,2,4-triazole-3-carboxamide

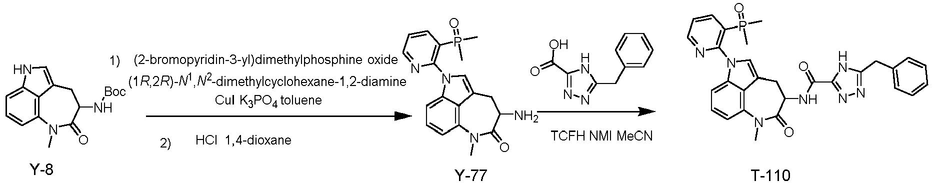

### Step 1: preparation of Y-77

Y-8 (30mg) and (2-bromopyridin-3-yl)dimethylphosphine oxide were used as starting materials to prepare 19.7mg of Y-77 as a solid under the same conditions as in the Step 1 of Example 80.

MS ESI: m/z=369, [M+1]⁺.

### Step 2: preparation of T-110

Y-77 was used as the starting material to obtain 19.8mg of T-110 as a white solid under the same conditions as in the Step 2 of Example 40.

¹H NMR (400 MHz, acetone-d₆) δ 8.78 (m, 1H), 8.66 (m, 1H), 8.62 - 8.53 (m, 1H), 7.73 (m, 2H), 7.30 (m, 6H), 7.16 (m, 2H), 4.86 (m, 1H), 4.18 (s, 2H), 3.63 (s, 3H), 3.47 (m, 1H), 3.22 - 3.03 (m, 1H), 1.49 (d, J = 13.5 Hz, 3H), 1.40 (d, J = 13.5 Hz, 3H).

MS ESI: m/z=554, [M+1]⁺.

### Example 118: T-111

### (±)-5-benzyl-N-(1-methyl-2-oxo-6-(3-((trifluoromethyl)thio)pyridin-2-yl)-2,3,4,6-tetrahydro-1H-aza [4,3,2-cd] indol-3-yl)-4H-1,2,4-triazole-3-carboxamide

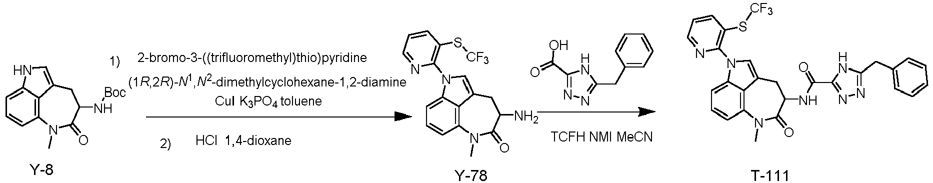

### Step 1: preparation of Y-78

Y-8 (30mg) and 2-bromo-3-(( trifluoromethyl)thio)pyridine were used as starting materials to obtain 15.9mg of Y-78 as a solid under the same conditions as in the Step 1 of Example 80.

MS ESI: m/z=393, [M+1]⁺.

### Step 2: preparation of T-111

Y-78 was used as the starting material to obtain 18.5mg of T-111 as a white solid under the same conditions as in the Step 2 of Example 40.

¹H NMR (400 MHz, acetone-d₆) δ 8.82 (dd, J = 4.7, 1.6 Hz, 1H), 8.70 (d, J = 6.1 Hz, 1H), 8.46 (d, J = 7.3 Hz, 1H), 7.68 (dd, J = 7.8, 4.7 Hz, 1H), 7.60 (s, 1H), 7.39 - 7.27 (m, 5H), 7.27 - 7.20 (m, 2H), 7.14 (m, 2H), 4.86 (m, 1H), 4.20 (s, 2H), 3.63 (s, 3H), 3.48 (m, 1H), 3.11 (m, 1H).

MS ESI: m/z=578, [M+1]⁺.

### Example 119: T-112

### (S)-5-benzyl-N-(6-(3-(difluoromethoxy)pyridin-2-yl)-1-methyl-2-oxo-2,3,4,6-tetrahydro-1H-aza [4,3,2-cd] indol-3-yl)-4H-1,2,4-triazole-3-carboxamide

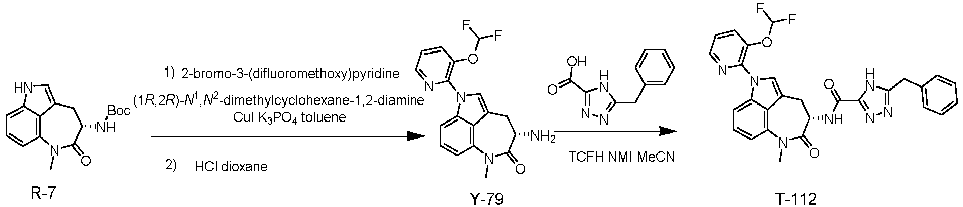

R-7 (200mg) was used as the starting material to obtain 306mg of T-112 as a white solid under the same conditions as in Example 114.

1H NMR (400 MHz, acetone-d₆) δ 8.68 (m, 1H), 8.51 (m, 1H), 7.98 (d, J = 7.9 Hz, 1H), 7.61 (s, 1H), 7.54 (m, 2H), 7.42 - 7.26 (m, 6H), 7.18 - 7.11 (m, 1H), 7.01 (t, J = 72.7 Hz, 1H), 4.85 (m, 1H), 4.20 (s, 2H), 3.62 (s, 3H), 3.49 (m, 1H), 3.20 - 3.00 (m, 1H).

MS ESI: m/z=544, [M+1]⁺.

### Example 120: T-113

### (S)-5-benzyl-N-(1-methyl-2-oxo-7-(3-(trifluoromethyl)-1H-pyrazol-4-yl)-1,2,3,4-tetrahydro-[1,4] diazepine [3,2,1-hi] indol-3-yl)-4H-1,2,4-triazole-3-carboxamide

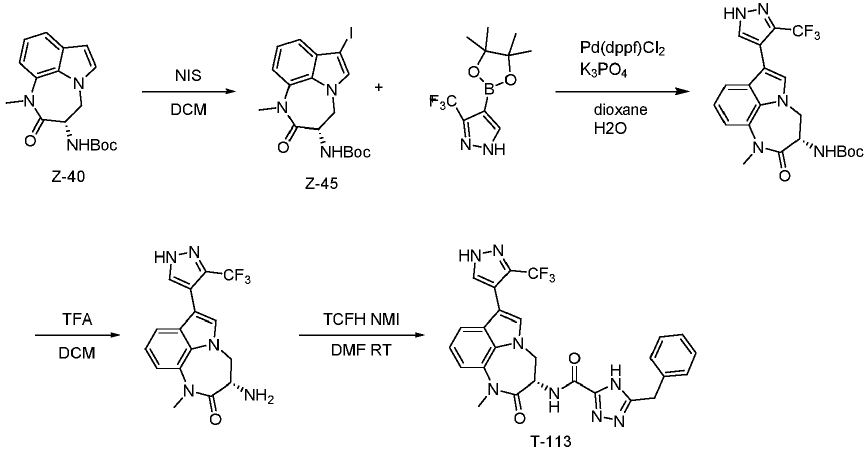

Z-45 was obtained under the same conditions as in the Example 4. 3-trifluoromethyl-1H-pyrazole-4-boric acid phenyl ester and the intermediate Z-45 were used to obtain T-113 under the same conditions as in the Example 16.

¹H NMR (400 MHz, acetone-*d*₆) δ 8.56 (s, 1H), 8.20 (s, 1H), 7.49 (m, 2H), 7.40 - 7.29 (m, 4H), 7.29 - 7.17 (m, 3H), 4.84 (m, 2H), 4.51 - 4.41 (m, 1H), 4.21 (s, 2H), 3.63 (s, 3H).

MS ESI: m/z=535 [M+H]⁺ .

### Example 121: T-114

### (±)-5-benzyl-N-(1-methyl-2-oxo-7-(1,3,5-trimethyl-1H-pyrazol-4-yl)-1,2,3,4-tetrahydro-[1,4] diazepine [3,2,1-hi] indol-3-yl)-4H-1,2,4-triazole-3-carboxamide

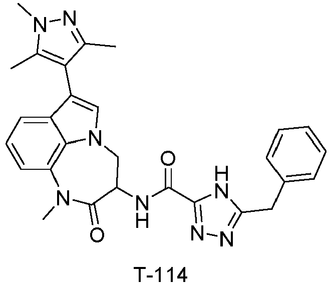

1,3,5-trimethyl-1H-pyrazol-4-boronic acid pinacol ester and Z-9 were used to obtain T-114 under the same conditions as in Example 16.

¹H NMR (400 MHz, acetone-*d*₆) δ 8.44 (s, 1H), 7.25 - 7.15 (m, 5H), 7.07 (m, 4H), 4.74 (m, 1H), 4.66 (m, 1H), 4.30 (m, 1H), 4.08 (s, 2H), 3.63 (s, 3H), 3.50 (s, 3H), 2.07 (s, 3H), 1.98 (s, 3H).

MS ESI: m/z=509[M+H]⁺ .

### Example 122: T-115

### (S)-5-benzyl-N-(7-(1-(difluoromethyl)-1H-pyrazol-4-yl)-1-methyl-2-oxo-1,2,3,4-tetrahydro-1,4-diazepine [3,2,1-hi] indol-3-yl)-4H-1,2,4-triazole-3-carboxamide

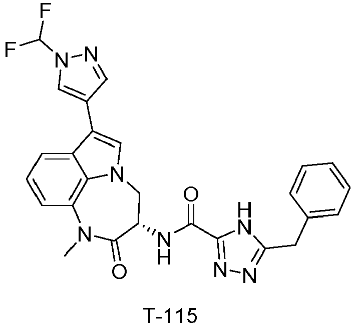

1-difluoromethyl-pyrrole-4-boronic acid pinacol ester and the intermediate Z-45 were used to obtain T-115 under the same conditions as in Example 16.

¹H NMR (400 MHz, acetone-*d*₆) δ 8.58 (s, 1H), 8.49 (s, 1H), 8.12 (s, 1H), 7.75 (m, 2H), 7.38 - 7.28 (m, 4H), 7.27 - 7.19 (m, 3H), 4.84 (m, 1H), 4.76 (m, 1H), 4.43 (m, 1H), 4.20 (s, 2H), 3.61 (s, 3H).

MS ESI: m/z=517[M+H]⁺ .

### Example 123: T-116

### (S)-5-benzyl-N-(1-methyl-2-oxo-7-(1H-pyrazol-5-yl)-1,2,3,4-tetrahydro-[1,4] diazepine [3,2,1-hi] indol-3-yl)-4H-1,2,4-triazole-3-carboxamide

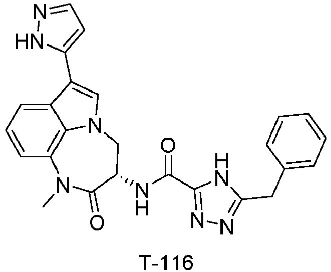

1H-pyrazol-3-boronic acid pinacol ester and Z-45 were used to obtain T-116 under the same conditions as in Example 16.

¹H NMR (400 MHz, acetone-d₆) δ 8.57 (s, 1H), 8.12 (s, 1H), 7.76 (m, 1H), 7.72 (m, 1H), 7.40 - 7.29 (m, 4H), 7.25 (m, 3H), 6.65 (m, 1H), 4.86 (m, 1H), 4.78 (m, 1H), 4.49 - 4.40 (m, 1H), 4.21 (s, 2H), 3.63 (s, 3H).

MS ESI: m/z=467 [M+H]⁺ .

### Example 124: T-117

### (S)-5-benzyl-N-(7-(1,3-dimethyl-1H-pyrazol-4-yl)-1-methyl-2-oxo-1,2,3,4-tetrahydro-1,4-diazepine [3,2,1-hi] indol-3-yl)-4H-1,2,4-triazole-3-carboxamide

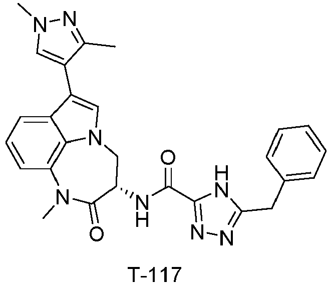

1,3-dimethyl-1H-pyrazol-4-boronic acid pinacol ester and Z-45 were used to obtain T-117 under the same conditions as in Example 16.

¹H NMR (400 MHz, acetone-*d*₆) δ 8.56 (s, 1H), 7.83 (s, 1H), 7.54 (m, 1H), 7.45 (s, 1H), 7.34 (m, 4H), 7.27 - 7.16 (m, 3H), 4.88 - 4.82 (m, 1H), 4.78 (m, 1H), 4.47 - 4.38 (m, 1H), 4.21 (s, 2H), 3.88 (s, 3H), 3.62 (s, 3H), 2.31 (s, 3H).

MS ESI: m/z=495 [M+H]⁺.

### Example 125: T-118

### (S)-5-benzyl-N-(1-methyl-7-(1-methyl-3-(trifluoromethyl)-1H-pyrazol-4-yl)-2-oxo-1,2,3,4-tetrahydro-[1,4] diazepine [3,2,1-hi] indol-3-yl)-4H-1,2,4-triazole-3-carboxamide

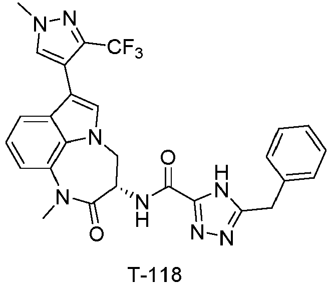

Use 1-methyl-3-trifluoromethyl-1H-pyrazole-4-boronic acid pinacol ester and Z-45 were used to obtain the T-118 under the same conditions as in Example 16.

¹H NMR (400 MHz, acetone-*d*₆) δ 8.57 (s, 1H), 8.09 (s, 1H), 7.49 (m, 1H), 7.46 (s, 1H), 7.32 (m, 4H), 7.27 - 7.15 (m, 3H), 4.89 - 4.76 (m, 2H), 4.49 - 4.39 (m, 1H), 4.20 (s, 2H), 4.06 (s, 3H).

MS ESI: m/z=549 [M+H]⁺ .

### Example 126: T-119

### (±)-5-benzyl-N-(1-methyl-2-oxo-7-(1,3,5-trimethyl-1H-pyrazol-4-yl)-1,2,3,4-tetrahydro-[1,4] diazepine [3,2,1-hi] indol-3-yl)-4H-1,2,4-triazole-3-carboxamide

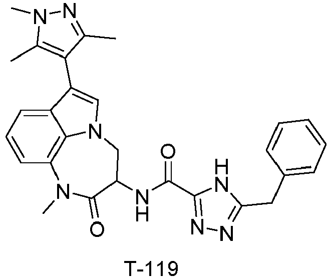

1,3,5-trimethyl-1H-pyrazol-4-boronic acid pinacol ester and Z-9 were used to obtain T-119 under the same conditions as in Example 16.

¹H NMR (400 MHz, acetone-*d*₆) δ 8.44 (s, 1H), 7.25 - 7.15 (m, 5H), 7.14 - 7.00 (m, 4H), 4.74 (m, 1H), 4.65 (m, 1H), 4.35 - 4.25 (m, 1H), 4.08 (s, 2H), 3.64 (s, 3H), 3.50 (s, 3H), 2.07 (s, 3H), 1.98 (s, 3H).

MS ESI: m/z=509 [M+H]⁺ .

### Example 127: T-120

### (S)-5-benzyl-N-(1-methyl-2-oxo-7-(1-(tetrahydro-2H-pyran-4-yl)-1H-pyrazol-4-yl)-1,2,3,4-tetrahydro-1,4-diazepine[3,2,1-hi]indol-3-yl)-4H-1,2,4-triazole-3-carboxamide

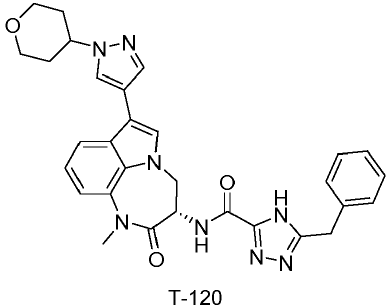

1-(tetrahydropyran-4-yl)-1H-pyrazole-4-boronic acid pinacol ester and Z-45 were used to obtain the T-120 under the same conditions as in Example 16.

¹H NMR (400 MHz, acetone-*d*₆) δ 8.56 (s, 1H), 8.09 (s, 1H), 7.81 (s, 1H), 7.72 (m, 1H), 7.56 (s, 1H), 7.34 (m, 4H), 7.24 (m, 3H), 4.83 (m, 1H), 4.75 (m, 1H), 4.49 (m, 1H), 4.45 - 4.35 (m, 1H), 4.21 (s, 2H), 4.05 (m, 2H), 3.62 (s, 3H), 3.56 (m, 2H), 2.19 - 2.08 (m, 4H).

MS ESI: m/z=551 [M+H]⁺ .

### Example 128: T-121

### (S)-5-benzyl-N-(7-(1-cyclopropyl-1H-pyrazol-4-yl)-1-methyl-2-oxo-1,2,3,4-tetrahydro-1,4-diazepino [3,2,1-hi] indol-3-yl)-4H-1,2,4-triazole-3-carboxamide

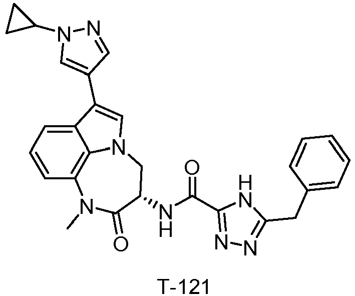

1-cyclopropylpyrazole-4-boronic acid pinacol ester and Z-45 were used to obtain T-121 under the same conditions as in Example 16.

¹H NMR (400 MHz, acetone-*d*₆) δ 8.56 (s, 1H), 8.06 (s, 1H), 7.75 (s, 1H), 7.70 (m, 1H), 7.55 (s, 1H), 7.34 (m, 4H), 7.22 (m, 3H), 4.83 (m, 1H), 4.74 (m, 1H), 4.43 (m, 1H), 4.21 (s, 2H), 3.77 (m, 1H), 3.62 (s, 3H), 1.18 (m, 2H), 1.02 (m, 2H).

MS ESI: m/z=507 [M+H]⁺ .

### Example 129: T-122

### (S)-5-benzyl-N-(1-methyl-7-(1-methyl-3-phenyl-1H-pyrazol-4-yl)-2-oxo-1,2,3,4-tetrahydro-[1,4] diazepine [3,2,1-hi] indol-3-yl)-4H-1,2,4-triazole-3-carboxamide

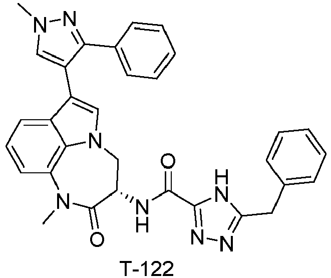

1-methyl-3-phenylpyrazole-4-boronic acid pinacol ester and Z-45 were used to obtain T-122 under the same conditions as in Example 16.

¹H NMR (400 MHz, acetone-*d*₆) δ 8.55 (s, 1H), 7.79 (s, 1H), 7.60 (m, 2H), 7.38 - 7.29 (m, 4H), 7.28 - 7.13 (m, 7H), 7.05 (m, 1H), 4.84 (m, 1H), 4.71 (m, 1H), 4.40 (m, 1H), 4.23 (s, 2H), 4.00 (s, 3H), 3.61 (s, 3H).

MS ESI: m/z=557 [M+H]⁺ .

### Example 130: T-123

### (S)-5-benzyl-N-(1-methyl-2-oxo-7-(thiazol-5-yl)-1,2,3,4-tetrahydro-[1,4] diazepine [3,2,1-hi] indol-3-yl)-4H-1,2,4-triazole-3-carboxamide

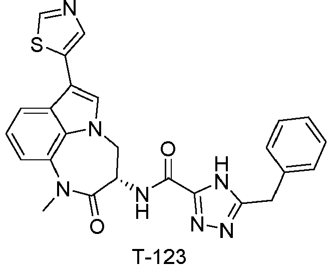

Thiazole-5-pyridylboronic acid pinacol ester and Z-45 were used to obtain T-123 under the same conditions as in Example 16.

¹H NMR (400 MHz, acetone-*d*₆) δ 13.42 (s, 1H), 8.93 (s, 1H), 8.56 (s, 1H), 8.16 (s, 1H), 7.84 (s, 1H), 7.80 - 7.73 (m, 1H), 7.40 - 7.28 (m, 6H), 7.26 (m, 1H), 4.88 (m, 1H), 4.81 (m, 1H), 4.49 (m, 1H), 4.22 (s, 2H), 3.63 (s, 3H).

MS ESI: m/z=484 [M+H]⁺ .

### Example 131: T-124

### (S)-5-benzyl-N-(1-methyl-2-oxo-7-(1-(2,2,2-trifluoroethyl)-1H-pyrazol-4-yl)-1,2,3,4-tetrahydro-[1,4] diazepine [3,2,1-hi] indol-3-yl)-4H-1,2,4-triazole-3-carboxamide

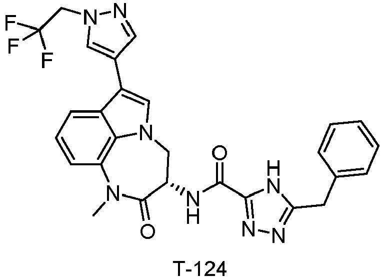

1-(2,2,2-trifluoroethyl)-1H-pyrazole-4-boronic acid pinacol ester and Z-45 were used to obtain the T-124 under the same conditions as in Example 16.

¹H NMR (400 MHz, acetone-d₆) δ 8.56 (s, 1H), 8.22 (s, 1H), 7.94 (s, 1H), 7.72 - 7.67 (m, 1H), 7.64 (s, 1H), 7.34 (m, 4H), 7.24 (m, 3H), 5.09 (q, *J* = 8.9 Hz, 2H), 4.84 (m, 1H), 4.76 (m, 1H), 4.43 (m, 1H), 4.22 (s, 2H), 3.62 (s, 3H).

MS ESI: m/z=549[M+H]⁺ .

### Example 132: T-125

### (S)-5-benzyl-N-(7-(1,5-dimethyl-1H-pyrazol-4-yl)-1-methyl-2-oxo-1,2,3,4-tetrahydro-1,4-diazepine [3,2,1-hi] indol-3-yl)-4H-1,2,4-triazole-3-carboxamide

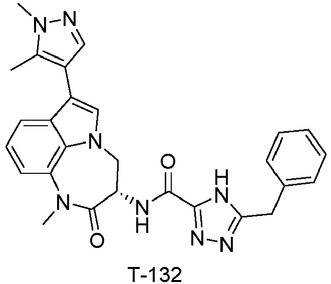

1,5-dimethyl-1H-pyrazol-4-boronic acid pinacol ester and Z-45 were used to obtain T-125 under the same conditions as in Example 16.

¹H NMR (400 MHz, acetone-*d*₆) δ 8.56 (s, 1H), 7.55 (s, 1H), 7.49 (m, 1H), 7.36 (m, 5H), 7.20 (m, 3H), 4.85 (m, 1H), 4.78 (m, 1H), 4.42 (m, 1H), 4.26 (s, 2H), 3.85 (s, 3H), 3.63 (s, 3H), 2.36 (s, 3H).

MS ESI: m/z=495[M+H]⁺ .

### Example 133: T-126

### (S)-5-benzyl-N-(7-(2-chloro-1-methyl-1H-imidazol-5-yl)-1-methyl-2-oxo-1,2,3,4-tetrahydro-1,4-diazepine [3,2,1-hi] indol-3-yl)-4H-1,2,4-triazole-3-carboxamide

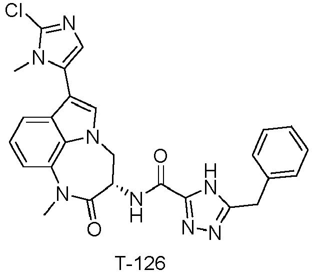

1-methyl-2-chlorimidazole-5-boronic acid pinacol ester and Z-45 were used to obtain T-126 under the same conditions as in Example 16.

¹H NMR (400 MHz, acetone-*d*₆) δ 8.57 (s, 1H), 7.65 (s, 1H), 7.47 (m, 1H), 7.39 - 7.21 (m, 7H), 7.04 (s, 1H), 4.93 - 4.77 (m, 2H), 4.48 (m, 1H), 4.21 (s, 2H), 3.63 (s, 6H).

MS ESI: m/z=515[M+H]⁺ .

### Example 134: T-127

### (S)-5-benzyl-N-(1-methyl-7-(1-methyl-3-(trifluoromethyl)-1H-pyrazol-4-yl)-2-oxo-1,2,3,4-tetrahydro-[1,4] diazepine [3,2,1-hi] indol-3-yl)oxazole-2-carboxamide

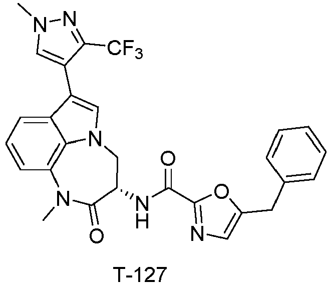

1-methyl-3-trifluoromethyl-1H-pyrazol-4-boronic acid pinacol ester, Z-45 and acid-2 were used to obtain the T-127 under the same conditions in the Example 16.

¹H NMR (400 MHz, acetone-d₆) δ 8.47 (s, 1H), 8.10 (s, 1H), 7.50 (m, 1H), 7.46 (s, 1H), 7.38 - 7.30 (m, 4H), 7.30 - 7.18 (m, 3H), 7.09 (s, 1H), 4.87 - 4.76 (m, 2H), 4.45 (m, 1H), 4.16 (s, 2H), 4.06 (s, 3H), 3.62 (s, 3H).

MS ESI: m/z=549[M+H]⁺ .

### Example 135: T-128

### (±)-5-benzyl-N-(6-methyl-7-oxo-6,7,8,9-tetrahydro-5, 6, 9a-triazabenzene[cd] azulene-8-yl)-4H-1,2,4-triazole-3-carboxamide

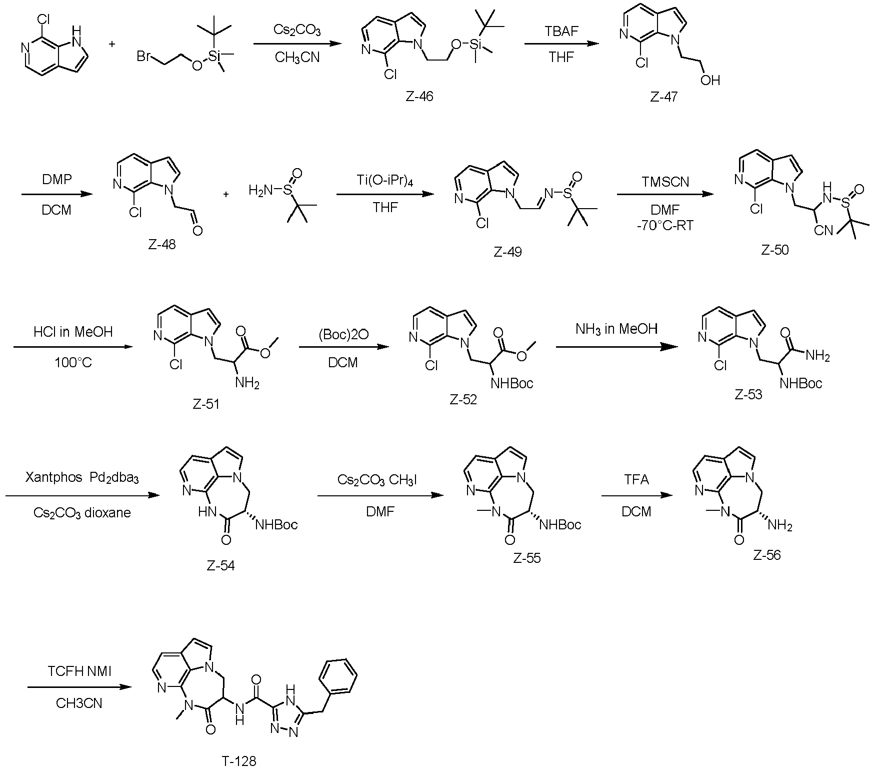

### Step 1: preparation of intermediate Z-46

A mixture of 7-chloro-6- azindole (1eq), (2-bromoethoxy)-tert-butyldimethylsilane (1.1eq), and cesium carbonate (1.5eq) in anhydrous acetonitrile was stirred at room temperature for 2 hours. The the reaction was quenched with water, and the mixture was extracted with EA. The extract was washed with water three times, and the organic phase was concentrated. The residue was purified by SGCC to give Z-46.

MS ESI: m/z=312, [M+H]⁺.

### Step 2: preparation of intermediate Z-47

Toe a solution of Z-46 (1eq) in anhydrous THF, was added 2eq of 1M tetrabutylammonium fluoride tetrahydrofuran solution. The mixture was stirred at room temperature for 1 hour and then poured into water. The mixture was extracted with EA, washed with water three times, and the organic phase was concentrated. The residue was purified by SGCC to give Z-47.

MS ESI: m/z=197, [M+H]⁺.

### Step 3: preparation of intermediate Z-48

To a solution of Z-47 (1eq) in anhydrous DCM cooled at 0 °C was added 1.2eq of Dess-Martin periodinane. After stirring at 0 °C for 2 hours, the reaction was quenched with water. The mixture was diluted with EA, and washed three times respectively with saturated aqueous solutions of sodium thiosulfate and sodium bicarbonate. The organic phase was concentrated and the residue was purified by SGCC to give Z-48.

MS ESI: m/z=195, [M+H]⁺.

### Step 4: preparation of intermediate Z-49

A mixture of Z-48 (1eq), Ti(Or-Pr)₄ (2.5eq), tert-butylsulfinamide (1.2eq) in anhydrous THF was stirred at room temperature for 6 hours and then quenched with water. The mixture was extracted with EA. The extract was washed with water three times and concentrated. The residue was purified by SGCC to give Z-49.

MS ESI: m/z=298, [M+H]⁺.

### Step 5: preparation of intermediate Z-50

To a solution of Z-49 (1eq) in anhydrous DMF was added trimethylsilyl cyanide (3eq) was added slowly at 0 °C. After stirring at -70°C to room temperature for 12 hours, the reaction was quenched with water and the mixture was extracted with EA. The extract was washed with water three times, and concentrated. The residue was purified by SGCC to give Z-50.

MS ESI: m/z=325, [M+H]⁺.

### Step 6: preparation of intermediate Z-51

A mixture of Z-50 (1eq) in 4M of hydrochloric methanol solution was stirred at 100°C for 1 hour. The mixture was cooled to room temperature and concentrated. The residue was purified by SGCC to give Z-51.

MS ESI: m/z=254, [M+H]⁺.

### Step 7: preparation of intermediate Z-52

To a solution of Z-51 (1eq) in dichloromethane were added triethylamine (1eq) and di-tert-butyl dicarbonate (1.2eq). After stirring at room temperature for 3 hours, the reaction mixture was quenched with water, and extracted with EA. The extract was washed with water three times and concentrated. The residue was purified by SGCC to give Z-52.

MS ESI: m/z=354, [M+H]⁺.

### Step 8: preparation of intermediate Z-53

A solution of Z-52 (1eq) in a methylamine solution was stirred at room temperature for 3 hours and then concentrated to obtain Z-53.

MS ESI: m/z=339, [M+H]⁺.

### Step 9: preparation of intermediate Z-54

A mixture of Z-53 (1eq), tris (dibenzalacetone) dipalladium (0.15eq), 4, 5-bis (diphenylphosphino)-9, 9-dimethyloxyanthracene (0.3eq), and cesium carbonate (2eq) in anhydrous 1,4-dioxane was heated to 110°C and stirred for 10 hours. The reaction was quenched with water. The mixture was extracted with EA, washed with water three times, and concentrated. The residue was purified by SGCC to give Z-54.

### Step 10: preparation of intermediate Z-55

Toe a solution of Z-54 (1eq) in anhydrous DMF were added cesium carbonate (1.5eq) and iodomethane (1.2eq). The mixture was stirred at room temperature for 3 hours, and quenched with water. The mixture was extracted with EA. The extract was washed with water three times and concentrated. The residue was purified by SGCC to give Z-55.

MS ESI: m/z=317, [M+H]⁺.

### Step 11: preparation of intermediate Z-56

A solution ofZ-40 (1eq) and trifluoroacetic acid (1.5eq) in DMF was stirred at room temperature for 2 hours. The reaction was quenched with ethanol, and concentrated to obtain Z-56.

MS ESI: m/z=217, [M+H]⁺.

### Step 12: preparation of T-128

Acid-1 (1.5eq) and Z-56 (1eq) were used as starting materials to obtain T-128 under the same conditions as in Example 2.

¹H NMR (400 MHz, acetone-d₆) δ 8.66 - 8.57 (m, 1H), 8.05 (d, *J* = 5.3 Hz, 1H), 7.61 (d, *J* = 3.2 Hz, 1H), 7.42 (d, *J* = 5.2 Hz, 1H), 7.34 (m, 4H), 7.26 (m, 1H), 6.64 (d, *J* = 3.2 Hz, 1H), 4.93 - 4.77 (m, 2H), 4.46 (m, 1H), 4.23 (s, 2H), 3.67 (s, 3H).

MS ESI: m/z=402, [M+H]⁺.

### Example 136: T-129

### (±)-5-benzyl-N-(6-methyl-7-oxo-6,7,8,9-tetrahydro-2H-2, 5, 6-triazabenzene[cd] azulene-8-yl)-4H-1,2,4-triazol-3-carboxamide

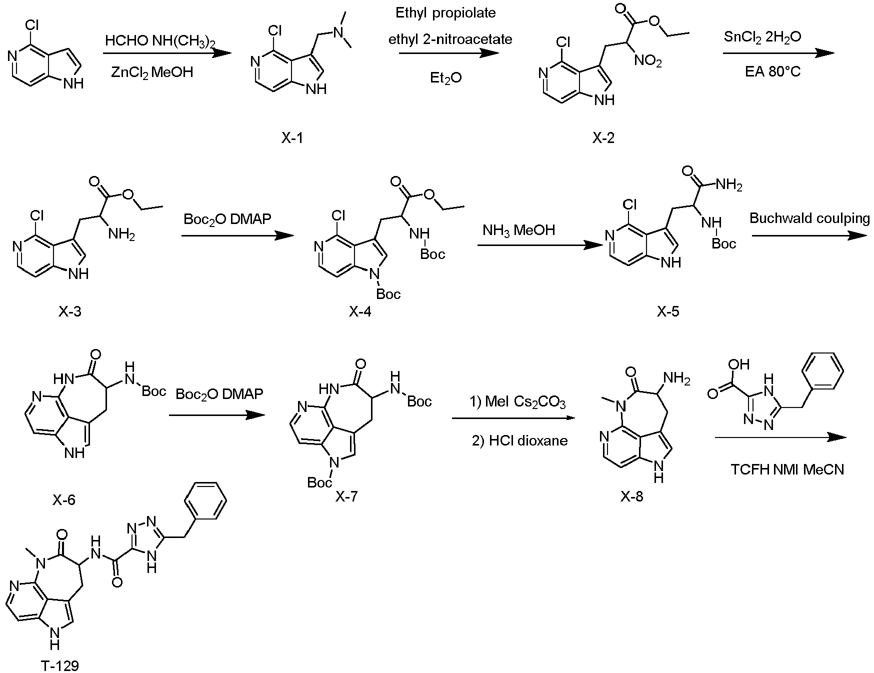

### Step 1: preparation of intermediate X-1

4-chloro-1H-pyrrolo [3,2-c] pyridine was used as the starting material to prepare X-1 under the same conditions as in the Step 1 of Example 20.

MS ESI: m/z=210, [M+H]⁺.

### Step 2: preparation of intermediate X-2

Using X-1 was used as the starting material, and ethyl nitroacetate as the starting materials under the conditions in the Step 2 of Example 20, X-2 was prepared with a longer reaction time.

MS ESI: m/z=298, [M+H]⁺.

### Step 3: preparation of intermediate X-3

A mixture of X-2 (1,000mg), stannous chloride dihydrate (3,789.9mg), and ethyl acetate (17mL) was heated at 80°C overnight. The reaction was then quenched with a saturated solution of NaHCOs. The mixture was filtered, extracted with EA. The extract concentrated and the residue was purified by SGCC to obtain 465mg of X-3 as a pale-yellow solid.

MS ESI: m/z=268, [M+H]⁺.

### Step 4: preparation of intermediate X-4

A mixture of X-3 (465mg), Boc2O (796.57mg), DMAP (21.2mg) and NEt3 (0.6mL) in THF (9mL) was stirred for 2 hours and then concentrated. The residue was purified by SGCC to give 606mg of X-4 as a solid.

### Step 5: preparation of intermediate X-5

A mixture of X-4 (200mg) and NH₃ MeOH (2mL) in a sealed tube was heated at 100°C for 2 days. After evaporation, the residue was purified by SGCC to obtain 34mg of X-5. This experiment was repeated to prepare more X-5.

MS ESI: m/z=339, [M+H]⁺.

### Step 6: preparation of intermediate X-6

A mixture of X-5 (100mg), Pd₂(dba)₃ (137.4mg), Xphos (71.5mg) and Cs₂CO₃ (293.2mg) in anhydrous toluene (3mL) was heated at 120°C under N₂ for 26 hours. The reaction was quenched with saturated NH₄Cl solution, and the mixture was extracted with EA. The extract was concentrated and the residue was purified by SGCC to obtain 34mg of X-6.

MS ESI: m/z=303, [M+H]⁺.

### Step 7: preparation of intermediate X-7

The X-6 (34mg) was used as the starting material to prepare X-7 (18.3mg) under the same conditions as in the Step 4.

### Step 8: preparation of intermediate X-8

A mixture of X-7 (18.3mg), MeI (3.4ul), Cs₂CO₃ (17.8mg) in DMF (2mL) was stirred at room temperature for 1 hour and then quenched with saturated NH₄Cl solution. The mixture was extracted with EA. The extract was concentrated and the solid obtained was dissolved in HCl (2mL, 4N in dioxane). The solution was stirred at room temperature for 2 hours and then concentrated to give 13.5mg of X-8.

MS ESI: m/z=217, [M+H]⁺.

### Step 9: preparation of T-129

X-7 was used as the starting material to prepare 11.6mg of T-129 as a white solid under the same conditions as in the Step 2 of Example 40.

¹H NMR (400 MHz, DMSO-d₆) δ 14.35 (s, 1H), 11.60 (s, 1H), 8.77 (s, 1H), 8.05 (d, J = 5.7 Hz, 1H), 7.39 - 7.22 (m, 6H), 7.20 (d, J = 5.7 Hz, 1H), 4.66 (m, 1H), 4.13 (s, 2H), 3.56 (s, 3H), 3.27 (m, 1H), 3.14 - 2.97 (m, 1H).

MS ESI: m/z=402, [M+H]⁺.

### Example 137: T-130

### (±)-5-benzyl-N-(6-methyl-7-oxo-6,7,8,9-tetrahydro-2H-2, 3, 6-triazabenzene[cd]azulene-8-yl)-4H-1,2,4-triazol-3-carboxamide

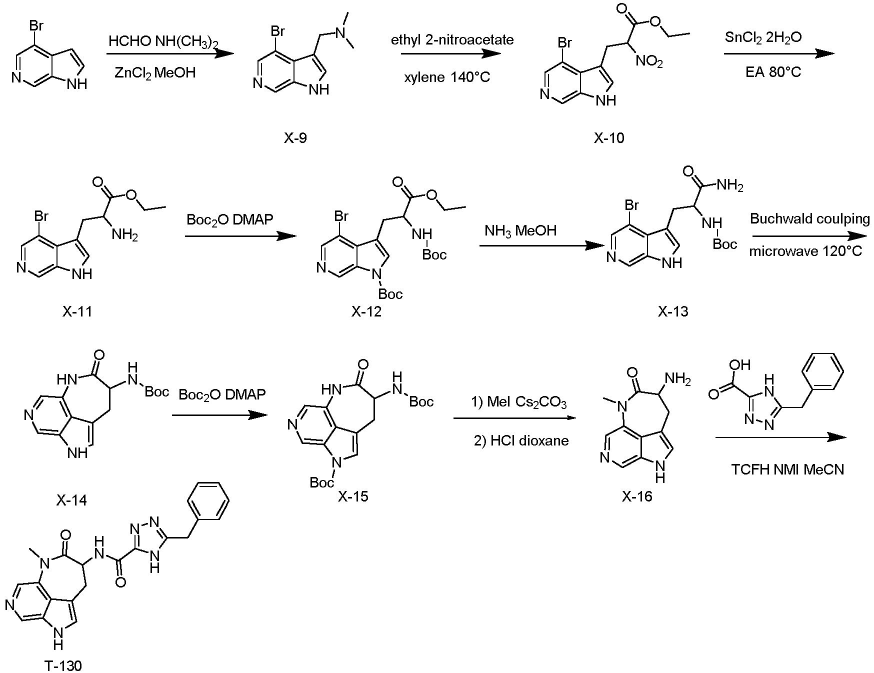

Using 4-bromo-1H-pyrrolo [2,3-c] pyridine as the starting material, T-140 was prepared under the same conditions as in the Example 136. For the preparation of intermediate X-10, the same conditions as in the Step 4 of Example 47 were used. For the preparation of intermediate X-14, the conventional heating was replaced with microwave heating.

¹H NMR (400 MHz, DMSO-d₆) δ 12.85 (s, 1H), 8.97 (s, 1H), 8.76 (s, 1H), 8.30 (s, 1H), 8.13 (s, 1H), 7.42 - 7.17 (m, 6H), 4.69 (m, 1H), 4.15 (s, 2H), 3.56 (s, 3H), 3.33 (m, 1H), 3.26 - 3.12 (m, 1H).

MS ESI: m/z=402, [M+H]⁺.

### Example 138: T-131

### (±)-5-benzyl-N-(9-fluoro-1-methyl-2-oxo-2,3,4,6-tetrahydroazepincd [4,3,2-cd]indol-3-yl)-4H-1,2,4-triazole-3-carboxamide

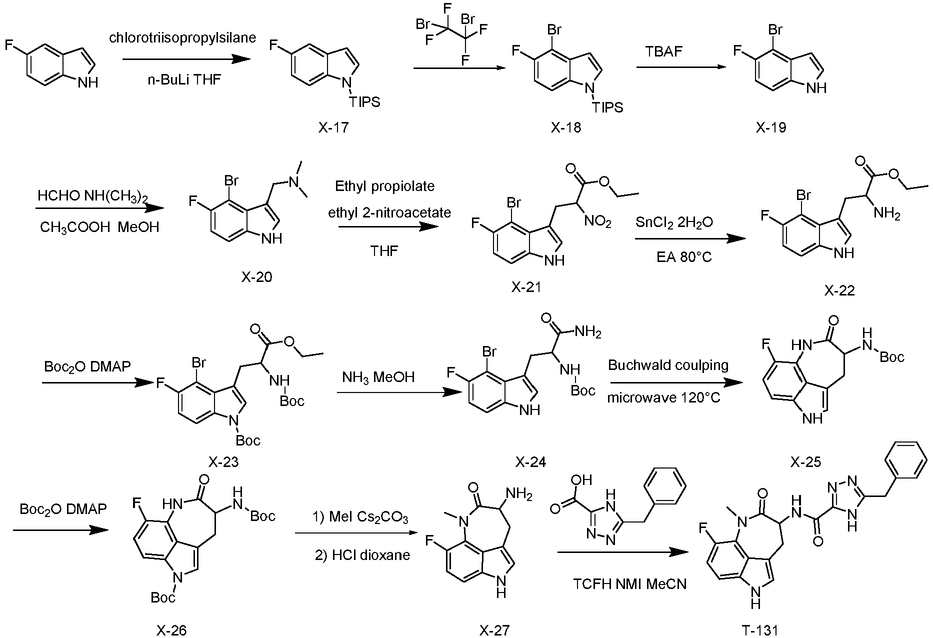

### Step 1: preparation of intermediate X-17

To a mixture of 5-fluoroindole (5g), triisopropylchlorosilane (8.72mL) in THF (150mL) cooled at -78°C was added n-BuLi (16.3mL, 2.5N in THF). After stirring at -78°C for 1 hour, the reaction was quenched with NH₄Cl saturated solution, and the mixture was extracted with EA. The organic phase was concentrated to give 12.1g of X-17 as an oil.

### Step 2: preparation of intermediate X-18

To a mixture of THF (140mL), n-BuLi (30.5mL, 2.5N in THF) cool at-78 °C was added t-BuOK (76.2mL, 1N in THF). After stirring at -78°C for 20 minutes, a solution of X-17 (11.1g) in THF (100mL) was added to the reaction mixture. After stirring at -78°C for 3 hours, 1,2-dibromotetrafluoroethane (5.91mL) was added and the mixture was slowly warmed to room temperature, and stirred overnight. The reaction mixture was concentrated and the residue was purified by SGCC to give 5.78g of X-18.

### Step 3: preparation of intermediate X-19

A solution of X-18 (5.78g) in TBAF (18.75mL, 1N in THF) was stirred at room temperature for 3 hours, and then concentrated. The residue was purified by SGCC to give X-19 (3.91g)..

MS ESI: m/z=214, [M+H]⁺.

### Step 4: preparation of intermediate X-20

Using X-19 (3,910mg) was used as the starting material, and X-20 (4.7 g) was prepared by the same methods described in Step 1 of Example 20 except ZnCl₂ was replaced with an equivalent amount of CH₃COOH.

MS ESI: m/z=271, [M+H]⁺.

### Step 5: preparation of intermediate X-21

Using X-20 was used as the starting material, and X-21 was prepared by the same methods described in Step 2 of Example 136 except THF was used as the solvent.

MS ESI: m/z=360, [M+H]⁺.

### Preparation of the final product T-131

X-21 was used as the starting material to obtain T-131 by following the same procedures described in Example 136.

¹H NMR (400 MHz, acetone-d₆) δ 13.40 (s, 1H), 10.46 (s, 1H), 8.43 (m, 1H), 7.38 - 7.28 (m, 6H), 7.25 (m, 1H), 7.05 (dd, *J* = 12.8, 8.9 Hz, 1H), 4.87 - 4.65 (m, 1H), 4.19 (s, 2H), 3.48 (d, *J* = 5.0 Hz, 2H), 3.39 (m, 1H), 3.06 - 2.94 (m, 1H).

MS ESI: m/z=419, [M+H]⁺.

Biological Activity test:
1. Cell activity testing system (TNF-α induces the necroptosis of Jurkat cells, the human leukemia cells deficient in FADD).
   (1) Cell culture: Jurkat FADD-deficient cells were cultured in an RPMI 1640 medium with 10% fetal bovine serum (FBS), 100U/mL penicillin, and 100U/mL streptomycin. The cells were cultured in a cell culture incubator at 37°C with 5% CO₂. And all the cells were cryopreserved in a complete medium containing 5-10% DMSO. Medium change and passage were performed 3-4 times per week.
   (2) Experiment materials Reagent:
      RPMI 1640 medium (GIBCO, USA), FBS (GIBCO, USA), Human recombinant TNF (Novoprotein C008), Cell Titer-Glo Kit (Promega, USA), Nec-1s (synthesized by the research group of Ma Dawei from the Shanghai Institute of Organic Chemistry, and compound GSK2982772 (Bidepharm).
   (3) Experimental consumables:
      Cell culture dishes (100/60/35mm) (BD or Corning, USA), cell culture plates (384/96/24/6 wells) (BD or Corning, USA), and centrifuge tube (15/50mL) (BD or Corning, USA).
   (4) Experimental scheme: The system of TNF-α inducing the necroptosis of Jurkat FADD-deficient cells was used to screen for compounds with RIPKI kinase inhibitory activity.

Jurkat FADD-deficient cells were evenly inoculated into a 384-well plate with each well added with a 40µL complete culture medium, followed by the addition of 5µL of the under-testing compound to each well. The initial concentration of the compounds was set at 25µM (for some compounds with higher activity, the initial concentration was set at 0.5µM), with a three-fold gradient dilution, totaling 12 gradient points. Three replicate wells were set for each compound. In addition, there was a blank control group and a control group with TNFα stimulation only. After the compound was added, the mixture needed to be centrifuged and incubated in a cell culture incubator at 37°C containing 5% CO₂ for 1 hour. After that, 5µL of 5ng/mL TNFα was added to stimulate the occurrence of necroptosis. After centrifugation and mixing, the mixture was placed in the cell culture incubator for 13 hours (until the cell death rate reaches 65% in the group with only TNF-α), with a total volume of 50µL per well. Then, the steps below were followed to test the cell survival rate.

The CellTiter-Glo^{®} Buffer was removed from the reagent kit, equilibrated to room temperature, and used to dissolve the CellTiter-Glo^{®} Substrate. The CellTiter-Glo^{®} Reagent obtained could be directly used for the experiment.

15µL of CellTiter-Glo^{®} Reagent which has been equilibrated to room temperature was added to each well, and incubated in the dark for 15 minutes. After that, a microplate reader (Enspire Multimode Plate Reader of PerkinElmer, USA) was used to measure the chemiluminescence. The value of the blank control group was taken as 100%, the relative change in ATP of the experimental group was calculated, and then the data was fitted through Graphpad Prism software to get the compound's IC₅₀: A(<0.01uM), B (0.01-0.1 uM), C (0.1-1.0uM), D (>1uM).

**Table 1 Inhibitory Activity Test Results of Jurkat FADD (-/-) Cells**

| Compound No. | IC₅₀ |
|---|---|
| T-1 | A |
| T-2 | A |
| T-3 | B |
| T-4 | B |
| T-5 | C |
| T-6 | C |
| T-7 | B |
| T-8 | A |
| T-9 | A |
| T-10 | B |
| T-11 | B |
| T-12 | C |
| T-13 | D |
| T-14 | B |
| T-15 | A |
| T-16 | A |
| T-17 | B |
| T-18 | A |
| T-19 | A |
| *S*-T-19 | A |
| *R*-T-19 | C |
| T-20 | B |
| T-21 | C |
| T-22 | B |
| T-23 | C |
| T-24 | B |
| T-25 | A |
| T-26 | B |
| T-27 | B |
| T-28 | A |
| T-29 | A |
| T-30 | C |
| T-31 | C |
| T-32 | A |
| T-33 | B |
| T-34 | A |
| T-35 | A |
| T-36 | A |
| T-37 | A |
| T-38 | A |
| T-39 | B |
| T-40 | A |
| T-41 | A |
| T-42 | A |
| T-43 | A |
| T-44 | A |
| T-45 | A |
| T-46 | C |
| T-47 | C |
| T-48 | A |
| T-49 | A |
| T-50 | A |
| T-51 | B |
| T-52 | A |
| T-53 | A |
| T-54 | A |
| T-55 | B |
| T-56 | B |
| T-57 | C |
| T-58 | B |
| T-59 | A |
| T-60 | A |
| T-61 | B |
| T-62 | A |
| T-63 | A |
| T-64 | A |
| T-65 | B |
| T-66 | A |
| T-67 | A |
| T-68 | A |
| T-69 | B |
| T-70 | A |
| T-71 | B |
| T-72 | C |
| T-73 | B |
| T-74 | A |
| T-75 | B |
| T-76 | B |
| T-77 | C |
| T-78 | A |
| T-79 | D |
| T-80 | B |
| T-81 | A |
| T-82 | A |
| T-83 | A |
| T-84 | B |
| T-85 | C |
| T-86 | A |
| T-87 | B |
| T-88 | B |
| T-89 | B |
| T-90 | A |
| T-91 | B |
| T-92 | D |
| T-93 | C |
| T-94 | D |
| T-95 | C |
| T-96 | A |
| T-97 | A |
| T-98 | A |
| T-99 | A |
| T-100 | B |
| T-101 | B |
| T-102 | B |
| T-103 | B |
| T-104 | B |
| T-105 | A |
| T-106 | C |
| T-107 | A |
| T-108 | B |
| T-109 | A |
| T-110 | D |
| T-111 | A |
| T-112 | A |
| T-113 | A |
| T-114 | A |
| T-115 | B |
| T-116 | B |
| T-117 | A |
| T-118 | A |
| T-119 | A |
| T-120 | A |
| T-121 | B |
| T-122 | B |
| T-123 | B |
| T-124 | B |
| T-125 | A |
| T-126 | B |
| T-127 | A |
| T-128 | B |
| T-129 | C |
| T-130 | D |
| T-131 | A |

### 2. RIPK1 activity inhibition test

The RIPK1 activity inhibition test was completed by NANOSYN from the USA (3100 Central Expressway, Santa Clara, CA 95051). The test compounds were dissolved in DMSO, with the highest concentration being 1µmol. Subsequently, the concentrations were serially diluted threefold, with the DMSO concentration in the test medium maintained at 1%. IC₅₀ values were calculated from data generated prom 12 tested concentrations.

**Table 2 Results of RIPK1 Activity Inhibition Test**

| Compound No. | IC₅₀ (nM) |
|---|---|
| Staurosporine (kinase inhibitor standard substance) | 79.8 |
| GSK2982772 (positive control) | 11.1 |
| T-16 | 47.8 |
| T-34 | 50.4 |
| T-96 | 15.2 |
| T-112 | 28.3 |
| T-118 | 24.9 |

### 3. Study of oral pharmacokinetics in rats:

Healthy male SD rats were used as experimental animals. The intragastric (p.o.) administration was performed, with a dosage of 5mg/kg or 15mg/kg. Preparation: the compound was dissolved in a formulation made of 1% tween 80, 1% HMPC, and 98% water. The mixture was ground and shaken until the compound was uniformly distributed with a small particle size, and then 1 equivalent of methanesulfonic acid was added. The intravenous administration was performed with a dosage of 1mg/kg. Preparation: 5% DMSO, 10% EtOH, 10% sulotol, and 75% physiological saline was added to the compound, and mixed well. Samples were taken at 5 minutes, 15 minutes, 30 minutes, 1 hour, 2 hours, 4 hours, 6 hours, and 8 hours after administration, and then the samples were analyzed using liquid chromatography tandem mass spectrometry (LC-MS/MS).

**Table 3 PK (p.o.)Data of Partial Compounds**

| Compound No. | T_{1/2}(h) | Tₘₐₓ(h) | Cₘₐₓ(ng/ml) | AUC₀₋₈ (h*ng/ml) | Bioavailability (%) | Dosage (mg/kg) |
|---|---|---|---|---|---|---|
| T-40 | 3.26 | 1 | 2230 | 9040 | 31.35 | 5 |
| T-96 | 3.76 | 1 | 1272 | 4899 | 28.23 | 5 |
| T-112 | 1.99 | 0.75 | 494.5 | 1702.93 | 18.3 | 5 |
| T-112 | 1.91 | 1.50 | 3930 | 15925.04 | 57.15 | 15 |

All the literature reviews in the present invention are used as the reference of the application, just like individual reference of each literature review. Besides, it's noteworthy that, after reading the above content, the technicians in this field can change or modify the present invention, and these equivalent forms are also in the scope restricted by the claims attached with the application.

## Claims

1. A compound as shown in general formula I, or a pharmaceutically acceptable salt thereof, its stereoisomer or its tautomer, or a prodrug: 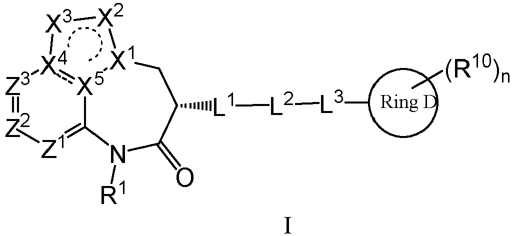 Wherein,
Z¹, Z², and Z³ are independently N, C(R);
R is independently H, halogen, -OH, -CN, -COOH, C₁-C₆ alkyl, -C₀-C₆ alkylene alkoxy, C₁-C₆ alkyl-, C₁-C₆ halogenated alkyl, C₁-C₆ alkoxy, C₁-C₆ halogenated alkoxy, C₂-C₁₀ alkoxyl-alkyl, C₂-C₁₀ halogenated alkoxyl-alkyl, C₁-C₆ hydroxyalkyl, -C₀-C₆ alkylene, -S-C₁-C₆ alkyl-, -C₀-C₆ alkylene, -C₆-C₁₀ aryl, -C₀-C₆ alkylene-X-C₆-C₁₀ aryl, -C₀-C₆ alkylene-5-10-membered heteroaryl, -C₀-C₆ alkoxy-X-5-10-membered heteroaryl, -C₀-C₆ alkylene-3-10-membered non-aromatic heterocyclyl (wherein, the heteroatoms are independently one or more of sulfur, oxygen, NH, or NR_{g}), -C₀-C₆ alkylene, -C₃-C₁₀ cycloalkyl, -C₀-C₆ alkylene, -C₃-C₁₀ cycloalkenyl, -C₀-C₆ alkylene-COR^{c}, -C₀-C₆ alkylene-CO₂, C₁-C₆ alkyl, -C₀-C₆ alkylene-CONR^{a}R^{b}, -C₀-C₆ alkylene-SO₂NR^{a}R^{b}, -C₀-C₆ alkyleneS(O)₂R^{c}, SF₅, -C₀-C₆ alkylene-NR^{a}R^{b}, -C₀-C₆ alkylene-NHC(O)R^{c}, -C₀-C₆ alkylene-NHC(O)C(O)NR^{a}R^{b}, -C₀-C₆ alkoxy-NHC(O)C(O)OR^{a}, -C₀-C₆ alkylene-NHC(O)NR^{a}R^{b}, -C₀-C₆ alkylene-P(O)Me₂, -C₀-C₆ alkylene-P(O)(OMe)₂, -C₀-C₆ alkylene, C₃-C₆ cycloalkyloxy, -C₀-C₆ alkylene, C₁-C₆ alkoxy, -C₀-C₆ alkylene, C₁-C₆ halogenated alkoxy, -C₀-C₆ alkylene, -C≡C-R₂, -O-C₁-C₆ alkylene, -C≡C-R², -S-C₁-C₆ alkylene, -C≡C-R², and -C0-C₆ alkylene-C(R^{a})=C(R^{b})-R²; R can be unsubstituted or substituted by 1 to 4 R^{f}; when R is connected to a nitrogen atom, R is not a halogen;
R is specifically selected from the following groups: 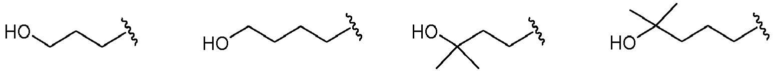 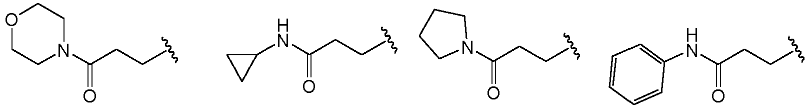 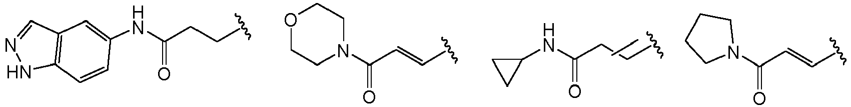 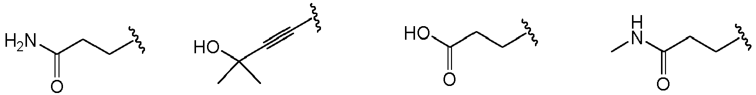 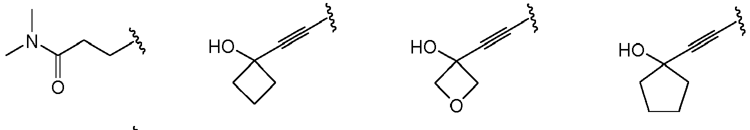 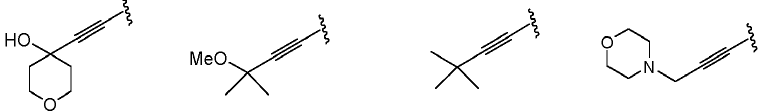 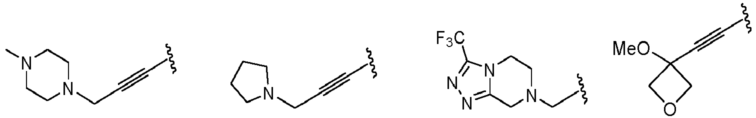 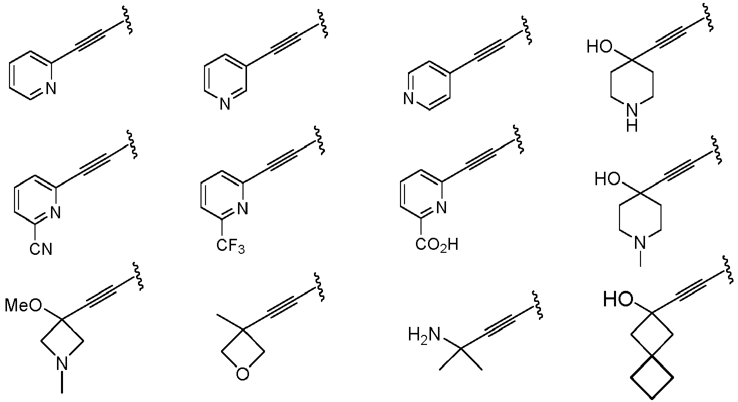 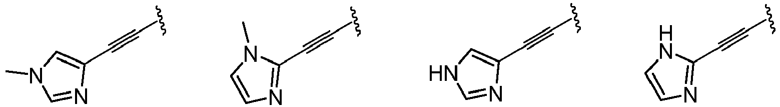 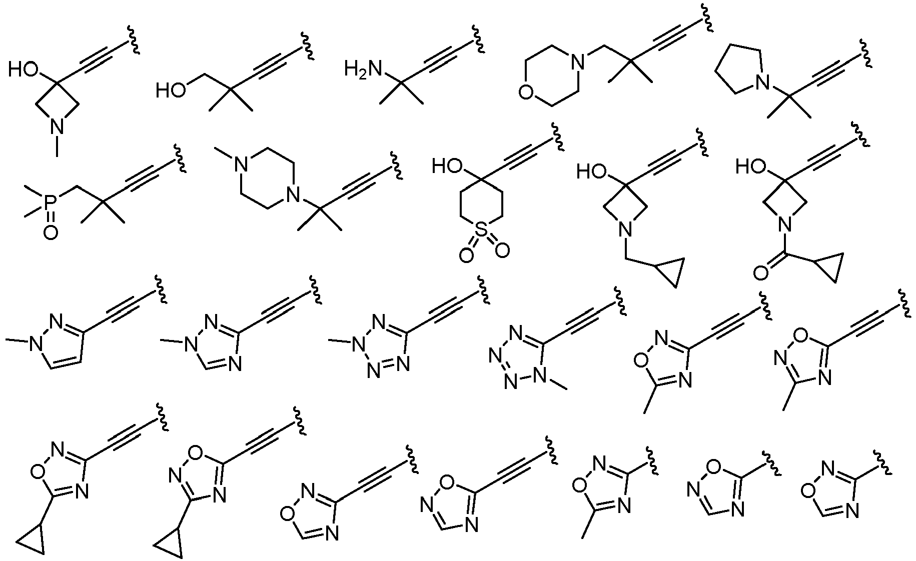 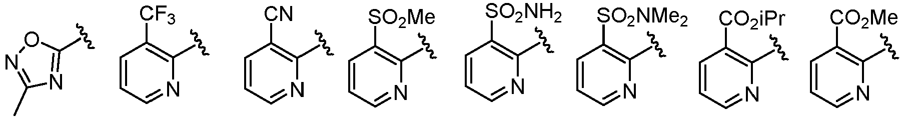 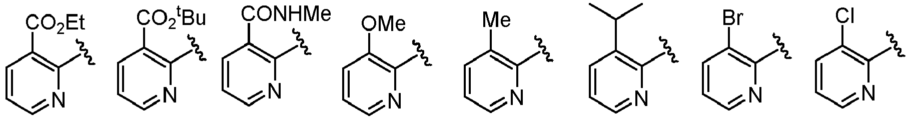 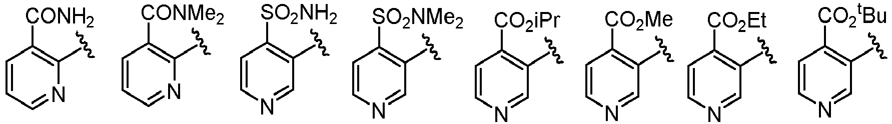 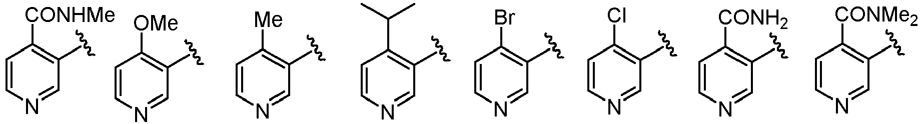 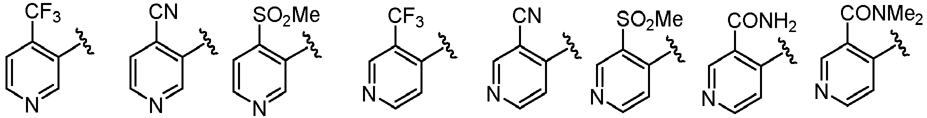 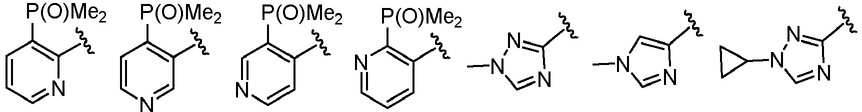 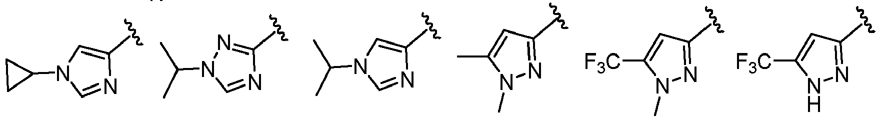
X can be O, S, SO, S(O)₂, NH, C(O), CH₂, CF₂, CH(CH₃), CH(OH), or N(CH₃);
R¹ and R^{1a} are independently of H, C₁-C₆ alkyl or -C₀-C₃ alkylene-C₃-C₆ cycloalkyl;
R² is hydrogen, C₁-C₁₀ alkyl, -C₀-C₆ alkylene-C₆-C₁₀ aryl, -C₀-C₆ alkylene-5-10-membered heteroaryl, -C₀-C₆ alkylene-3-10-membered non-aromatic heterocyclyl (wherein, the heteroatoms are independent one or more of sulfur, oxygen, NH, or NR^{g}), -C₀-C₆ alkylene-C₃-C₁₀ cycloalkyl, or -C₀-C₆ alkylene-C₁-C₁₀ alkoxy; R² can be independently unsubstituted or substituted by 1 to 4 R^{f};
R^{f}, at each occurrence, is independently halogen, -OH, carbonyl, -CN, -COOH, C₁-C₆ alkyl, - C₀-C₆ alkylene alkoxy, C₁-C₆ alkyl, C₁-C₆ halogenated alkyl, C₁-C₆ alkoxy, C₁-C₆ halogenated alkoxy, C₂-C₁₀ alkoxyl-alkyl, C₂-C₁₀ halogenated alkoxyl-alkyl, C₁-C₆ hydroxyalkyl, -C₀-C₆ alkylene-S-Ci-C₆ alkyl, -SF₅, -C₀-C₆ alkylene-COR^{c}, -C₀-C₆ alkylene-CO₂C₁-C₆ alkyl, -C₀-C₆ alkylene-CONR^{a}R^{b}, -C₀-C₆ alkylene-SO₂NR^{a}R^{b}, -C₀-C₆ alkylene-S(O)₂R^{c}, -C₀-C₆ alkylene-NR^{a}R^{b}, -C₀-C₆ alkylene-C(O)NR^{a}R^{b}, -C₀-C₆ alkylene-NHC(O)R^{c}, -C₀-C₆ alkylene-NHC(O)C(O)NR^{a}R^{b}, -C₀-C₆ alkylene-NHC(O)C(O)OR^{a}, -C₀-C₆ alkylene-NHC(O)NR^{a}R^{b}, -C₀-C₆ alkylene-P(O)Me₂, -C₀-C₆ alkylene-P(O)(OMe)₂. Two neighboring R^{f} or two R^{f} connected to the same carbon atom together form a 3- to 8-membered ring or a 4- to 8-membered heterocyclic ring, which may contain heteroatoms such as sulfur, oxygen, NH, or NR^{g};
R^{a} and R^{b} are independent hydrogen, substituted or unsubstituted C₁-C₁₀ alkyl, substituted or unsubstituted C₃-C₁₀ cycloalkyl, substituted or unsubstituted C₂-C₁₀ alkenyl, substituted or unsubstituted C₆-C₁₀ aryl, or substituted or unsubstituted C₃-C₁₀ heteroaryl respectively; R^{a} and R^{b} can form a 3- to 8-membered ring or a 4- to 8-membered heterocyclic ring when connected to a nitrogen or carbon atom, which may contain heteroatoms such as sulfur, oxygen, NH, or NR^{g}; the said 3- to 8-membered ring or 4- to 8-membered heterocyclic ring can be substituted by one or multiple R^{e};
R^{c} is independently H, C₁-C₆ alkyl, C₃-C₆ cycloalkyl, C₁-C₆ halogenated alkyl, C₃-C₁₀ cycloalkenyl, C₁-C₆ alkoxy, C₁-C₆ cycloalkyloxy, C₀-C₆ alkylene hydroxyl, C₆-C₁₀ aryl, 5- to 10-membered heteroaryl, 3- to 10-membered non-aromatic heterocyclyl, -C₀-C₆ alkylene CF₃, -C₁-C₆ alkylene CN, -C₁-C₆ alkylene-C₁-C₆ alkoxy, C₁-C₆ alkylene NR^{a}R^{b}, C₁-C₆ alkylene NR^{b}C(O)R^{a}, C₁-C₆ alkylene NR^{b}S(O)₂R^{a}, C₁-C₆ alkylene carboxyl, -C₁-C₆ alkylene CO₂, C₁-C₆ alkyl, C₁-C₆ alkylene COR^{a}, -C₀-C₆ alkylene CONR^{a}R^{b}; wherein, the said C₆-C₁₀ aryl, 5- to 10-membered heteroaryl, 3- to 10-membered non-aromatic heterocyclyl, C₃-C₆ cycloalkyl, or C₃-C₁₀ cycloalkenyl is each independently unsubstituted or substituted by one or two substituents independently selected from the following: halogen, C₁-C₆ alkyl, C₃-C₆ cycloalkyl, C₁-C₄ alkoxy, C₀-C₆ alkylene NR^{a}R^{b}, -C₀-C₆ alkylene CN, -C₀-C₆ alkylene OH;
R^{g} is C₁-C₁₀ alkyl, C₁-C₁₀ heteroalkyl, C₃-C₁₀ cycloalkyl, C₆-C₁₀ aryl, or C₃-C₁₀ heteroaryl;
X¹, X², X³, X⁴, and X⁵ are independently selected from C, CR, N, and NR, provided that the rules of valence bonding are conformed; 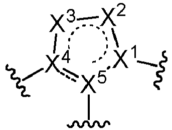 is selected from the following: 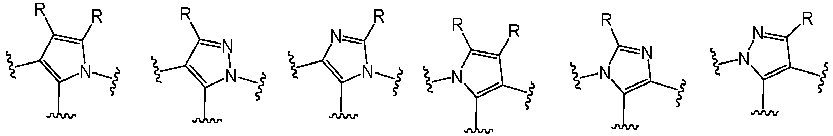 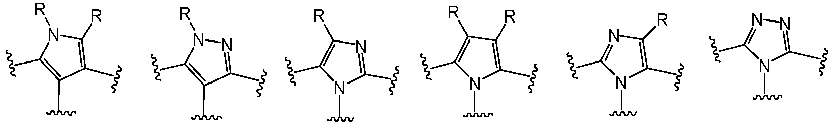 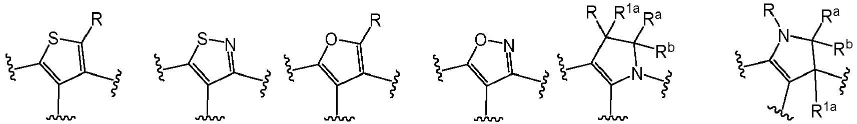 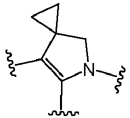
The said 3- to 10-membered non-aromatic heterocyclyl can also have the bicyclic or spiral ring structures as follows: 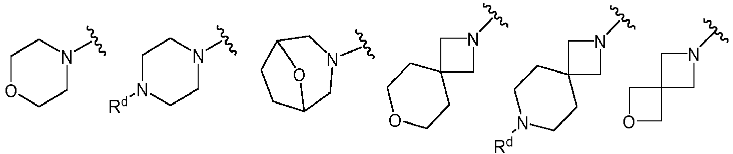 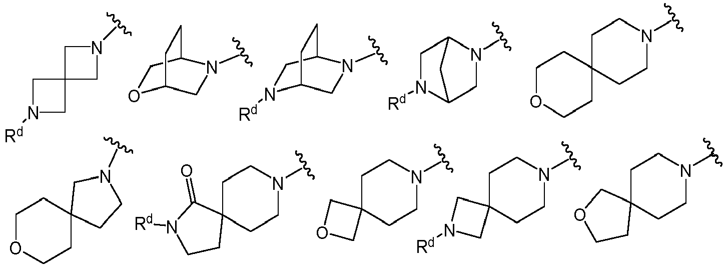 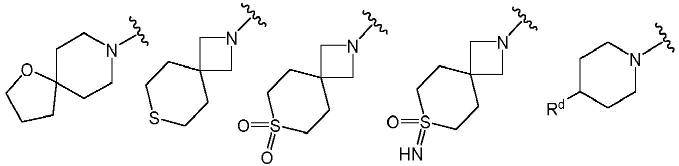
L¹ is structured as follows: 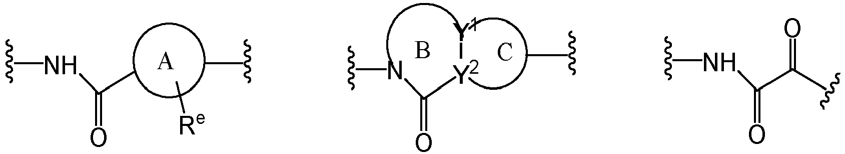
Ring A is a substituted or unsubstituted benzene ring, a 5- to 6-membered heteroaromatic ring, or a 5- to 6-membered non-aromatic heterocyclic ring; Ring A can be furan, thiophene, isoxazole, oxazole, thiazole, oxadiazole, pyrrole, pyrazole, imidazole, triazole, or tetrazole;
Preferably, Ring A is structured as follows: 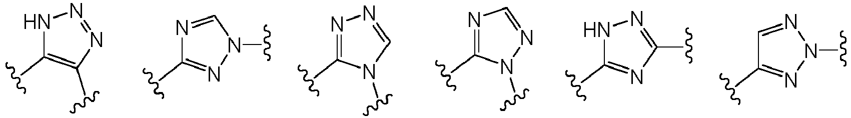 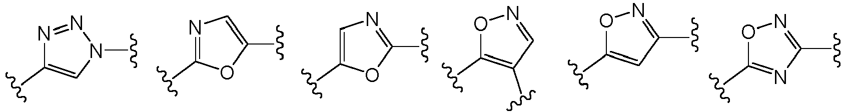 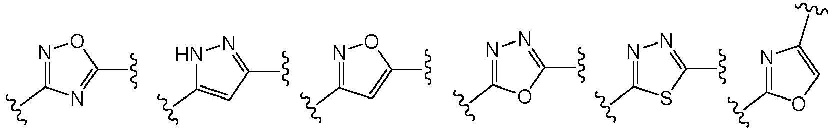 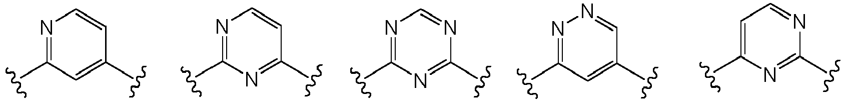 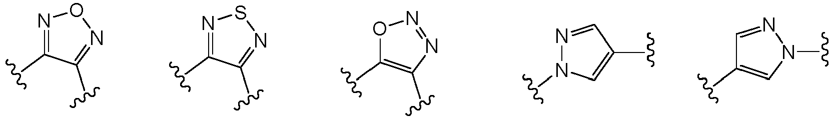
Ring A can be substituted by one or two halogens, -CN, C₁-C₆ alkyl, or C₁-C₆ halogenated alkyl;
Ring B is a substituted or unsubstituted 5- to 7-membered heterocyclic ring containing nitrogen atom;
Ring C is a substituted or unsubstituted benzene ring, or a 5- to 6-membered heteroaromatic ring;
Y¹ and Y² are independent carbon atoms or nitrogen atoms;
Preferably, 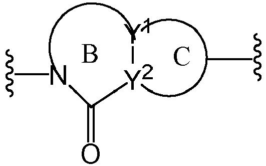 is structured as follows: 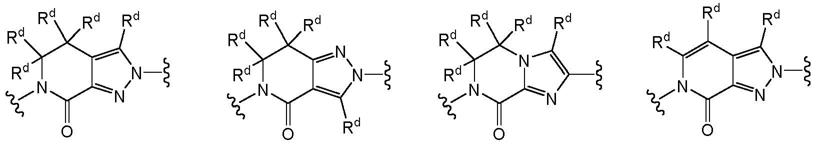 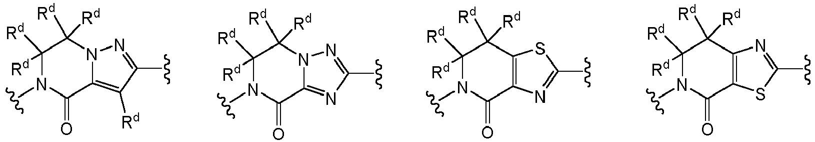 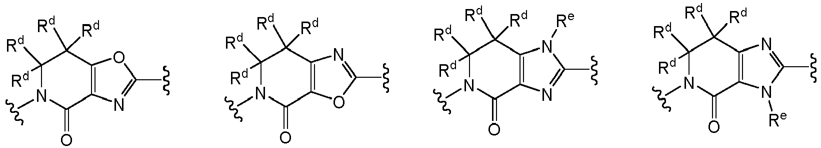 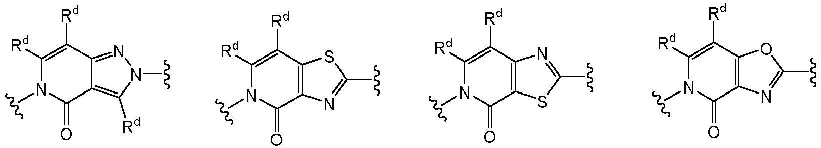 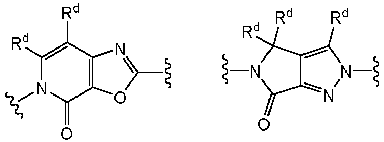
R^{d} is independently selected from H, halogen, C₁-C₆ alkyl, or C₁-C₆ halogenated alkyl;
R^{e} is H, C₁-C₆ alkyl, or C₁-C₆ halogenated alkyl;
L² is O, NR^{g}, CR^{a}R^{b}, or a chemical bond;
L³ is C₀-C₆ alkylene, C₂-C₆ alkenylene, C₃-C₆ cycloalkyl, benzene ring, 5- to 6-membered heteroaromatic ring, or 5- to 6-membered non-aromatic heterocyclic ring; L³ can be substituted by 1 to 3 R⁵;
R⁵ is independently H, halogen, -OH, -CN, carbonyl, C₁-C₆ alkyl, C₁-C₆ halogenated alkyl, C₁-C₆ alkoxy, or C₁-C₆ halogenated alkoxy;
Ring D is C₃-C₆ cycloalkyl, phenyl, naphthyl, C₃-C₆ cycloalkyl and phenyl, 5- to 6-membered heteroaryl, or 5- to 6-membered non-aromatic heterocyclyl;
R¹⁰ is independently selected from halogen, CN, C₁-C₆ alkyl, C₁-C₆ halogenated alkyl, OC₁-C₆ alkyl, or C₃-C₆ cycloalkyl;
n is 0, 1, 2, or 3.

2. According to claim 1, the said compound has the structure shown in general formula II, 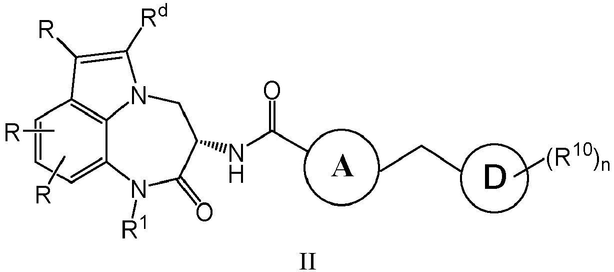
Wherein, the independent definitions of R and R¹⁰ are as stated in general formula I; R¹ is H, methyl or ethyl;
R^{d} is independent H, halogen, C₁-C₆ alkyl, or C₁-C₆ halogenated alkyl;
Ring A is structured as follows: 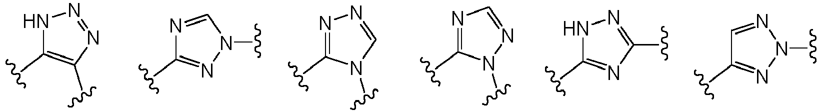 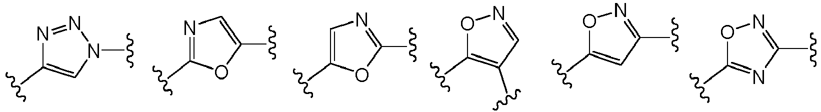 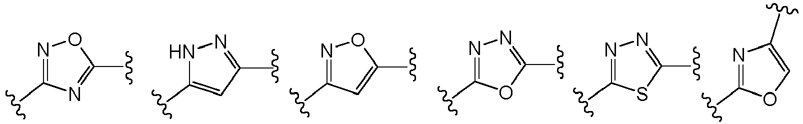 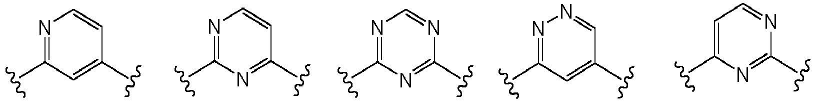 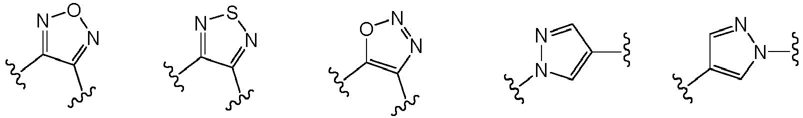
Ring D is a benzene ring or thiophene;
R¹⁰ is an independent halogen or -CN;
n is 0, 1, 2, or 3.

3. According to claim 1, the said compound has a structure shown in general formula III, 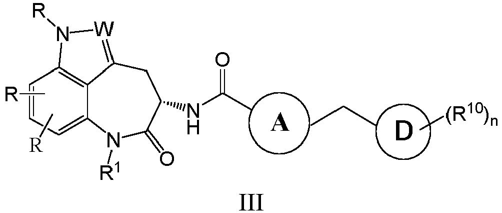
Wherein, the independent definitions of R and R¹⁰ are as stated in general formula I, provided that R is not a halogen when connected to a nitrogen atom; R¹ is H, methyl or ethyl;
W is N or CR^{d};
R^{d} is H, halogen, C₁-C₆ alkyl, or C₁-C₆ halogenated alkyl;
Ring A is structured as follows: 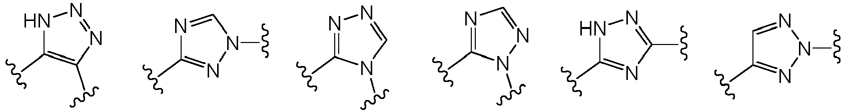 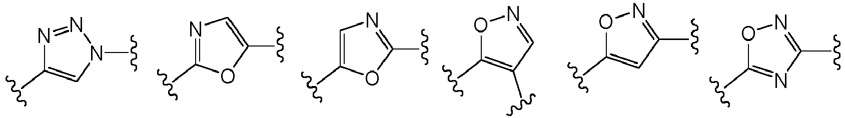 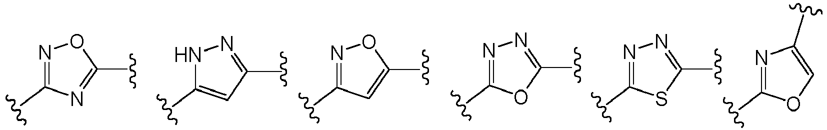 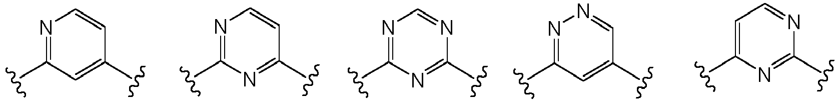 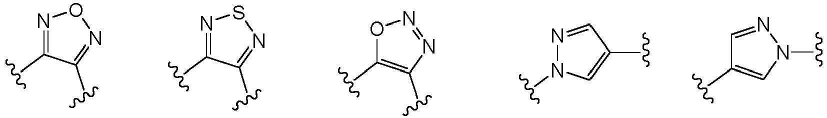
Ring D is a benzene ring or thiophene;
R¹⁰ is an independent halogen or -CN;
n is 0, 1, 2, or 3.

4. According to claim 1, the said compound has the structure shown in general formula IV, 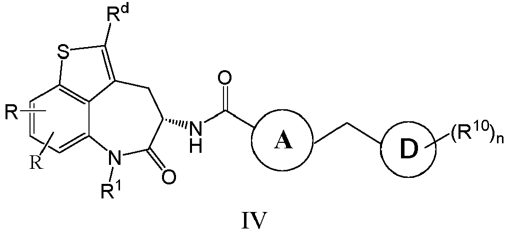
Wherein, the independent definitions of R and R¹⁰ are as stated in general formula I; R¹ is H, methyl or ethyl;
R^{d} is independent H, halogen, C₁-C₆ alkyl, or C₁-C₆ halogenated alkyl;
n is 0, 1, 2, or 3;
Ring A is structured as follows: 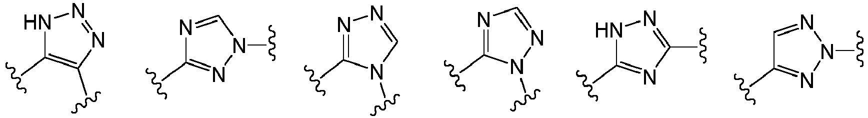 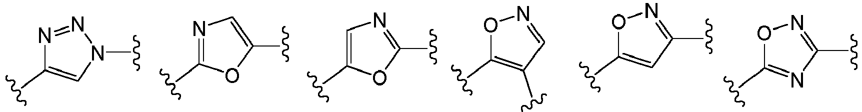 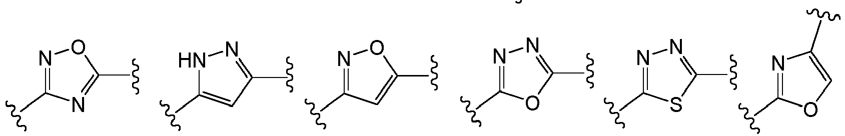 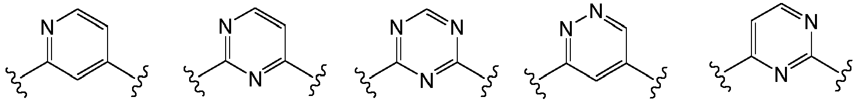 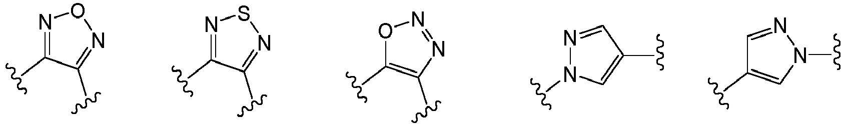
Ring D is a benzene ring or thiophene;
R¹⁰ is an independent halogen or -CN;
n is 0, 1, 2, or 3.

5. According to claim 1, the said compound is represented by general formula V, 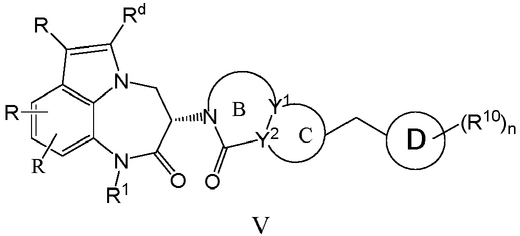
Wherein, the independent definitions of R are as stated in general formula I; R¹ is H, methyl or ethyl; 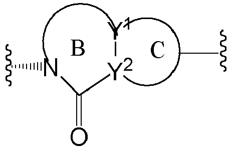 is structured as follows: 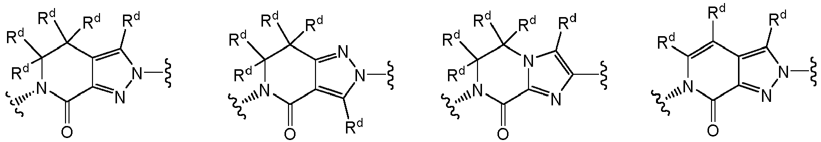 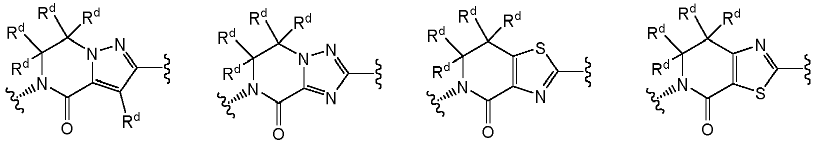 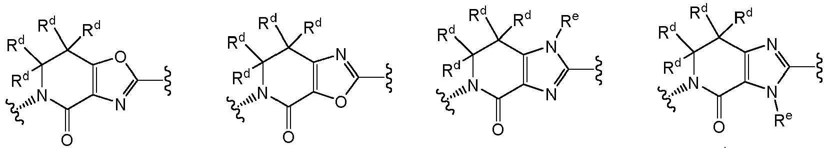 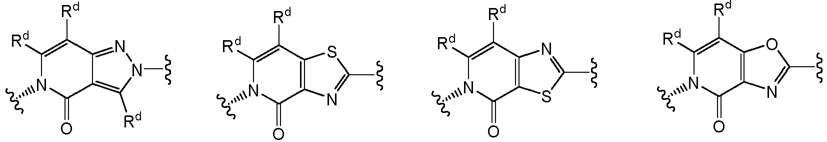 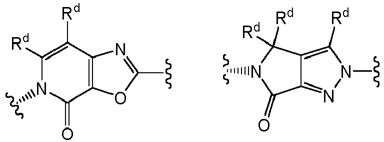
R^{d} is independent H, halogen, C₁-C₆ alkyl, or C₁-C₆ halogenated alkyl;
R^{e} is H, C₁-C₆ alkyl, or C₁-C₆ halogenated alkyl;
Ring D is a benzene ring or thiophene;
R¹⁰ is an independent halogen or -CN;
n is 0, 1, 2, or 3.

6. According to claim 1, the said compound is represented by general formula VI, 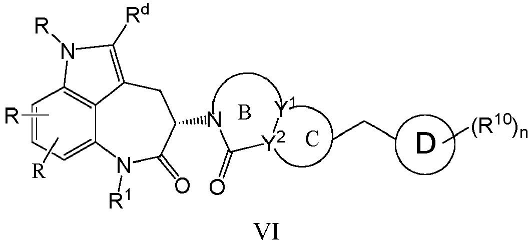
Wherein, the independent definitions of R are as stated in general formula I, provided that R is not a halogen when connected to a nitrogen atom; R¹ is H, methyl or ethyl; 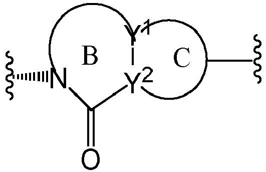 is structured as follows: 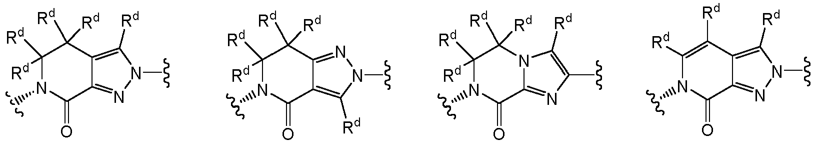 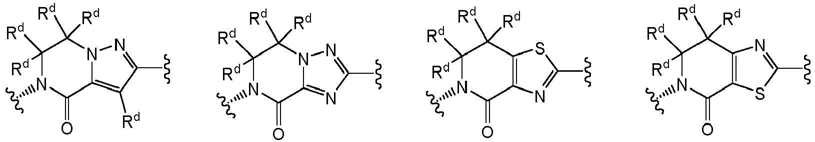 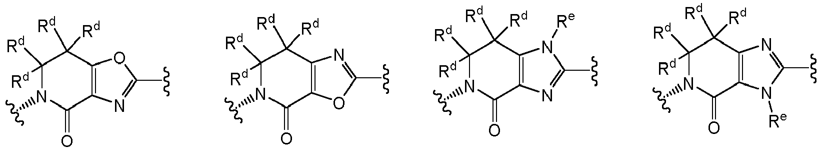 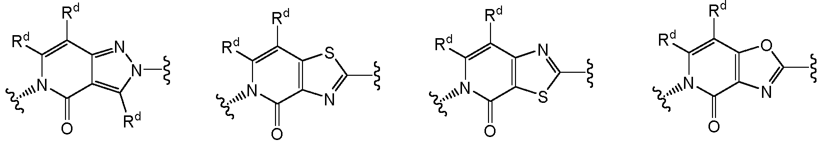 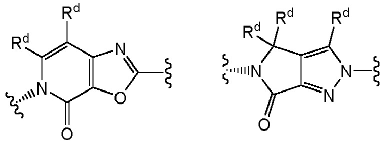
R^{d} is independent H, halogen, C₁-C₆ alkyl, or C₁-C₆ halogenated alkyl;
R^{e} is H, C₁-C₆ alkyl, or C₁-C₆ halogenated alkyl;
Ring D is a benzene ring or thiophene;
R¹⁰ is an independent halogen or -CN;
n is 0, 1, 2, or 3.

7. According to claim 1, the said compound is represented by general formula VII, 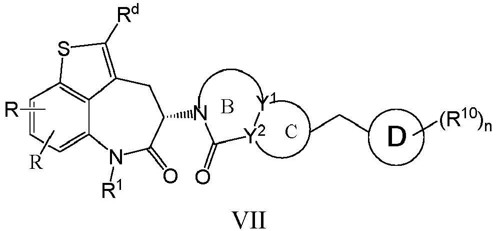
Wherein, the independent definitions of R and R¹⁰ are as stated in general formula I, provided that R is not a halogen when connected to a nitrogen atom; R¹ is H, methyl or ethyl; 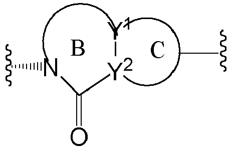 is structured as follows: 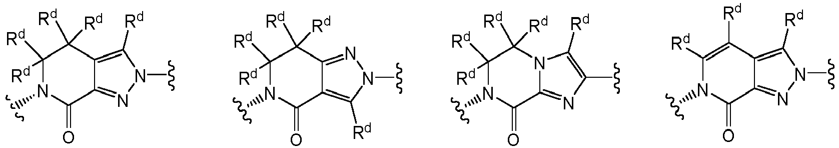 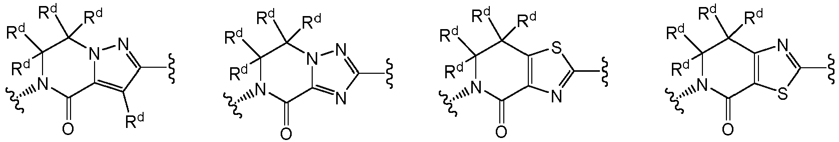 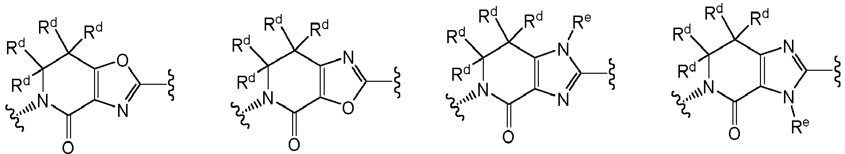 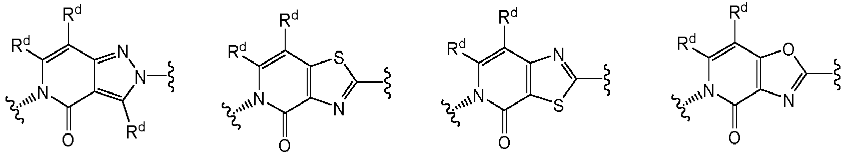 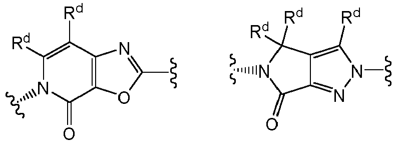
R^{d} is H, halogen, C₁-C₆ alkyl, or C₁-C₆ halogenated alkyl;
R^{e} is H, C₁-C₆ alkyl, or C₁-C₆ halogenated alkyl;
Ring D is a benzene ring or thiophene;
R¹⁰ is an independent halogen or -CN;
n is 0, 1, 2, or 3.

8. According to claim 1, the said compound is represented by general formula VIII, 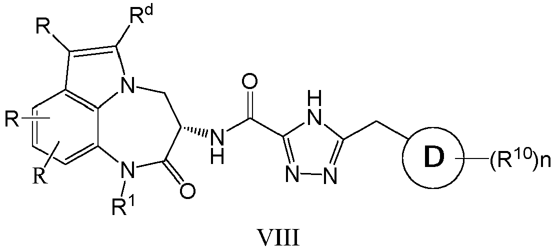
Wherein, the independent definitions of R and R¹⁰ are as stated in general formula I; R¹ is H, methyl or ethyl;
R^{d} is H, halogen, C₁-C₆ alkyl, or C₁-C₆ halogenated alkyl;
Ring D is a benzene ring or thiophene;
n is 0, 1, 2, or 3.

9. According to claim 1, the said compound is represented by general formula IX, 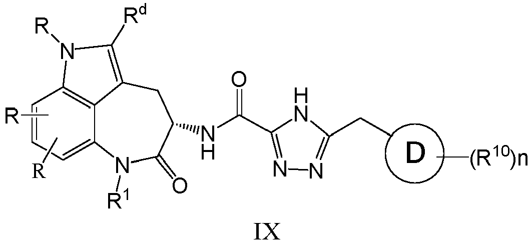
Wherein, the independent definitions of R and R¹⁰ are as stated in general formula I, provided that R is not a halogen when connected to a nitrogen atom; R¹ is H, methyl or ethyl;
R^{d} is H, halogen, C₁-C₆ alkyl, or C₁-C₆ halogenated alkyl;
Ring D is a benzene ring or thiophene;
n is 0, 1, 2, or 3.

10. According to claim 1, the said compound is represented by general formula X, 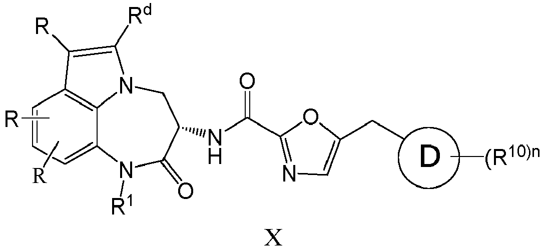
Wherein, the independent definitions of R and R¹⁰ are as stated in general formula I; R¹ is H, methyl or ethyl;
Ring D is a benzene ring or thiophene;
R^{d} is H, halogen, C₁-C₆ alkyl, or C₁-C₆ halogenated alkyl;
n is 0, 1, 2, or 3.

11. According to claim 1, the said compound is represented by general formula XI, 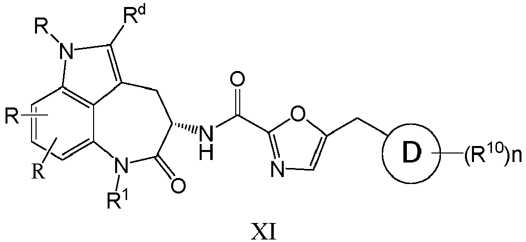
Wherein, the independent definitions of R and R¹⁰ are as stated in general formula I, provided that R is not a halogen when connected to a nitrogen atom; R¹ is H, methyl or ethyl;
Ring D is a benzene ring or thiophene;
R^{d} is H, halogen, C₁-C₆ alkyl, or C₁-C₆ halogenated alkyl;
n is 0, 1, 2, or 3.

12. According to claim 1, the said compound is represented by general formula XII, 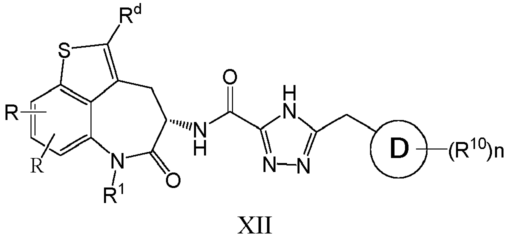
Wherein, the independent definitions of R and R¹⁰ are as stated in general formula I; R¹ is H, methyl or ethyl;
R^{d} is H, halogen, C₁-C₆ alkyl, or C₁-C₆ halogenated alkyl;
Ring D is a benzene ring or thiophene;
n is 0, 1, 2, or 3.

13. According to claim 1, the said compound is selected from the following structures:

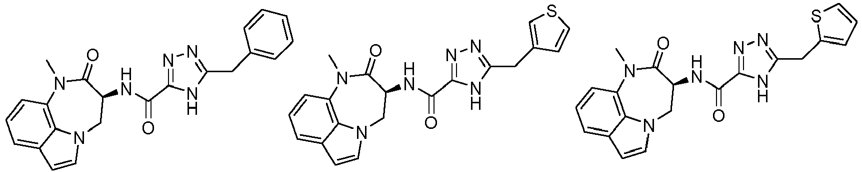

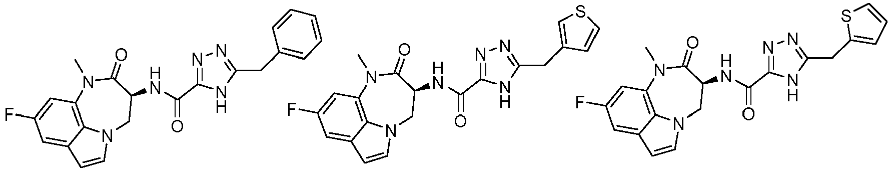

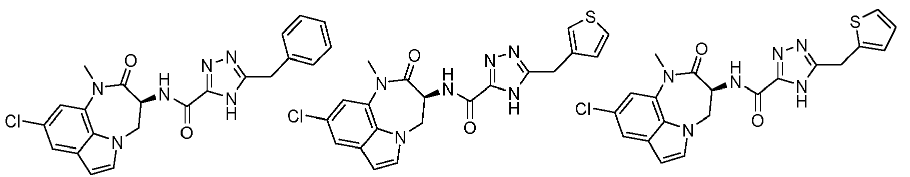

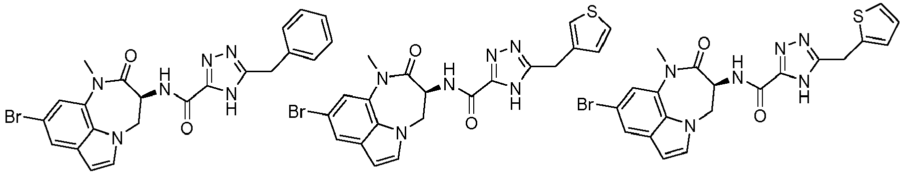

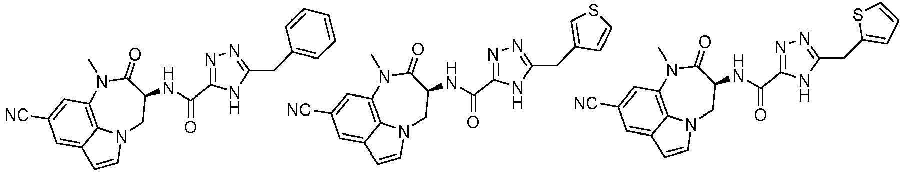

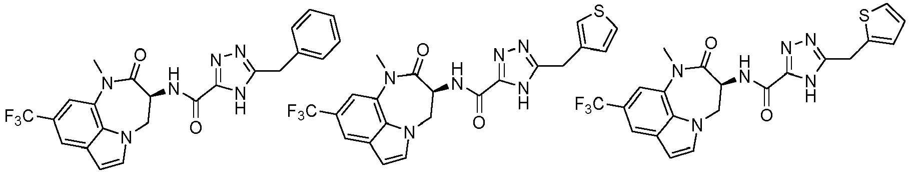

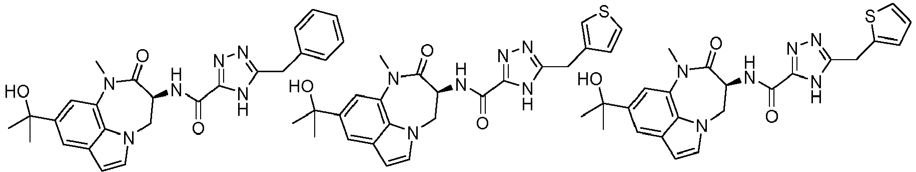

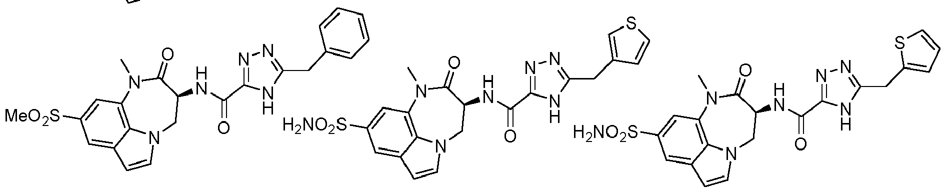

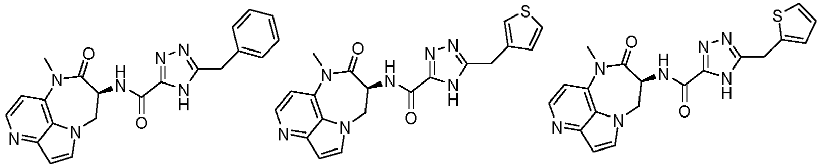

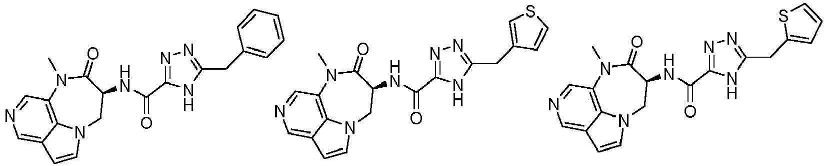

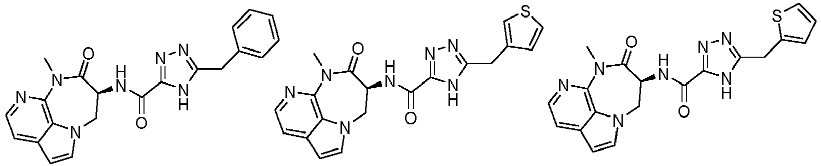

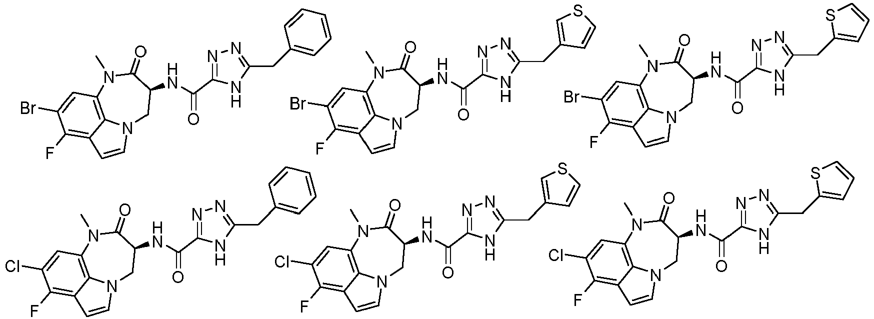

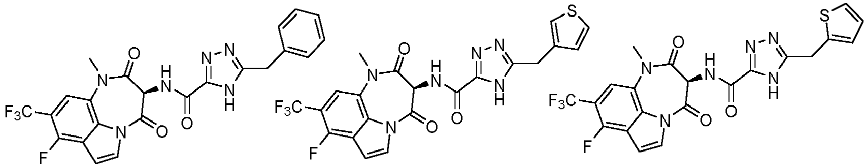

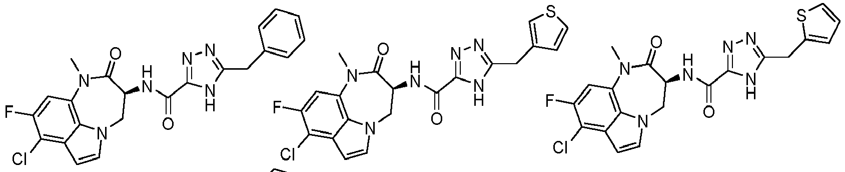

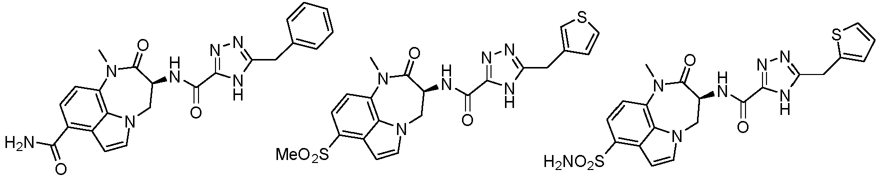

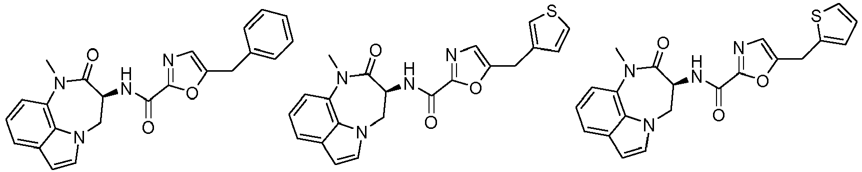

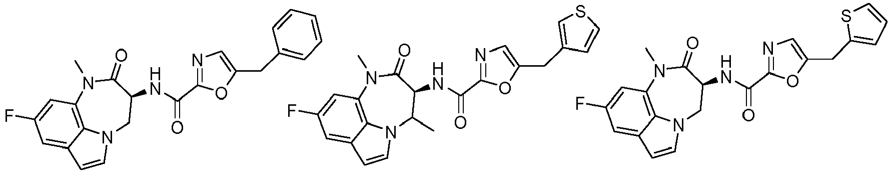

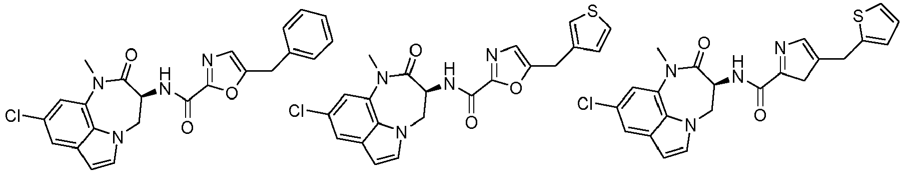

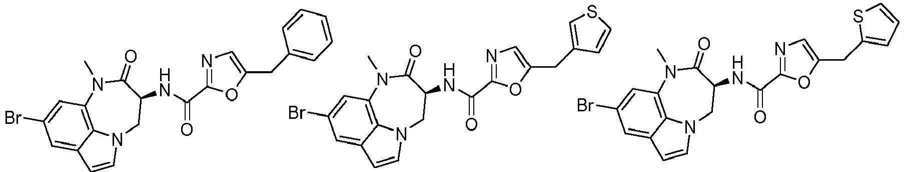

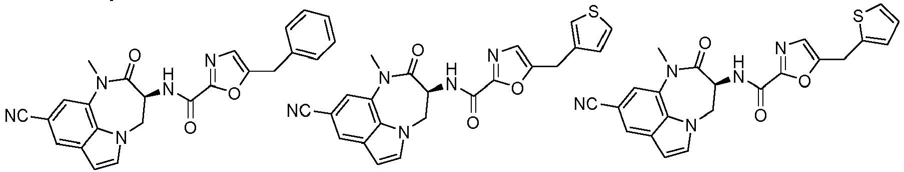

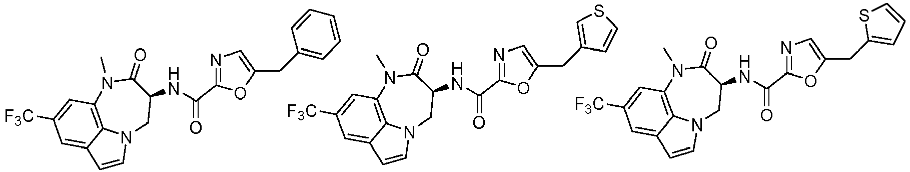

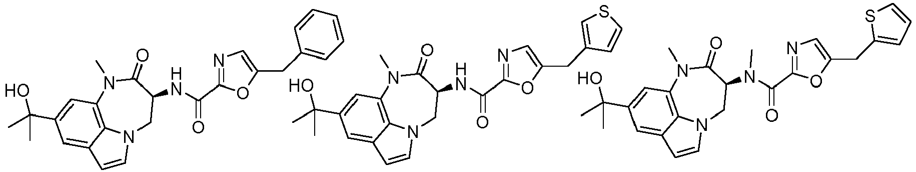

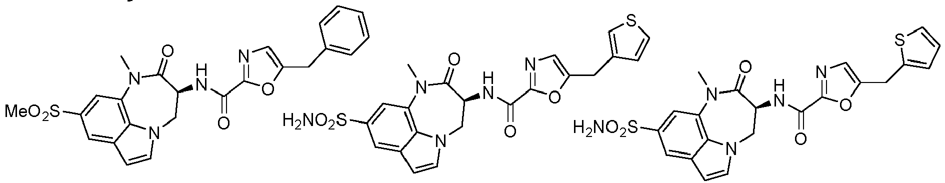

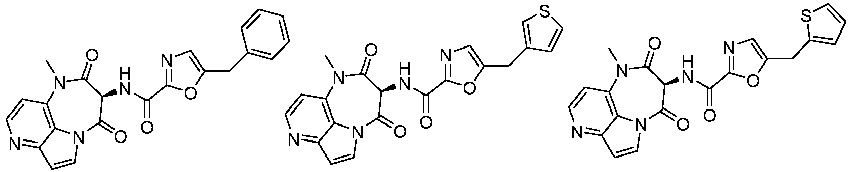

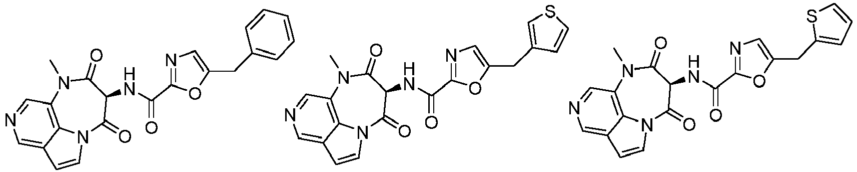

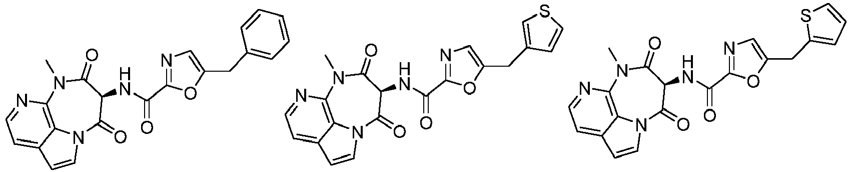

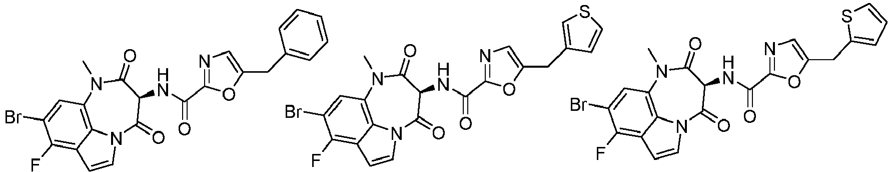

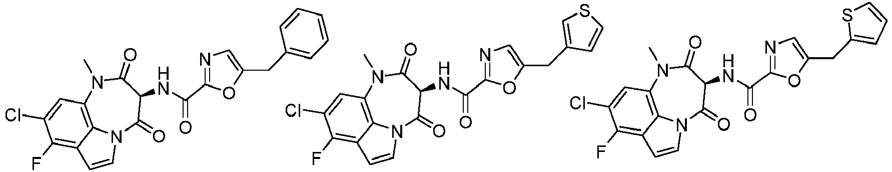

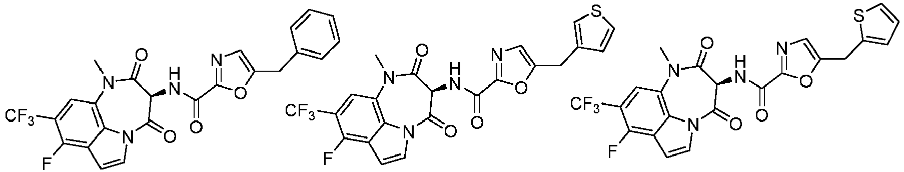

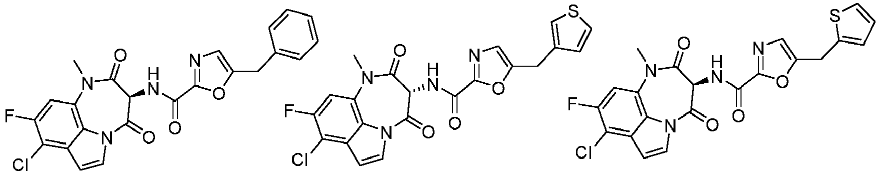

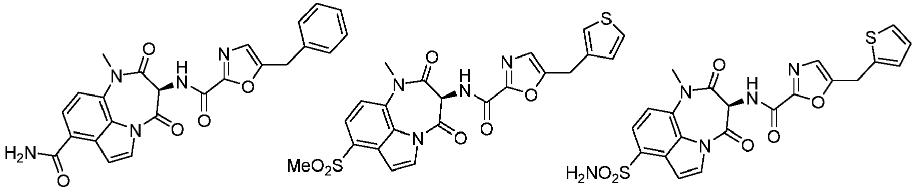

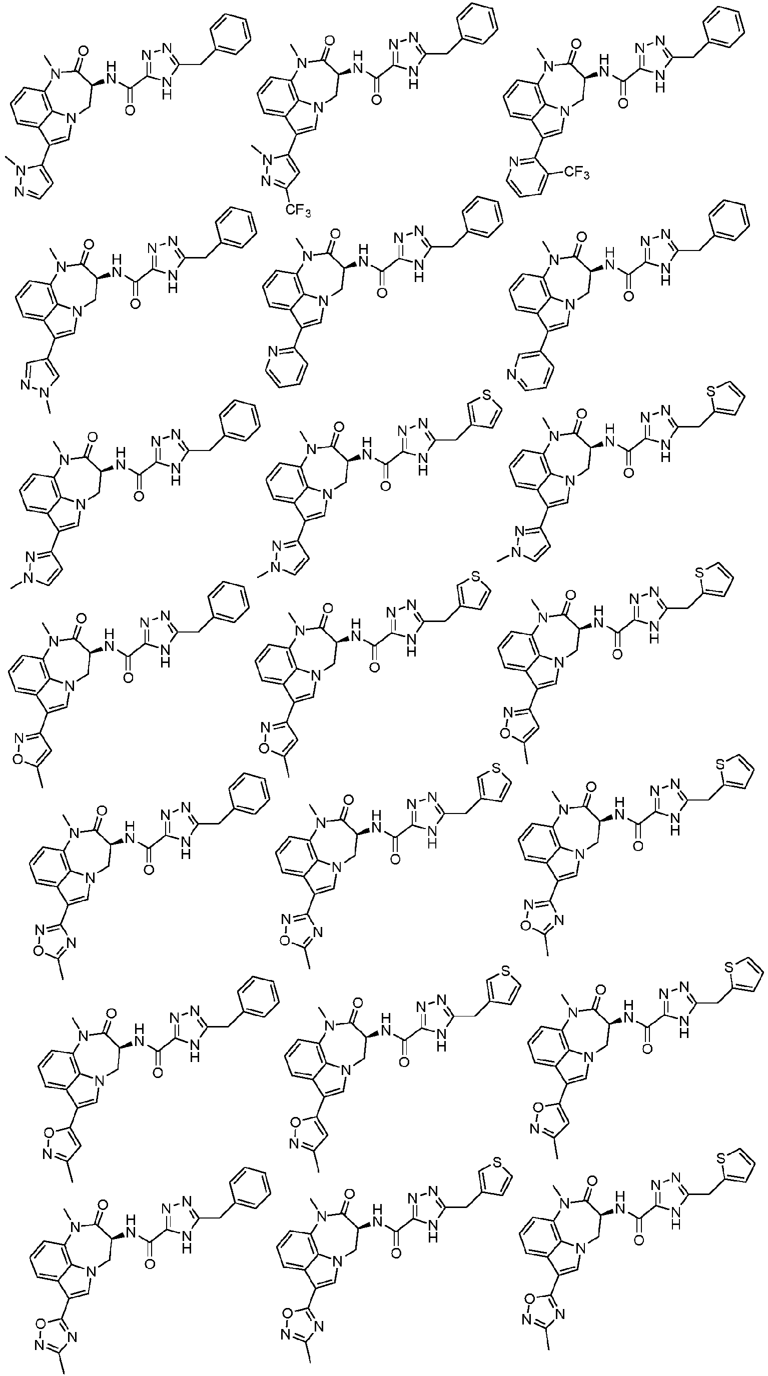

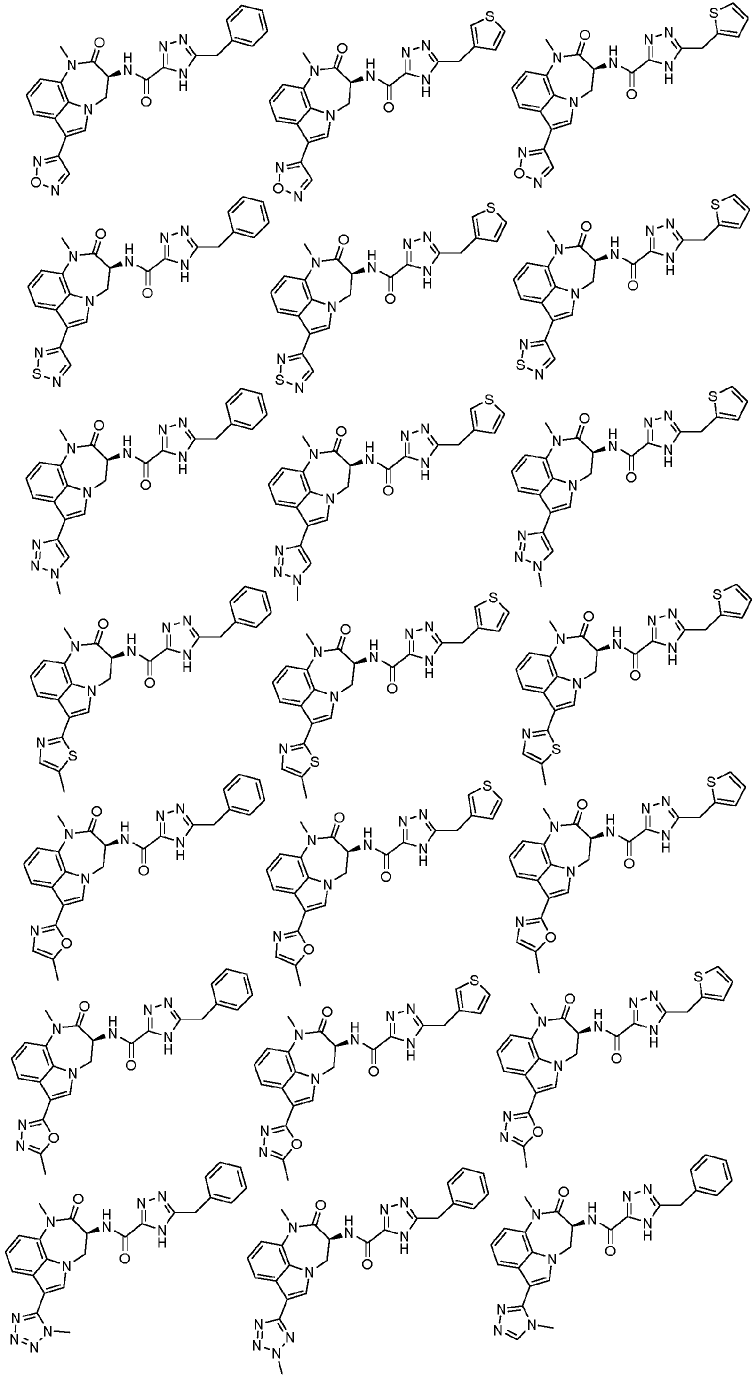

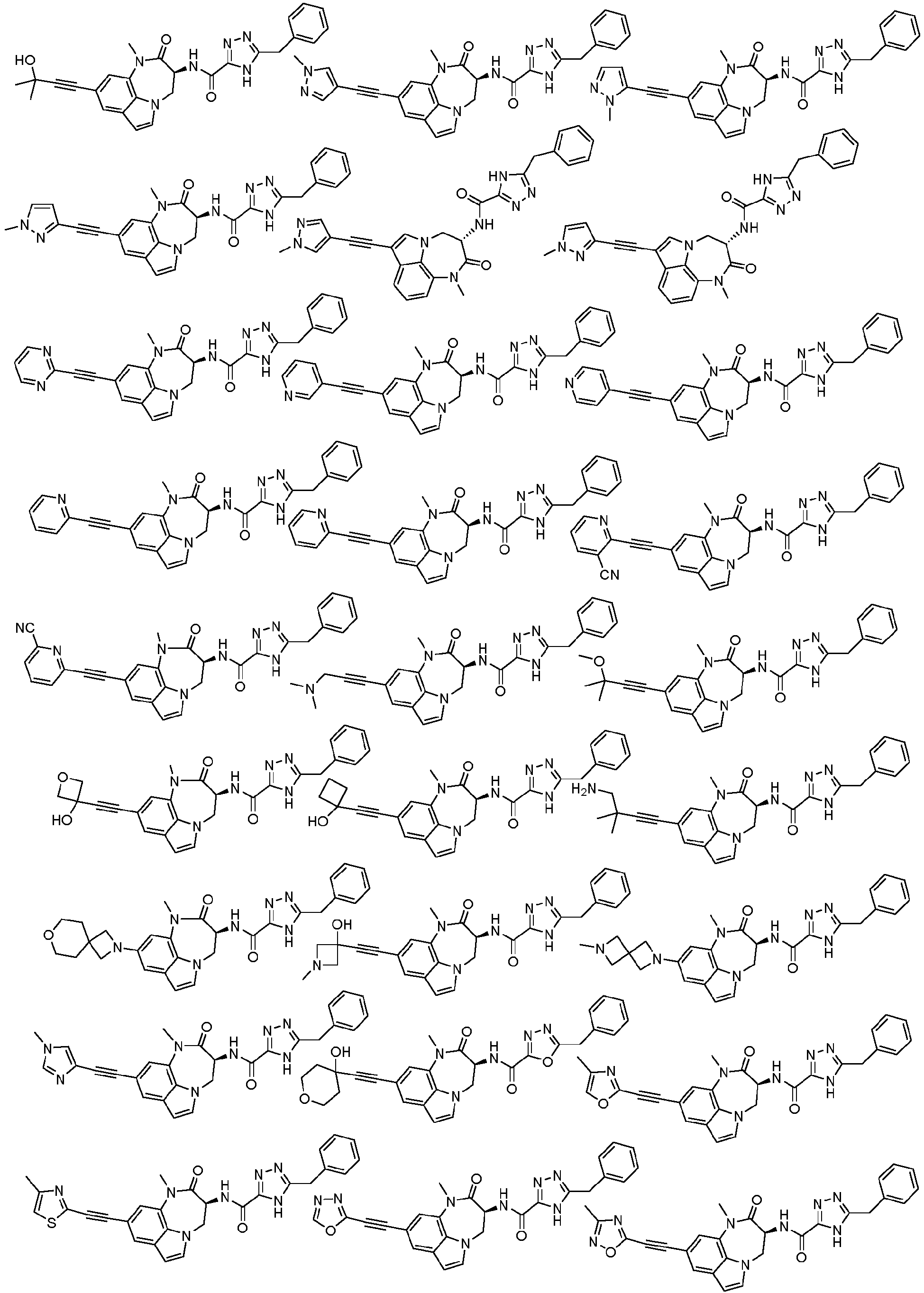

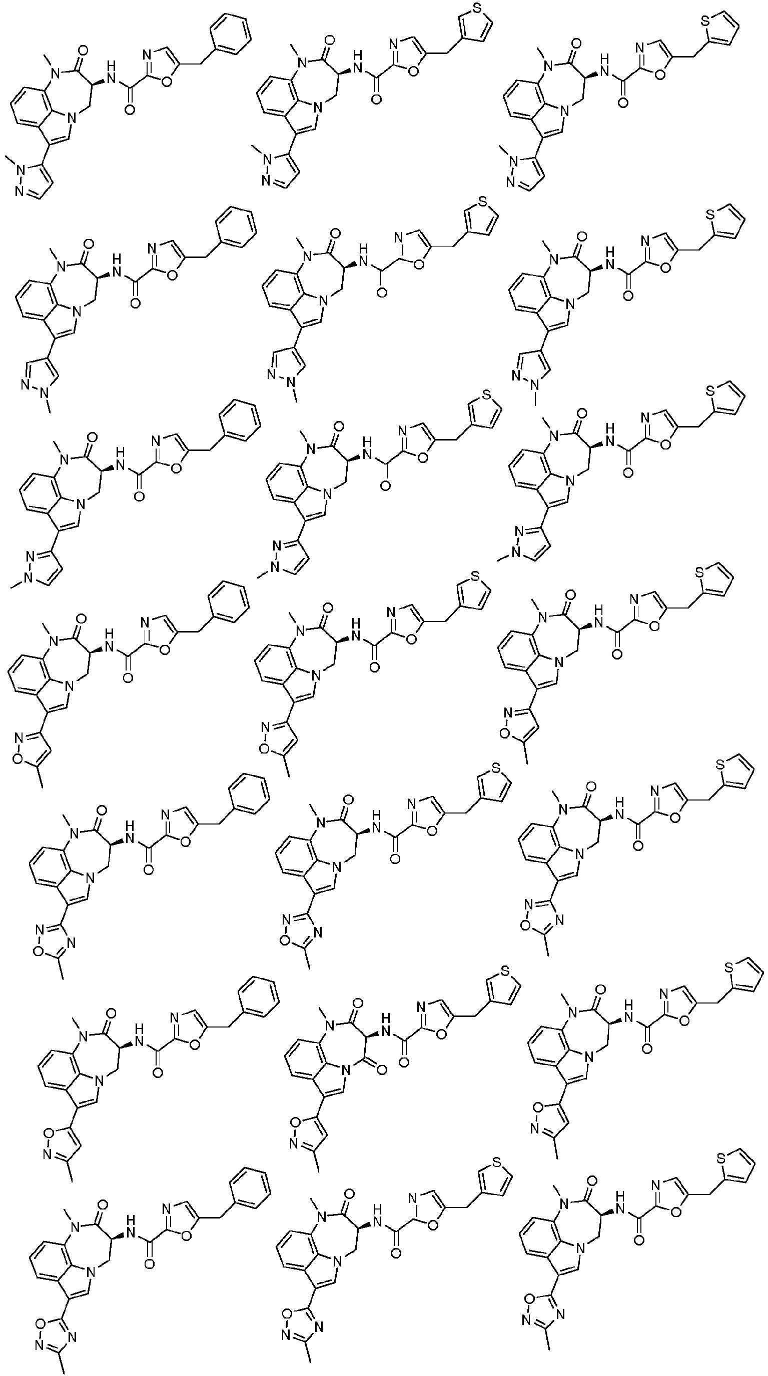

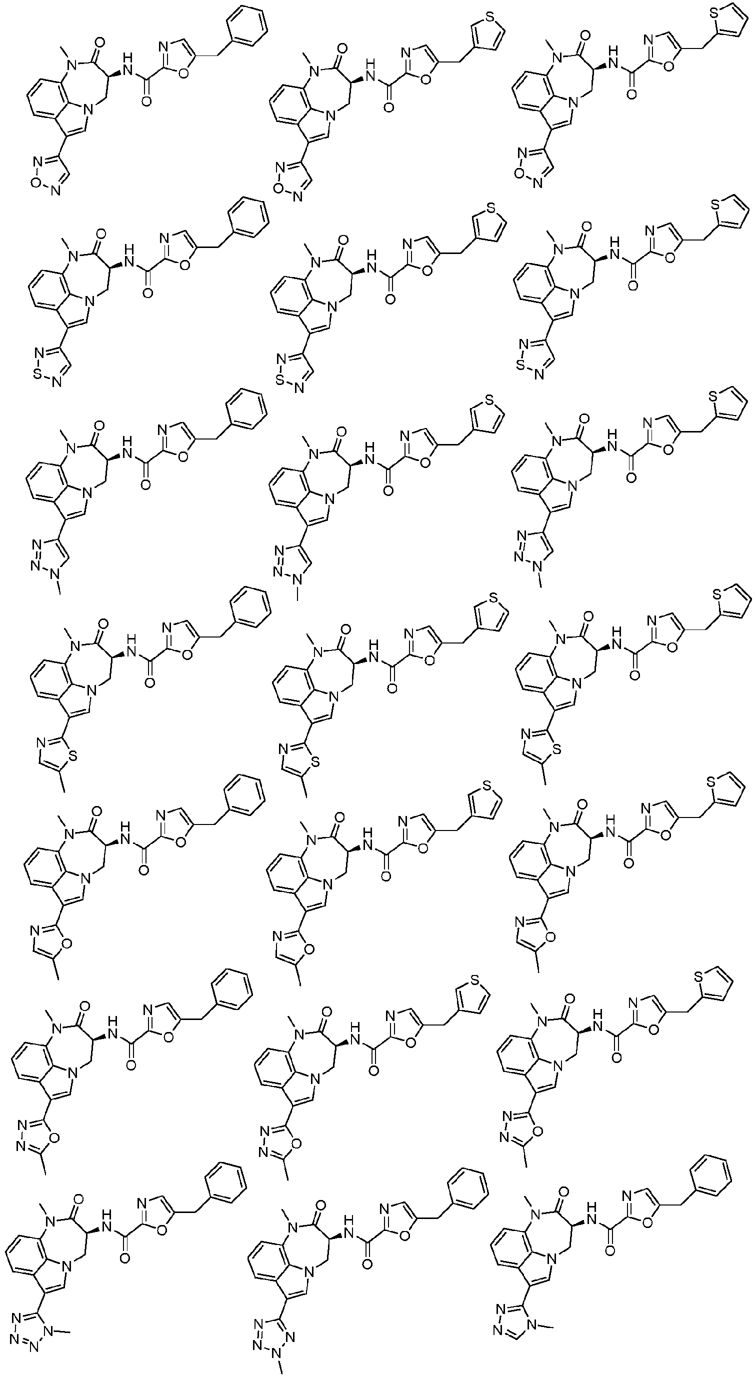

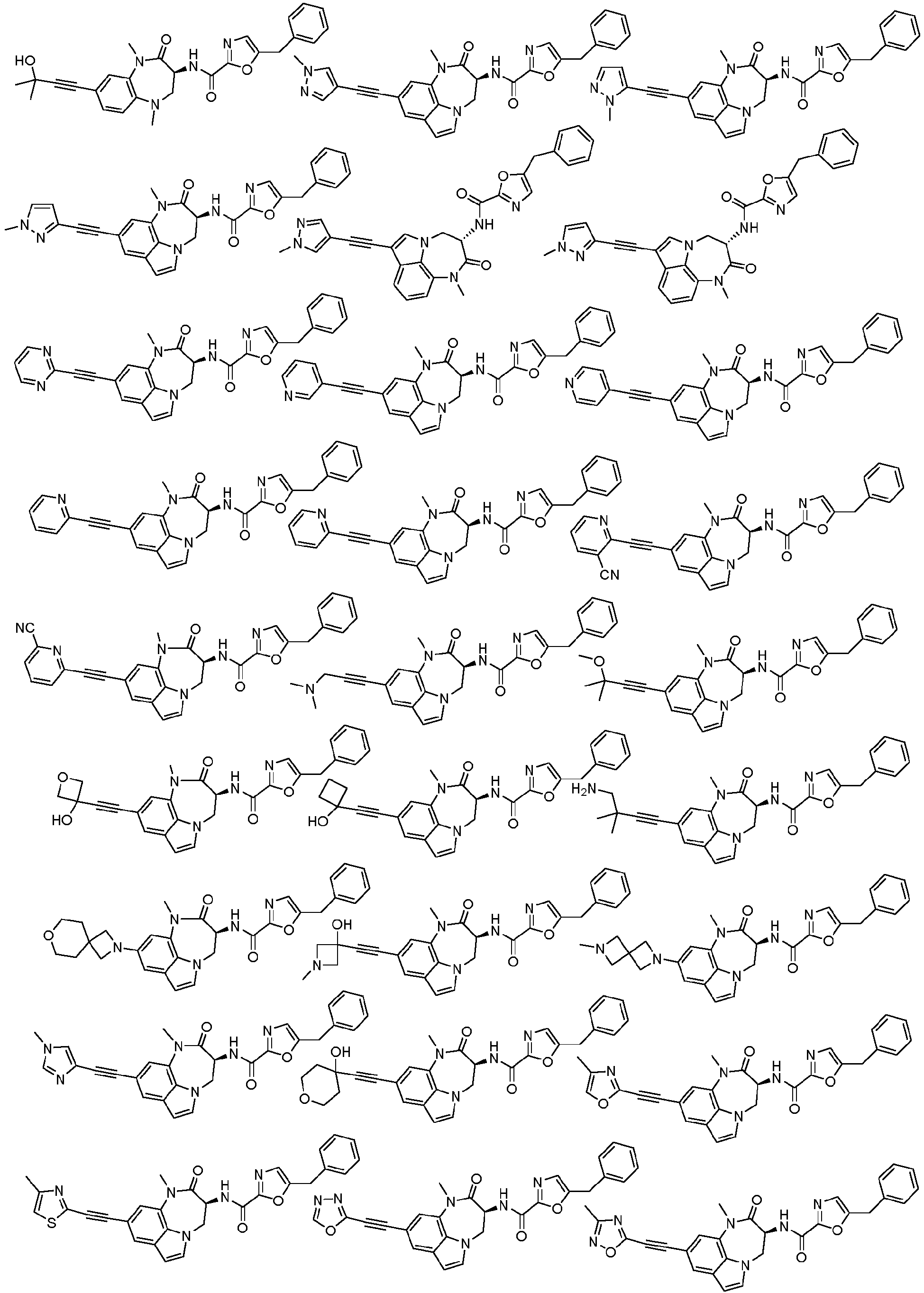

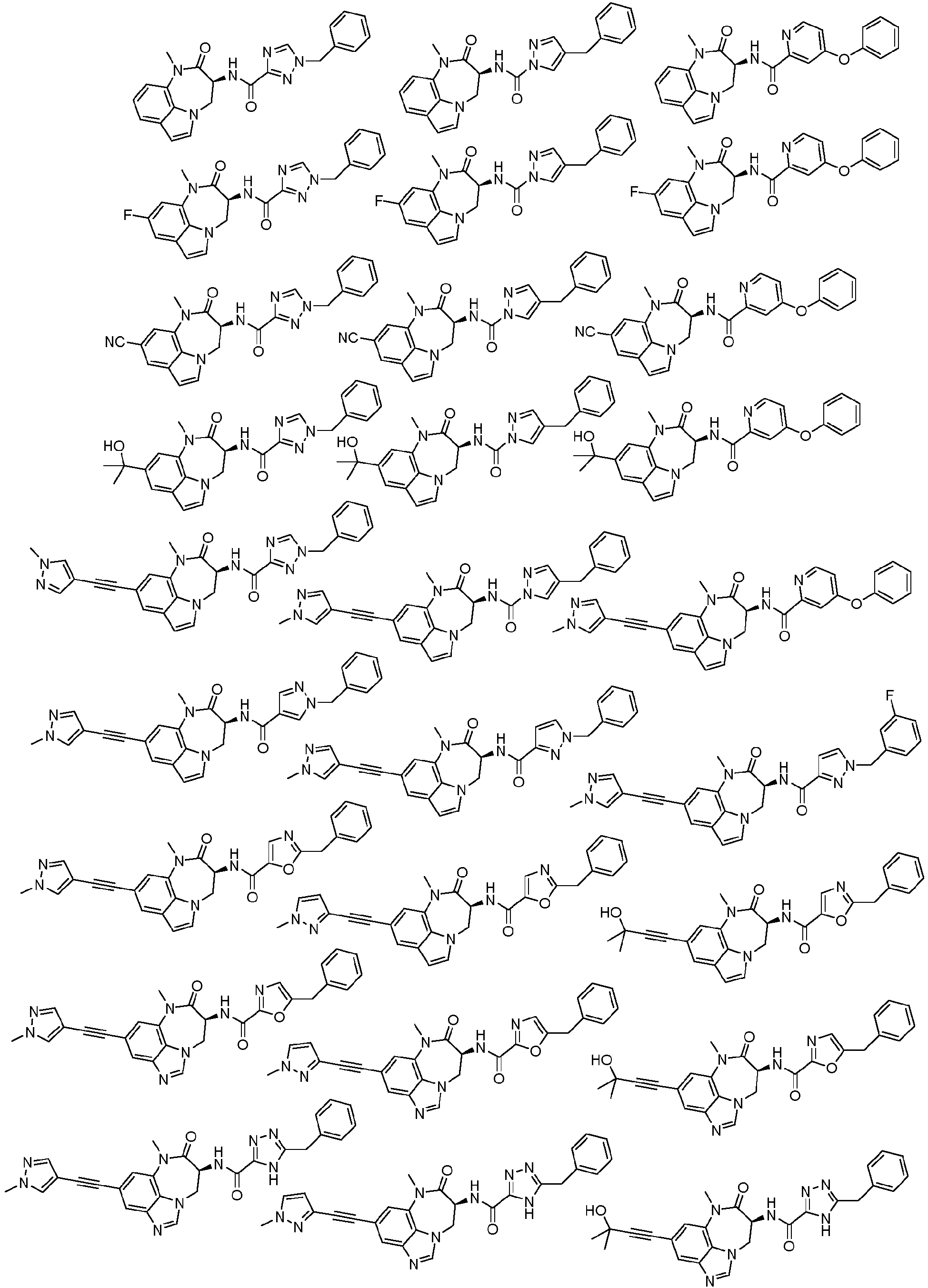

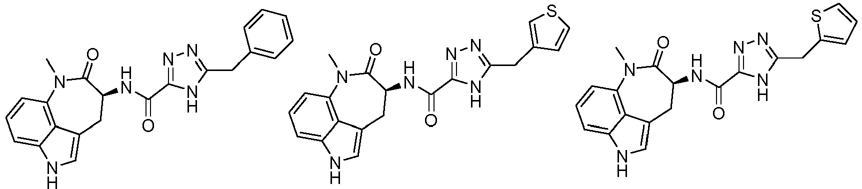

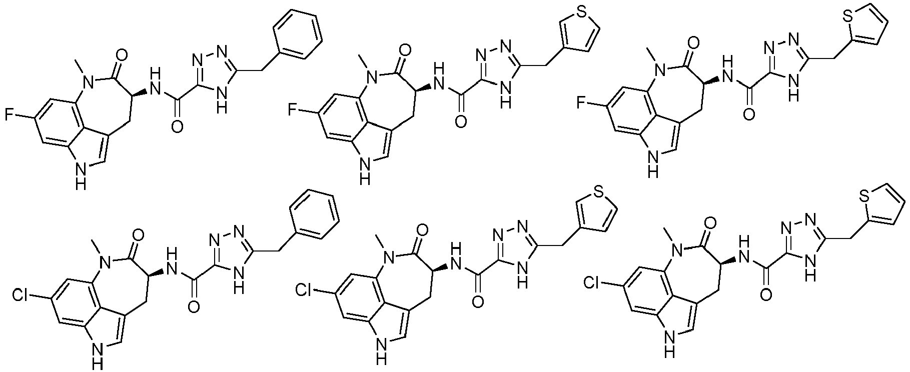

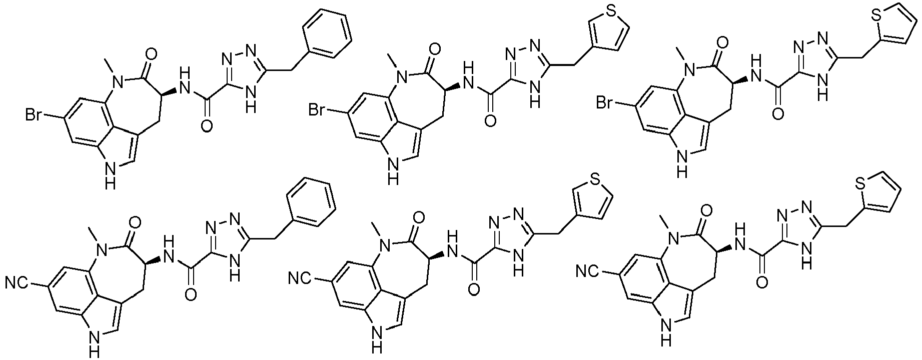

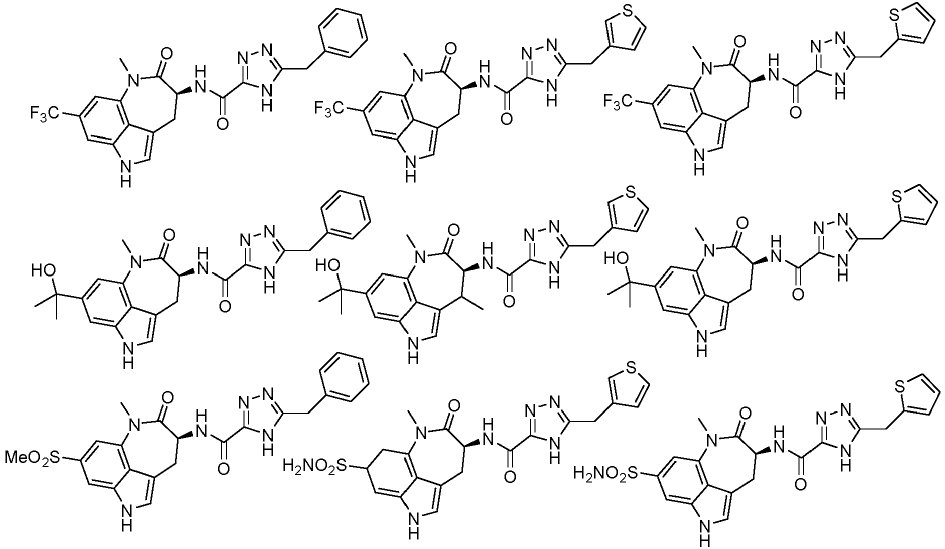

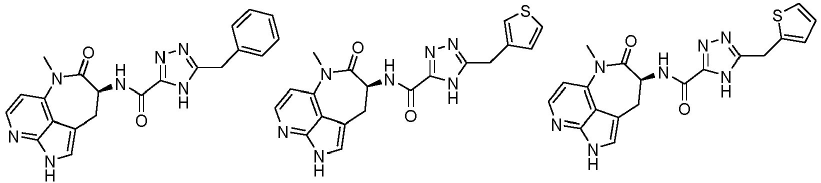

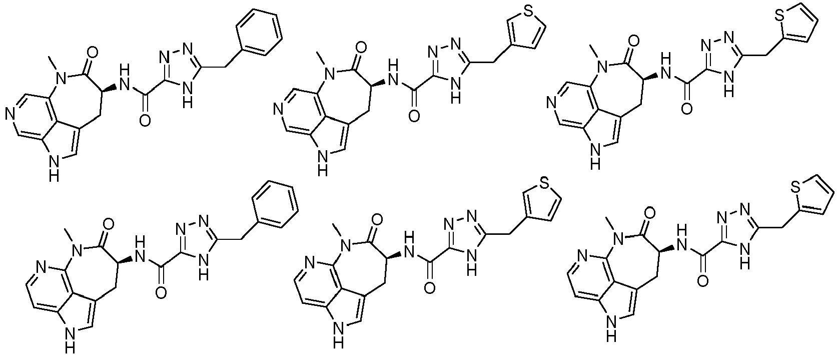

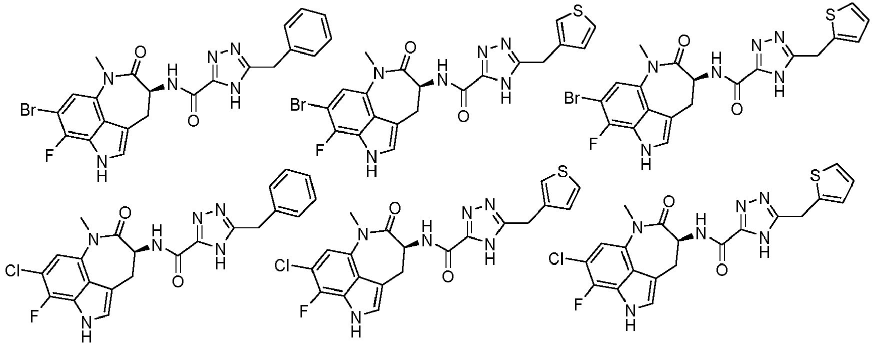

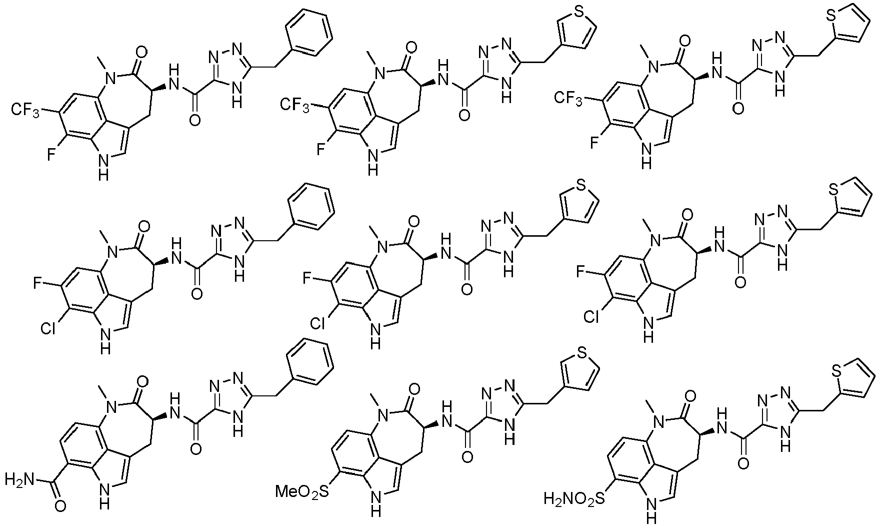

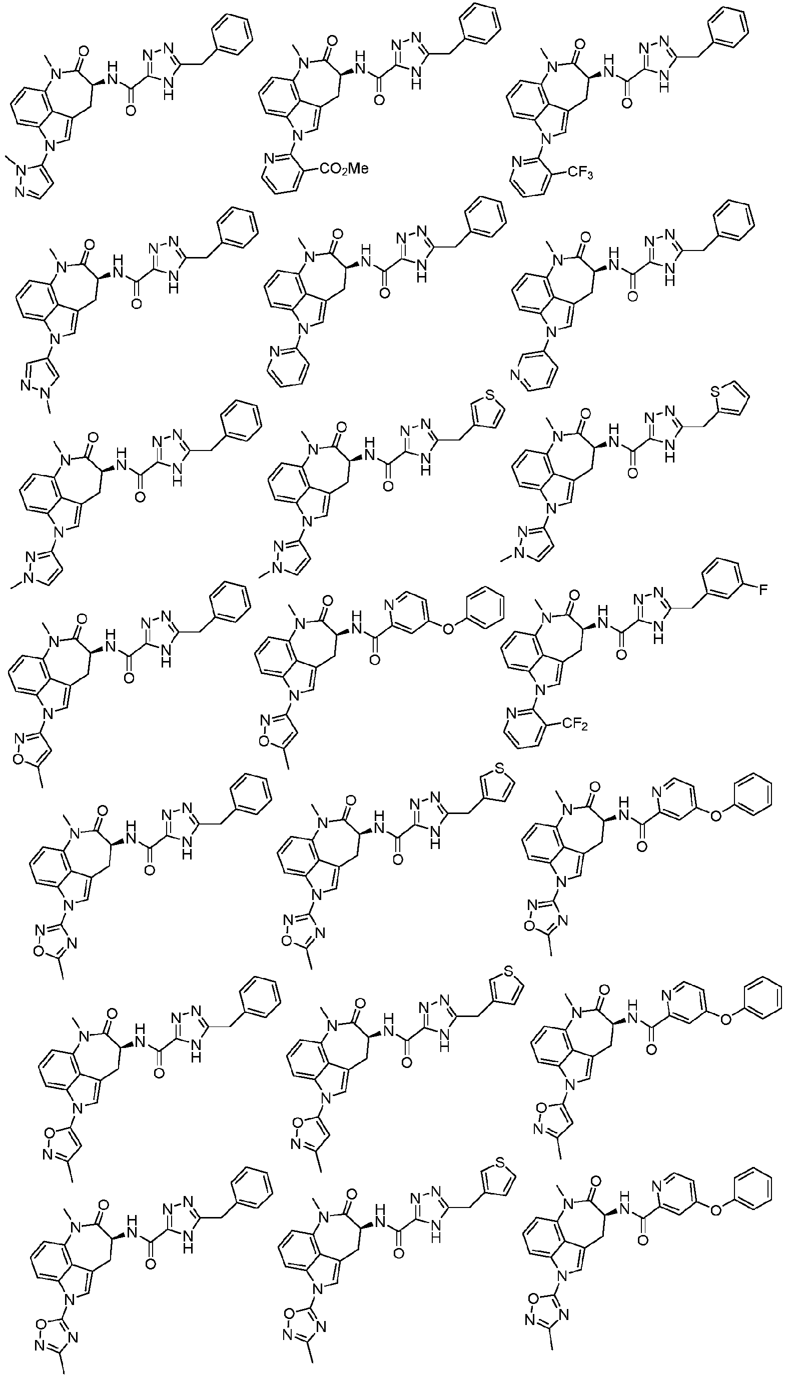

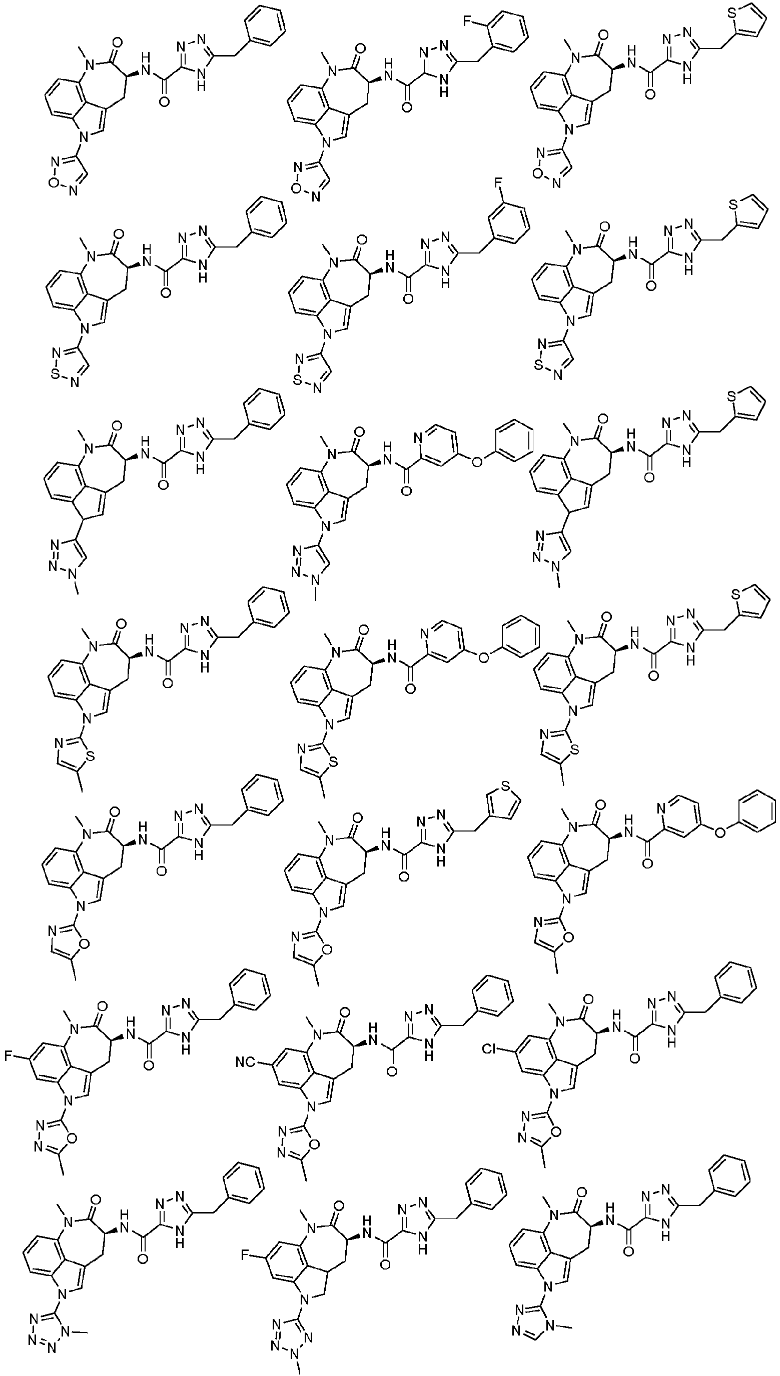

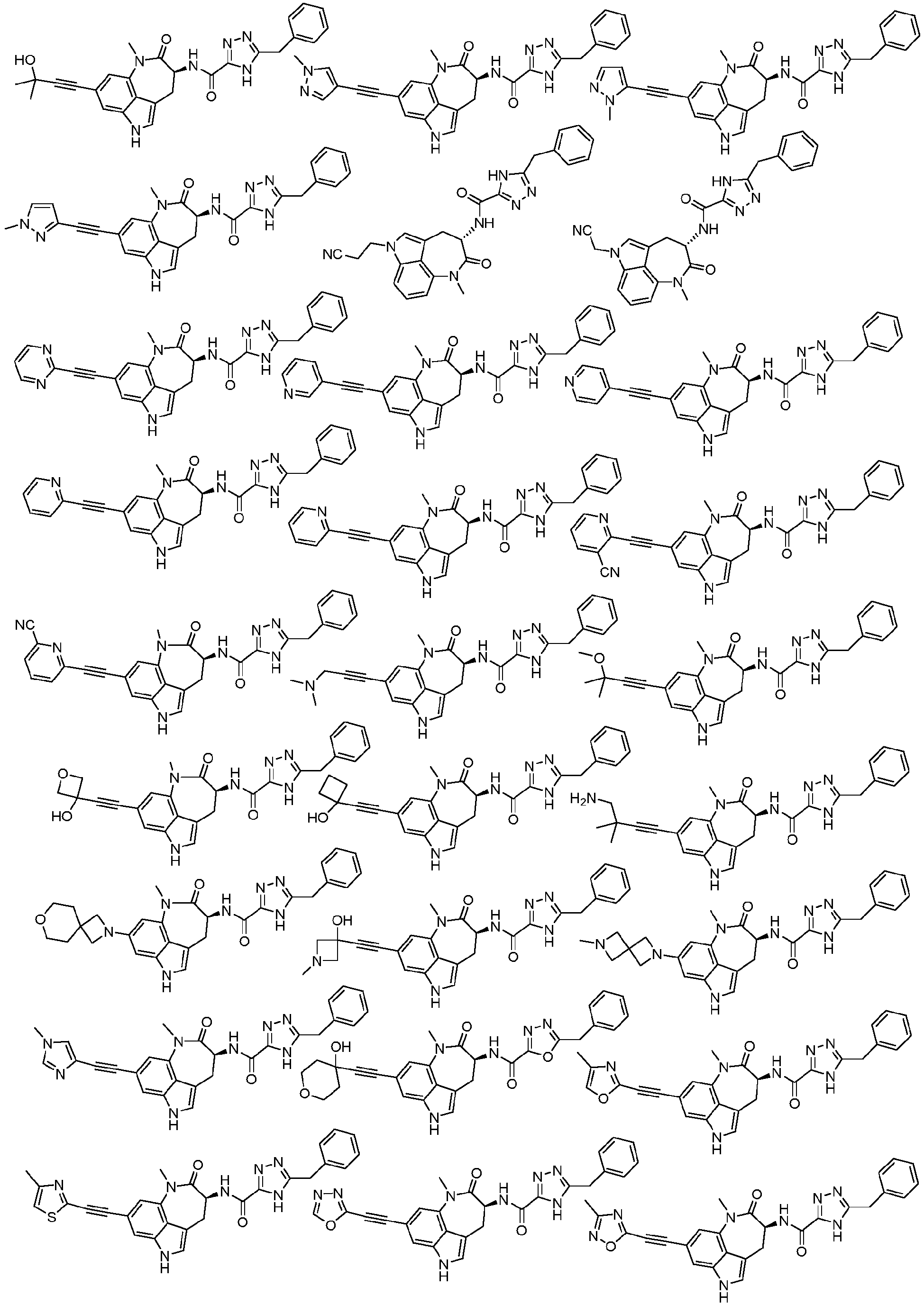

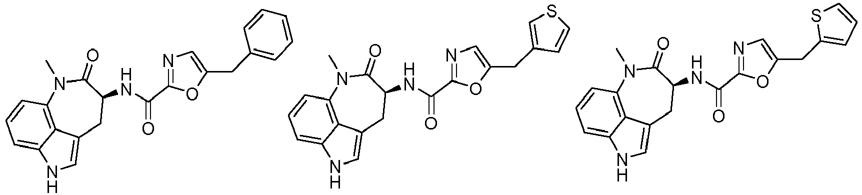

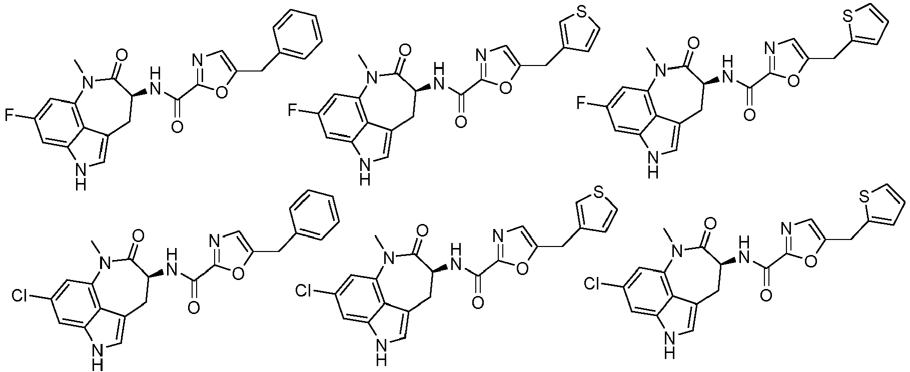

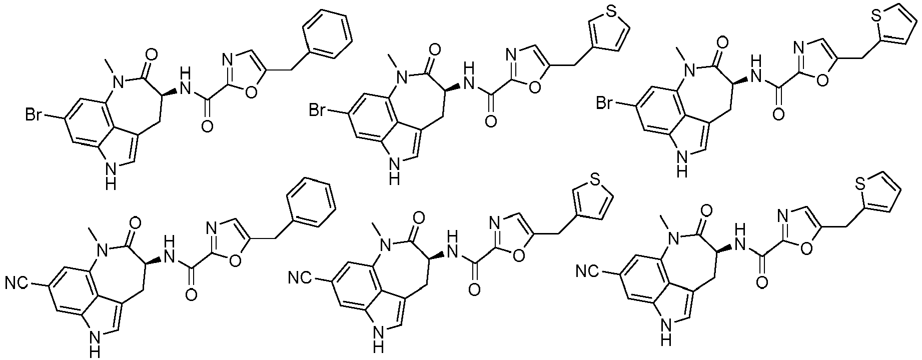

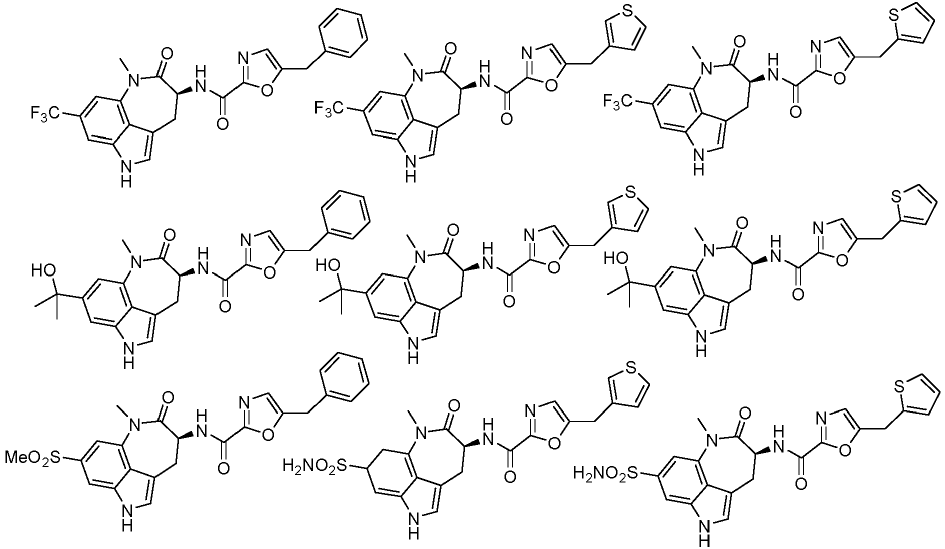

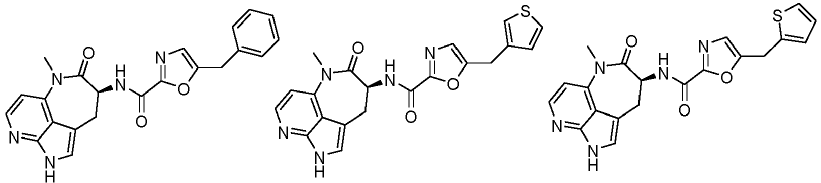

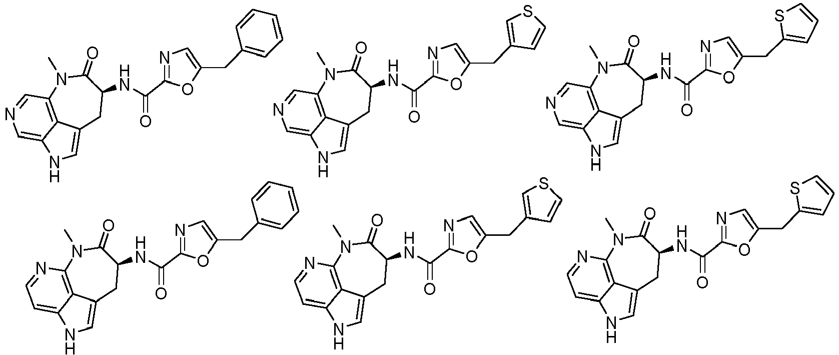

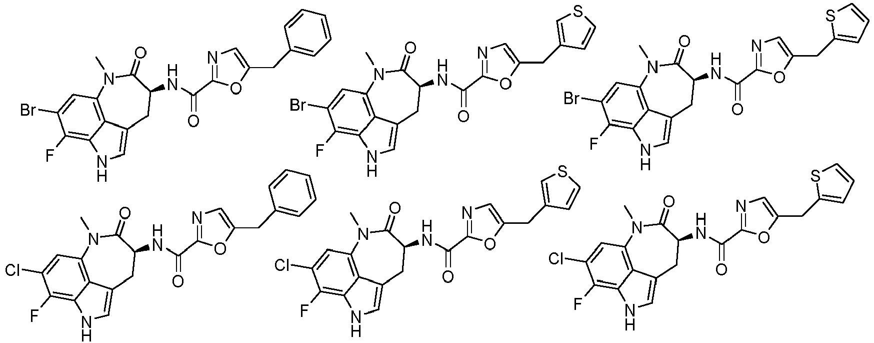

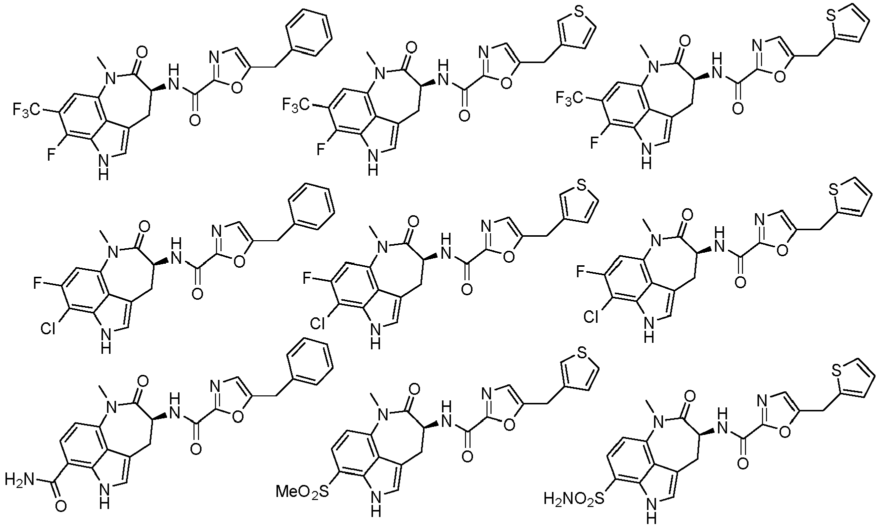

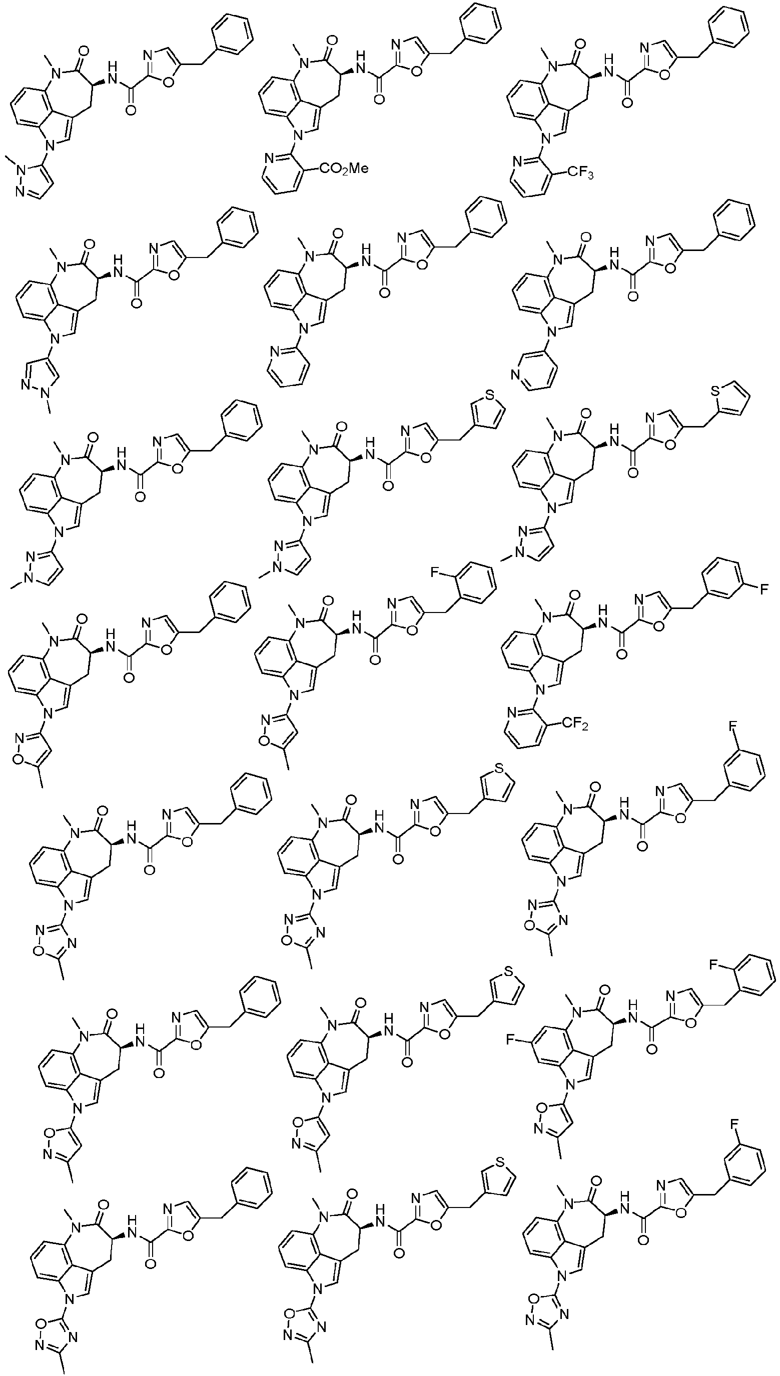

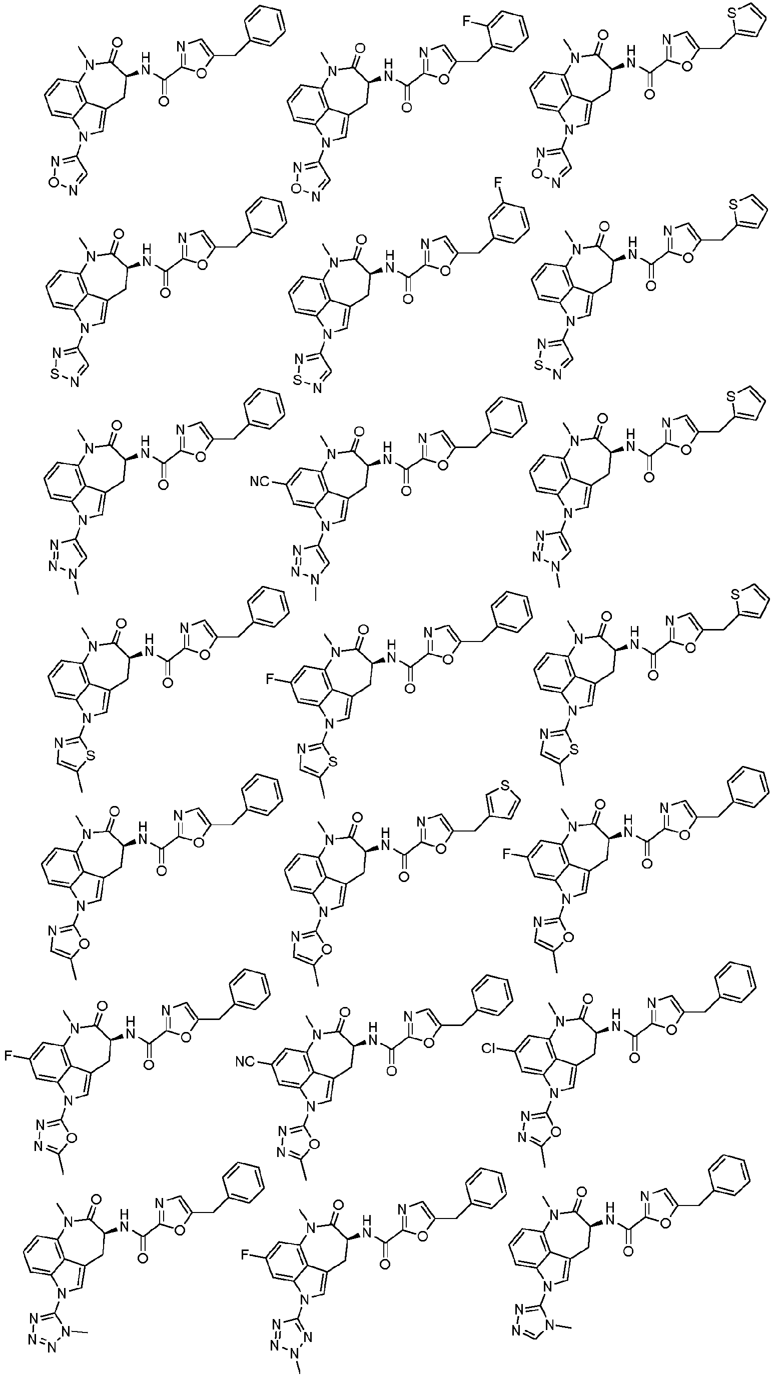

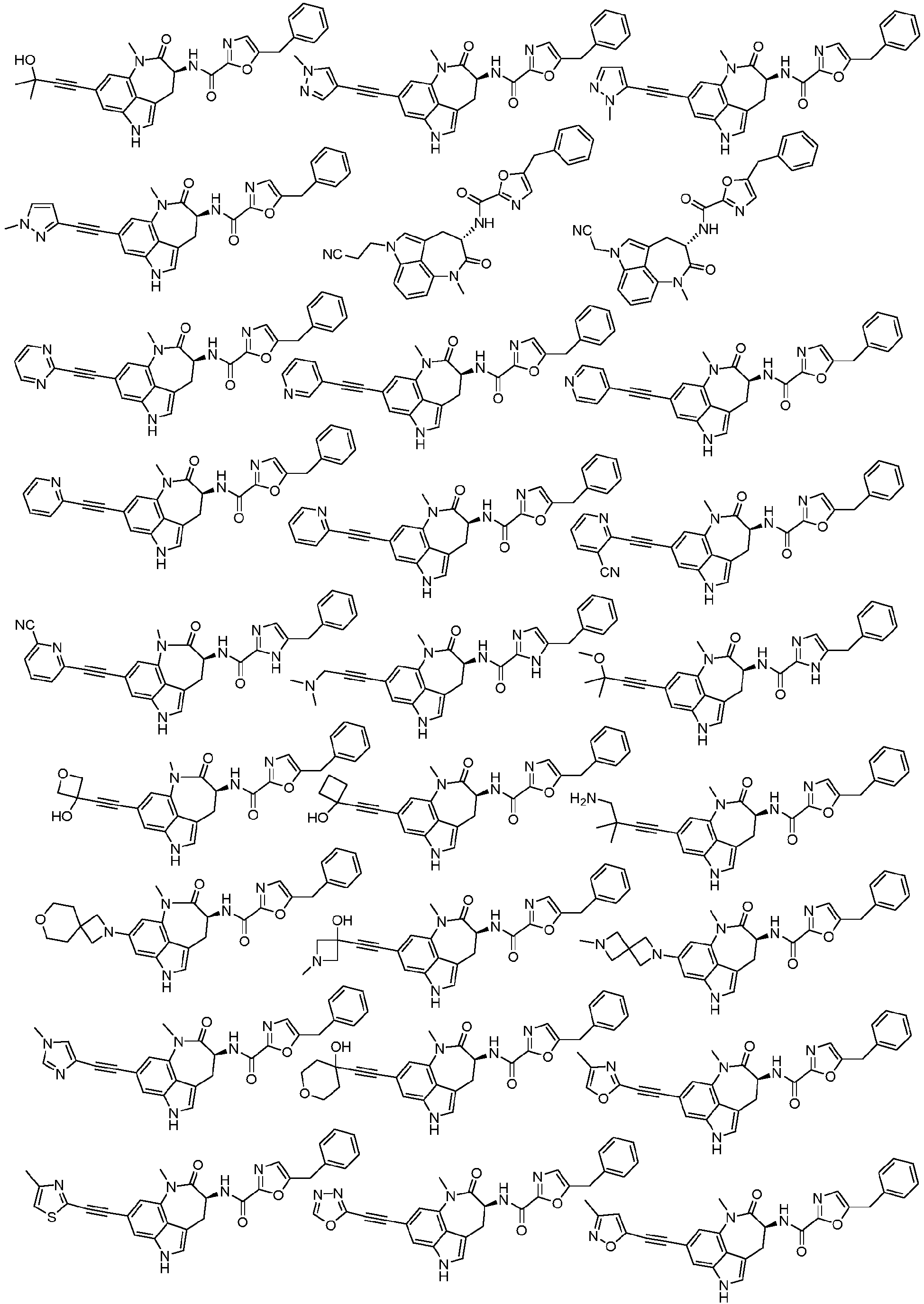

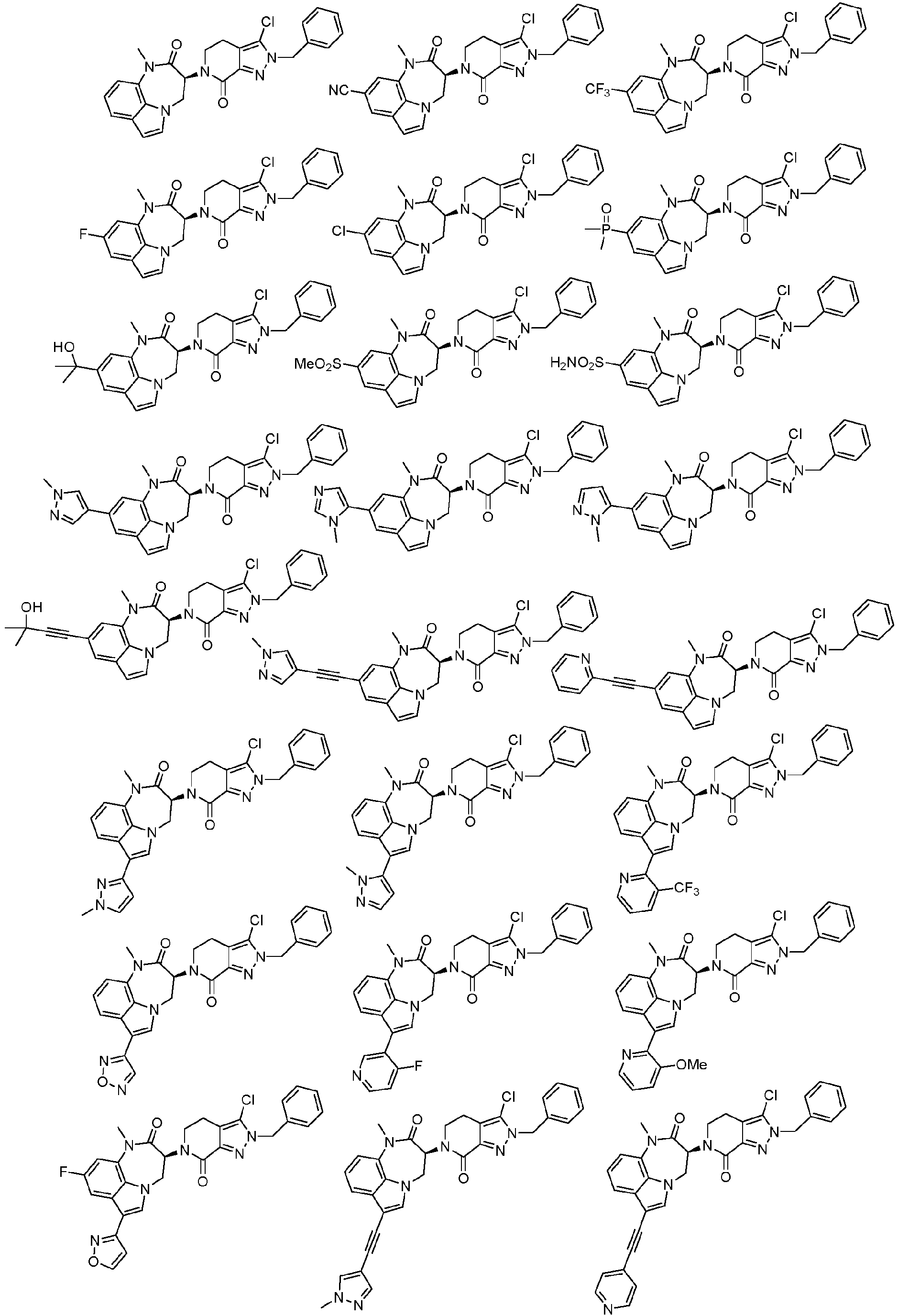

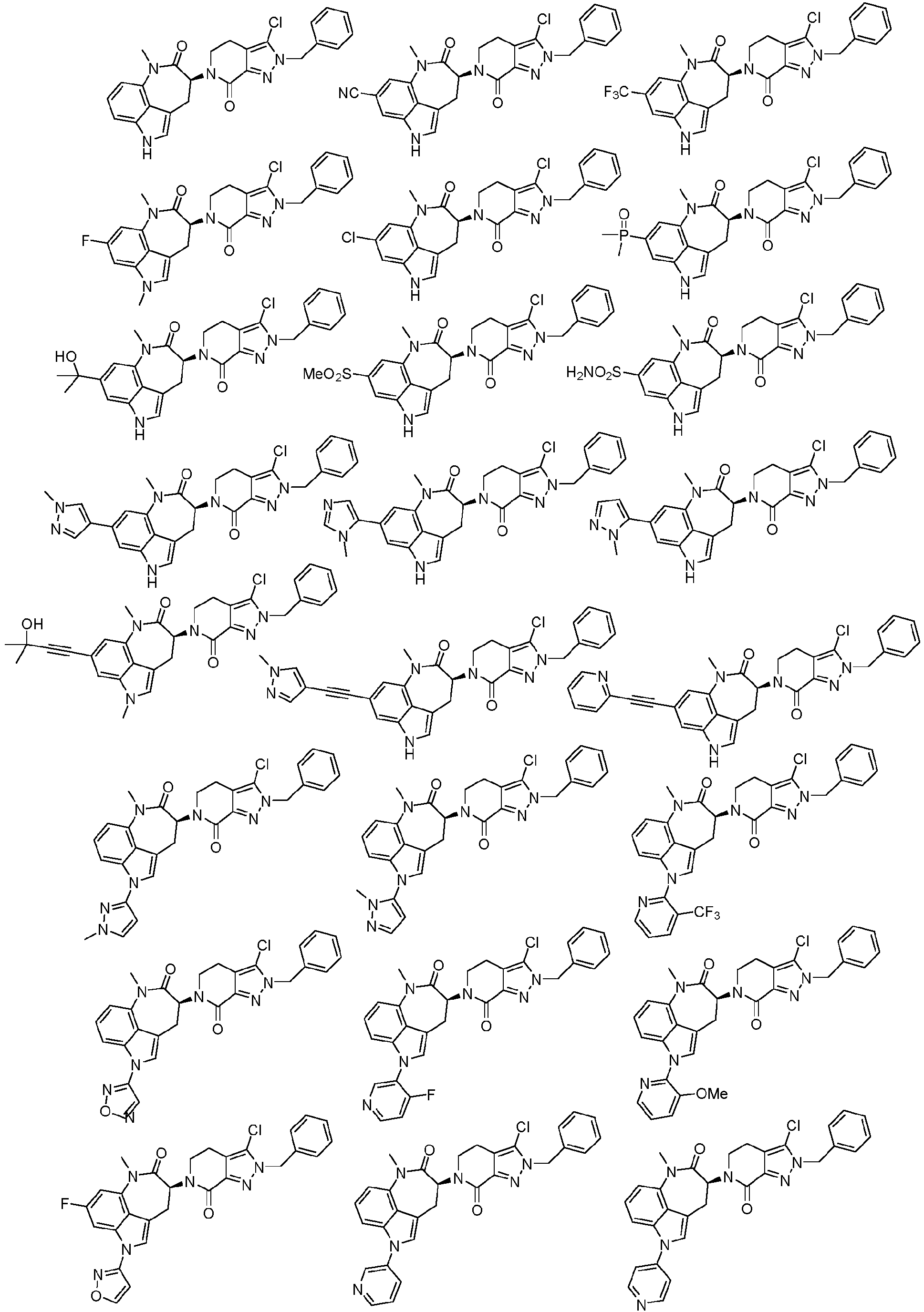

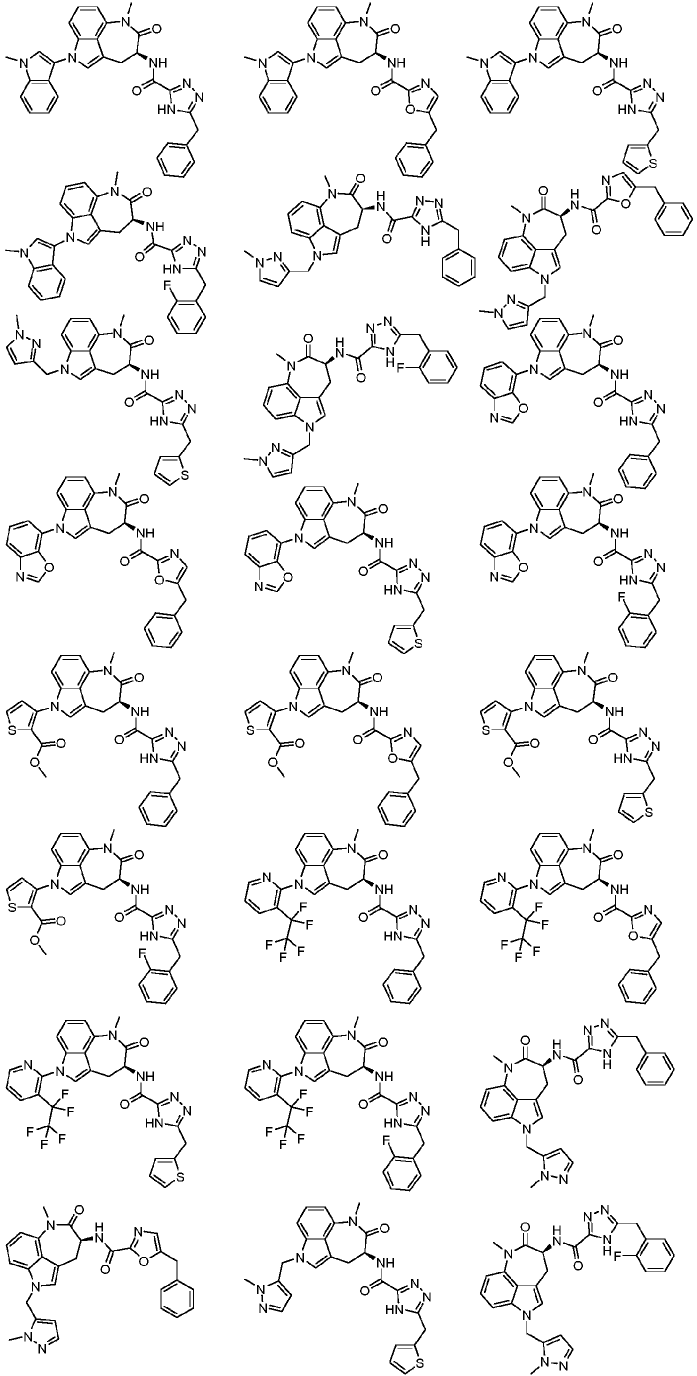

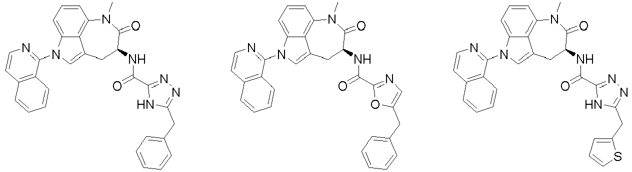

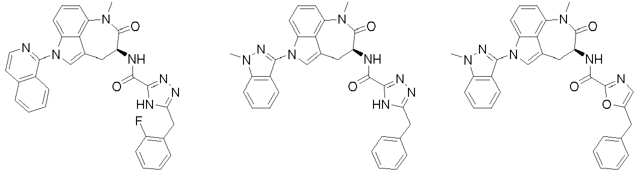

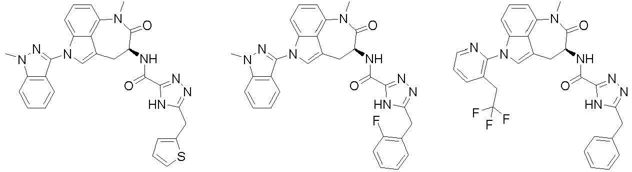

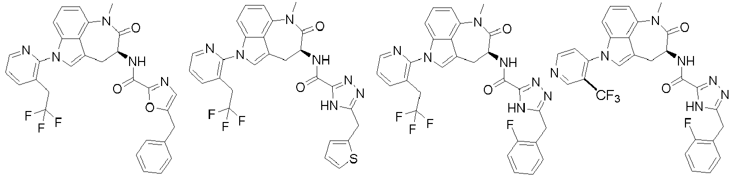

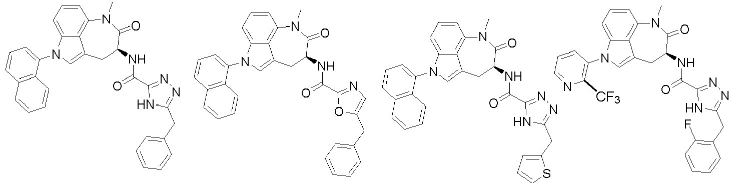

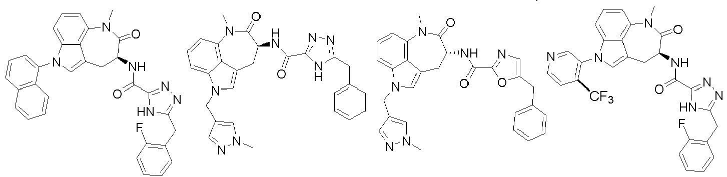

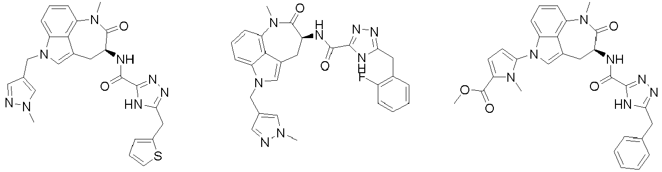

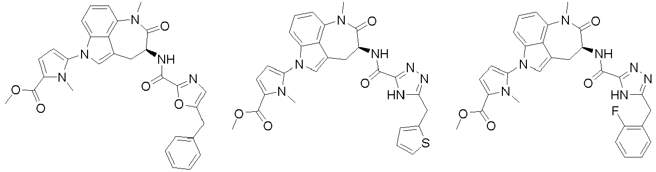

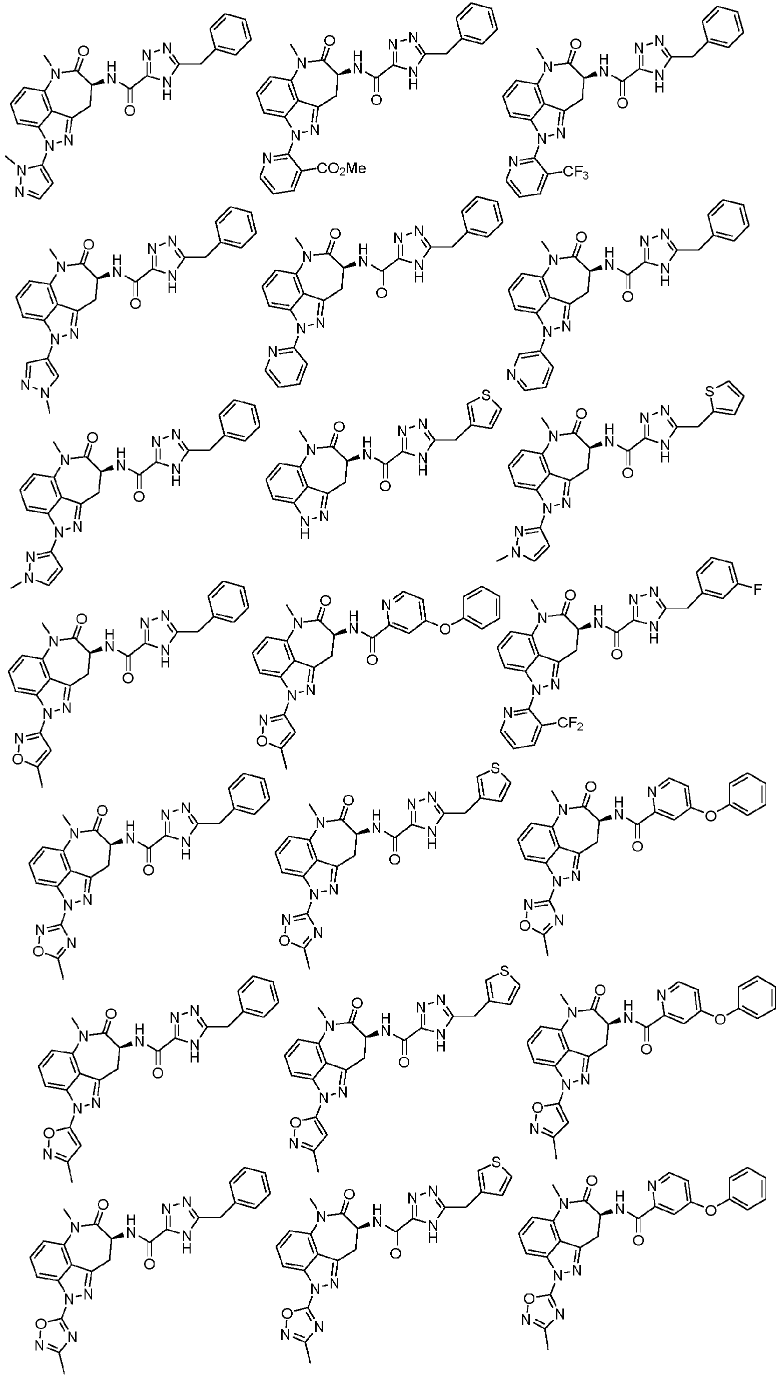

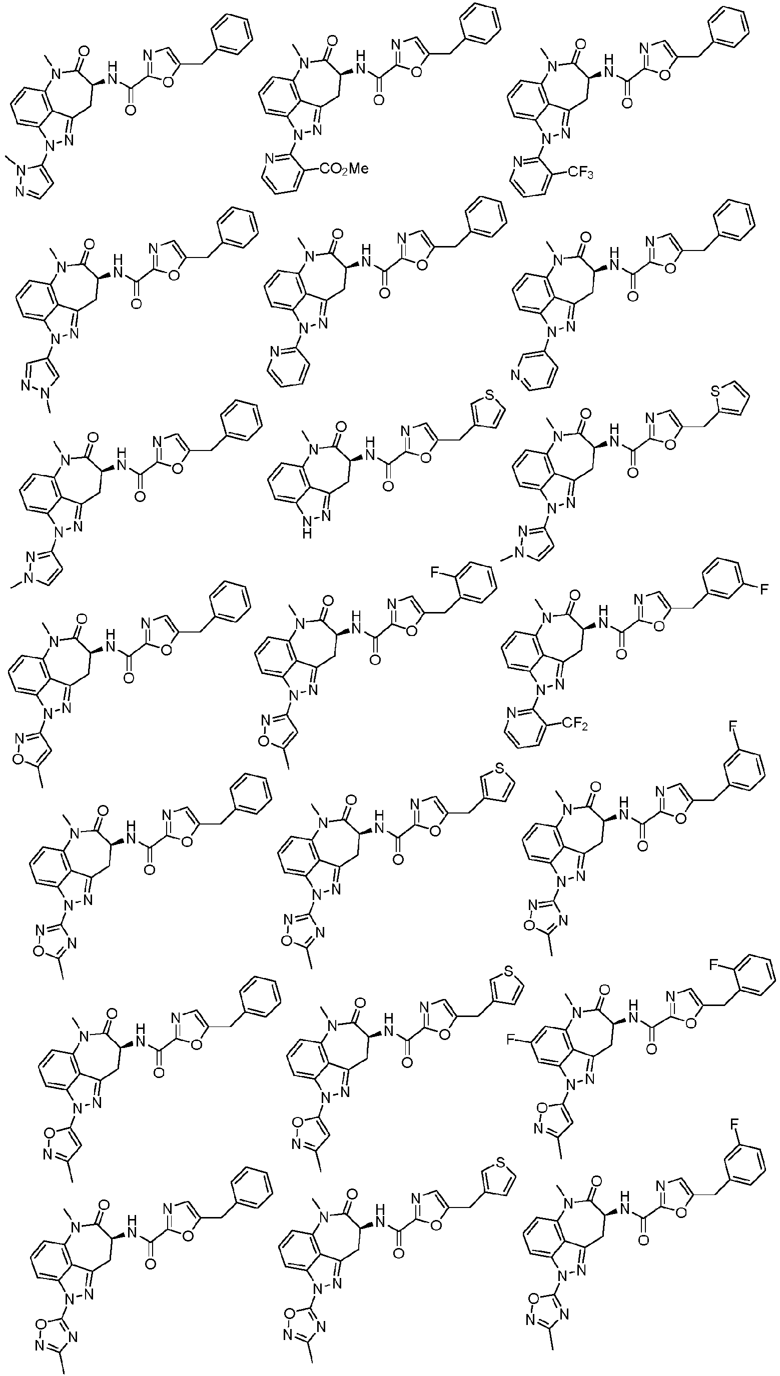

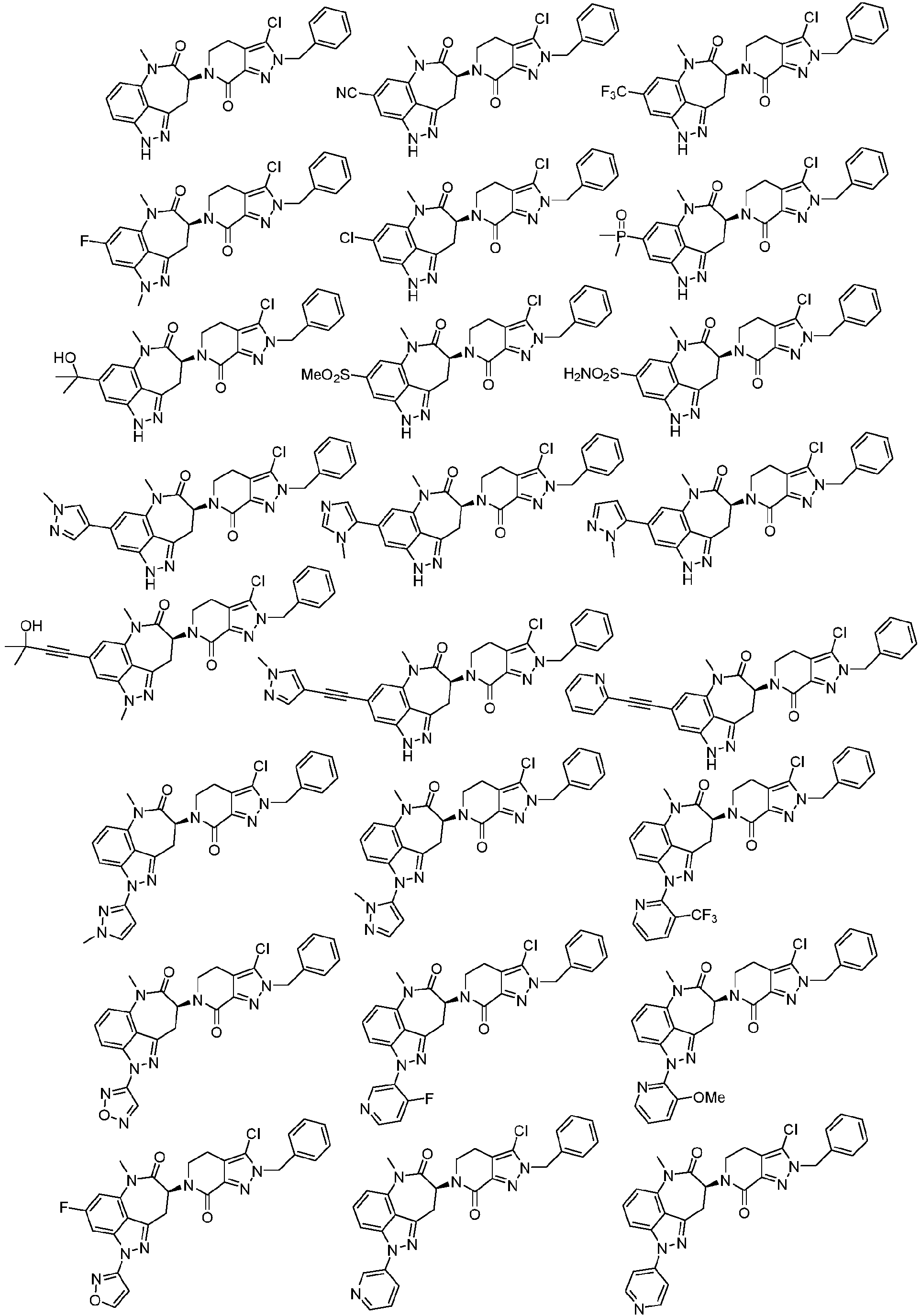

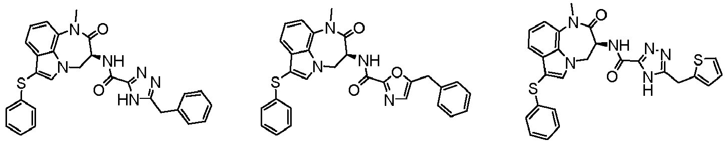

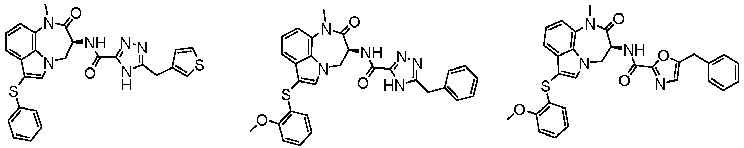

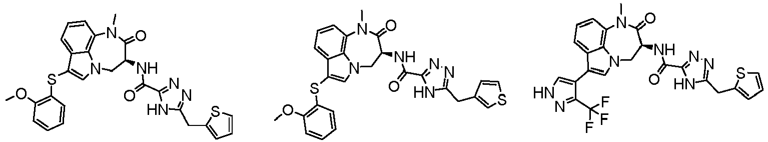

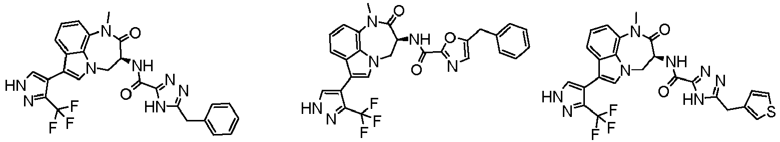

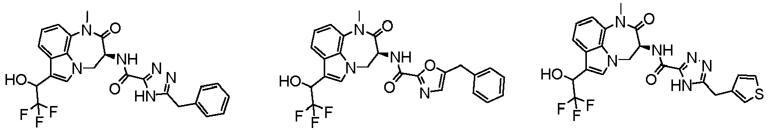

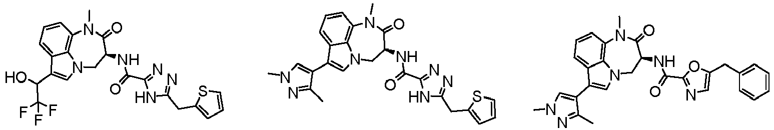

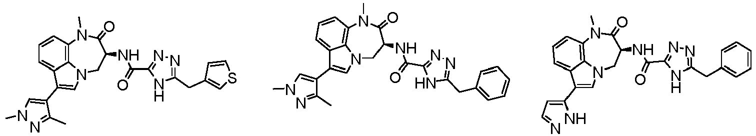

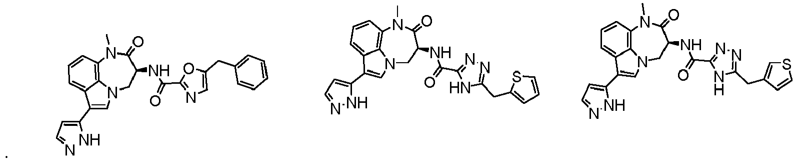

14. The purpose of the said compound according to any one of claims 1 to 13, **characterized in that**, it is used for:
(i) preparation of RIPK1 inhibitor;
(ii) preparation of necroptosis inhibitor;
(iii) preparation of medicine for the prevention and/or treatment of diseases mediated by necroptosis.

15. According to claim 14, the disease mediated by necroptosis is selected from the following group: cancer, COVID-19 infection, inflammatory bowel disease, Crohn's disease, ulcerative colitis, psoriasis, retinal detachment, pigmentary retinitis, macular degeneration, pancreatitis, atopic dermatitis, rheumatoid arthritis, spondyloarthritis, gout, systemic lupus erythematosus, Sjögren's syndrome, systemic scleroderma, antiphospholipid syndrome, vasculitis, osteoarthritis, non-alcoholic steatohepatitis, autoimmune hepatitis, autoimmune liver and gallbladder diseases, primary sclerosing cholangitis, nephritis, celiac sprue, primary immunologic thrombocytopenic purpura, transplant rejection, ischemia-reperfusion injury of solid organs, septicemia, systemic inflammatory response syndrome, cerebrovascular accident, myocardial infarction, Huntington's disease, Alzheimer's disease, Parkinson's disease, allergic diseases, asthma, multiple sclerosis, diabetes mellitus type 1, Wegener's granulomatosis, pulmonary sarcoidosis, Behcet's disease, interleukin-1 conversion enzyme-related fever syndrome, chronic obstructive pulmonary disease, tumor necrosis factor receptor-associated periodic syndrome, and periodontitis.
